# EUROPEAN PATENT SPECIFICATION

(11) **EP 3 443 350 B1**
(45) Date of publication and mention of the grant of the patent: **09.12.2020**
(21) Application number: 17735254.9
(22) Date of filing: 14.04.2017
(51) Int. Cl.: G01N 33/574, A61K 39/00

(54) **METHODS FOR MONITORING AND TREATING CANCER**
VERFAHREN ZUR ÜBERWACHUNG UND BEHANDLUNG VON KREBS
MÉTHODES DE SUIVI ET DE TRAITEMENT DU CANCER

(30) Priority: 15.04.2016 US 201662323297 P
(43) Date of publication of application: 20.02.2019
(60) Divisional application: 20205424.3
(73) Proprietor: F. Hoffmann-La Roche AG, 4070 Basel (CH)
(72) Inventor: WALLIN, Jeffrey, South San Francisco CA 94080-4990 (US); HEGDE, Priti, South San Francisco CA 94080-4990 (US)
(74) Representative: Aoki, Kenichiro
(86) International application number: PCT/US2017/027768
(87) International publication number: WO 2017/181111

(56) References cited:
- Wayne Kuznar: "Adding Anti-PD-L1 Antibody to Bevacizumab Induces Responses in mRCC", , 2 March 2015 (2015-03-02), XP055396450, Retrieved from the Internet: URL:http://www.onclive.com/conference-cove rage/gu-2015/adding-anti-pd-l1-antibody-to -bevacizumab-induces-responses-in-mrcc [retrieved on 2017-08-07]
- ROY S. HERBST ET AL: "Predictive correlates of response to the anti-PD-L1 antibody MPDL3280A in cancer patients", NATURE, vol. 515, no. 7528, 26 November 2014 (2014-11-26), pages 563-567, XP055262130, ISSN: 0028-0836, DOI: 10.1038/nature14011
- JEFFREY J. WALLIN ET AL: "Atezolizumab in combination with bevacizumab enhances antigen-specific T-cell migration in metastatic renal cell carcinoma", NATURE COMMUNICATIONS, vol. 7, 30 August 2016 (2016-08-30), page 12624, XP055396190, DOI: 10.1038/ncomms12624

### SEQUENCE LISTING

<110> Genentech, Inc.
   F. Hoffmann-La Roche AG
<120> METHODS FOR MONITORING AND TREATING CANCER
<130> 50474-132WO2
<150> US 62/323,297
   <151> 2016-04-15
<160> 71
<170> PatentIn version 3.5
<210> 1
   <211> 440
   <212> PRT
   <213> Artificial Sequence
<220>
   <223> Synthetic Polypeptide
<400> 1
   Gln Val Gln Leu Val Glu Ser Gly Gly Gly Val Val Gln Pro Gly Arg
   1               5                   10                  15
   Ser Leu Arg Leu Asp Cys Lys Ala Ser Gly Ile Thr Phe Ser Asn Ser
               20                  25                  30
   Gly Met His Trp Val Arg Gln Ala Pro Gly Lys Gly Leu Glu Trp Val
           35                  40                  45
   Ala Val Ile Trp Tyr Asp Gly Ser Lys Arg Tyr Tyr Ala Asp Ser Val
       50                  55                  60
   Lys Gly Arg Phe Thr Ile Ser Arg Asp Asn Ser Lys Asn Thr Leu Phe
   65                  70                  75                  80
   Leu Gln Met Asn Ser Leu Arg Ala Glu Asp Thr Ala Val Tyr Tyr Cys
                   85                  90                  95
   Ala Thr Asn Asp Asp Tyr Trp Gly Gln Gly Thr Leu Val Thr Val Ser
               100                 105                 110
   Ser Ala Ser Thr Lys Gly Pro Ser Val Phe Pro Leu Ala Pro Cys Ser
           115                 120                 125
   Arg Ser Thr Ser Glu Ser Thr Ala Ala Leu Gly Cys Leu Val Lys Asp
       130                 135                 140
   Tyr Phe Pro Glu Pro Val Thr Val Ser Trp Asn Ser Gly Ala Leu Thr
   145 150 155 160
   Ser Gly Val His Thr Phe Pro Ala Val Leu Gln Ser Ser Gly Leu Tyr
   165 170 175
   Ser Leu Ser Ser Val Val Thr Val Pro Ser Ser Ser Leu Gly Thr Lys
   180 185 190
   Thr Tyr Thr Cys Asn Val Asp His Lys Pro Ser Asn Thr Lys Val Asp
   195 200 205
   Lys Arg Val Glu Ser Lys Tyr Gly Pro Pro Cys Pro Pro Cys Pro Ala
   210 215 220
   Pro Glu Phe Leu Gly Gly Pro Ser Val Phe Leu Phe Pro Pro Lys Pro
   225 230 235 240
   Lys Asp Thr Leu Met Ile Ser Arg Thr Pro Glu Val Thr Cys Val Val
   245 250 255
   Val Asp Val Ser Gln Glu Asp Pro Glu Val Gln Phe Asn Trp Tyr Val
   260 265 270
   Asp Gly Val Glu Val His Asn Ala Lys Thr Lys Pro Arg Glu Glu Gln
   275 280 285
   Phe Asn Ser Thr Tyr Arg Val Val Ser Val Leu Thr Val Leu His Gln
   290 295 300
   Asp Trp Leu Asn Gly Lys Glu Tyr Lys Cys Lys Val Ser Asn Lys Gly
   305 310 315 320
   Leu Pro Ser Ser Ile Glu Lys Thr Ile Ser Lys Ala Lys Gly Gln Pro
   325 330 335
   Arg Glu Pro Gln Val Tyr Thr Leu Pro Pro Ser Gln Glu Glu Met Thr
   340 345 350
   Lys Asn Gln Val Ser Leu Thr Cys Leu Val Lys Gly Phe Tyr Pro Ser
   355 360 365
   Asp Ile Ala Val Glu Trp Glu Ser Asn Gly Gln Pro Glu Asn Asn Tyr
   370 375 380
   Lys Thr Thr Pro Pro Val Leu Asp Ser Asp Gly Ser Phe Phe Leu Tyr
   385 390 395 400
   Ser Arg Leu Thr Val Asp Lys Ser Arg Trp Gln Glu Gly Asn Val Phe
   405 410 415
   Ser Cys Ser Val Met His Glu Ala Leu His Asn His Tyr Thr Gln Lys
   420 425 430
   Ser Leu Ser Leu Ser Leu Gly Lys
   435 440
<210> 2
   <211> 214
   <212> PRT
   <213> Artificial Sequence
<220>
   <223> Synthetic Polypetide
<400> 2
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Ser Ser Asn Trp Pro Arg
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 3
   <211> 118
   <212> PRT
   <213> Artificial Sequence
<220>
   <223> Synthetic Polypeptide
<400> 3
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Ser
               20                  25                  30
   Trp Ile His Trp Val Arg Gln Ala Pro Gly Lys Gly Leu Glu Trp Val
           35                  40                  45
   Ala Trp Ile Ser Pro Tyr Gly Gly Ser Thr Tyr Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Arg His Trp Pro Gly Gly Phe Asp Tyr Trp Gly Gln Gly Thr
               100                 105                 110
   Leu Val Thr Val Ser Ala
           115
<210> 4
   <211> 108
   <212> PRT
   <213> Artificial Sequence
<220>
   <223> Synthetic Polypeptide
<400> 4
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Leu Tyr His Pro Ala
                   85                  90                  95
   Thr Phe Gly Gln Gly Thr Lys Val Glu Ile Lys Arg
               100                 105
<210> 5
   <211> 10
   <212> PRT
   <213> Artificial Sequence
<220>
   <223> Synthetic Polypeptide
<220>
   <221> MOD_RES
   <222> (6)..(6)
   <223> Xaa is Asp or Gly
<400> 5
   Gly Phe Thr Phe Ser Xaa Ser Trp Ile His
   1               5                   10
<210> 6
   <211> 18
   <212> PRT
   <213> Artificial Sequence
<220>
   <223> Synthetic Polypeptide
<220>
   <221> MOD_RES
   <222> (4)..(4)
   <223> Xaa is Ser or Leu
<220>
   <221> MOD_RES
   <222> (10)..(10)
   <223> Xaa is Thr or Ser
<400> 6
   Ala Trp Ile Xaa Pro Tyr Gly Gly Ser Xaa Tyr Tyr Ala Asp Ser Val
   1               5                   10                  15
   Lys Gly
<210> 7
   <211> 9
   <212> PRT
   <213> Artificial Sequence
<220>
   <223> Synthetic Polypeptide
<400> 7
   Arg His Trp Pro Gly Gly Phe Asp Tyr
   1               5
<210> 8
   <211> 25
   <212> PRT
   <213> Artificial Sequence
<220>
   <223> Synthetic Polypeptide
<400> 8
<210> 9
   <211> 13
   <212> PRT
   <213> Artificial Sequence
<220>
   <223> Synthetic Polypeptide
<400> 9
   Trp Val Arg Gln Ala Pro Gly Lys Gly Leu Glu Trp Val
   1               5                   10
<210> 10
   <211> 32
   <212> PRT
   <213> Artificial Sequence
<220>
   <223> Synthetic Polypeptide
<400> 10
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Tyr Tyr Cys Ala Arg
               20                  25                  30
<210> 11
   <211> 11
   <212> PRT
   <213> Artificial Sequence
<220>
   <223> Synthetic Polypeptide
<400> 11
   Trp Gly Gln Gly Thr Leu Val Thr Val Ser Ala
   1               5                   10
<210> 12
   <211> 11
   <212> PRT
   <213> Artificial Sequence
<220>
   <223> Synthetic Polypeptide
<220>
   <221> MOD_RES
   <222> (5)..(5)
   <223> Xaa is Asp or Val
<220>
   <221> MOD_RES
   <222> (6)..(6)
   <223> Xaa is Val or Ile
<220>
   <221> MOD_RES
   <222> (7)..(7)
   <223> Xaa is Ser or Asn
<220>
   <221> MOD_RES
   <222> (9)..(9)
   <223> Xaa is Ala or Phe
<220>
   <221> MOD_RES
   <222> (10)..(10)
   <223> Xaa is Val or Leu
<400> 12
   Arg Ala Ser Gln Xaa Xaa Xaa Thr Xaa Xaa Ala
   1               5                   10
<210> 13
   <211> 7
   <212> PRT
   <213> Artificial Sequence
<220>
   <223> Synthetic Polypeptide
<220>
   <221> MOD_RES
   <222> (4)..(4)
   <223> Xaa is Phe or Thr
<220>
   <221> MOD_RES
   <222> (6)..(6)
   <223> Xaa is Tyr or Ala
<400> 13
   Ser Ala Ser Xaa Leu Xaa Ser
   1               5
<210> 14
   <211> 9
   <212> PRT
   <213> Artificial Sequence
<220>
   <223> Synthetic Polypeptide
<220>
   <221> MOD_RES
   <222> (3)..(3)
   <223> Xaa is Tyr, Gly, Phe, or Ser
<220>
   <221> MOD_RES
   <222> (4)..(4)
   <223> Xaa is Leu, Tyr, Phe, or Trp
<220>
   <221> MOD_RES
   <222> (5)..(5)
   <223> Xaa is Tyr, Asn, Ala, Thr, Gly, Phe, or Ile
<220>
   <221> MOD_RES
   <222> (6)..(6)
   <223> Xaa is His, Val, Pro, Thr, or Ile
<220>
   <221> MOD_RES
   <222> (8)..(8)
   <223> Xaa is Ala, Trp, Arg, Pro, or Thr
<400> 14
   Gln Gln Xaa Xaa Xaa Xaa Pro Xaa Thr
   1               5
<210> 15
   <211> 23
   <212> PRT
   <213> Artificial Sequence
<220>
   <223> Synthetic Polypeptide
<400> 15
   Asp Ile Gln Met Thr Gln Ser Pro Ser Ser Leu Ser Ala Ser Val Gly
   1               5                   10                  15
   Asp Arg Val Thr Ile Thr Cys
               20
<210> 16
   <211> 15
   <212> PRT
   <213> Artificial Sequence
<220>
   <223> Synthetic Polypeptide
<400> 16
   Trp Tyr Gln Gln Lys Pro Gly Lys Ala Pro Lys Leu Leu Ile Tyr
   1               5                   10                  15
<210> 17
   <211> 32
   <212> PRT
   <213> Artificial Sequence
<220>
   <223> Synthetic Polypeptide
<400> 17
   Gly Val Pro Ser Arg Phe Ser Gly Ser Gly Ser Gly Thr Asp Phe Thr
   1               5                   10                  15
   Leu Thr Ile Ser Ser Leu Gln Pro Glu Asp Phe Ala Thr Tyr Tyr Cys
               20                  25                  30
<210> 18
   <211> 11
   <212> PRT
   <213> Artificial Sequence
<220>
   <223> Synthetic Polypeptide
<400> 18
   Phe Gly Gln Gly Thr Lys Val Glu Ile Lys Arg
   1               5                   10
<210> 19
   <211> 10
   <212> PRT
   <213> Artificial Sequence
<220>
   <223> Synthetic Polypeptide
<400> 19
   Gly Phe Thr Phe Ser Asp Ser Trp Ile His
   1               5                   10
<210> 20
   <211> 18
   <212> PRT
   <213> Artificial Sequence
<220>
   <223> Synthetic Polypeptide
<400> 20
   Ala Trp Ile Ser Pro Tyr Gly Gly Ser Thr Tyr Tyr Ala Asp Ser Val
   1               5                   10                  15
   Lys Gly
<210> 21
   <211> 9
   <212> PRT
   <213> Artificial Sequence
<220>
   <223> Synthetic Polypeptide
<400> 21
   Arg His Trp Pro Gly Gly Phe Asp Tyr
   1               5
<210> 22
   <211> 11
   <212> PRT
   <213> Artificial Sequence
<220>
   <223> Synthetic Polypeptide
<400> 22
   Arg Ala Ser Gln Asp Val Ser Thr Ala Val Ala
   1               5                   10
<210> 23
   <211> 7
   <212> PRT
   <213> Artificial Sequence
<220>
   <223> Synthetic Polypeptide
<400> 23
   Ser Ala Ser Phe Leu Tyr Ser
   1               5
<210> 24
   <211> 9
   <212> PRT
   <213> Artificial Sequence
<220>
   <223> Synthetic Polypeptide
<400> 24
   Gln Gln Tyr Leu Tyr His Pro Ala Thr
   1               5
<210> 25
   <211> 118
   <212> PRT
   <213> Artificial Sequence
<220>
   <223> Synthetic Polypeptide
<400> 25
<210> 26
   <211> 122
   <212> PRT
   <213> Artificial Sequence
<220>
   <223> Synthetic Polypeptide
<400> 26
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Asp Ser
   20 25 30
   Trp Ile His Trp Val Arg Gln Ala Pro Gly Lys Gly Leu Glu Trp Val
   35 40 45
   Ala Trp Ile Ser Pro Tyr Gly Gly Ser Thr Tyr Tyr Ala Asp Ser Val
   50 55 60
   Lys Gly Arg Phe Thr Ile Ser Ala Asp Thr Ser Lys Asn Thr Ala Tyr
   65 70 75 80
   Leu Gln Met Asn Ser Leu Arg Ala Glu Asp Thr Ala Val Tyr Tyr Cys
   85 90 95
   Ala Arg Arg His Trp Pro Gly Gly Phe Asp Tyr Trp Gly Gln Gly Thr
   100 105 110
   Leu Val Thr Val Ser Ser Ala Ser Thr Lys
   115 120
<210> 27
   <211> 11
   <212> PRT
   <213> Artificial Sequence
<220>
   <223> Synthetic Polypeptide
<400> 27
   Trp Gly Gln Gly Thr Leu Val Thr Val Ser Ser
   1               5                   10
<210> 28
   <211> 10
   <212> PRT
   <213> Artificial Sequence
<220>
   <223> Synthetic Polypeptide
<400> 28
   Phe Gly Gln Gly Thr Lys Val Glu Ile Lys
   1               5                   10
<210> 29
   <211> 30
   <212> PRT
   <213> Artificial Sequence
<220>
   <223> Synthetic Polypeptide
<400> 29
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser
               20                  25                  30
<210> 30
   <211> 14
   <212> PRT
   <213> Artificial Sequence
<220>
   <223> Synthetic Polypeptide
<400> 30
   Trp Val Arg Gln Ala Pro Gly Lys Gly Leu Glu Trp Val Ala
   1               5                   10
<210> 31
   <211> 15
   <212> PRT
   <213> Artificial Sequence
<220>
   <223> Synthetic Polypeptide
<400> 31
   Trp Gly Gln Gly Thr Leu Val Thr Val Ser Ser Ala Ser Thr Lys
   1               5                   10                  15
<210> 32
   <211> 447
   <212> PRT
   <213> Artificial Sequence
<220>
   <223> Synthetic Polypeptide
<400> 32
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Ser
               20                  25                  30
   Trp Ile His Trp Val Arg Gln Ala Pro Gly Lys Gly Leu Glu Trp Val
           35                  40                  45
   Ala Trp Ile Ser Pro Tyr Gly Gly Ser Thr Tyr Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Arg His Trp Pro Gly Gly Phe Asp Tyr Trp Gly Gln Gly Thr
               100                 105                 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Ala Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Glu Glu Met Thr Lys Asn Gln Val Ser Leu Thr Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly
   435 440 445
<210> 33
   <211> 214
   <212> PRT
   <213> Artificial Sequence
<220>
   <223> Synthetic Polypeptide
<400> 33
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Leu Tyr His Pro Ala
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 34
   <211> 1975
   <212> RNA
   <213> Homo sapiens
<400> 34
   acuuuccccc cucggcgccc caccggcucc cgcgcgccuc cccucgcgcc cgagcuucga      60
   gccaagcagc guccugggga gcgcgucaug gccuuaccag ugaccgccuu gcuccugccg     120
   cuggccuugc ugcuccacgc cgccaggccg agccaguucc gggugucgcc gcuggaucgg     180
   accuggaacc ugggcgagac aguggagcug aagugccagg ugcugcuguc caacccgacg     240
   ucgggcugcu cguggcucuu ccagccgcgc ggcgccgccg ccagucccac cuuccuccua     300
   uaccucuccc aaaacaagcc caaggcggcc gaggggcugg acacccagcg guucucgggc     360
   aagagguugg gggacaccuu cguccucacc cugagcgacu uccgccgaga gaacgagggc     420
   uacuauuucu gcucggcccu gagcaacucc aucauguacu ucagccacuu cgugccgguc     480
   uuccugccag cgaagcccac cacgacgcca gcgccgcgac caccaacacc ggcgcccacc     540
   aucgcgucgc agccccuguc ccugcgccca gaggcgugcc ggccagcggc ggggggcgca     600
   gugcacacga gggggcugga cuucgccugu gauaucuaca ucugggcgcc ccuggccggg     660
   acuugugggg uccuucuccu gucacugguu aucacccuuu acugcaacca caggaaccga     720
   agacguguuu gcaaaugucc ccggccugug gucaaaucgg gagacaagcc cagccuuucg     780
   gcgagauacg ucuaacccug ugcaacagcc acuacauuac uucaaacuga gauccuuccu     840
   uuugagggag caaguccuuc ccuuucauuu uuuccagucu uccucccugu guauucauuu     900
   ucaugauuau uauuuuagug ggggcggggu gggaaagauu acuuuuucuu uauguguuug     960
   acgggaaaca aaacuaggua aaaucuacag uacaccacaa gggucacaau acuguugugc    1020
   gcacaucgcg guagggcgug gaaaggggca ggccagagcu acccgcagag uucucagaau    1080
   caugcugaga gagcuggagg cacccaugcc aucucaaccu cuuccccgcc cguuuuacaa    1140
   agggggaggc uaaagcccag agacagcuug aucaaaggca cacagcaagu caggguugga    1200
   gcaguagcug gagggaccuu gucucccagc ucagggcucu uuccuccaca ccauucaggu    1260
   cuuucuuucc gaggccccug ucucagggug aggugcuuga gucuccaacg gcaagggaac    1320
   aaguacuucu ugauaccugg gauacugugc ccagagccuc gaggagguaa ugaauuaaag    1380
   aagagaacug ccuuuggcag aguucuauaa uguaaacaau aucagacuuu uuuuuuuuau    1440
   aaucaagccu aaaauuguau agaccuaaaa uaaaaugaag uggugagcuu aacccuggaa    1500
   aaugaauccc ucuaucucua aagaaaaucu cugugaaacc ccuacgugga ggcggaauug    1560
   cucucccagc ccuugcauug cagaggggcc caugaaagag gacaggcuac cccuuuacaa      1620
   auagaauuug agcaucagug agguuaaacu aaggcccucu ugaaucucug aauuugagau      1680
   acaaacaugu uccugggauc acugaugacu uuuuauacuu uguaaagaca auuguuggag      1740
   agccccucac acagcccugg ccuccgcuca acuagcagau acagggauga ggcagaccug      1800
   acucucuuaa ggaggcugag agcccaaacu gcugucccaa acaugcacuu ccuugcuuaa      1860
   gguaugguac aagcaaugcc ugcccauugg agagaaaaaa cuuaaguaga uaaggaaaua      1920
   agaaccacuc auaauucuuc accuuaggaa uaaucuccug uuaauauggu guaca           1975
<210> 35
   <211> 235
   <212> PRT
   <213> Homo sapiens
<400> 35
<210> 36
   <211> 1411
   <212> RNA
   <213> Homo sapiens
<400> 36
   gcgacugucu ccgccgagcc cccggggcca ggugucccgg gcgcgccacg augcggccgc 60
   ggcuguggcu ccuccuggcc gcgcagcuga caguucucca uggcaacuca guccuccagc 120
   agaccccugc auacauaaag gugcaaacca acaagauggu gaugcugucc ugcgaggcua 180
   aaaucucccu caguaacaug cgcaucuacu ggcugagaca gcgccaggca ccgagcagug 240
   acagucacca cgaguuccug gcccucuggg auuccgcaaa agggacuauc cacggugaag 300
   agguggaaca ggagaagaua gcuguguuuc gggaugcaag ccgguucauu cucaaucuca 360
   caagcgugaa gccggaagac aguggcaucu acuucugcau gaucgucggg agccccgagc 420
   ugaccuucgg gaagggaacu cagcugagug ugguugauuu ccuucccacc acugcccagc 480
   ccaccaagaa guccacccuc aagaagagag ugugccgguu acccaggcca gagacccaga 540
   agggcccacu uuguagcccc aucacccuug gccugcuggu ggcuggcguc cugguucugc 600
   ugguuucccu gggaguggcc auccaccugu gcugccggcg gaggagagcc cggcuucguu 660
   ucaugaaaca auuuuacaaa uaagcagaga auacgguuuu gguguccugc uacaaaaaga 720
   caucggucag uaaugagcac gauguggaaa aaugagagaa gggacacauu caacccugga     780
   gaguucaaug gcugcugaag cugccugcuu uucacugcug caaggccuuu cuguguguga     840
   ugugcauggg agcaacuugu ucguggguca ucgggaauac uagggagaag guuucauugc 900
   ccccagggca cuucacagag ugugcuggag gacugaguaa gaaaugcugc ccaugccacc 960
   gcuuccggcu ccugugcuuu cccugaacug ggaccuuuag ugguggccau uuagccacca 1020
   ucuuugcagg uugcuuugcc cugguagggc aguaacauug gguccugggu cuuucauggg 1080
   gugaugcugg gcuggcuccc ucuuggucuu cccaggcugg ggcugaccuu ccucgcagag 1140
   aggccaggug cagguuggga augaggcuug cugagagggg cuguccaguu cccagaaggc 1200
   auaucagucu cugagggcuu ccuuuggggc cgggaacuug cggguuugag gauaggaguu 1260
   cacuucaucu ucucagcucc cauuucuacu cuuaaguuuc uccccauuuc uacucuuaag 1320
   uuucucagcu cccauuucua cucucccaug gcuucaugcu ucuuucauuu uucuguuugu 1380
   uuuauacaaa uguguuaguu guacaaauaa a 1411
<210> 37
   <211> 210
   <212> PRT
   <213> Homo sapiens
<400> 37
   Met Arg Pro Arg Leu Trp Leu Leu Leu Ala Ala Gln Leu Thr Val Leu
   1               5                   10                  15
   His Gly Asn Ser Val Leu Gln Gln Thr Pro Ala Tyr Ile Lys Val Gln
               20                  25                  30
   Thr Asn Lys Met Val Met Leu Ser Cys Glu Ala Lys Ile Ser Leu Ser
           35                  40                  45
   Asn Met Arg Ile Tyr Trp Leu Arg Gln Arg Gln Ala Pro Ser Ser Asp
       50                  55                  60
   Ser His His Glu Phe Leu Ala Leu Trp Asp Ser Ala Lys Gly Thr Ile
   65                  70                  75                  80
   His Gly Glu Glu Val Glu Gln Glu Lys Ile Ala Val Phe Arg Asp Ala
                   85                  90                  95
   Ser Arg Phe Ile Leu Asn Leu Thr Ser Val Lys Pro Glu Asp Ser Gly
               100                 105                 110
   Ile Tyr Phe Cys Met Ile Val Gly Ser Pro Glu Leu Thr Phe Gly Lys
           115                 120                 125
   Gly Thr Gln Leu Ser Val Val Asp Phe Leu Pro Thr Thr Ala Gln Pro
       130                 135                 140
   Thr Lys Lys Ser Thr Leu Lys Lys Arg Val Cys Arg Leu Pro Arg Pro
   145                 150                 155                 160
   Glu Thr Gln Lys Gly Pro Leu Cys Ser Pro Ile Thr Leu Gly Leu Leu
                   165                 170                 175
   Val Ala Gly Val Leu Val Leu Leu Val Ser Leu Gly Val Ala Ile His
               180                 185                 190
   Leu Cys Cys Arg Arg Arg Arg Ala Arg Leu Arg Phe Met Lys Gln Phe
           195                 200                 205
   Tyr Lys
       210
<210> 38
   <211> 2061
   <212> RNA
   <213> Homo sapiens
<400> 38
   augcaguugg gagagcagcu cuuggugagc ucagugaacc ugccuggcgc gcacuucuac      60
   ccgcuggaga gugcgcgagg cggcagcggc gggagcgcug gccaccuccc cagcgcggcc     120
   cccucuccuc agaaguugga cuuaggcaaa gcguccaaga aguuuuccgg cagucucucc     180
   ugcgaggcgg ugagcgggga gccugcagcc gccagcgcag gggcccccgc ggccaugcuu     240
   agugacaccg acgccgggga cgcauuugcc agcgcugcgg caguggccaa gccggggccc     300
   ccggacggcc gcaagggcuc ccccugcggg gaggaggagc ugcccuccgc cgcugcagcc     360
   gccgccgccg ccgccgccgc ggcugcggcc acugcgcgcu acuccaugga cagccugagc     420
   uccgugcggu acuaccucca gucccccggu ccucaggggu cggagcuggc ugcgcccugc     480
   ucacucuucc cguaccaggc ggcggcuggg gcgccccacg gaccugugua cccggcuccu     540
   aacggggcgc gcuaccccua cggcuccaug cugccccccg gcggcuuccc cgcggcugcg      600
   ugcccacccg ggagggcgca guucggccca ggagccggug cgggcagugg cgcgggcggu     660
   aucaacggcg ggggcggcgg cccgggcacc uaucaguaca gccagggggc uccgcucuac    720
   gggccguacc cuggagccgc agcggcggga ucuugcggag gacugggggg ccuggggguu     780
   ccaaguucug gcuuccgugc ccacgucuac cugugcaacc ggccucugug gcucaaauuc     840
   caccggcacc aaacugagau gaucaucacc aaacagggca ggcgcauguu uccuuucuug     900
   agcuucaaca uaaacggacu caaccccacc gcccacuaca auguuuucgu ggaagugguu     960
   cuggccgacc cuaaccacug gcgcuuccag gggggcaaau gggugaccug uggcaaagcc    1020
   gacaauaaca ugcagggcaa caaaauguau guucacccag agucuccuaa uacugguucc    1080
   cacuggauga gacaggagau uucauucggg aaauuaaaac ucaccaauaa caaaggcgca    1140
   aauaacaaca acacccagau gauagucuua caauccuuac acaaauacca accccgacug    1200
   cauauuguug aaguuacaga ggauggcgug gaggacuuga augagcccuc aaagacccag    1260
   acuuuuaccu ucucagaaac gcaauucauu gcagugacug ccuaccaaaa caccgauauu    1320
   acucaacuaa agauugauca uaaccccuuu gcaaaaggcu ucagagacaa cuaugauuca    1380
   ucccaucaga uugucccugg aggucgguac ggcguucaau ccuucuuccc ggagcccuuu 1440
   gucaacacuu uaccucaagc ccgcuauuau aauggcgaga gaaccgugcc acagaccaac 1500
   ggccuccuuu caccccaaca gagcgaagag guggccaacc cuccccagcg guggcuuguc 1560
   acgccugucc agcaaccugg gaccaacaaa cuagacauca guuccuauga aucugaauau 1620
   acuucuagca cauugcuccc auauggcauu aaauccuugc cccuucagac aucccaugcc 1680
   cugggguauu acccagaccc aaccuuuccu gcaauggcag gguggggagg ucgagguucu 1740
   uaccagagga agauggcagc uggacuacca uggaccucca gaacaagccc cacuguguuc 1800
   ucugaagauc agcucuccaa ggagaaagug aaagaggaaa uuggcucuuc uuggauagag 1860
   acacccccuu ccaucaaauc ucuagauucc aaugauucag gaguauacac cagugcuugu 1920
   aagcgaaggc ggcugucucc uagcaacucc aguaaugaaa auucacccuc cauaaagugu 1980
   ggggacauua augcugaaga guauaguaaa gacaccucaa aaggcauggg aggguauuau 2040
   gcuuuuuaca caagucccua g 2061
<210> 39
   <211> 686
   <212> PRT
   <213> Homo sapiens
<400> 39
   Met Gln Leu Gly Glu Gln Leu Leu Val Ser Ser Val Asn Leu Pro Gly
   1               5                   10                  15
   Ala His Phe Tyr Pro Leu Glu Ser Ala Arg Gly Gly Ser Gly Gly Ser
               20                  25                  30
   Ala Gly His Leu Pro Ser Ala Ala Pro Ser Pro Gln Lys Leu Asp Leu
           35                  40                  45
   Asp Lys Ala Ser Lys Lys Phe Ser Gly Ser Leu Ser Cys Glu Ala Val
       50                  55                  60
   Ser Gly Glu Pro Ala Ala Ala Ser Ala Gly Ala Pro Ala Ala Met Leu
   65                  70                  75                  80
   Ser Asp Thr Asp Ala Gly Asp Ala Phe Ala Ser Ala Ala Ala Val Ala
                   85                  90                  95
   Lys Pro Gly Pro Pro Asp Gly Arg Lys Gly Ser Pro Cys Gly Glu Glu
               100                 105                 110
   Glu Leu Pro Ser Ala Ala Ala Ala Ala Ala Ala Ala Ala Ala Ala Ala
           115                 120                 125
   Ala Ala Thr Ala Arg Tyr Ser Met Asp Ser Leu Ser Ser Glu Arg Tyr
   130 135 140
   Tyr Leu Gln Ser Pro Gly Pro Gln Gly Ser Glu Leu Ala Ala Pro Cys
   145 150 155 160
   Ser Leu Phe Pro Tyr Gln Ala Ala Ala Gly Ala Pro His Gly Pro Val
   165 170 175
   Tyr Pro Ala Pro Asn Gly Ala Arg Tyr Pro Tyr Gly Ser Met Leu Pro
   180 185 190
   Pro Gly Gly Phe Pro Ala Ala Val Cys Pro Pro Gly Arg Ala Gln Phe
   195 200 205
   Gly Pro Gly Ala Gly Ala Gly Ser Gly Ala Gly Gly Ser Ser Gly Gly
   210 215 220
   Gly Gly Gly Pro Gly Thr Tyr Gln Tyr Ser Gln Gly Ala Pro Leu Tyr
   225 230 235 240
   Gly Pro Tyr Pro Gly Ala Ala Ala Ala Gly Ser Cys Gly Gly Leu Gly
   245 250 255
   Gly Leu Gly Val Pro Gly Ser Gly Phe Arg Ala His Val Tyr Leu Cys
   260 265 270
   Asn Arg Pro Leu Trp Leu Lys Phe His Arg His Gln Thr Glu Met Ile
   275 280 285
   Ile Thr Lys Gln Gly Arg Arg Met Phe Pro Phe Leu Ser Phe Asn Ile
   290 295 300
   Asn Gly Leu Asn Pro Thr Ala His Tyr Asn Val Phe Val Glu Val Val
   305 310 315 320
   Leu Ala Asp Pro Asn His Trp Arg Phe Gln Gly Gly Lys Trp Val Thr
   325 330 335
   Cys Gly Lys Ala Asp Asn Asn Met Gln Gly Asn Lys Met Tyr Val His
   340 345 350
   Pro Glu Ser Pro Asn Thr Gly Ser His Trp Met Arg Gln Glu Ile Ser
   355 360 365
   Phe Gly Lys Leu Lys Leu Thr Asn Asn Lys Gly Ala Asn Asn Asn Asn
   370 375 380
   Thr Gln Met Ile Val Leu Gln Ser Leu His Lys Tyr Gln Pro Arg Leu
   385 390 395 400
   His Ile Val Glu Val Thr Glu Asp Gly Val Glu Asp Leu Asn Glu Pro
   405 410 415
   Ser Lys Thr Gln Thr Phe Thr Phe Ser Glu Thr Gln Phe Ile Ala Val
   420 425 430
   Thr Ala Tyr Gln Asn Thr Asp Ile Thr Gln Leu Lys Ile Asp His Asn
   435 440 445
   Pro Phe Ala Lys Gly Phe Arg Asp Asn Tyr Asp Ser Ser His Gln Ile
   450 455 460
   Val Pro Gly Gly Arg Tyr Gly Val Gln Ser Phe Phe Pro Glu Pro Phe
   465 470 475 480
   Val Asn Thr Leu Pro Gln Ala Arg Tyr Tyr Asn Gly Glu Arg Thr Val
   485 490 495
   Pro Gln Thr Asn Gly Leu Leu Ser Pro Gln Gln Ser Glu Glu Val Ala
   500 505 510
   Asn Pro Pro Gln Arg Trp Leu Val Thr Pro Val Gln Gln Pro Gly Thr
   515 520 525
   Asn Lys Leu Asp Ile Ser Ser Tyr Glu Ser Glu Tyr Thr Ser Ser Thr
   530 535 540
   Leu Leu Pro Tyr Gly Ile Lys Ser Leu Pro Leu Gln Thr Ser His Ala
   545 550 555 560
   Leu Gly Tyr Tyr Pro Asp Pro Thr Phe Pro Ala Met Ala Gly Trp Gly
   565 570 575
   Gly Arg Gly Ser Tyr Gln Arg Lys Met Ala Ala Gly Leu Pro Trp Thr
   580 585 590
   Ser Arg Thr Ser Pro Thr Val Phe Ser Glu Asp Gln Leu Ser Lys Glu
   595 600 605
   Lys Val Lys Glu Glu Ile Gly Ser Ser Trp Ile Glu Thr Pro Pro Ser
   610 615 620
   Ile Lys Ser Leu Asp Ser Asn Asp Ser Gly Val Tyr Thr Ser Ala Cys
   625                 630                 635                 640
   Lys Arg Arg Arg Leu Ser Pro Ser Asn Ser Ser Asn Glu Asn Ser Pro
                   645                 650                 655
   Ser Ile Lys Cys Glu Asp Ile Asn Ala Glu Glu Tyr Ser Lys Asp Thr
               660                 665                 670
   Ser Lys Gly Met Gly Gly Tyr Tyr Ala Phe Tyr Thr Thr Pro
           675                 680                 685
<210> 40
   <211> 849
   <212> RNA
   <213> Homo sapiens
<400> 40
<210> 41
   <211> 262
   <212> PRT
   <213> Homo sapiens
<400> 41
   Met Arg Asn Ser Tyr Arg Phe Leu Ala Ser Ser Leu Ser Val Val Val
   1               5                   10                  15
   Ser Leu Leu Leu Ile Pro Glu Asp Val Cys Glu Lys Ile Ile Gly Gly
               20                  25                  30
   Asn Glu Val Thr Pro His Ser Arg Pro Tyr Met Val Leu Leu Ser Leu
           35                  40                  45
   Asp Arg Lys Thr Ile Cys Ala Gly Ala Leu Ile Ala Lys Asp Trp Val
       50                  55                  60
   Leu Thr Ala Ala His Cys Asn Leu Asn Lys Arg Ser Gln Val Ile Leu
   65                  70                  75                  80
   Gly Ala His Ser Ile Thr Arg Glu Glu Pro Thr Lys Gln Ile Met Leu
                   85                  90                  95
   Val Lys Lys Glu Phe Pro Tyr Pro Cys Tyr Asp Pro Ala Thr Arg Glu
               100                 105                 110
   Gly Asp Leu Lys Leu Leu Gln Leu Met Glu Lys Ala Lys Ile Asn Lys
           115                 120                 125
   Tyr Val Thr Ile Leu His Leu Pro Lys Lys Gly Asp Asp Val Lys Pro
       130                 135                 140
   Gly Thr Met Cys Gln Val Ala Gly Trp Gly Arg Thr His Asn Ser Ala
   145                 150                 155                 160
   Ser Trp Ser Asp Thr Leu Arg Glu Val Asn Ile Thr Ile Ile Asp Arg
                   165                 170                 175
   Lys Val Cys Asn Asp Arg Asn His Tyr Asn Phe Asn Pro Val Ile Gly
               180                 185                 190
   Met Asn Met Val Cys Ala Gly Ser Leu Arg Gly Gly Arg Asp Ser Cys
           195                 200                 205
   Asn Gly Asp Ser Gly Ser Pro Leu Leu Cys Glu Gly Val Phe Arg Gly
       210                 215                 220
   Val Thr Ser Phe Gly Leu Glu Asn Lys Cys Gly Asp Pro Arg Gly Pro
   225                 230                 235                 240
   Gly Val Tyr Ile Leu Leu Ser Lys Lys His Leu Asn Trp Ile Ile Met
                   245                 250                 255
   Thr Ile Lys Gly Ala Val
               260
<210> 42
   <211> 934
   <212> RNA
   <213> Homo sapiens
<400> 42
   ugagaagaug caaccaaucc ugcuucugcu ggccuuccuc cugcugccca gggcagaugc      60
   aggggagauc aucgggggac augaggccaa gccccacucc cgccccuaca uggcuuaucu     120
   uaugaucugg gaucagaagu cucugaagag gugcgguggc uuccugauac aagacgacuu      180
   cgugcugaca gcugcucacu guuggggaag cuccauaaau gucaccuugg gggcccacaa     240
   uaucaaagaa caggagccga cccagcaguu uaucccugug aaaagaccca ucccccaucc     300
   agccuauaau ccuaagaacu ucuccaacga caucaugcua cugcagcugg agagaaaggc     360
   caagcggacc agagcugugc agccccucag gcuaccuagc aacaaggccc aggugaagcc     420
   agggcagaca ugcagugugg ccggcugggg gcagacggcc ccccugggaa aacacucaca     480
   cacacuacaa gaggugaaga ugacagugca ggaagaucga aagugcgaau cugacuuacg     540
   ccauuauuac gacaguacca uugaguugug cgugggggac ccagagauua aaaagacuuc     600
   cuuuaagggg gacucuggag gcccucuugu guguaacaag guggcccagg gcauugucuc     660
   cuauggacga aacaauggca ugccuccacg agccugcacc aaagucucaa gcuuuguaca     720
   cuggauaaag aaaaccauga aacgcuacua acuacaggaa gcaaacuaag cccccgcugu     780
   aaugaaacac cuucucugga gccaagucca gauuuacacu gggagaggug ccagcaacug     840
   aauaaauacc ucucccagug uaaaucugga gccaagucca gauuuacacu gggagaggug     900
   ccagcaacug aauaaauacc ucuuagcuga gugg                                 934
<210> 43
   <211> 247
   <212> PRT
   <213> Homo sapiens
<400> 43
   Met Gln Pro Ile Leu Leu Leu Leu Ala Phe Leu Leu Leu Pro Arg Ala
   1               5                   10                  15
   Asp Ala Gly Glu Ile Ile Gly Gly His Glu Ala Lys Pro His Ser Arg
               20                  25                  30
   Pro Tyr Met Ala Tyr Leu Met Ile Trp Asp Gln Lys Ser Leu Lys Arg
           35                  40                  45
   Cys Gly Gly Phe Leu Ile Arg Asp Asp Phe Val Leu Thr Ala Ala His
       50                  55                  60
   Cys Trp Gly Ser Ser Ile Asn Val Thr Leu Gly Ala His Asn Ile Lys
   65                  70                  75                  80
   Glu Gln Glu Pro Thr Gln Gln Phe Ile Pro Val Lys Arg Pro Ile Pro
                   85                  90                  95
   His Pro Ala Tyr Asn Pro Lys Asn Phe Ser Asn Asp Ile Met Leu Leu
               100                 105                 110
   Gln Leu Glu Arg Lys Ala Lys Arg Thr Arg Ala Val Gln Pro Leu Arg
           115                 120                 125
   Leu Pro Ser Asn Lys Ala Gln Val Lys Pro Gly Gln Thr Cys Ser Val
       130                 135                 140
   Ala Gly Trp Gly Gln Thr Ala Pro Leu Gly Lys His Ser His Thr Leu
   145                 150                 155                 160
   Gln Glu Val Lys Met Thr Val Gln Glu Asp Arg Lys Cys Glu Ser Asp
                   165                 170                 175
   Leu Arg His Tyr Tyr Asp Ser Thr Ile Glu Leu Cys Val Gly Asp Pro
               180                 185                 190
   Glu Ile Lys Lys Thr Ser Phe Lys Gly Asp Ser Gly Gly Pro Leu Val
           195                 200                 205
   Cys Asn Lys Val Ala Gln Gly Ile Val Ser Tyr Gly Arg Asn Asn Gly
       210                 215                 220
   Met Pro Pro Arg Ala Cys Thr Lys Val Ser Ser Phe Val His Trp Ile
   225                 230                 235                 240
   Lys Lys Thr Met Lys Arg Tyr
                   245
<210> 44
   <211> 1193
   <212> RNA
   <213> Homo sapiens
<400> 44
   ugaagaucag cuauuagaag agaaagauca guuaaguccu uuggaccuga ucagcuugau     60
   acaagaacua cugauuucaa cuucuuuggc uuaauucucu cggaaacgau gaaauauaca    120
   aguuauaucu uggcuuuuca gcucugcauc guuuuggguu cucuuggcug uuacugccag    180
   gacccauaug uaaaagaagc agaaaaccuu aagaaauauu uuaaugcagg ucauucagau    240
   guagcggaua auggaacucu uuucuuaggc auuuugaaga auuggaaaga ggagagugac    300
   agaaaaauaa ugcagagcca aauugucucc uuuuacuuca aacuuuuuaa aaacuuuaaa    360
   gaugaccaga gcauccaaaa gaguguggag accaucaagg aagacaugaa ugucaaguuu    420
   uucaauagca acaaaaagaa acgagaugac uucgaaaagc ugacuaauua uucgguaacu    480
   gacuugaaug uccaacgcaa agcaauacau gaacucaucc aagugauggc ugaacugucg    540
   ccagcagcua aaacagggaa gcgaaaaagg agucagaugc uguuucaagg ucgaagagca    600
   ucccaguaau gguuguccug ccugcaauau uugaauuuua aaucuaaauc uauuuauuaa    660
   uauuuaacau uauuuauaug gggaauauau uuuuagacuc aucaaucaaa uaaguauuua    720
   uaauagcaac uuuuguguaa ugaaaaugaa uaucuauuaa uauauguauu auuuauaauu    780
   ccuauauccu gugacugucu cacuuaaucc uuuguuuucu gacuaauuag gcaaggcuau    840
   gugauuacaa ggcuuuaucu caggggccaa cuaggcagcc aaccuaagca agaucccaug    900
   gguugugugu uuauuucacu ugaugauaca augaacacuu auaagugaag ugauacuauc    960
   caguuacugc cgguuugaaa auaugccugc aaucugagcc agugcuuuaa uggcauguca   1020
   gacagaacuu gaauguguca ggugacccug augaaaacau agcaucucag gagauuucau   1080
   gccuggugcu uccaaauauu guugacaacu gugacuguac ccaaauggaa aguaacucau   1140
   uuguuaaaau uaucaauauc uaauauauau gaauaaagug uaaguucaca acu          1193
<210> 45
   <211> 166
   <212> PRT
   <213> Homo sapiens
<400> 45
   Met Lys Tyr Thr Ser Tyr Ile Leu Ala Phe Gln Leu Cys Ile Val Leu
   1               5                   10                  15
   Gly Ser Leu Gly Cys Tyr Cys Gln Asp Pro Tyr Val Lys Glu Ala Glu
               20                  25                  30
   Asn Leu Lys Lys Tyr Phe Asn Ala Gly His Ser Asp Val Ala Asp Asn
           35                  40                  45
   Gly Thr Leu Phe Leu Gly Ile Leu Lys Asn Trp Lys Glu Glu Ser Asp
       50                  55                  60
   Arg Lys Ile Met Gln Ser Gln Ile Val Ser Phe Tyr Phe Lys Leu Phe
   65                  70                  75                  80
   Lys Asn Phe Lys Asp Asp Gln Ser Ile Gln Lys Ser Val Glu Thr Ile
                   85                  90                  95
   Lys Glu Asp Met Asn Val Lys Phe Phe Asn Ser Asn Lys Lys Lys Arg
               100                 105                 110
   Asp Asp Phe Glu Lys Leu Thr Asn Tyr Ser Val Thr Asp Leu Asn Val
           115                 120                 125
   Gln Arg Lys Ala Ile His Glu Leu Ile Gln Val Met Ala Glu Leu Ser
       130                 135                 140
   Pro Ala Ala Lys Thr Gly Lys Arg Lys Arg Ser Gln Met Leu Phe Arg
   145                 150                 155                 160
   Gly Arg Arg Ala Ser Gln
                   165
<210> 46
   <211> 1668
   <212> RNA
   <213> Homo sapiens
<400> 46
   auggcagccc gucugcuccu ccugggcauc cuucuccugc ugcugccccu gcccgucccu 60
   gccccgugcc acacagccgc acgcucagag ugcaagcgca gccacaaguu cgugccuggu 120
   gcauggcugg ccggggaggg uguggacgug accagccucc gccgcucggg cuccuuccca 180
   guggacacac aaagguuccu gcggcccgac ggcaccugca cccucuguga aaaugcccua 240
   caggagggca cccuccagcg ccugccucug gcgcucacca acuggcgggc ccagggcucu 300
   ggcugccagc gccauguaac cagggccaaa gucagcucca cugaagcugu ggcccgggau 360
   gcggcucgua gcauccgcaa cgacuggaag gucgggcugg acgugacucc uaagcccacc 420
   agcaaugugc augugucugu ggccggcuca cacucacagg cagccaacuu ugcagcccag 480
   aagacccacc aggaccagua cagcuucagc acugacacgg uggagugccg cuucuacagu 540
   uuccaugugg uacacacucc cccgcugcac ccugacuuca agagggcccu cggggaccug 600
   ccccaccacu ucaacgccuc cacccagccc gccuaccuca ggcuuaucuc caacuacggc 660
   acccacuuca uccgggcugu ggagcugggu ggccgcauau cggcccucac ugcccugcgc 720
   accugcgagc uggcccugga agggcucacg gacaacgagg uggaggacug ccugacuguc 780
   gaggcccagg ucaacauagg cauccacggc agcaucucug ccgaagccaa ggccugugag 840
   gagaagaaga agaagcacaa gaugacggcc uccuuccacc aaaccuaccg ggagcgccac 900
   ucggaagugg uuggcggcca ucacaccucc auuaacgacc ugcuguucgg gauccaggcc 960
   gggcccgagc aguacucagc cuggguaaac uccgugcccg gcagcccugg ccugguggac 1020
   uacacccugg aaccccugca cgugcugcug gacagccagg acccgcggcg ggaggcacug 1080
   aggagggccc ugagucagua ccugacggac agggcucgcu ggagggacug cagccggccg 1140
   ugcccaccag ggcggcagaa gagcccccga gacccaugcc agugugugug ccauggcuca 1200
   gcggucacca cccaggacug cugcccucgg cagaggggcc uggcccagcu ggaggugacc 1260
   uucauccaag cauggagccu guggggggac ugguucacug ccacggaugc cuaugugaag 1320
   cucuucuuug guggccagga gcugaggacg agcaccgugu gggacaauaa caaccccauc 1380
   uggucagugc ggcuggauuu uggggaugug cuccuggcca caggggggcc ccugagguug 1440
   caggucuggg aucaggacuc uggcagggac gaugaccucc uuggcaccug ugaucaggcu 1500
   cccaagucug guucccauga ggugagaugc aaccugaauc auggccaccu aaaauuccgc 1560
   uaucaugcca ggugcuugcc ccaccuggga ggaggcaccu gccuggacua ugucccccaa 1620
   augcuucugg gggagccucc aggaaaccgg aguggggccg uguggtga 1668
<210> 47
   <211> 555
   <212> PRT
   <213> Homo sapiens
<400> 47
<210> 48
   <211> 2545
   <212> RNA
   <213> Homo sapiens
<400> 48
   auccaauaca ggagugacuu ggaacuccau ucuaucacua ugaagaaaag ugguguucuu      60
   uuccucuugg gcaucaucuu gcugguucug auuggagugc aaggaacccc aguagugaga     120
   aagggucgcu guuccugcau cagcaccaac caagggacua uccaccuaca auccuugaaa     180
   gaccuuaaac aauuugcccc aagcccuucc ugcgagaaaa uugaaaucau ugcuacacug     240
   aagaauggag uucaaacaug ucuaaaccca gauucagcag augugaagga acugauuaaa     300
   aagugggaga aacaggucag ccaaaagaaa aagcaaaaga augggaaaaa acaucaaaaa     360
   aagaaaguuc ugaaaguucg aaaaucucaa cguucucguc aaaagaagac uacauaagag     420
   accacuucac caauaaguau ucuguguuaa aaauguucua uuuuaauuau accgcuauca     480
   uuccaaagga ggauggcaua uaauacaaag gcuuauuaau uugacuagaa aauuuaaaac 540
   auuacucuga aauuguaacu aaaguuagaa aguugauuuu aagaauccaa acguuaagaa 600
   uuguuaaagg cuaugauugu cuuuguucuu cuaccaccca ccaguugaau uucaucaugc 660
   uuaaggccau gauuuuagca auacccaugu cuacacagau guucacccaa ccacauccca 720
   cucacaacag cugccuggaa gagcagcccu aggcuuccac guacugcagc cuccagagag 780
   uaucugaggc acaugucagc aaguccuaag ccuguuagca ugcuggugag ccaagcaguu 840
   ugaaauugag cuggaccuca ccaagcugcu guggccauca accucuguau uugaaucagc 900
   cuacaggccu cacacacaau gugucugaga gauucaugcu gauuguuauu ggguaucacc 960
   acuggagauc accagugugu ggcuuucaga gccuccuuuc uggcuuugga agccauguga 1020
   uuccaucuug cccgcucagg cugaccacuu uauuucuuuu uguuccccuu ugcuucauuc 1080
   aagucagcuc uucuccaucc uaccacaaug cagugccuuu cuucucucca gugcaccugu 1140
   cauaugcucu gauuuaucug agucaacucc uuucucaucu uguccccaac accccacaga 1200
   agugcuuucu ucucccaauu cauccucacu caguccagcu uaguucaagu ccugccucuu 1260
   aaauaaaccu uuuuggacac acaaauuauc uuaaaacucc uguuucacuu gguucaguac 1320
   cacaugggug aacacucaau gguuaacuaa uucuugggug uuuauccuau cucuccaacc 1380
   agauugucag cuccuugagg gcaagagcca caguauauuu cccuguuucu uccacagugc 1440
   cuaauaauac uguggaacua gguuuuaaua auuuuuuaau ugauguuguu augggcagga 1500
   uggcaaccag accauugucu cagagcaggu gcuggcucuu uccuggcuac uccauguugg 1560
   cuagccucug guaaccucuu acuuauuauc uucaggacac ucacuacagg gaccagggau 1620
   gaugcaacau ccuugucuuu uuaugacagg auguuugcuc agcuucucca acaauaagaa 1680
   gcacguggua aaacacuugc ggauauucug gacuguuuuu aaaaaauaua caguuuaccg 1740
   aaaaucauau aaucuuacaa ugaaaaggac uuuauagauc agccagugac caaccuuuuc 1800
   ccaaccauac aaaaauuccu uuucccgaag gaaaagggcu uucucaauaa gccucagcuu 1860
   ucuaagaucu aacaagauag ccaccgagau ccuuaucgaa acucauuuua ggcaaauaug 1920
   aguuuuauug uccguuuacu uguuucagag uuuguauugu gauuaucaau uaccacacca 1980
   ucucccauga agaaagggaa cggugaagua cuaagcgcua gaggaagcag ccaagucggu 2040
   uaguggaagc augauuggug cccaguuagc cucugcagga uguggaaacc uccuuccagg 2100
   ggagguucag ugaauugugu aggagagguu gucuguggcc agaauuuaaa ccuauacuca 2160
   cuuucccaaa uugaaucacu gcucacacug cugaugauuu agagugcugu ccgguggaga 2220
   ucccacccga acgucuuauc uaaucaugaa acucccuagu uccuucaugu aacuucccug 2280
   aaaaaucuaa guguuucaua aauuugagag ucugugaccc acuuaccuug caucucacag 2340
   guagacagua uauaacuaac aaccaaagac uacauauugu cacugacaca cacguuauaa 2400
   ucauuuauca uauauauaca uacaugcaua cacucucaaa gcaaauaauu uuucacuuca 2460
   aaacaguauu gacuuguaua ccuuguaauu ugaaauauuu ucuuuguuaa aauagaaugg 2520
   uaucaauaaa uagaccauua aucag 2545
<210> 49
   <211> 125
   <212> PRT
   <213> Homo sapiens
<400> 49
   Met Lys Lys Ser Gly Val Leu Phe Leu Leu Gly Ile Ile Leu Leu Val
   1 5 10 15
   Leu Ile Gly Val Gln Gly Thr Pro Val Val Arg Lys Gly Arg Cys Ser
   20 25 30
   Cys Ile Ser Thr Asn Gln Gly Thr Ile His Leu Gln Ser Leu Lys Asp
   35 40 45
   Leu Lys Gln Phe Ala Pro Ser Pro Ser Cys Glu Lys Ile Glu Ile Ile
   50 55 60
   Ala Thr Leu Lys Asn Gly Val Gln Thr Cys Leu Asn Pro Asp Ser Ala
   65 70 75 80
   Asp Val Lys Glu Leu Ile Lys Lys Trp Glu Lys Gln Val Ser Gln Lys
   85 90 95
   Lys Lys Gln Lys Asn Gly Lys Lys His Gln Lys Lys Lys Val Leu Lys
   100 105 110
   Val Arg Lys Ser Gln Arg Ser Arg Gln Lys Lys Thr Thr
   115 120 125
<210> 50
   <211> 1172
   <212> RNA
   <213> Homo sapiens
<400> 50
   gagacauucc ucaauugcuu agacauauuc ugagccuaca gcagaggaac cuccagucuc 60
   agcaccauga aucaaacugc gauucugauu ugcugccuua ucuuucugac ucuaaguggc 120
   auucaaggag uaccucucuc uagaaccgua cgcuguaccu gcaucagcau uaguaaucaa 180
   ccuguuaauc caaggucuuu agaaaaacuu gaaauuauuc cugcaagcca auuuugucca 240
   cguguugaga ucauugcuac aaugaaaaag aagggugaga agagaugucu gaauccagaa 300
   ucgaaggcca ucaagaauuu acugaaagca guuagcaagg aaaugucuaa aagaucuccu 360
   uaaaaccaga ggggagcaaa aucgaugcag ugcuuccaag gauggaccac acagaggcug 420
   ccucucccau cacuucccua cauggaguau augucaagcc auaauuguuc uuaguuugca 480
   guuacacuaa aaggugacca augaugguca ccaaaucagc ugcuacuacu ccuguaggaa 540
   gguuaauguu caucauccua agcuauucag uaauaacucu acccuggcac uauaauguaa 600
   gcucuacuga ggugcuaugu ucuuagugga uguucugacc cugcuucaaa uauuucccuc 660
   accuuuccca ucuuccaagg guacuaagga aucuuucugc uuugggguuu aucagaauuc 720
   ucagaaucuc aaauaacuaa aagguaugca aucaaaucug cuuuuuaaag aaugcucuuu 780
   acuucaugga cuuccacugc cauccuccca aggggcccaa auucuuucag uggcuaccua 840
   cauacaauuc caaacacaua caggaaggua gaaauaucug aaaauguaug uguaaguauu 900
   cuuauuuaau gaaagacugu acaaaguaua agucuuagau guauauauuu ccuauauugu 960
   uuucagugua cauggaauaa cauguaauua aguacuaugu aucaaugagu aacaggaaaa 1020
   uuuuaaaaau acagauagau auaugcucug cauguuacau aagauaaaug ugcugaaugg 1080
   uuuucaaaua aaaaugaggu acucuccugg aaauauuaag aaagacuauc uaaauguuga 1140
   aagaucaaaa gguuaauaaa guaauuauaa cu 1172
<210> 51
   <211> 98
   <212> PRT
   <213> Homo sapiens
<400> 51
   Met Asn Gln Thr Ala Ile Leu Ile Cys Cys Leu Ile Phe Leu Thr Leu
   1               5                   10                  15
   Ser Gly Ile Gln Gly Val Pro Leu Ser Arg Thr Val Arg Cys Thr Cys
               20                  25                  30
   Ile Ser Ile Ser Asn Gln Pro Val Asn Pro Arg Ser Leu Glu Lys Leu
           35                  40                  45
   Glu Ile Ile Pro Ala Ser Gln Phe Cys Pro Arg Val Glu Ile Ile Ala
       50                  55                  60
   Thr Met Lys Lys Lys Gly Glu Lys Arg Cys Leu Asn Pro Glu Ser Lys
   65                  70                  75                  80
   Ala Ile Lys Asn Leu Leu Lys Ala Val Ser Lys Glu Arg Ser Lys Arg
                   85                  90                  95
   Ser Pro
<210> 52
   <211> 995
   <212> RNA
   <213> Homo sapiens
<400> 52
   gaauucggcc aaagaggccu acuuccaaga agagcagcaa agcugaagua gcagcaacag        60
   caccagcagc aacagcaaaa aacaaacaug agugugaagg gcauggcuau agccuuggcu       120
   gugauauugu gugcuacagu uguucaaggc uuccccaugu ucaaaagagg acgcugucuu       180
   ugcauaggcc cugggguaaa agcagugaaa guggcagaua uugagaaagc cuccauaaug      240
   uacccaagua acaacuguga caaaauagaa gugauuauua cccugaaaga aaauaaagga       300
   caacgaugcc uaaaucccaa aucgaagcaa gcaaggcuua uaaucaaaaa aguugaaaga       360
   aagaauuuuu aaaaauauca aaacauauga aguccuggaa aagggcaucu gaaaaaccua       420
   gaacaaguuu aacugugacu acugaaauga caagaauucu acaguaggaa acugagacuu       480
   uucuaugguu uugugacuuu caacuuuugu acaguuaugu gaaggaugaa agguggggga        540
   aaggaccaaa aacagaaaua cagucuuccu gaaugaauga caaucagaau uccacugccc       600
   aaaggagucc aacaauuaaa uggauuucua ggaaaagcua ccuuaagaaa ggcugguuac       660
   caucggaguu uacaaagugc uuucacguuc uuacuuguug uauuauacau ucaugcauuu       720
   cuaggcuaga gaaccuucua gauuugaugc uuacaacuau ucuguuguga cuaugagaac       780
   auuucugucu cuagaaguua ucugucugua uugaucuuua ugcuauauua cuaucugugg       840
   uuacagugga gacauugaca uuauuacugg agucaagccc uuauaaguca aaagcaccua       900
   ugugucguaa agcauuccuc aaacauuuaa aaaaaaaaaa aaaaaaaaaa aaaaaaaaaa       960
   aaaaaaaaaa aaaaaaaaaa aaaaaaagcg gccgc                                  995
<210> 53
   <211> 94
   <212> PRT
   <213> Homo sapiens
<400> 53
   Met Ser Val Lys Gly Met Ala Ile Ala Leu Ala Val Ile Leu Cys Ala
   1               5                   10                  15
   Thr Val Val Gln Gly Phe Pro Met Phe Lys Arg Gly Arg Cys Leu Cys
               20                  25                  30
   Ile Gly Pro Gly Val Lys Ala Val Lys Val Ala Asp Ile Glu Lys Ala
           35                  40                  45
   Ser Ile Met Tyr Pro Ser Asn Asn Cys Asp Lys Ile Glu Val Ile Ile
       50                  55                  60
   Thr Leu Lys Glu Asn Lys Gly Gln Arg Cys Leu Asn Pro Lys Ser Lys
   65                  70                  75                  80
   Gln Ala Arg Leu Ile Ile Lys Lys Val Glu Arg Lys Asn Phe
                   85                  90
<210> 54
   <211> 1216
   <212> RNA
   <213> Homo sapiens
<400> 54
   uucggcacuu gggagaagau guuugaaaaa acugacucug cuaaugagcc uggacucaga      60
   gcucaagucu gaacucuacc uccagacaga augaaguuca ucucgacauc ucugcuucuc     120
   augcugcugg ucagcagccu cucuccaguc caagguguuc uggaggucua uuacacaagc     180
   uugaggugua gaugugucca agagagcuca gucuuuaucc cuagacgcuu cauugaucga     240
   auucaaaucu ugccccgugg gaaugguugu ccaagaaaag aaaucauagu cuggaagaag     300
   aacaagucaa uugugugugu ggacccucaa gcugaaugga uacaaagaau gauggaagua     360
   uugagaaaaa gaaguucuuc aacucuacca guuccagugu uuaagagaaa gauucccuga     420
   ugcugauauu uccacuaaga acaccugcau ucuucccuua ucccugcucu ggauuuuagu     480
   uuugugcuua guuaaaucuu uuccagggag aaagaacuuc cccauacaaa uaaggcauga     540
   ggacuaugug aaaaauaacc uugcaggagc ugauggggca aacucaagcu ucuucacuca     600
   cagcacccua uauacacuug gaguuugcau ucuuauucau cagggaggaa aguuucuuug     660
   aaaauaguua uucaguuaua aguaauacag gauuauuuug auuauauacu uguuguuuaa     720
   uguuuaaaau uucuuagaaa acaauggaau gagaauuuaa gccucaaauu ugaacaugug     780
   gcuugaauua agaagaaaau uauggcauau auuaaaagca ggcuucuaug aaagacucaa     840
   aaagcugccu gggaggcaga uggaacuuga gccugucaag aggcaaagga auccauguag     900
   uagauauccu cugcuuaaaa acucacuacg gaggagaauu aaguccuacu uuuaaagaau     960
   uucuuuauaa aauuuacugu cuaagauuaa uagcauucga agauccccag acuucauaga    1020
   auacucaggg aaagcauuua aagggugaug uacacaugua uccuuucaca cauuugccuu    1080
   gacaaacuuc uuucacucac aucuuuuuca cugacuuuuu uugugggggc ggggccgggg    1140
   ggacucuggu aucuaauucu uuaaugauuc cuauaaaucu aaugacauuc aauaaaguug    1200
   agcaaacauu uuacuu                                                    1216
<210> 55
   <211> 109
   <212> PRT
   <213> Homo sapiens
<400> 55
   Met Lys Phe Ile Ser Thr Ser Leu Leu Leu Met Leu Leu Val Ser Ser
   1               5                   10                  15
   Leu Ser Pro Val Gln Gly Val Leu Glu Val Tyr Tyr Thr Ser Leu Arg
               20                  25                  30
   Cys Arg Cys Val Gln Glu Ser Ser Val Phe Ile Pro Arg Arg Phe Ile
           35                  40                  45
   Asp Arg Ile Gln Ile Leu Pro Arg Gly Asn Gly Cys Pro Arg Lys Glu
       50                  55                  60
   Ile Ile Val Trp Lys Lys Asn Lys Ser Ile Val Cys Val Asp Pro Gln
   65                  70                  75                  80
   Ala Glu Trp Ile Gln Arg Met Met Glu Val Leu Arg Lys Arg Ser Ser
                   85                  90                  95
   Ser Thr Leu Pro Val Pro Val Phe Lys Arg Lys Ile Pro
               100                 105
<210> 56
   <211> 1770
   <212> RNA
   <213> Homo sapiens
<400> 56
   gaggagugga uuacauauuc caacaguugu uauuacauug guaaggaaag aagaacuugg 60
   gaagaaagag uuugcuggcc ugugcuucga agaacucuga ucugcuuucu auagauaaug 120
   aggaagaaau gguaugugug gggacuuccc aguuggcugu aaguugccau uugaacuaaa 180
   cgaaauagau caggaacuga ggacauaucu aaauuuucua guuuuauaga aggcuuuuau 240
   ccacaagaau caagaucuuc ccucucugag caggaauccu uugugcauug aagacuuuag 300
   auuccucucu gcgguagacg ugcacuuaua aguauuugau ggggguggau cguggucgga 360
   ggucucgaca cagcugggag augagugaau uucauaauua uaacuuggau cugaagaaga 420
   gugauuuuuc aacacgaugg caaaagcaaa gauguccagu agucaaaagc aaauguagag 480
   aaaaugcauc uccauuuuuu uucugcugcu ucaucgcugu agccauggga auccguuuca 540
   uuauuauggu agcaauaugg agugcuguau uccuaaacuc auuauucaac caagaaguuc 600
   aaauucccuu gaccgaaagu uacuguggcc cauguccuaa aaacuggaua uguuacaaaa 660
   auaacugcua ccaauuuuuu gaugagagua aaaacuggua ugagagccag gcuucuugua 720
   ugucucaaaa ugccagccuu cugaaaguau acagcaaaga ggaccaggau uuacuuaaac 780
   uggugaaguc auaucauugg augggacuag uacacauucc aacaaaugga ucuuggcagu 840
   gggaagaugg cuccauucuc ucacccaacc uacuaacaau aauugaaaug cagaagggag 900
   acugugcacu cuaugccucg agcuuuaaag gcuauauaga aaacuguuca acuccaaaua 960
   cauacaucug caugcaaagg acuguguaaa gaugaucaac caucucaaua aaagccagga 1020
   acagagaaga gauuacacca gcgguaacac ugccaaccga gacuaaagga aacaaacaaa 1080
   aacaggacaa aaugaccaaa gacugucaga uuucuuagac uccacaggac caaaccauag 1140
   aacaauuuca cugcaaacau gcaugauucu ccaagacaaa agaagagaga uccuaaaggc 1200
   aauucagaua uccccaaggc ugccucuccc accacaagcc cagaguggau gggcuggggg 1260
   aggggugcug uuuuaauuuc uaaagguagg accaacaccc aggggaucag ugaaggaaga 1320
   gaaggccagc agaucaguga gagugcaacc ccacccucca caggaaauug ccucaugggc 1380
   agggccacag cagagagaca cagcaugggc agugccuucc cugccugugg gggucaugcu 1440
   gccacuuuua auggguccuc cacccaacgg ggucagggag guggugcugc cccagugggc 1500
   caugauuauc uuaaaggcau uauucuccag ccuuaagauc uuaggacguu uccuuugcua 1560
   ugauuuguac uugcuugagu cccaugacug uuucucuucc ucucuuucuu ccuuuuggaa 1620
   uaguaauauc cauccuaugu uugucccacu auuguauuuu ggaagcacau aacuuguuug 1680
   guuucacagg uucacaguua agaaggaauu uugccucuga auaaauagaa ucuugagucu 1740
   caugcaaaaa aaaaaaaaaa aaaaaaaaaa 1770
<210> 57
   <211> 216
   <212> PRT
   <213> Homo sapiens
<400> 57
   Met Gly Trp Ile Arg Gly Arg Arg Ser Arg His Ser Trp Glu Met Ser
   1               5                   10                  15
   Glu Phe His Asn Tyr Asn Leu Asp Leu Lys Lys Ser Asp Phe Ser Thr
               20                  25                  30
   Arg Trp Gln Lys Gln Arg Cys Pro Val Val Lys Ser Lys Cys Arg Glu
           35                  40                  45
   Asn Ala Ser Pro Phe Phe Phe Cys Cys Phe Ile Ala Val Ala Met Gly
       50                  55                  60
   Ile Arg Phe Ile Ile Met Val Ala Ile Trp Ser Ala Val Phe Leu Asn
   65                  70                  75                  80
   Ser Leu Phe Asn Gln Glu Val Gln Ile Pro Leu Thr Glu Ser Tyr Cys
                   85                  90                  95
   Gly Pro Cys Pro Lys Asn Trp Ile Cys Tyr Lys Asn Asn Cys Tyr Gln
               100                 105                 110
   Phe Phe Asp Glu Ser Lys Asn Trp Tyr Glu Ser Gln Ala Ser Cys Met
           115                 120                 125
   Ser Gln Asn Ala Ser Leu Leu Lys Val Tyr Ser Lys Glu Asp Gln Asp
       130                 135                 140
   Leu Leu Lys Leu Val Lys Ser Tyr His Trp Met Gly Leu Val His Ile
   145                 150                 155                 160
   Pro Thr Asn Gly Ser Trp Gln Trp Glu Asp Gly Ser Ile Leu Ser Pro
                   165                 170                 175
   Asn Leu Leu Thr Ile Ile Glu Met Gln Lys Gly Asp Cys Ala Leu Tyr
               180                 185                 190
   Ala Ser Ser Phe Lys Gly Tyr Ile Glu Asn Cys Ser Thr Pro Asn Thr
           195                 200                 205
   Tyr Ile Cys Met Gln Arg Thr Val
       210                 215
<210> 58
   <211> 1083
   <212> RNA
   <213> Homo sapiens
<400> 58
   cagagagcaa uauggcuggu uccccaacau gccucacccu caucuauauc cuuuggcagc      60
   ucacaggguc agcagccucu ggacccguga aagagcuggu cgguuccguu gguggggccg     120
   ugacuuuccc ccugaagucc aaaguaaagc aaguugacuc uauugucugg accuucaaca     180
   caaccccucu ugucaccaua cagccagaag ggggcacuau cauagugacc caaaaucgua      240
   auagggagag aguagacuuc ccagauggag gcuacucccu gaagcucagc aaacugaaga     300
   agaaugacuc agggaucuac uaugugggga uauacagcuc aucacuccag cagcccucca     360
   cccaggagua cgugcugcau gucuacgagc accugucaaa gccuaaaguc accauggguc    420
   ugcagagcaa uaagaauggc accuguguga ccaaucugac augcugcaug gaacaugggg     480
   aagaggaugu gauuuauacc uggaaggccc uggggcaagc agccaaugag ucccauaaug     540
   gguccauccu ccccaucucc uggagauggg gagaaaguga uaugaccuuc aucugcguug     600
   ccaggaaccc ugucagcaga aacuucucaa gccccauccu ugccaggaag cucugugaag     660
   gugcugcuga ugacccagau uccuccaugg uccuccugug ucuccuguug gugccccucc     720
   ugcucagucu cuuuguacug gggcuauuuc uuugguuucu gaagagagag agacaagaag      780
   aguacauuga agagaagaag agaguggaca uuugucggga aacuccuaac auaugccccc     840
   auucuggaga gaacacagag uacgacacaa ucccucacac uaauagaaca auccuaaagg     900
   aagauccagc aaauacgguu uacuccacug uggaaauacc gaaaaagaug gaaaaucccc     960
   acucacugcu cacgaugcca gacacaccaa ggcuauuugc cuaugagaau guuaucuaga    1020
   cagcagugca cugccccuaa gucucugcuc aaaaaaaaaa caauucucgg cccaaagaaa     1080
   aca                                                                  1083
<210> 59
   <211> 335
   <212> PRT
   <213> Homo sapiens
<400> 59
   Met Ala Gly Ser Pro Thr Cys Leu Thr Leu Ile Tyr Ile Leu Trp Gln
   1               5                   10                  15
   Leu Thr Gly Ser Ala Ala Ser Gly Pro Val Lys Glu Leu Val Gly Ser
               20                  25                  30
   Val Gly Gly Ala Val Thr Phe Pro Leu Lys Ser Lys Val Lys Gln Val
           35                  40                  45
   Asp Ser Ile Val Trp Thr Phe Asn Thr Thr Pro Leu Val Thr Ile Gln
       50                  55                  60
   Pro Glu Gly Gly Thr Ile Ile Val Thr Gln Asn Arg Asn Arg Glu Arg
   65                  70                  75                  80
   Val Asp Phe Pro Asp Gly Gly Tyr Ser Leu Lys Leu Ser Lys Leu Lys
                   85                  90                  95
   Lys Asn Asp Ser Gly Ile Tyr Tyr Val Gly Ile Tyr Ser Ser Ser Leu
               100                 105                 110
   Gln Gln Pro Ser Thr Gln Glu Tyr Val Leu His Val Tyr Glu His Leu
           115                 120                 125
   Ser Lys Pro Lys Val Thr Met Gly Leu Gln Ser Asn Lys Asn Gly Thr
       130                 135                 140
   Cys Val Thr Asn Leu Thr Cys Cys Met Glu His Gly Glu Glu Asp Val
   145                 150                 155                 160
   Ile Tyr Thr Trp Lys Ala Leu Gly Gln Ala Ala Asn Glu Ser His Asn
                   165                 170                 175
   Gly Ser Ile Leu Pro Ile Ser Trp Arg Trp Gly Glu Ser Asp Met Thr
               180                 185                 190
   Phe Ile Cys Val Ala Arg Asn Pro Val Ser Arg Asn Phe Ser Ser Pro
           195                 200                 205
   Ile Leu Ala Arg Lys Leu Cys Glu Gly Ala Ala Asp Asp Pro Asp Ser
       210                 215                 220
   Ser Met Val Leu Leu Cys Leu Leu Leu Val Pro Leu Leu Leu Ser Leu
   225                 230                 235                 240
   Phe Val Leu Gly Leu Phe Leu Trp Phe Leu Lys Arg Glu Arg Gln Glu
                   245                 250                 255
   Glu Tyr Ile Glu Glu Lys Lys Arg Val Asp Ile Cys Arg Glu Thr Pro
               260                 265                 270
   Asn Ile Cys Pro His Ser Gly Glu Asn Thr Glu Tyr Asp Thr Ile Pro
           275                 280                 285
   His Thr Asn Arg Thr Ile Leu Lys Glu Asp Pro Ala Asn Thr Val Tyr
       290                 295                 300
   Ser Thr Val Glu Ile Pro Lys Lys Met Glu Asn Pro His Ser Leu Leu
   305                 310                 315                 320
   Thr Met Pro Asp Thr Pro Arg Leu Phe Ala Tyr Glu Asn Val Ile
                   325                 330                 335
<210> 60
   <211> 741
   <212> RNA
   <213> Homo sapiens
<400> 60
   aaccgagagg cugagacuaa cccagaaaga uccaauucuc aaacugaagc ucgcacucuc 60
   gccuccagca ugaaagucuc ugccgcccuu cugugccugc ugcucauagc agccaccuuc 120
   auuccccaag ggcucgcuca gccagaugca aucaaugccc cagucaccug cuguuauaac 180
   uucaccaaua ggaagaucuc agugcagagg cucgcgagcu auagaagaau caccagcagc 240
   aaguguccca aagaagcugu gaucuucaag accauugugg ccaaggagau cugugcugac 300
   cccaagcaga aguggguuca ggauuccaug gaccaccugg acaagcaaac ccaaacuccg 360
   aagacuugaa cacucacucc acaacccaag aaucugcagc uaacuuauuu uccccuagcu 420
   uuccccagac acccuguuuu auuuuauuau aaugaauuuu guuuguugau gugaaacauu 480
   augccuuaag uaauguuaau ucuuauuuaa guuauugaug uuuuaaguuu aucuuucaug 540
   guacuagugu uuuuuagaua cagagacuug gggaaauugc uuuuccucuu gaaccacagu 600
   ucuaccccug ggauguuuug agggucuuug caagaaucau uaauacaaag aauuuuuuuu 660
   aacauuccaa ugcauugcua aaauauuauu guggaaauga auauuuugua acuauuacac 720
   caaauaaaua uauuuuugua c 741
<210> 61
   <211> 99
   <212> PRT
   <213> Homo sapiens
<400> 61
   Met Lys Val Ser Ala Ala Leu Leu Cys Leu Leu Leu Ile Ala Ala Thr
   1 5 10 15
   Phe Ile Pro Gln Gly Leu Ala Gln Pro Asp Ala Ile Asn Ala Pro Val
   20 25 30
   Thr Cys Cys Tyr Asn Phe Thr Asn Arg Lys Ile Ser Val Gln Arg Leu
   35 40 45
   Ala Ser Tyr Arg Arg Ile Thr Ser Ser Lys Cys Pro Lys Glu Ala Val
   50 55 60
   Ile Phe Lys Thr Ile Val Ala Lys Glu Ile Cys Ala Asp Pro Lys Gln
   65 70 75 80
   Lys Trp Val Gln Asp Ser Met Asp His Leu Asp Lys Gln Thr Gln Thr
   85 90 95
   Pro Lys Thr
<210> 62
   <211> 1160
   <212> RNA
   <213> Homo sapiens
<400> 62
   ccuccgacag ccucuccaca gguaccauga aggucuccgc ggcacgccuc gcugucaucc 60
   ucauugcuac ugcccucugc gcuccugcau cugccucccc auauuccucg gacaccacac 120
   ccugcugcuu ugccuacauu gcccgcccac ugccccgugc ccacaucaag gaguauuucu 180
   acaccagugg caagugcucc aacccagcag ucgucuuugu cacccgaaag aaccgccaag 240
   ugugugccaa cccagagaag aaauggguuc gggaguacau caacucuuug gagaugagcu 300
   aggauggaga guccuugaac cugaacuuac acaaauuugc cuguuucugc uugcucuugu 360
   ccuagcuugg gaggcuuccc cucacuaucc uaccccaccc gcuccuugaa gggcccagau 420
   ucugaccacg acgagcagca guuacaaaaa ccuuccccag gcuggacgug guggcucagc 480
   cuuguaaucc cagcacuuug ggaggccaag guggguggau cacuugaggu caggaguucg 540
   agacagccug gccaacauga ugaaacccca uguguacuaa aaauacaaaa aauuagccgg 600
   gcgugguagc gggcgccugu agucccagcu acucgggagg cugaggcagg agaauggcgu 660
   gaacccggga gcggagcuug cagugagccg agaucgcgcc acugcacucc agccugggcg 720
   acagagcgag acuccgucuc aaaaaaaaaa aaaaaaaaaa aaaaaauaca aaaauuagcc 780
   gcgugguggc ccacgccugu aaucccagcu acucgggagg cuaaggcagg aaaauuguuu 840
   gaacccagga gguggaggcu gcagugagcu gagauugugc cacuucacuc cagccugggu 900
   gacaaaguga gacuccguca caacaacaac aacaaaaagc uuccccaacu aaagccuaga 960
   agagcuucug aggcgcugcu uugucaaaag gaagucucua gguucugagc ucuggcuuug 1020
   ccuuggcuuu gcaagggcuc ugugacaagg aaggaaguca gcaugccucu agaggcaagg 1080
   aagggaggaa cacugcacuc uuaagcuucc gccgucucaa ccccucacag gagcuuacug 1140
   gcaaacauga aaaaucgggg 1160
<210> 63
   <211> 91
   <212> PRT
   <213> Homo sapiens
<400> 63
   Met Lys Val Ser Ala Ala Ala Leu Ala Val Ile Leu Ile Ala Thr Ala
   1               5                   10                  15
   Leu Cys Ala Pro Ala Ser Ala Ser Pro Tyr Ser Ser Asp Thr Thr Pro
               20                  25                  30
   Cys Cys Phe Ala Tyr Ile Ala Arg Pro Leu Pro Arg Ala His Ile Lys
           35                  40                  45
   Glu Tyr Phe Tyr Thr Ser Gly Lys Cys Ser Asn Pro Ala Val Val Phe
       50                  55                  60
   Val Thr Arg Lys Asn Arg Gln Val Cys Ala Asn Pro Glu Lys Lys Trp
   65                  70                  75                  80
   Val Arg Glu Tyr Ile Asn Ser Leu Glu Met Ser
                   85                  90
<210> 64
   <211> 3383
   <212> RNA
   <213> Homo sapiens
<400> 64
   agaagagcug agacauccgu uccccuacaa gaaacucucc ccggguggaa caagauggau 60
   uaucaagugu caaguccaau cuaugacauc aauuauuaua caucggagcc cugccaaaaa 120
   aucaauguga agcaaaucgc agcccgccuc cugccuccgc ucuacucacu gguguucauc 180
   uuugguuuug ugggcaacau gcuggucauc cucauccuga uaaacugcaa aaggcugaag 240
   agcaugacug acaucuaccu gcucaaccug gccaucucug accuguuuuu ccuucuuacu 300
   guccccuucu gggcucacua ugcugccgcc cagugggacu uuggaaauac aaugugucaa 360
   cucuugacag ggcucuauuu uauaggcuuc uucucuggaa ucuucuucau cauccuccug 420
   acaaucgaua gguaccuggc ugucguccau gcuguguuug cuuuaaaagc caggacgguc 480
   accuuugggg uggugacaag ugugaucacu uggguggugg cuguguuugc gucucuccca 540
   ggaaucaucu uuaccagauc ucaaaaagaa ggucuucauu acaccugcag cucucauuuu 600
   ccauacaguc aguaucaauu cuggaagaau uuccagacau uaaagauagu caucuugggg 660
   cugguccugc cgcugcuugu cauggucauc ugcuacucgg gaauccuaaa aacucugcuu 720
   cggugucgaa augagaagaa gaggcacagg gcugugaggc uuaucuucac caucaugauu 780
   guuuauuuuc ucuucugggc ucccuacaac auuguccuuc uccugaacac cuuccaggaa 840
   uucuuuggcc ugaauaauug caguagcucu aacagguugg accaagcuau gcaggugaca 900
   gagacucuug ggaugacgca cugcugcauc aaccccauca ucuaugccuu ugucggggag 960
   aaguucagaa acuaccucuu agucuucuuc caaaagcaca uugccaaacg cuucugcaaa 1020
   ugcuguucua uuuuccagca agaggcuccc gagcgagcaa gcucaguuua cacccgaucc 1080
   acuggggagc aggaaauauc ugugggcuug ugacacggac ucaagugggc uggugaccca    1140
   gucagaguug ugcacauggc uuaguuuuca uacacagccu gggcuggggg ugggguggga    1200
   gaggucuuuu uuaaaaggaa guuacuguua uagagggucu aagauucauc cauuuauuug 1260
   gcaucuguuu aaaguagauu agaucuuuua agcccaucaa uuauagaaag ccaaaucaaa 1320
   auauguugau gaaaaauagc aaccuuuuua ucuccccuuc acaugcauca aguuauugac 1380
   aaacucuccc uucacuccga aaguuccuua uguauauuua aaagaaagcc ucagagaauu 1440
   gcugauucuu gaguuuagug aucugaacag aaauaccaaa auuauuucag aaauguacaa 1500
   cuuuuuaccu aguacaaggc aacauauagg uuguaaaugu guuuaaaaca ggucuuuguc 1560
   uugcuauggg gagaaaagac augaauauga uuaguaaaga aaugacacuu uucaugugug 1620
   auuuccccuc caagguaugg uuaauaaguu ucacugacuu agaaccaggc gagagacuug 1680
   uggccuggga gagcugggga agcuucuuaa augagaagga auuugaguug gaucaucuau 1740
   ugcuggcaaa gacagaagcc ucacugcaag cacugcaugg gcaagcuugg cuguagaagg 1800
   agacagagcu gguugggaag acauggggag gaaggacaag gcuagaucau gaagaaccuu 1860
   gacggcauug cuccgucuaa gucaugagcu gagcagggag auccugguug guguugcaga 1920
   agguuuacuc uguggccaaa ggagggucag gaaggaugag cauuuagggc aaggagacca 1980
   ccaacagccc ucaggucagg gugaggaugg ccucugcuaa gcucaaggcg ugaggauggg 2040
   aaggagggag guauucguaa ggaugggaag gagggaggua uucgugcagc auaugaggau 2100
   gcagagucag cagaacuggg guggauuugg uuuggaagug agggucagag aggagucaga 2160
   gagaaucccu agucuucaag cagauuggag aaacccuuga aaagacauca agcacagaag 2220
   gaggaggagg agguuuaggu caagaagaag auggauuggu guaaaaggau gggucugguu 2280
   ugcagagcuu gaacacaguc ucacccagac uccaggcugu cuuucacuga augcuucuga 2340
   cuucauagau uuccuuccca ucccagcuga aauacugagg ggucuccagg aggagacuag 2400
   auuuaugaau acacgaggua ugaggucuag gaacauacuu cagcucacac augagaucua 2460
   ggugaggauu gauuaccuag uagucauuuc auggguuguu gggaggauuc uaugaggcaa 2520
   ccacaggcag cauuuagcac auacuacaca uucaauaagc aucaaacucu uaguuacuca 2580
   uucagggaua gcacugagca aagcauugag caaagggguc ccauauaggu gagggaagcc      2640
   ugaaaaacua agaugcugcc ugcccagugc acacaagugu agguaucauu uucugcauuu 2700
   aaccgucaau aggcaaaggg gggaagggac auauucauuu ggaaauaagc ugccuugagc 2760
   cuuaaaaccc acaaaaguac aauuuaccag ccuccguauu ucagacugaa uggggguggg 2820
   gggggcgccu uagguacuua uuccagaugc cuucuccaga caaaccagaa gcaacagaaa 2880
   aaaucgucuc ucccucccuu ugaaaugaau auaccccuua guguuugggu auauucauuu 2940
   caaagggaga gagagagguu uuuuucuguu cuuucucaua ugauugugca cauacuugag 3000
   acuguuuuga auuuggggga uggcuaaaac caucauagua cagguaaggu gagggaauag 3060
   uaagugguga gaacuacuca gggaaugaag gugucagaau aauaagaggu gcuacugacu 3120
   uucucagccu cugaauauga acggugagca uuguggcugu cagcaggaag caacgaaggg 3180
   aaaugucuuu ccuuuugcuc uuaaguugug gagagugcaa caguagcaua ggacccuacc 3240
   cucugggcca agucaaagac auucugacau cuuaguauuu gcauauucuu auguauguga 3300
   aaguuacaaa uugcuugaaa gaaaauaugc aucuaauaaa aaacaccuuc uaaaauaaaa 3360
   aaaaaaaaaa aaaaaaaaaa aaa 3383
<210> 65
   <211> 352
   <212> PRT
   <213> Homo sapiens
<400> 65
<210> 66
   <211> 1635
   <212> RNA
   <213> Homo sapiens
<400> 66
   ggcacgaggg cacugagcuc ugccgccugg cucuagccgc cugccuggcc cccgccggga 60
   cucuugccca cccucagcca uggcuccgau aucucugucg uggcugcucc gcuuggccac 120
   cuucugccau cugacugucc ugcuggcugg acagcaccac ggugugacga aaugcaacau 180
   cacgugcagc aagaugacau caaagauacc uguagcuuug cucauccacu aucaacagaa 240
   ccaggcauca ugcggcaaac gcgcaaucau cuuggagacg agacagcaca ggcuguucug 300
   ugccgacccg aaggagcaau gggucaagga cgcgaugcag caucuggacc gccaggcugc 360
   ugcccuaacu cgaaauggcg gcaccuucga gaagcagauc ggcgagguga agcccaggac 420
   caccccugcc gccgggggaa uggacgaguc ugugguccug gagcccgaag ccacaggcga 480
   aagcaguagc cuggagccga cuccuucuuc ccaggaagca cagagggccc uggggaccuc 540
   cccagagcug ccgacgggcg ugacugguuc cucagggacc aggcuccccc cgacgccaaa 600
   ggcucaggau ggagggccug ugggcacgga gcuuuuccga gugccucccg ucuccacugc 660
   cgccacgugg cagaguucug cuccccacca accugggccc agccucuggg cugaggcaaa 720
   gaccucugag gccccgucca cccaggaccc cuccacccag gccuccacug cguccucccc 780
   agccccagag gagaaugcuc cgucugaagg ccagcgugug uggggucagg gacagagccc 840
   caggccagag aacucucugg agcgggagga gauggguccc gugccagcgc acacggaugc 900
   cuuccaggac ugggggccug gcagcauggc ccacgucucu guggucccug ucuccucaga 960
   agggaccccc agcagggagc caguggcuuc aggcagcugg accccuaagg cugaggaacc 1020
   cauccaugcc accauggacc cccagaggcu gggcguccuu aucacuccug ucccugacgc 1080
   ccaggcugcc acccggaggc aggcgguggg gcugcuggcc uuccuuggcc uccucuucug 1140
   ccugggggug gccauguuca ccuaccagag ccuccagggc ugcccucgaa agauggcagg 1200
   agagauggcg gagggccuuc gcuacauccc ccggagcugu gguaguaauu cauauguccu 1260
   ggugcccgug ugaacuccuc uggccugugu cuaguuguuu gauucagaca gcugccuggg    1320
   aucccucauc cucauaccca cccccaccca agggccuggc cugagcuggg augauuggag 1380
   gggggaggug ggauccucca ggugcacaag cuccaagcuc ccaggcauuc cccaggaggc 1440
   cagccuugac cauucuccac cuuccaggga cagagggggu ggccucccaa cucaccccag 1500
   ccccaaaacu cuccucugcu gcuggcuggu uagagguucc cuuugacgcc aucccagccc 1560
   caaugaacaa uuauuuauua aaugcccagc cccuucugaa aaaaaaaaaa aaaaaaaaaa 1620
   aaaaaaaaaa aaaaa 1635
<210> 67
   <211> 397
   <212> PRT
   <213> Homo sapiens
<400> 67
   Met Ala Pro Ile Ser Leu Ser Trp Leu Leu Arg Leu Ala Thr Phe Cys
   1               5                   10                  15
   His Leu Thr Val Leu Leu Ala Gly Gln His His Gly Val Thr Lys Cys
               20                  25                  30
   Asn Ile Thr Cys Ser Lys Met Thr Ser Lys Ile Pro Val Ala Leu Leu
           35                  40                  45
   Ile His Tyr Gln Gln Asn Gln Ala Ser Cys Gly Lys Arg Ala Ile Ile
       50                  55                  60
   Leu Glu Thr Arg Gln His Arg Leu Phe Cys Ala Asp Pro Lys Glu Gln
   65                  70                  75                  80
   Trp Val Lys Asp Ala Met Gln His Leu Asp Arg Gln Ala Ala Ala Leu
                   85                  90                  95
   Thr Arg Asn Gly Gly Thr Phe Glu Lys Gln Ile Gly Glu Val Lys Pro
               100                 105                 110
   Arg Thr Thr Pro Ala Ala Gly Gly Met Asp Glu Ser Val Val Leu Glu
           115                 120                 125
   Pro Glu Ala Thr Gly Glu Ser Ser Ser Leu Glu Pro Thr Pro Ser Ser
       130                 135                 140
   Gln Glu Ala Gln Arg Ala Leu Gly Thr Ser Pro Glu Leu Pro Thr Gly
   145                 150                 155                 160
   Val Thr Gly Ser Ser Gly Thr Arg Leu Pro Pro Thr Pro Lys Ala Gln
                   165                 170                 175
   Asp Gly Gly Pro Val Gly Thr Glu Leu Phe Arg Val Pro Pro Val Ser
               180                 185                 190
   Thr Ala Ala Thr Trp Gln Ser Ser Ala Pro His Gln Pro Gly Pro Ser
           195                 200                 205
   Leu Trp Ala Glu Ala Lys Thr Ser Glu Ala Pro Ser Thr Gln Asp Pro
       210                 215                 220
   Ser Thr Gln Ala Ser Thr Ala Ser Ser Pro Ala Pro Glu Glu Asn Ala
   225                 230                 235                 240
   Pro Ser Glu Gly Gln Arg Val Trp Gly Gln Gly Gln Ser Pro Arg Pro
                   245                 250                 255
   Glu Asn Ser Leu Glu Arg Glu Glu Met Gly Pro Val Pro Ala His Thr
               260                 265                 270
   Asp Ala Phe Gln Asp Trp Gly Pro Gly Ser Met Ala His Val Ser Val
           275                 280                 285
   Val Pro Val Ser Ser Glu Gly Thr Pro Ser Arg Glu Pro Val Ala Ser
       290                 295                 300
   Gly Ser Trp Thr Pro Lys Ala Glu Glu Pro Ile His Ala Thr Met Asp
   305                 310                 315                 320
   Pro Gln Arg Leu Gly Val Leu Ile Thr Pro Val Pro Asp Ala Gln Ala
                   325                 330                 335
   Ala Thr Arg Arg Gln Ala Val Gly Leu Leu Ala Phe Leu Gly Leu Leu
               340                 345                 350
   Phe Cys Leu Gly Val Ala Met Phe Thr Tyr Gln Ser Leu Gln Gly Cys
   355 360 365
   Pro Arg Lys Met Ala Gly Glu Met Ala Glu Gly Leu Arg Tyr Ile Pro
   370 375 380
   Arg Ser Cys Gly Ser Asn Ser Tyr Val Leu Val Pro Val
   385 390 395
<210> 68
   <211> 2154
   <212> RNA
   <213> Homo sapiens
<400> 68
   ggaauuccgu agugcgaggc cgggcacagc cuuccugugu gguuuuaccg cccagagagc 60
   gucauggacc uggggaaacc aaugaaaagc gugcuggugg uggcucuccu ugucauuuuc 120
   cagguaugcc ugugucaaga ugaggucacg gacgauuaca ucggagacaa caccacagug 180
   gacuacacuu uguucgaguc uuugugcucc aagaaggacg ugcggaacuu uaaagccugg 240
   uuccucccua ucauguacuc caucauuugu uucgugggcc uacugggcaa ugggcugguc 300
   guguugaccu auaucuauuu caagaggcuc aagaccauga ccgauaccua ccugcucaac 360
   cuggcggugg cagacauccu cuuccuccug acccuucccu ucugggccua cagcgcggcc 420
   aaguccuggg ucuucggugu ccacuuuugc aagcucaucu uugccaucua caagaugagc 480
   uucuucagug gcaugcuccu acuucuuugc aucagcauug accgcuacgu ggccaucguc 540
   caggcugucu cagcucaccg ccaccgugcc cgcguccuuc ucaucagcaa gcuguccugu 600
   gugggcagcg ccauacuagc cacagugcuc uccaucccag agcuccugua cagugaccuc 660
   cagaggagca gcagugagca agcgaugcga ugcucucuca ucacagagca uguggaggcc     720
   uuuaucacca uccagguggc ccagauggug aucggcuuuc uggucccccu gcuggccaug     780
   agcuucuguu accuugucau cauccgcacc cugcuccagg cacgcaacuu ugagcgcaac 840
   aaggccauca aggugaucau cgcugugguc guggucuuca uagucuucca gcugcccuac 900
   aauggggugg uccuggccca gacgguggcc aacuucaaca ucaccaguag caccugugag 960
   cucaguaagc aacucaacau cgccuacgac gucaccuaca gccuggccug cguccgcugc 1020
   ugcgucaacc cuuucuugua cgccuucauc ggcgucaagu uccgcaacga uaucuucaag 1080
   cucuucaagg accugggcug ccucagccag gagcagcucc ggcagugguc uuccugucgg 1140
   cacauccggc gcuccuccau gaguguggag gccgagacca ccaccaccuu cuccccauag 1200
   gcgacucuuc ugccuggacu agagggaccu cucccagggu cccuggggug gggauaggga 1260
   gcagaugcaa ugacucagga cauccccccg ccaaaagcug cucaggggaa aaagcagcuc 1320
   uccccucaga gugcaagccc cugcuccaga agauagcuuc accccaaucc cagcuaccuc 1380
   aaccaaugcc aaaaaaagac agggcugaua agcuaacacc agacagacaa cacugggaaa 1440
   cagaggcuau uguccccuaa accaaaaacu gaaagugaaa guccagaaac uguucccacc 1500
   ugcuggagug aaggggccaa ggagggugag ugcaaggggc gugggagugg ccugaagagu 1560
   ccucugaaug aaccuucugg ccucccacag acucaaaugc ucagaccagc ucuuccgaaa 1620
   accaggccuu aucuccaaga ccagagauag uggggagacu ucuuggcuug gugaggaaaa 1680
   gcggacauca gcuggucaaa caaacucucu gaaccccucc cuccaucguu uucuucacug 1740
   uccuccaagc cagcgggaau ggcagcugcc acgccgcccu aaaagcacac ucauccccuc 1800
   acuugccgcg ucgcccuccc aggcucucaa caggggagag ugugguguuu ccugcaggcc 1860
   aggccagcug ccuccgcgug aucaaagcca cacucugggc uccagagugg ggaugacaug 1920
   cacucagcuc uuggcuccac ugggauggga ggagaggaca agggaaaugu caggggcggg 1980
   gagggugaca guggccgccc aaggccacga gcuuguucuu uguucuuugu cacagggacu 2040
   gaaaaccucu ccucauguuc ugcuuucgau ucguuaagag agcaacauuu uacccacaca 2100
   cagauaaagu uuucccuuga ggaaacaaca gcuuuaaaaa aaaaaaagga auuc 2154
<210> 69
   <211> 378
   <212> PRT
   <213> Homo sapiens
<400> 69
   Met Asp Leu Gly Lys Pro Met Lys Ser Val Leu Val Val Ala Leu Leu
   1               5                   10                  15
   Val Ile Phe Gln Val Cys Leu Cys Gln Asp Glu Val Thr Asp Asp Tyr
               20                  25                  30
   Ile Gly Asp Asn Thr Thr Val Asp Tyr Thr Leu Phe Glu Ser Leu Cys
           35                  40                  45
   Ser Lys Lys Asp Val Arg Asn Phe Lys Ala Trp Phe Leu Pro Ile Met
       50                  55                  60
   Tyr Ser Ile Ile Cys Phe Val Gly Leu Leu Gly Asn Gly Leu Val Val
   65                  70                  75                  80
   Leu Thr Tyr Ile Tyr Phe Lys Arg Leu Lys Thr Met Thr Asp Thr Tyr
                   85                  90                  95
   Leu Leu Asn Leu Ala Val Ala Asp Ile Leu Phe Leu Leu Thr Leu Pro
               100                 105                 110
   Phe Trp Ala Tyr Ser Ala Ala Lys Ser Trp Val Phe Gly Val His Phe
           115                 120                 125
   Cys Lys Leu Ile Phe Ala Ile Tyr Lys Met Ser Phe Phe Ser Gly Met
       130                 135                 140
   Leu Leu Leu Leu Cys Ile Ser Ile Asp Arg Tyr Val Ala Ile Val Gln
   145                 150                 155                 160
   Ala Val Ser Ala His Arg His Arg Ala Arg Val Leu Leu Ile Ser Lys
                   165                 170                 175
   Leu Ser Cys Val Gly Ile Trp Ile Leu Ala Thr Val Leu Ser Ile Pro
               180                 185                 190
   Glu Leu Leu Tyr Ser Asp Leu Gln Arg Ser Ser Ser Glu Gln Ala Met
           195                 200                 205
   Arg Cys Ser Leu Ile Thr Glu His Val Glu Ala Phe Ile Thr Ile Gln
       210                 215                 220
   Val Ala Gln Met Val Ile Gly Phe Leu Val Pro Leu Leu Ala Met Ser
   225                 230                 235                 240
   Phe Cys Tyr Leu Val Ile Ile Arg Thr Leu Leu Gln Ala Arg Asn Phe
                   245                 250                 255
   Glu Arg Asn Lys Ala Ile Lys Val Ile Ile Ala Val Val Val Val Phe
               260                 265                 270
   Ile Val Phe Gln Leu Pro Tyr Asn Gly Val Val Leu Ala Gln Thr Val
           275                 280                 285
   Ala Asn Phe Asn Ile Thr Ser Ser Thr Cys Glu Leu Ser Lys Gln Leu
       290                 295                 300
   Asn Ile Ala Tyr Asp Val Thr Tyr Ser Leu Ala Cys Val Arg Cys Cys
   305                 310                 315                 320
   Val Asn Pro Phe Leu Tyr Ala Phe Ile Gly Val Lys Phe Arg Asn Asp
                   325                 330                 335
   Leu Phe Lys Leu Phe Lys Asp Leu Gly Cys Leu Ser Gln Glu Gln Leu
               340                 345                 350
   Arg Gln Trp Ser Ser Cys Arg His Ile Arg Arg Ser Ser Met Ser Val
           355                 360                 365
   Glu Ala Glu Thr Thr Thr Thr Phe Ser Pro
   370 375
<210> 70
   <211> 3100
   <212> RNA
   <213> Homo sapiens
<400> 70
   acucgucucu gguaaagucu gagcaggaca ggguggcuga cuggcagauc cagagguucc 60
   cuuggcaguc cacgccaggc cuucaccaug gaucaguucc cugaaucagu gacagaaaac 120
   uuugaguacg augauuuggc ugaggccugu uauauugggg acaucguggu cuuugggacu 180
   guguuccugu ccauauucua cuccgucauc uuugccauug gccugguggg aaauuuguug 240
   guaguguuug cccucaccaa cagcaagaag cccaagagug ucaccgacau uuaccuccug 300
   aaccuggccu ugucugaucu gcuguuugua gccacuuugc ccuucuggac ucacuauuug 360
   auaaaugaaa agggccucca caaugccaug ugcaaauuca cuaccgccuu cuucuucauc 420
   ggcuuuuuug gaagcauauu cuucaucacc gucaucagca uugauaggua ccuggccauc 480
   guccuggccg ccaacuccau gaacaaccgg accgugcagc auggcgucac caucagccua 540
   ggcgucuggg cagcagccau uuugguggca gcaccccagu ucauguucac aaagcagaaa 600
   gaaaaugaau gccuugguga cuaccccgag guccuccagg aaaucuggcc cgugcuccgc 660
   aauguggaaa caaauuuucu uggcuuccua cucccccugc ucauuaugag uuauugcuac 720
   uucagaauca uccagacgcu guuuuccugc aagaaccaca agaaagccaa agccauuaaa 780
   cugauccuuc ugguggucau cguguuuuuc cucuucugga cacccuacaa cguuaugauu 840
   uuccuggaga cgcuuaagcu cuaugacuuc uuucccaguu gugacaugag gaaggaucug 900
   aggcuggccc ucagugugac ugagacgguu gcauuuagcc auuguugccu gaauccucuc 960
   aucuaugcau uugcugggga gaaguucaga agauaccuuu accaccugua ugggaaaugc 1020
   cuggcugucc ugugugggcg cucaguccac guugauuucu ccucaucuga aucacaaagg 1080
   agcaggcaug gaaguguucu gagcagcaau uuuacuuacc acacgaguga uggagaugca 1140
   uugcuccuuc ucugaaggga aucccaaagc cuugugucua cagagaaccu ggaguuccug 1200
   aaccugaugc ugacuaguga ggaaagauuu uuguuguuau uucuuacagg cacaaaauga 1260
   uggacccaau gcacacaaaa caacccuaga guguuguuga gaauugugcu caaaauuuga 1320
   agaaugaaca aauugaacuc uuugaaugac aaagaguaga cauuucucuu acugcaaaug 1380
   ucaucagaac uuuuugguuu gcagaugaca aaaauucaac ucagacuagu uuaguuaaau 1440
   gaggguggug aauauuguuc auauuguggc acaagcaaaa gggugucuga gcccucaaag 1500
   ugaggggaaa ccagggccug agccaagcua gaauucccuc ucucugacuc ucaaaucuuu 1560
   uagucauuau agauccccca gacuuuacau gacacagcuu uaucaccaga gagggacuga 1620
   cacccauguu ucucuggccc caagggaaaa uucccaggga agugcucuga uaggccaagu 1680
   uuguaucagg ugcccauccc uggaaggugc uguuauccau ggggaaggga uauauaagau 1740
   ggaagcuucc aguccaaucu cauggagaag cagaaauaca uauuuccaag aaguuggaug 1800
   gguggguacu auucugauua cacaaaacaa augccacaca ucacccuuac caugugccug 1860
   auccagccuc uccccugauu acaccagccu cgucuucauu aagcccucuu ccaucauguc 1920
   cccaaaccug caagggcucc ccacugccua cugcaucgag ucaaaacuca aaugcuuggc 1980
   uucucauacg uccaccaugg gguccuacca auagauuccc cauugccucc uccuucccaa 2040
   aggacuccac ccauccuauc agccugucuc uuccauauga ccucaugcau cuccaccugc 2100
   ucccaggcca guaagggaaa uagaaaaacc cugcccccaa auaagaaggg auggauucca 2160
   accccaacuc caguagcuug ggacaaauca agcuucaguu uccuggucug uagaagaggg 2220
   auaagguacc uuucacauag agaucauccu uuccagcaug aggaacuagc caccaacucu 2280
   ugcaggucuc aacccuuuug ucugccucuu agacuucugc uuuccacacc ugcacugcug 2340
   ugcugugccc aaguuguggu gcugacaaag cuuggaagag ccugcaggug ccuuggccgc 2400
   gugcauagcc cagacacaga agaggcuggu ucuuacgaug gcacccagug agcacuccca 2460
   agucuacaga gugauagccu uccguaaccc aacucuccug gacugccuug aauauccccu 2520
   cccagucacc uugugcaagc cccugcccau cugggaaaau accccaucau ucaugcuacu 2580
   gccaaccugg ggagccaggg cuaugggagc agcuuuuuuu uccccccuag aaacguuugg 2640
   aacaauguaa aacuuuaaag cucgaaaaca auuguaauaa ugcuaaagaa aaagucaucc 2700
   aaucuaacca caucaauauu gucauuccug uauucacccg uccagaccuu guucacacuc 2760
   ucacauguuu agaguugcaa ucguaaugua cagaugguuu uauaaucuga uuuguuuucc 2820
   ucuuaacguu agaccacaaa uagugcucgc uuucuaugua guuugguaau uaucauuuua 2880
   gaagacucua ccagacugug uauucauuga agucagaugu gguaacuguu aaauugcugu 2940
   guaucugaua gcucuuuggc agucuauaug uuuguauaau gaaugagaga auaagucaug 3000
   uuccuucaag aucauguacc ccaauuuacu ugccauuacu caauugauaa acauuuaacu 3060
   uguuuccaau guuuagcaaa uacauauuuu auagaacuuc 3100
<210> 71
   <211> 355
   <212> PRT
   <213> Homo sapiens
<400> 71
   Met Asp Gln Phe Pro Glu Ser Val Thr Glu Asn Phe Glu Tyr Asp Asp
   1 5 10 15
   Leu Ala Glu Ala Cys Tyr Ile Gly Asp Ile Val Val Phe Gly Thr Val
               20                  25                  30
   Phe Leu Ser Ile Phe Tyr Ser Val Ile Phe Ala Ile Gly Leu Val Gly
           35                  40                  45
   Asn Leu Leu Val Val Phe Ala Leu Thr Asn Ser Lys Lys Pro Lys Ser
       50                  55                  60
   Val Thr Asp Ile Tyr Leu Leu Asn Leu Ala Leu Ser Asp Leu Leu Phe
   65                  70                  75                  80
   Val Ala Thr Leu Pro Phe Trp Thr His Tyr Leu Ile Asn Glu Lys Gly
                   85                  90                  95
   Leu His Asn Ala Met Cys Lys Phe Thr Thr Ala Phe Phe Phe Ile Gly
               100                 105                 110
   Phe Phe Gly Ser Ile Phe Phe Ile Thr Val Ile Ser Ile Asp Arg Tyr
           115                 120                 125
   Leu Ala Ile Val Leu Ala Ala Asn Ser Met Asn Asn Arg Thr Val Gln
       130                 135                 140
   His Gly Val Thr Ile Ser Leu Gly Val Trp Ala Ala Ala Ile Leu Val
   145                 150                 155                 160
   Ala Ala Pro Gln Phe Met Phe Thr Lys Gln Lys Glu Asn Glu Cys Leu
                   165                 170                 175
   Gly Asp Tyr Pro Glu Val Leu Gln Glu Ile Trp Pro Val Leu Arg Asn
               180                 185                 190
   Val Glu Thr Asn Phe Leu Gly Phe Leu Leu Pro Leu Leu Ile Met Ser
           195                 200                 205
   Tyr Cys Tyr Phe Arg Ile Ile Gln Thr Leu Phe Ser Cys Lys Asn His
       210                 215                 220
   Lys Lys Ala Lys Ala Ile Lys Leu Ile Leu Leu Val Val Ile Val Phe
   225                 230                 235                 240
   Phe Leu Phe Trp Thr Pro Tyr Asn Val Met Ile Phe Leu Glu Thr Leu
                   245                 250                 255
   Lys Leu Tyr Asp Phe Phe Pro Ser Cys Asp Met Arg Lys Asp Leu Arg
                   260                 265                 270
   Leu Ala Leu Ser Val Thr Glu Thr Val Ala Phe Ser His Cys Cys Leu
           275                 280                 285
   Asn Pro Leu Ile Tyr Ala Phe Ala Gly Glu Lys Phe Arg Arg Tyr Leu
       290                 295                 300
   Tyr His Leu Tyr Gly Lys Cys Leu Ala Val Leu Cys Gly Arg Ser Val
   305                 310                 315                 320
   His Val Asp Phe Ser Ser Ser Glu Ser Gln Arg Ser Arg His Gly Ser
                   325                 330                 335
   Val Leu Ser Ser Asn Phe Thr Tyr His Thr Ser Asp Gly Asp Ala Leu
               340                 345                 350
   Leu Leu Leu
           355

## Description

### FIELD OF THE INVENTION

The present invention relates generally to methods for diagnosing, monitoring, and treating kidney cancer.

### BACKGROUND OF THE INVENTION

Cancer remains one of the most deadly threats to human health. In the U.S. alone, cancer affects nearly 1.3 million new patients each year, and is the second leading cause of death after cardiovascular disease, accounting for approximately 1 in 4 deaths. Solid tumors are responsible for most of those deaths. Although there have been significant advances in the medical treatment of certain cancers, the overall 5-year survival rate for all cancers has improved only by about 10% in the past 20 years. Kidney cancer, in particular, has been rising in incidence since the 1990s and is now among the 10 most common cancers in both men and women.

Combination therapies pairing anti-angiogenesis agents with other anti-cancer agents, such as drugs that promote anti-tumor immunity, offer a promising new approach for the treatment of cancer, such as kidney cancer.

Accordingly, there is an unmet need in the field for improved therapies and diagnostic methods for cancers including kidney cancer.

Herbst et al. Nature 515(7528):563-567, 2014 discusses various factors predictive of response to anti-PD-L1 antibody atezolizmab monotherapy. Kuznar, "Adding Anti-PD-L1 Antibody to Bevacizumab Induces Responses in mRCC," 2015 (https://www.onclive.com/conference-coverage/gu-2015/adding-anti-pd-l1-antibody-to-bevacizumab-induces-responses-in-mrcc) concerns the combination therapy of anti-PD-L1 antibody atezolizumab and anti-VEGF antibody bevacizumab for mRCC and discloses that an increase in CD8+ cell infiltration was observed after treatment.

### SUMMARY OF THE INVENTION

The present invention provides compositions and methods for monitoring, diagnosing, and treating kidney cancer (e.g., metastatic renal cell carcinoma (mRCC)).

In one aspect, the invention features a method of monitoring the response of a patient having a kidney cancer to treatment with an anti-cancer therapy comprising a VEGF antagonist and a PD-L1 axis binding antagonist, the method comprising: (a) determining, in a biological sample obtained from the patient at a time point following administration of the anti-cancer therapy, the mRNA expression level of CD8A, CD8B, EOMES, GZMA, GZMB, IFNG, and PRF1; and (b) comparing the mRNA expression level of CD8A, CD8B, EOMES, GZMA, GZMB, IFNG, and PRF1 in the biological sample with a reference level, thereby monitoring the response in the patient to treatment with the anti-cancer therapy.

In some embodiments, the expression level of CD8A, CD8B, EOMES, GZMA, GZMB, IFNG, and PRF1 is correlated with the presence of CD8⁺ T effector (T_{eff}) cells in the tumor microenvironment. In some embodiments, the expression level of one or more of CXCL9, CXCL10, CXCL11, or CXCL13 is correlated with the presence of Th1 chemokines in the tumor microenvironment.
In some embodiments, the presence of GZMB, KLRK1, or SLAMF7 is correlated with the presence of natural killer (NK) cells in the tumor microenvironment.

In some embodiments, the expression level of one or more of CXCL9, CXCL10, CXCL11, or CXCL13 is determined. In some embodiments, the expression level of at least two or at least three of CXCL9, CXCL10, CXCL11, or CXCL13 is determined. In some embodiments, the expression level of CXCL9, CXCL10, CXCL11, and CXCL13 is determined.

In some embodiments, the expression level of one or more of GZMB, KLRK1, or SLAMF7 is determined. In some embodiments, the expression level of at least two of GZMB, KLRK1, or SLAMF7 is determined. In some embodiments, the expression level of GZMB, KLRK1, and SLAMF7 is determined.

In some embodiments, the reference level is selected from the group consisting of (i) the expression level of the one or more genes in a biological sample from the patient obtained prior to administration of the anti-cancer therapy; (ii) the expression level of the one or more genes in a reference population; (iii) a pre-assigned expression level for the one or more genes; (iv) the expression level of the one or more genes in a biological sample obtained from the patient at a previous time point, wherein the previous time point is following administration of the anti-cancer therapy; or (v) the expression level of the one or more genes in a biological sample obtained from the patient at a subsequent time point. In some embodiments, the expression level of the one or more genes is increased in the biological sample obtained from the patient relative to the reference level.

In some embodiments, the expression level of CD8A, CD8B, EOMES, GZMA, GZMB, IFNG, and PRF1 is increased at least about 2-fold relative to the reference level. In some embodiments, the expression level of CD8A, CD8B, EOMES, GZMA, GZMB, IFNG, and PRF1 is increased at least about 15-fold relative to the reference level. In some embodiments, the expression level of CD8A, CD8B, EOMES, GZMA, GZMB, IFNG, and PRF1 is increased at least about 50-fold relative to the reference level.

In some embodiments, the expression level of one or more of CXCL9, CXCL10, CXCL11, or CXCL13 is increased at least about 3-fold relative to the reference level. In some embodiments, the expression level of one or more of CXCL9, CXCL10, CXCL11, or CXCL13 is increased at least about 80-fold relative to the reference level. In some embodiments, the expression level of one or more of CXCL9, CXCL10, CXCL11, or CXCL13 is increased at least about 250-fold relative to the reference level.

In some embodiments, the expression level of one or more of GZMB, KLRK1, or SLAMF7 is increased at least about 2-fold relative to the reference level. In some embodiments, the expression level of one or more of GZMB, KLRK1, or SLAMF7 is increased at least about 8-fold relative to the reference level. In some embodiments, the expression level of one or more of GZMB, KLRK1, or SLAMF7 is increased at least about 13-fold relative to the reference level.

In some embodiments, the increased expression level of the one or more genes indicates that the patient is responding to the VEGF antagonist.

In some embodiments of any of the preceding aspects, the biological sample from the patient is obtained about 4 to about 6 weeks following administration of the anti-cancer therapy.

In another aspect, the invention features an anti-cancer therapy comprising a VEGF antagonist and a PD-L1 axis binding antagonist for use in a method of treating a patient having a kidney cancer, the method comprising: (a) determining, in a biological sample obtained from the patient at a time point following administration of the anti-cancer therapy, the expression level of CD8A, CD8B, EOMES, GZMA, GZMB, IFNG, and PRF1; CXCL9, CXCL10, CXCL11, or CXCL13; or GZMB, KLRK1, or SLAMF7; (b) comparing the expression level of the one or more genes in the biological sample with a reference level; and (c) continuing to administer the anti-cancer therapy to the patient if the expression level of their one or more genes is increased relative to the reference level.

In some embodiments, the expression level of CD8A, CD8B, EOMES, GZMA, GZMB, IFNG, and PRF1 is correlated with the presence of CD8⁺ T effector (T_{eff}) cells in the tumor microenvironment. In some embodiments, the expression level of one or more of CXCL9, CXCL10, CXCL11, or CXCL13 is correlated with the presence of Th1 chemokines in the tumor microenvironment. In some embodiments, the presence of GZMB, KLRK1, or SMALF7 is correlated with the presence of natural killer (NK) cells in the tumor microenvironment.

In some embodiments, the expression level of one or more of CXCL9, CXCL10, CXCL11, or CXCL13 is determined. In some embodiments, the expression level of at least two or at least three of CXCL9, CXCL10, CXCL11, or CXCL13 is determined. In some embodiments, the expression level of CXCL9, CXCL10, CXCL11, and CXCL13 is determined.

In some embodiments, the expression level of one or more of GZMB, KLRK1, or SLAMF7 is determined. In some embodiments, the expression level of at least two of GZMB, KLRK1, or SLAMF7 is determined. In some embodiments, the expression level of GZMB, KLRK1, and SLAMF7 is determined.

In some embodiments, the reference level is selected from the group consisting of (i) the expression level of the one or more genes in a biological sample from the patient obtained prior to administration of the anti-cancer therapy; (ii) the expression level of the one or more genes in a reference population; (iii) a pre-assigned expression level for the one or more genes; (iv) the expression level of the one or more genes in a biological sample obtained from the patient at a previous time point, wherein the previous time point is following administration of the anti-cancer therapy; or (v) the expression level of the one or more genes in a biological sample obtained from the patient at a subsequent time point.

In some embodiments, the expression level of CD8A, CD8B, EOMES, GZMA, GZMB, IFNG, anbd PRF1 is increased at least about 2-fold relative to the reference level. In some embodiments, the expression level of CD8A, CD8B, EOMES, GZMA, GZMB, IFNG, and PRF1 is increased at least about 15-fold relative to the reference level. In some embodiments, the expression level of CD8A, CD8B, EOMES, GZMA, GZMB, IFNG, and PRF1 is increased at least about 50-fold relative to the reference level.

In some embodiments, the expression level of one or more of CXCL9, CXCL10, CXCL11, or CXCL13 is increased at least about 3-fold relative to the reference level. In some embodiments, the expression level of one or more of CXCL9, CXCL10, CXCL11, or CXCL13 is increased at least about 80-fold relative to the reference level. In some embodiments, the expression level of one or more of CXCL9, CXCL10, CXCL11, or CXCL13 is increased at least about 250-fold relative to the reference level.

In some embodiments, the expression level of one or more of GZMB, KLRK1, or SLAMF7 is increased at least about 2-fold relative to the reference level. In some embodiments, the expression level of one or more of GZMB, KLRK1, or SLAMF7 is increased at least about 8-fold relative to the reference level. In some embodiments, the expression level of one or more of GZMB, KLRK1, or SLAMF7 is increased at least about 13-fold relative to the reference level.

In some embodiments of any of the preceding aspects, the biological sample from the patient is obtained about 4 to about 6 weeks following administration of the anti-cancer therapy. In some embodiments, the VEGF antagonist is an anti-VEGF antibody, e.g., bevacizumab. In some embodiments, the PD-L1 axis binding antagonist is selected from the group consisting of a PD-L1 binding antagonist, a PD-1 binding antagonist, and a PD-L2 binding antagonist.

In some embodiments of any of the preceding aspects, the PD-L1 axis binding antagonist is a PD-L1 binding antagonist. In some embodiments, the PD-L1 binding antagonist inhibits the binding of PD-L1 to one or more of its ligand binding partners. In some embodiments, the PD-L1 binding antagonist inhibits the binding of PD-L1 to PD-1. In some embodiments, the PD-L1 binding antagonist inhibits the binding of PD-L1 to B7-1. In some embodiments, the PD-L1 binding antagonist inhibits the binding of PD-L1 to both PD-1 and B7-1.

In some embodiments, the PD-L1 binding antagonist is an antibody. In some embodiments, the antibody is selected from the group consisting of: MPDL3280A (atezolizumab), YW243.55.S70, MDX-1105, MEDI4736 (durvalumab), and MSB0010718C (avelumab). In some embodiments, the antibody comprises a heavy chain comprising HVR-H1 sequence of SEQ ID NO:19, HVR-H2 sequence of SEQ ID NO:20, and HVR-H3 sequence of SEQ ID NO:21; and a light chain comprising HVR-L1 sequence of SEQ ID NO:22, HVR-L2 sequence of SEQ ID NO:23, and HVR-L3 sequence of SEQ ID NO:24. In some embodiments, the antibody comprises a heavy chain variable region comprising the amino acid sequence of SEQ ID NO:26 and a light chain variable region comprising the amino acid sequence of SEQ ID NO:4.

In some embodiments, the PD-L1 axis binding antagonist is a PD-1 binding antagonist. In some embodiments, the PD-1 binding antagonist inhibits the binding of PD-1 to one or more of its ligand binding partners. In some embodiments, the PD-1 binding antagonist inhibits the binding of PD-1 to PD-L1. In some embodiments, the PD-1 binding antagonist inhibits the binding of PD-1 to PD-L2. In some embodiments, the PD-1 binding antagonist inhibits the binding of PD-1 to both PD-L1 and PD-L2.

In some embodiments, the PD-1 binding antagonist is an antibody. In some embodiments, the antibody is selected from the group consisting of: MDX-1106 (nivolumab), MK-3475 (pembrolizumab), CT-011 (pidilizumab), MEDI-0680 (AMP-514), PDR001, REGN2810, and BGB-108. In some embodiments, the PD-1 binding antagonist is an Fc-fusion protein. In some embodiments, the Fc-fusion protein is AMP-224.

In some embodiments of any of the preceding aspects, the method further comprises administering an additional therapeutic agent to the patient. In some embodiments, the additional therapeutic agent is selected from the group consisting of an immunotherapy agent, a cytotoxic agent, a growth inhibitory agent, a radiation therapy agent, an anti-angiogenic agent, and combinations thereof.

In some embodiments of any of the preceding aspects, the kidney cancer is a renal cell carcinoma. In some embodiments, the renal cell carcinoma is a metastatic renal cell carcinoma.

In some embodiments of any of the preceding aspects, the expression level is an mRNA expression level. In some embodiments, the mRNA expression level is determined using a method selected from the group consisting of quantitative polymerase chain reaction (qPCR), reverse transcription qPCR (RT-qPCR), RNA sequencing, microarray analysis, in situ hybridization, and serial analysis of gene expression (SAGE).

In some embodiments of any of the preceding aspects, the expression level is a protein expression level. In some embodiments, the protein expression level is determined using a method selected from the group consisting of immunohistochemistry (IHC), immunofluorescence, mass spectrometry, flow cytometry, and Western blot.

In some embodiments of any of the preceding aspects, the biological sample obtained from the patient is a tumor sample or a cell sample. In some embodiments, the tumor sample is formalin-fixed and paraffin-embedded, fresh, archival, or frozen. In some embodiments, the cell sample comprises peripheral CD8⁺ T cells.

In some embodiments of any of the preceding aspects, the patient is a human patient.

### BRIEF DESCRIPTION OF THE DRAWINGS

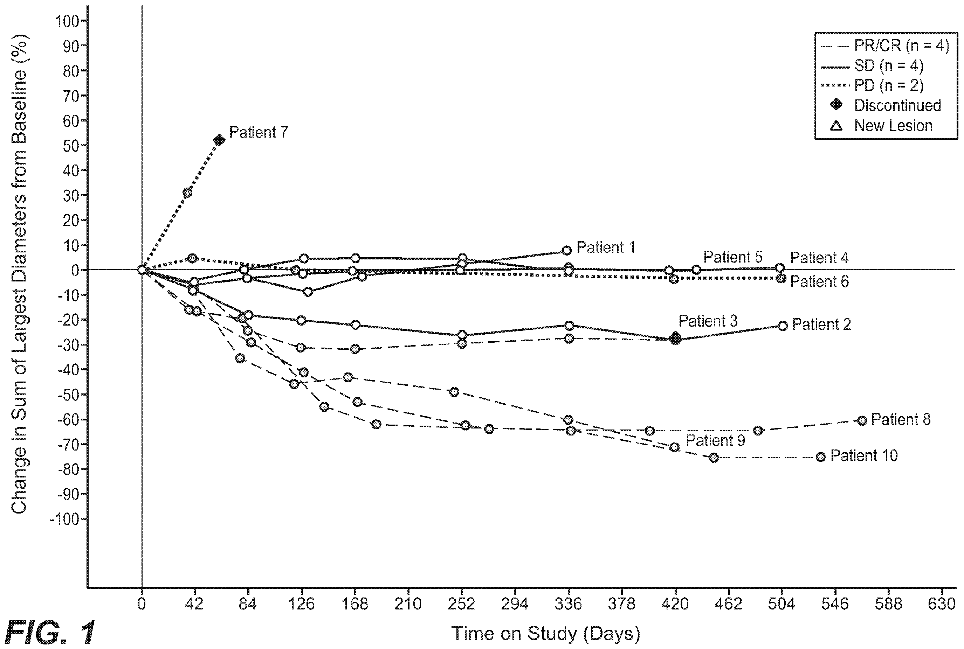

FIG. 1 is a graph showing the tumor burden over time among renal cell carcinoma (RCC) patients receiving atezolizumab and bevacizumab combination treatment. Points on the graph show the maximum reduction from baseline in the sum of the longest diameter (SLD) for target lesions. PR, partial response; PD, progressive disease; SD, stable disease.

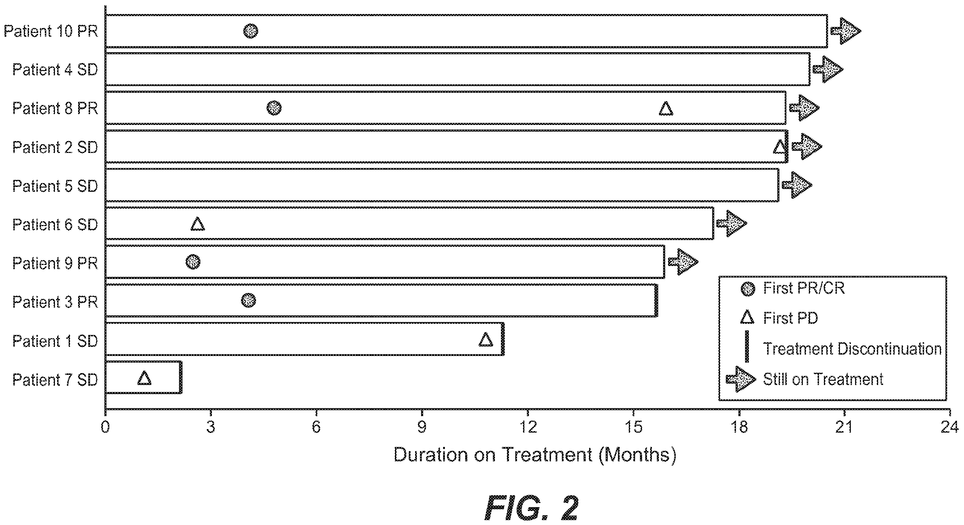

FIG. 2 is a graph showing the duration of study treatment for each RCC patient. Time of first PR or CR is indicated with a circle; time of first PD is indicated with a triangle; treatment discontinuation is indicated with a black bar; and patients still on treatment as of the time of analysis are identified with an arrow.

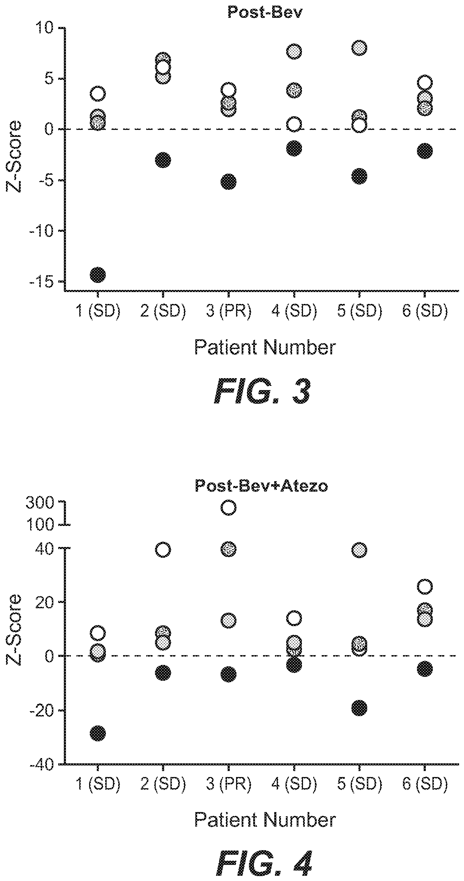

FIG. 3 is a graph showing gene expression levels of tumor biomarkers following bevacizumab ("Bev") treatment. The expression levels of on-treatment tumor samples are shown relative to the baseline expression levels (pre-treatment). Vascular signature genes (ANGPT2, CD34, DLL4, EGFL7, and ESM1) are shown in black, CD8 T cell effector genes (CD8A, CD8B, EOMES, GZMA, IFNG, and PRF1) are shown in patterned gray, Th1 chemokines (CXCL10, CXCL11, CXCL13, and CXCL9) are shown in white, and natural killer (NK) cell genes (GZMB, KLRK1, and SLAMF7) are shown in solid gray.
FIG. 4 is a graph showing gene expression levels of tumor biomarkers following atezolizumab and bevacizumab combination ("Bev+Atezo") treatment. The expression levels of on-treatment tumor samples are shown relative to the baseline expression levels (pre-treatment). Vascular signature genes (ANGPT2, CD34, DLL4, EGFL7, and ESM1) are shown in black, CD8 T cell effector genes (CD8A, CD8B, EOMES, GZMA, IFNG, and PRF1) are shown in patterned gray, Th1 chemokines (CXCL10, CXCL11, CXCL13, and CXCL9) are shown in white, and NK cell genes (GZMB, KLRK1, and SLAMF7) are shown in solid gray.

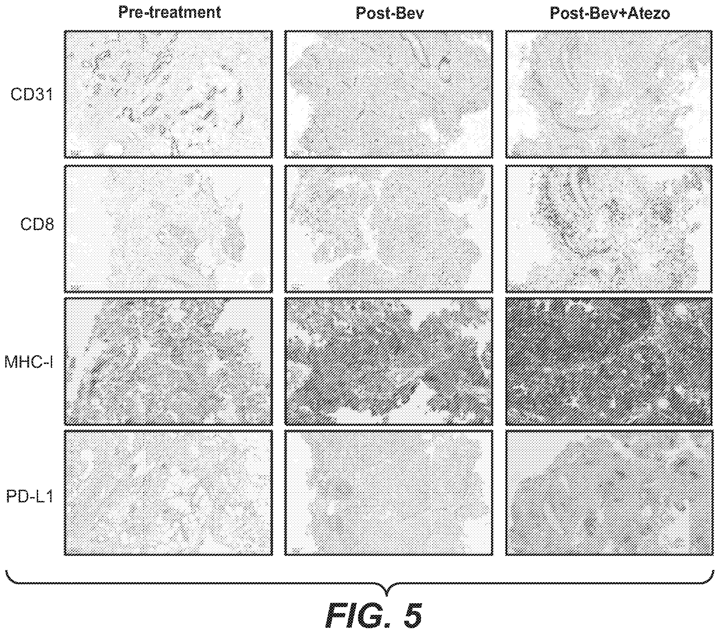

FIG. 5 is a series of representative images showing protein expression of immune and vasculature markers in tumor samples from patient 3, as assessed by immunohistochemistry (IHC). CD31 is stained dark gray (first row), CD8 is stained dark gray (second row), MHC-I is stained dark gray (third row), and PD-L1 is stained dark gray (fourth row). Pre-treatment samples are shown in the left-hand column, post-bevacizumab samples are shown in the middle column, and post-bevacizumab+atezolizumab samples are shown in the right-hand column.

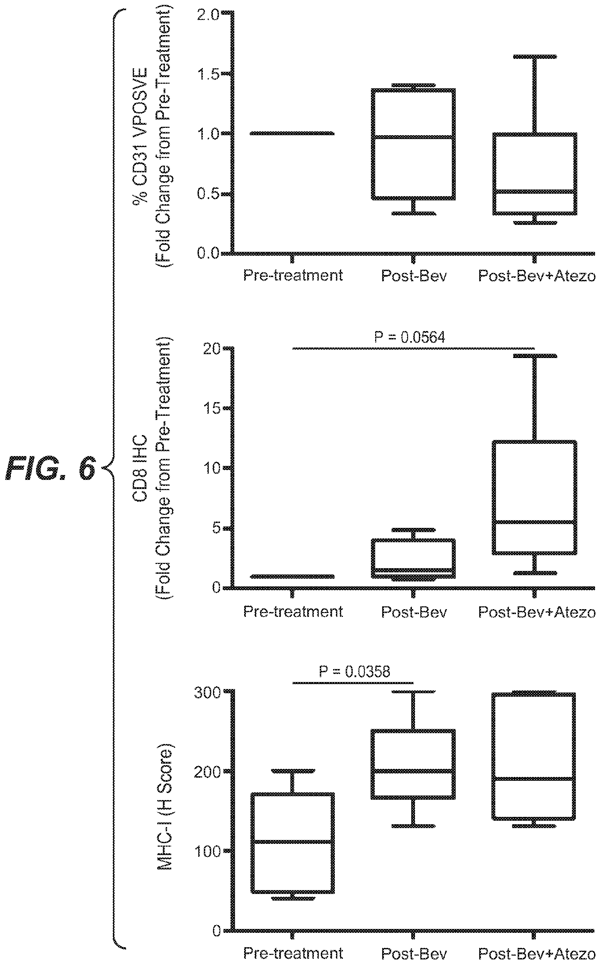

FIG. 6 is a series of graphs showing quantification of immune and vasculature markers at the indicated time points, as assessed by IHC. CD31 expression is shown in the top panel, CD8 expression is shown in the middle panel, and MHC-I expression is shown in the bottom panel. P values were determined by paired t-test. "VPOSVE" is a measure of vessel density.

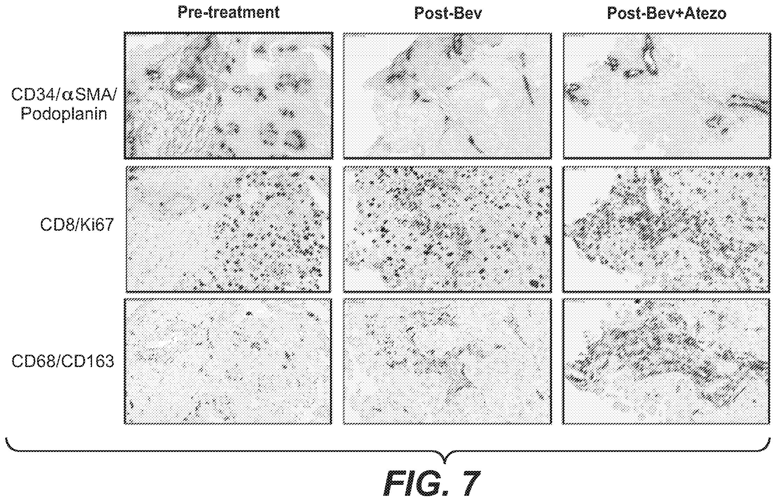

FIG. 7 is a series of representative images showing protein expression of immune and vasculature markers from serial sections of tumor samples from patient 3, as assessed by IHC. The first row shows expression of CD34, alpha smooth muscle actin (αSMA), and podoplanin. The second row shows expression of CD8 and Ki67. The third row shows expression of CD68 and CD163. Pre-treatment samples are shown in the left-hand column, post-bevacizumab samples are shown in the middle column, and post-bevacizumab+atezolizumab samples are shown in the right-hand column.

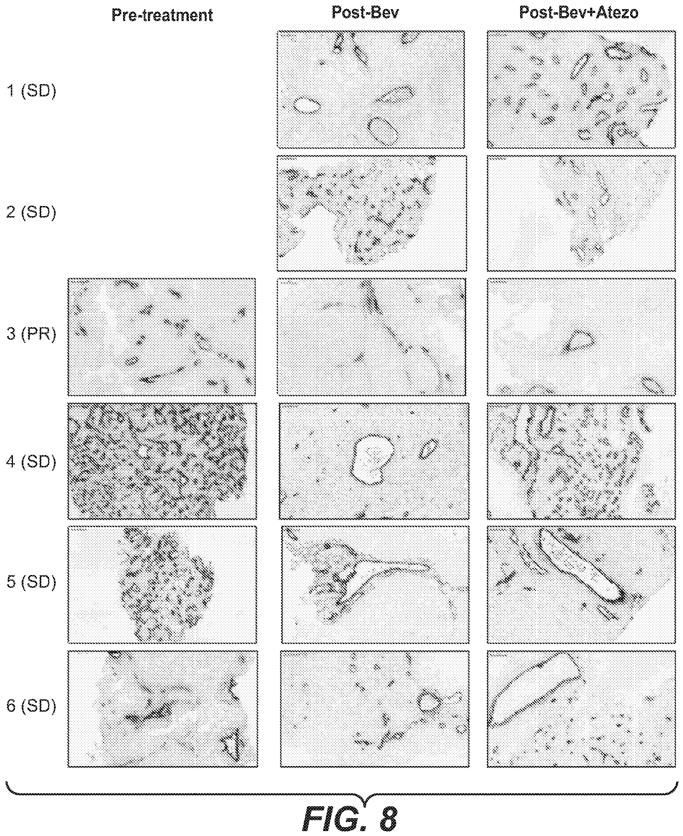

FIG. 8 is a series of representative images showing protein expression of CD34 and αSMA in tumor samples, as assessed by IHC. Pre-treatment samples are shown in the left-hand column, post-bevacizumab samples are shown in the middle column, and post-bevacizumab+atezolizumab samples are shown in the right-hand column. Sections of individual patients 1-6 are arranged on each row. The response of each patient is also indicated.

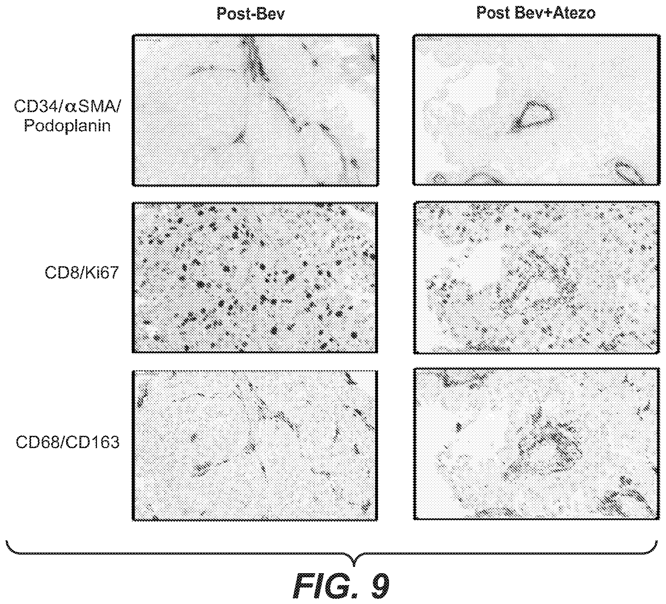

FIG. 9 is a series of representative images showing protein expression of immune and vasculature markers from serial sections of tumors, as assessed by IHC. The first row shows expression of CD34, αSMA, and podoplanin. The second row shows expression of CD8 and Ki67. The third row shows expression of CD68 and CD163. Post-bevacizumab samples are shown in the left-hand column, and post-bevacizumab+atezolizumab samples are shown in the right-hand column.

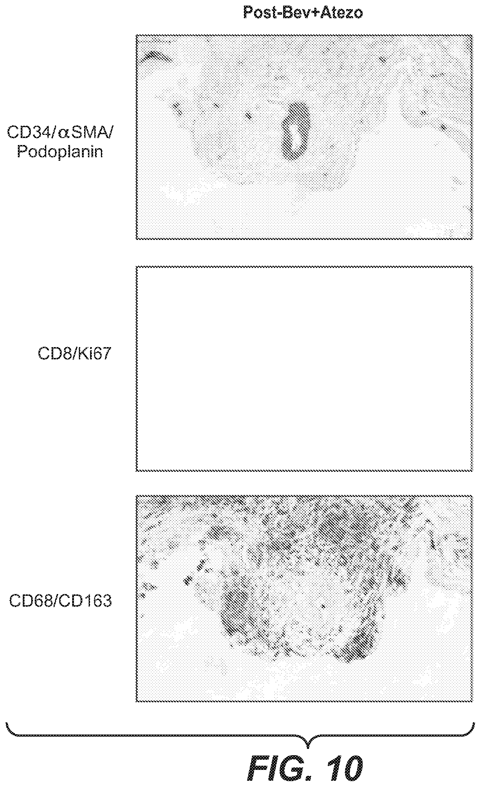

FIG. 10 is a series of representative images showing protein expression of immune and vasculature markers from serial sections of tumors post-bevacizumab+atezolizumab, as assessed by IHC. The top image shows expression of CD34, αSMA, and podoplanin. The middle image shows expression of CD8 and Ki67. The bottom image shows expression of CD68 and CD163.

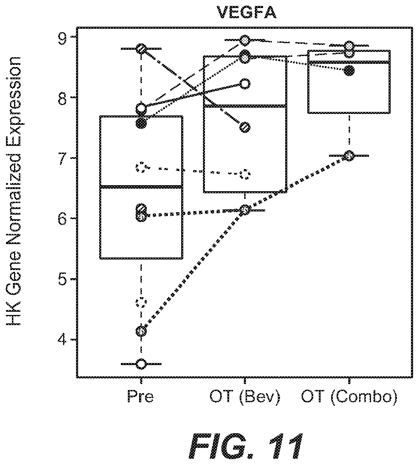

FIG. 11 is a graph showing the upregulation of VEGF transcript expression in tumors in response to bevacizumab and bevacizumab+atezolizumab treatment. Expression is normalized to housekeeping (HK) gene expression. Each line represents an individual patient.

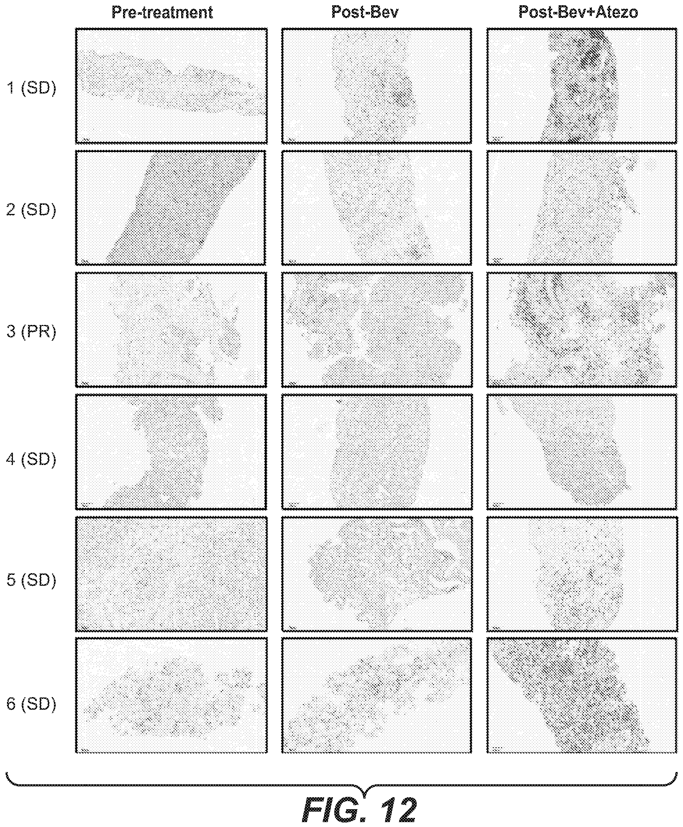

FIG. 12 is a series of representative images showing protein expression of CD8 (stained dark gray) from tumor sections, as assessed by IHC. Pre-treatment samples are shown in the left-hand column, post-bevacizumab samples are shown in the middle column, and post-bevacizumab+atezolizumab samples are shown in the right-hand column. Sections of individual patients 1-6 are arranged on each row. The response of each patient is also indicated.

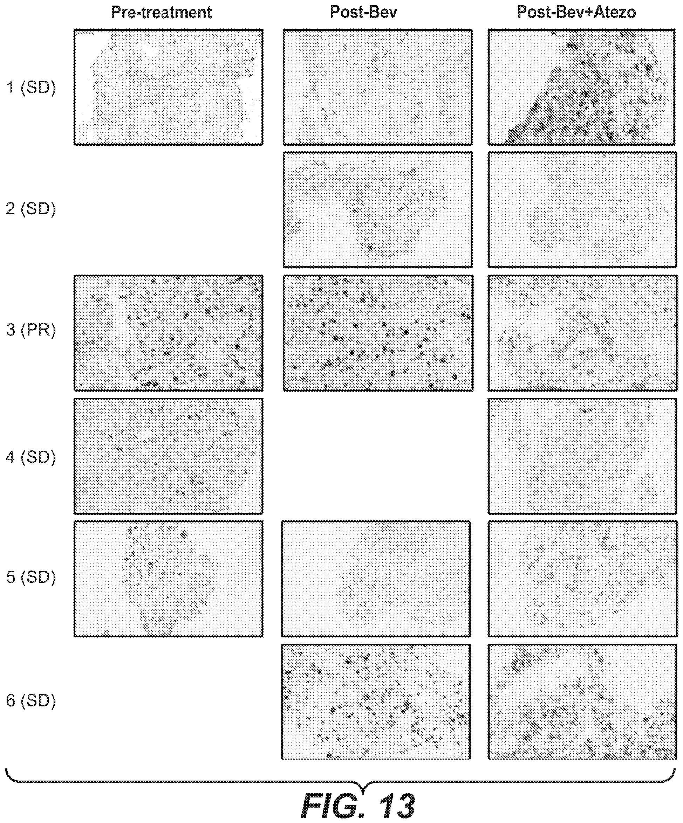

FIG. 13 is a series of representative images showing protein expression of CD8 and Ki67 from tumor sections, as assessed by IHC. Pre-treatment samples are shown in the left-hand column, post-bevacizumab samples are shown in the middle column, and post-bevacizumab+atezolizumab samples are shown in the right-hand column. Sections of individual patients 1-6 are arranged on each row.

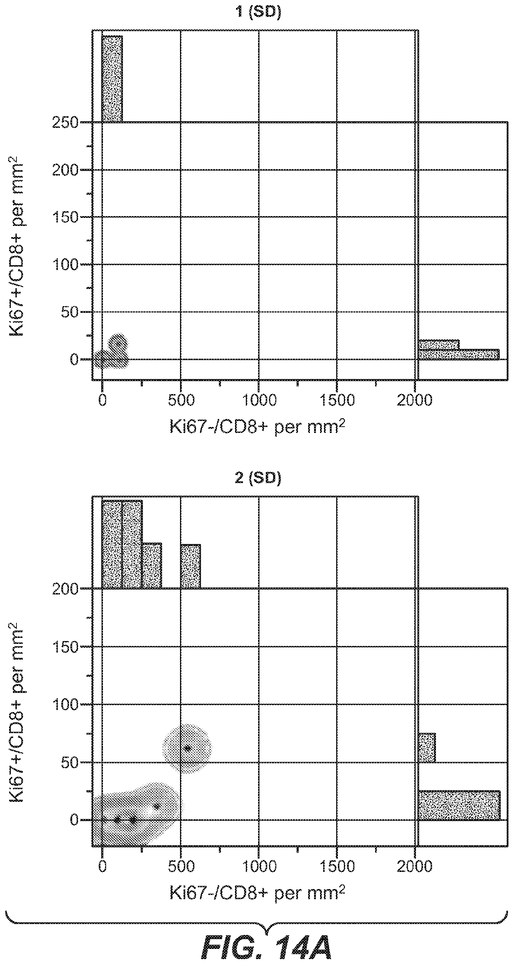

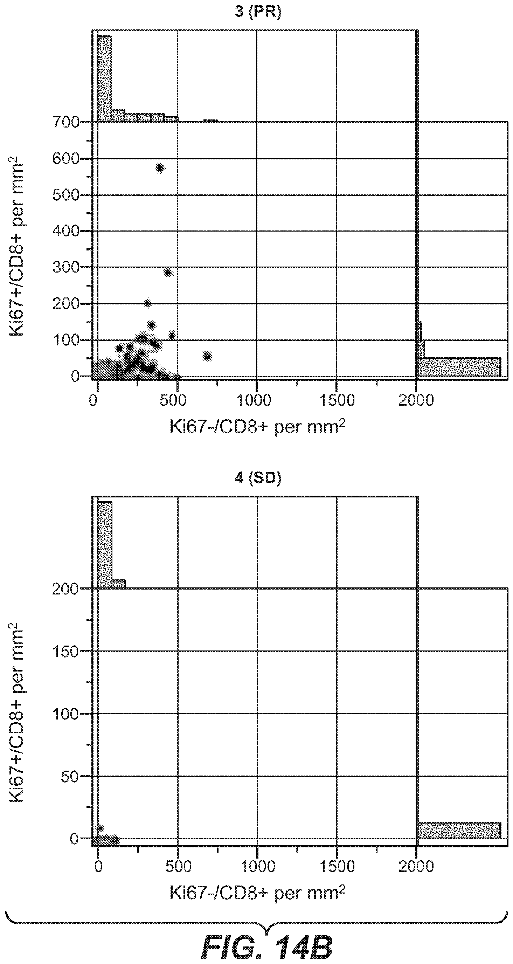

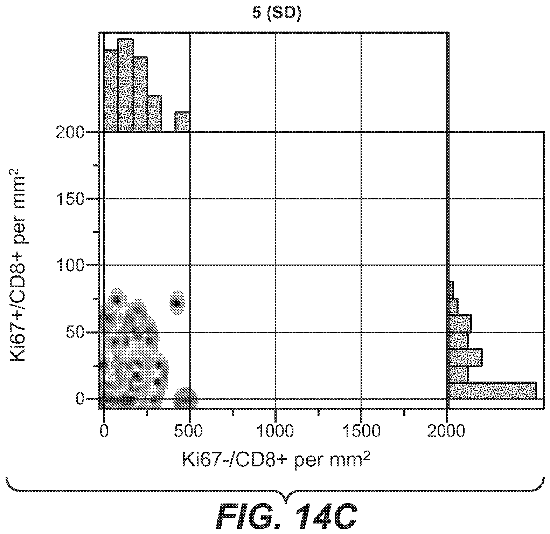

FIGS. 14A-14C are a series of graphs showing the number of cells expressing CD8 or both Ki67 and CD8 per square millimeter (mm²) tumor area for each patient at the pre-treatment time point. Fig. 14A shows data for patients 1 and 2. Fig. 14B shows data for patients 3 and 4. Fig. 14C shows data for patient 5. The response of each patient is also indicated.

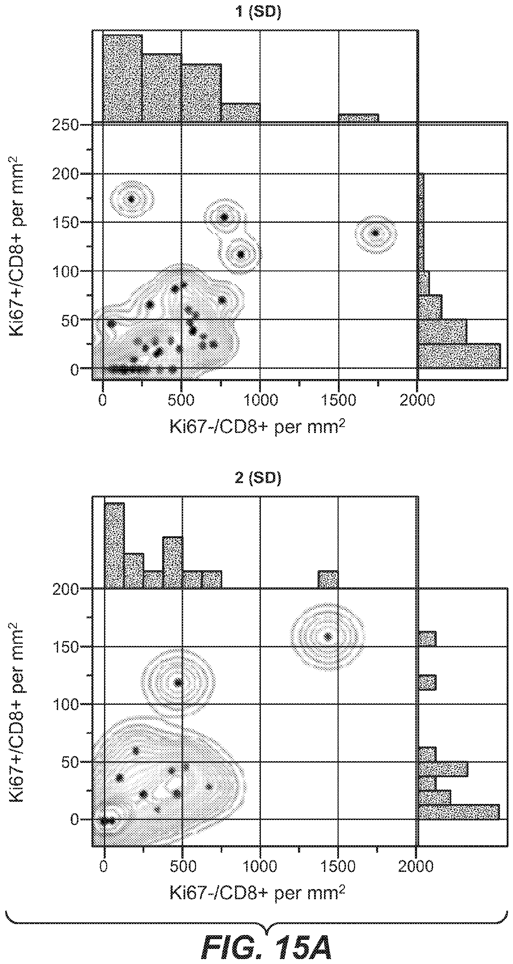

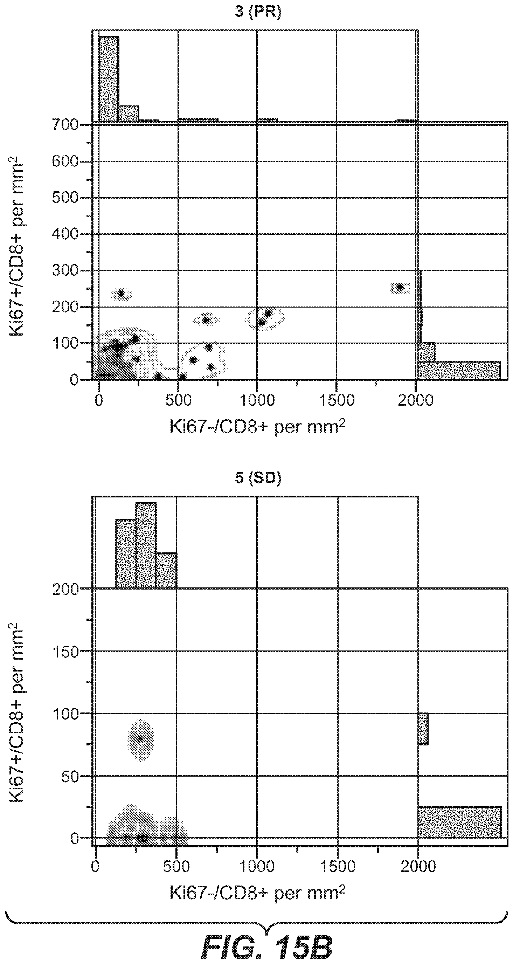

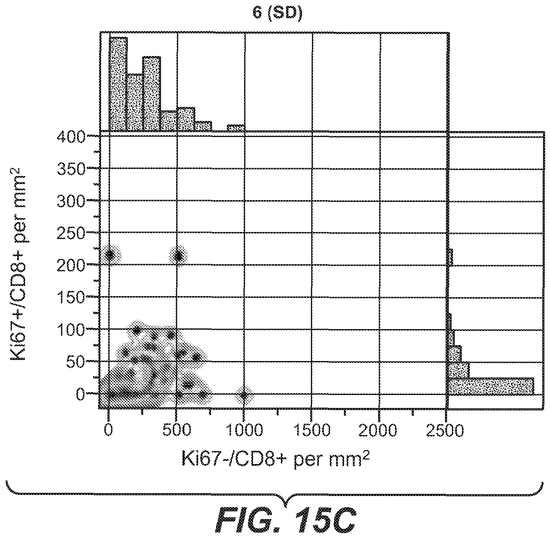

FIGS. 15A-15C are a series of graphs showing the number of cells expressing CD8 or both Ki67 and CD8 per mm² tumor area for each patient at the post-bevacizumab time point. Fig. 15A shows data for patients 1 and 2. Fig. 15B shows data for patients 3 and 5. Fig. 15C shows data for patient 6. The response of each patient is also indicated.

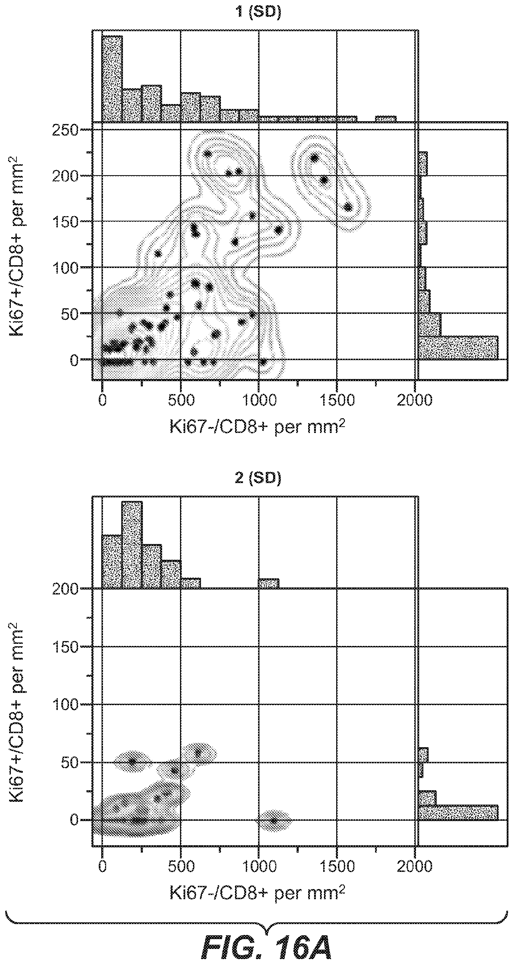

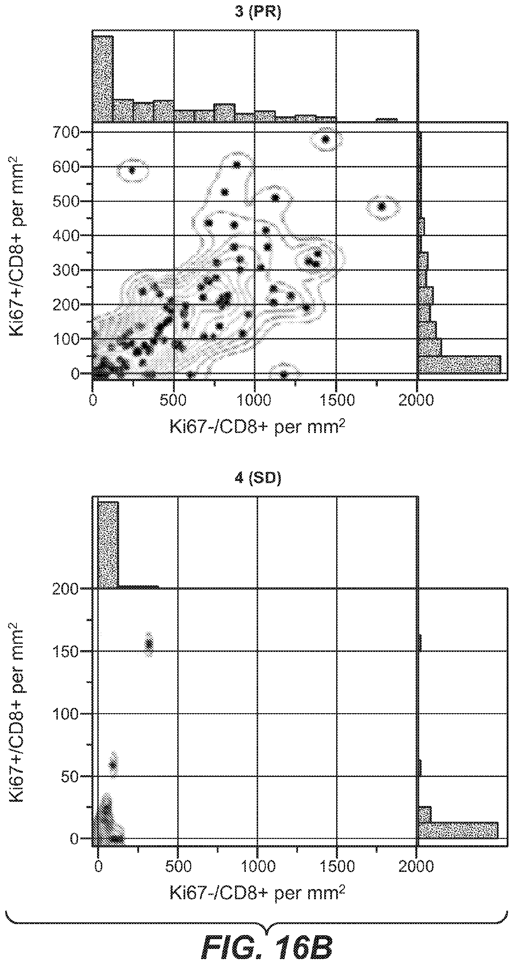

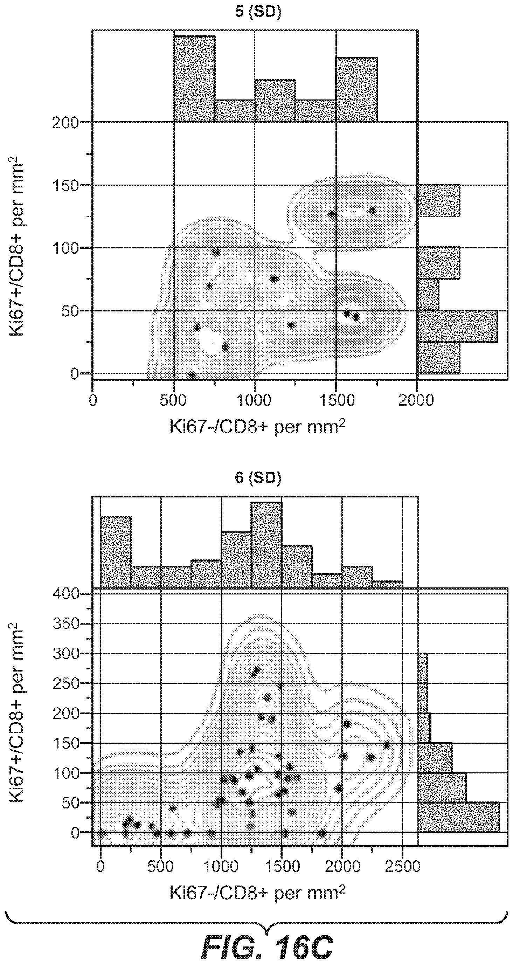

FIGS. 16A-16C are a series of graphs showing the number of cells expressing CD8 or both Ki67 and CD8 per mm² tumor area for each patient at the post-bevacizumab+atezolizumab time point. Fig. 16A shows data for patients 1 and 2. Fig. 16B shows data for patients 3 and 4. Fig. 16C shows data for patients 5 and 6. The response of each patient is also indicated.

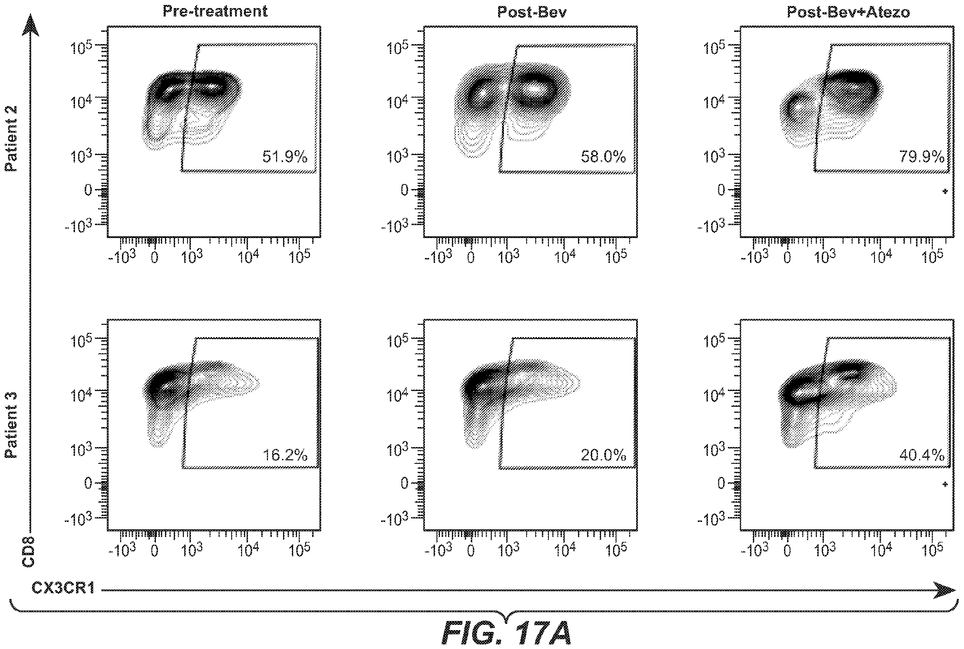

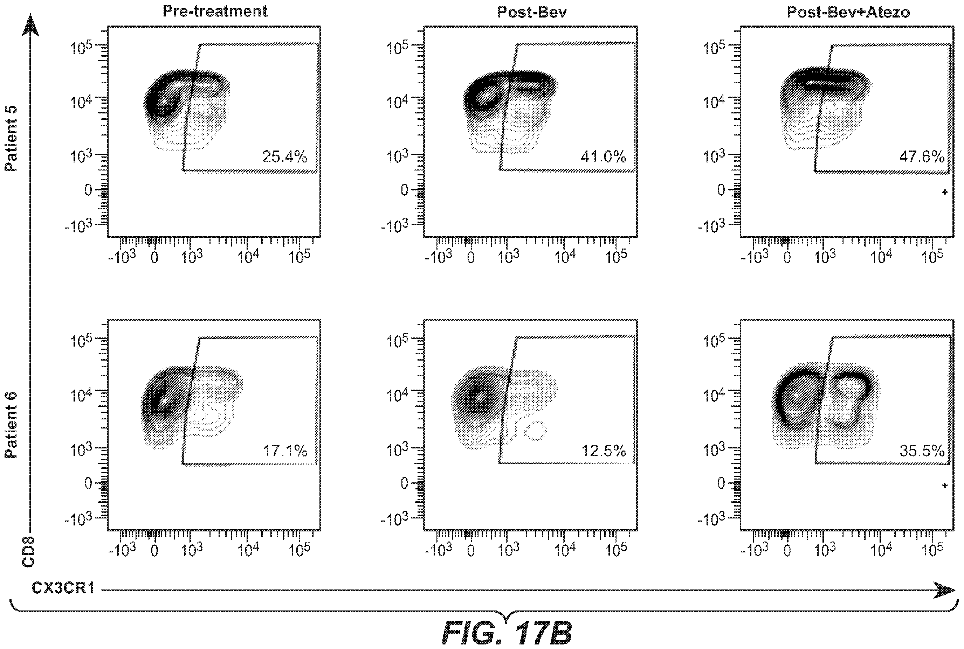

FIGS. 17A and 17B are a series of flow cytometry plots showing the percentage of CD8⁺ cells in tumor samples expressing CX3CR1. Pre-treatment samples are shown in the left-hand column, post-bevacizumab samples are shown in the middle column, and post-bevacizumab+atezolizumab samples are shown in the right-hand column. Each row shows the results from an individual patient. Fig. 17A shows data for patients 2 and 3. Fig. 17B shows data for patients 5 and 6.

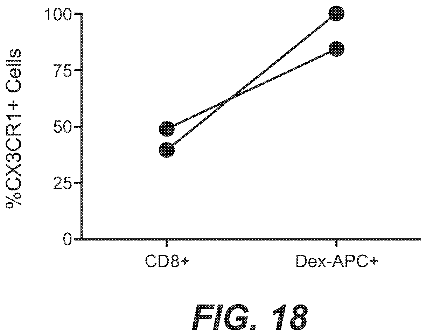

FIG. 18 is a graph showing the expression of CX3CR1 as a percentage of total CD8⁺ cells (left) and as a percentage of tumor antigen-specific (Dex-APC⁺) T cells (right), based on flow cytometry analysis.

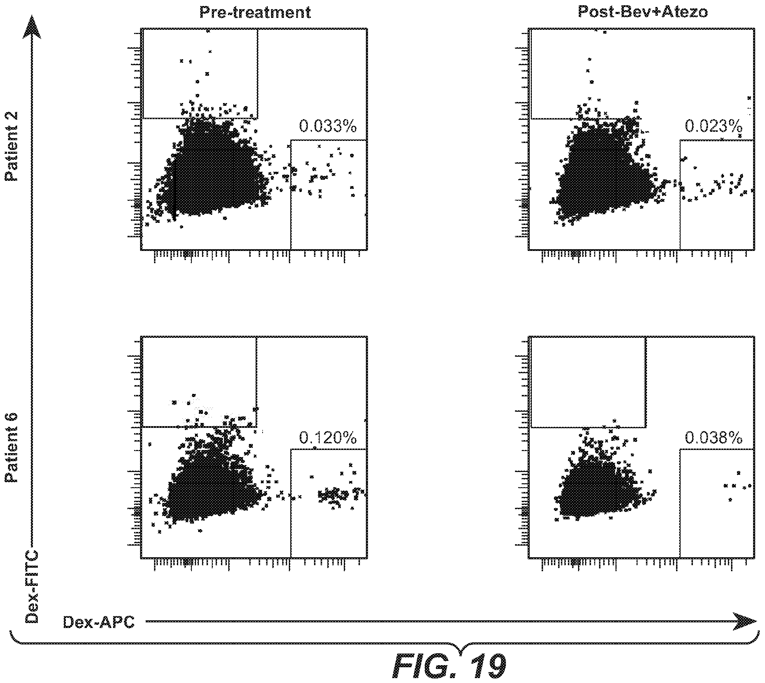

FIG. 19 is a series of flow cytometry plots showing representative frequencies of antigen-specific T cells in the blood. Representative data from two HLA-A2-positive patients with blood draws matched to tumor biopsy time points are shown.

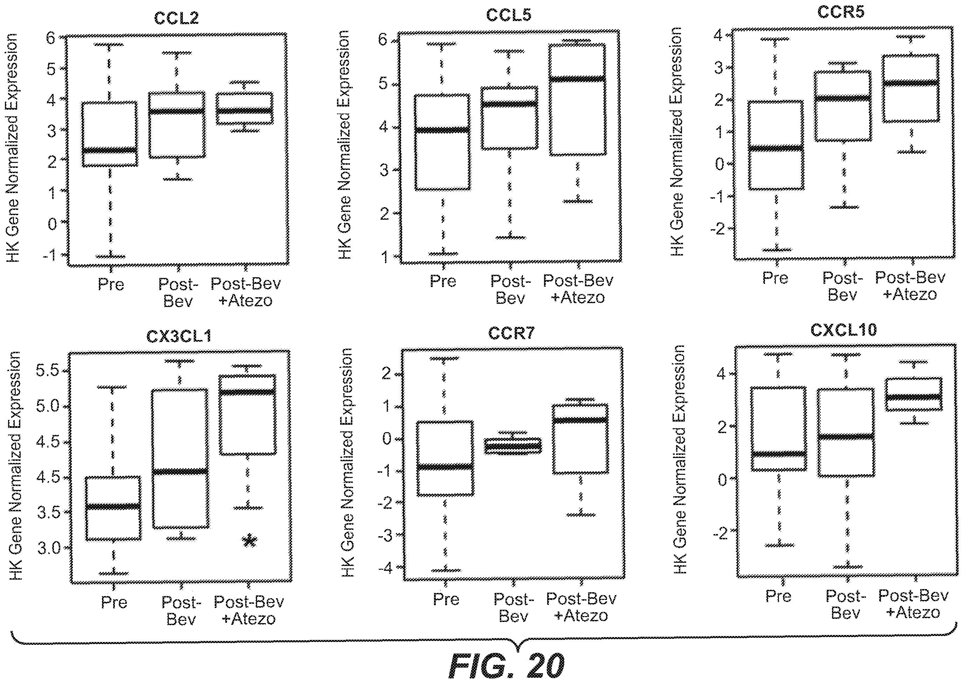

FIG. 20 is a series of graphs showing the change in chemokine expression (CCL2, CCL5, CCR5, CX3CL1, CCR7, and CXCL10) in tumors at the indicated treatment time points. Expression was normalized to housekeeping (HK) gene expression.

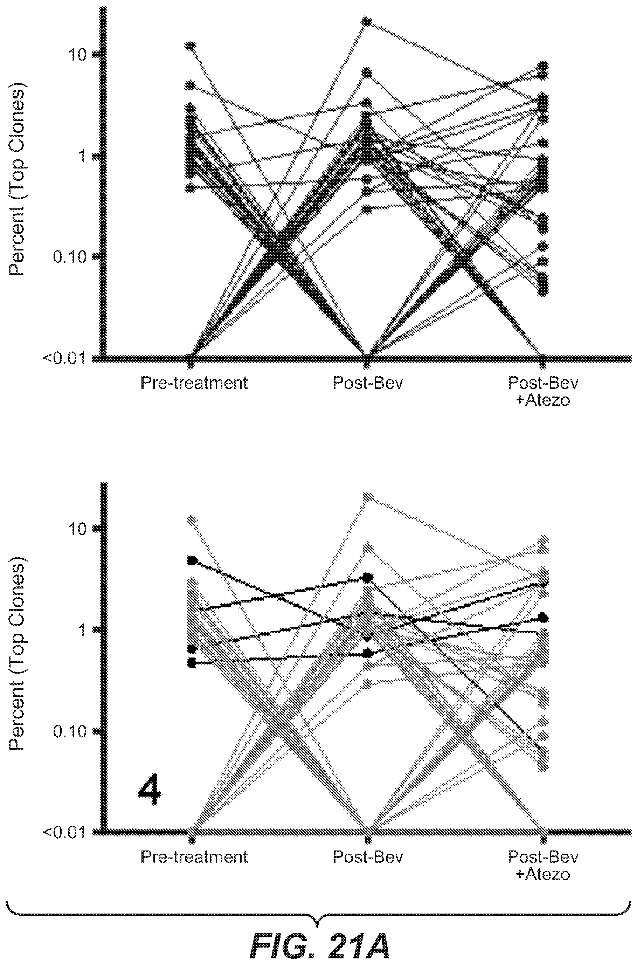

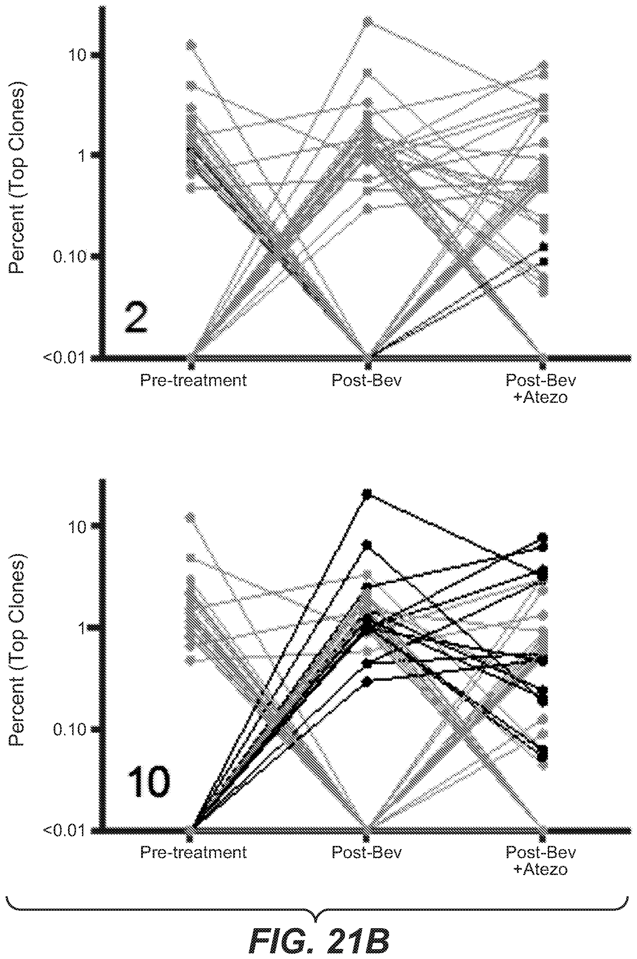

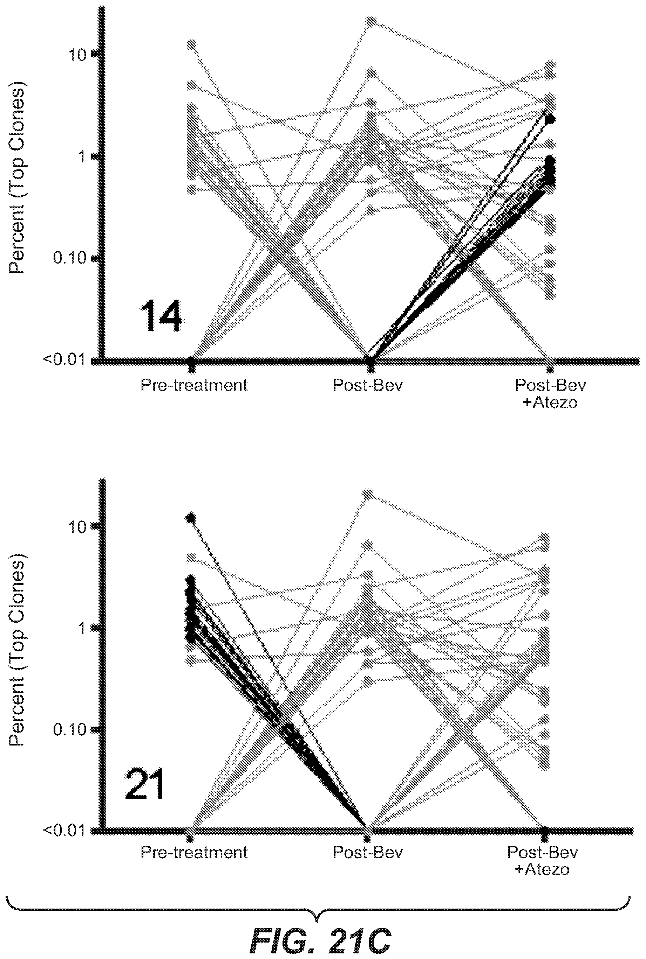

FIGS. 21A-21C are a series of graphs showing the results of TCRβ sequencing of infiltrating lymphocytes (TILs) before and after treatment in tumor samples from patient 6. The top clones (up to 25) for each group are shown. The prevalence of trending TCRβ clone populations are shown in the darker lines in Figs. 21A (bottom panel), 22B, and 22C.

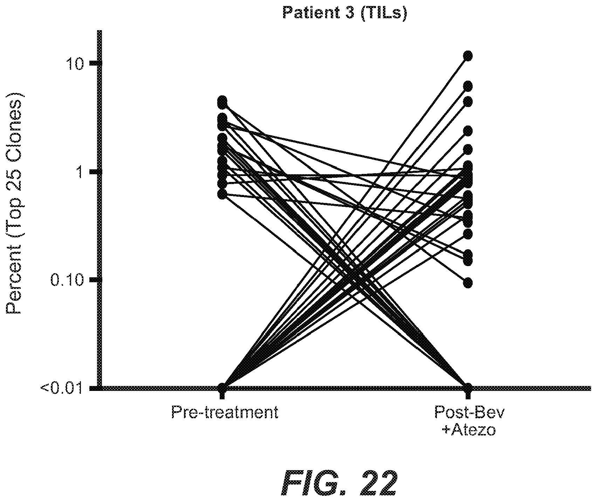

FIG. 22 is a graph showing the results of TCRβ sequencing from patient 3 TILs before and after bevacizumab+atezolizumab treatment.

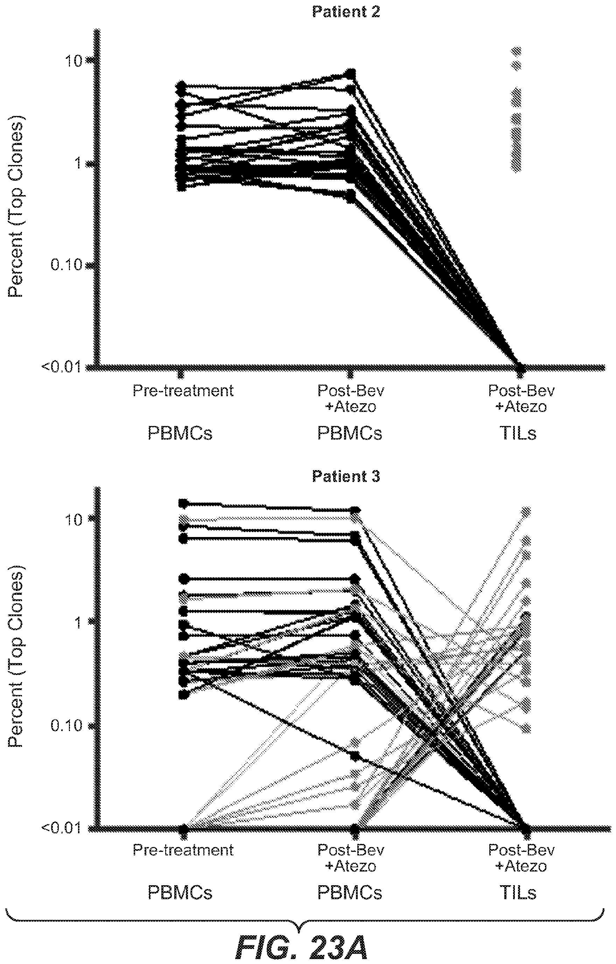

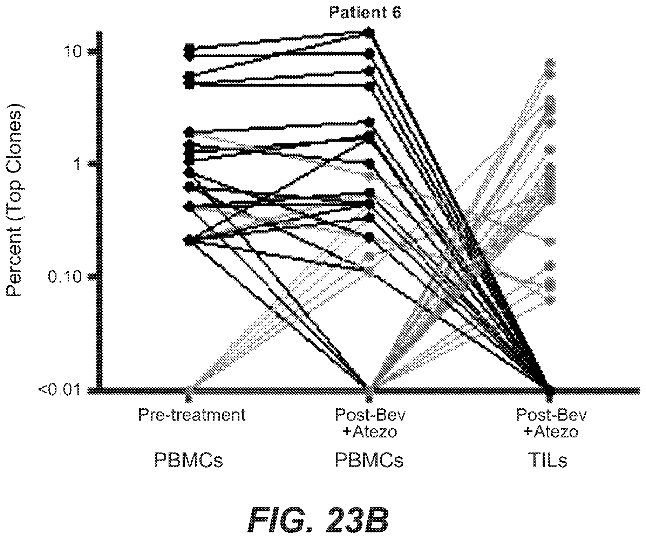

FIGS. 23A and 23B are a series of graphs showing the results of TCRβ sequencing of pre-treatment PBMCs, post-bevacizumab+atezolizumab peripheral blood mononuclear cells (PBMCs), and post-bevacizumab+atezolizumab TILs from patients 2, 3, and 6. Fig. 23A shows data for patients 2 and 3, and Fig. 23B shows data for patient 6. The top clones (up to 25) for each group are shown.

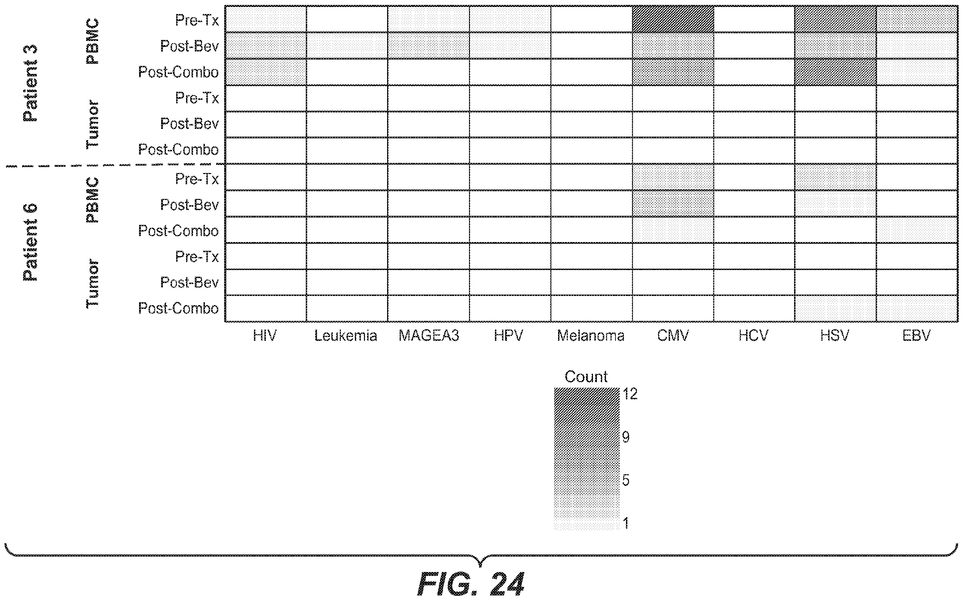

FIG. 24 is a heat map showing the number of viral antigen-specific clones in the PBMC pool versus the TIL pool at each treatment time point, for patients 3 and 6.

### DETAILED DESCRIPTION OF THE INVENTION

### I. Introduction

The present invention provides methods for diagnosing, monitoring, and treating kidney cancer (e.g., renal cell carcinoma (RCC), e.g., metastatic RCC). The invention is based, at least in part, on the discovery that treatment with an anti-cancer therapy that includes a VEGF antagonist (e.g., an anti-VEGF antibody, e.g., bevacizumab) and a PD-L1 axis binding antagonist (e.g., an anti-PD-L1 antibody, e.g., atezolizumab) results in changes in the expression levels of biomarkers (e.g., immunological biomarkers, e.g., genes associated with CD8⁺ T_{eff} cells (e.g., CD8A, CD8B, EOMES, GZMA, GZMB, IFNG, and PRF1), Th1 chemokines (e.g., CXCL9, CXLC10, CXCL11, and CXCL13), NK cells (e.g., GZMB, KLRK1, and SLAMF7), antigen presentation (e.g., MHC-I), and immune trafficking molecules (e.g., CCL2, CCL5, CCR5, CXCL1, and CCR7), and/or tumor infiltration of immune cells (e.g., CD8⁺ T_{eff} cells and/or CD68⁺/CD163⁺ macrophages)). The invention provides methods for monitoring the response of a patient having kidney cancer to treatment with a combination anti-cancer therapy including a VEGF antagonist (e.g., an anti-VEGF antibody, e.g., bevacizumab) and a PD-L1 axis binding antagonist (e.g., an anti-PD-L1 antibody, e.g., atezolizumab) by detecting and comparing expression levels of biomarkers of the invention. The disclosure also provides methods for treating a patient having cancer (e.g., kidney cancer) by administering VEGF antagonists (e.g., an anti-VEGF antibody, e.g., bevacizumab) and PD-L1 axis binding antagonists (e.g., an anti-PD-L1 antibody, e.g., atezolizumab).

### II. Definitions

It is to be understood that aspects and embodiments of the invention described herein include "comprising," "consisting," and "consisting essentially of" aspects and embodiments. As used herein, the singular form "a," "an," and "the" includes plural references unless indicated otherwise.

The term "about" as used herein refers to the usual error range for the respective value readily known to the skilled person in this technical field. Reference to "about" a value or parameter herein includes (and describes) embodiments that are directed to that value or parameter *per se.*

As used herein, the terms "individual," "patient," or "subject" are used interchangeably and refer to any single animal, more preferably a mammal (including such non-human animals as, for example, dogs, cats, horses, rabbits, zoo animals, cows, pigs, sheep, and non-human primates) for which treatment is desired. In particular embodiments, the patient herein is a human. The patient may be a "cancer patient," i.e., one who is suffering from cancer, or at risk for suffering from cancer, or suffering from one or more symptoms of cancer.

The terms "cancer" and "cancerous" refer to or describe the physiological condition in mammals that is typically characterized by unregulated cell growth. Included in this definition are benign and malignant cancers as well as dormant tumors or micrometastases. Examples of cancer include, but are not limited to, carcinoma, lymphoma, blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, melanoma, and leukemia or lymphoid malignancies. More particular examples of such cancers include kidney cancer (e.g., renal cell carcinoma (RCC), e.g., metastatic RCC), squamous cell cancer (e.g., epithelial squamous cell cancer), lung cancer (including small-cell lung cancer (SCLC), non-small cell lung cancer (NSCLC), adenocarcinoma of the lung, and squamous carcinoma of the lung), cancer of the peritoneum, hepatocellular cancer, gastric or stomach cancer including gastrointestinal cancer, pancreatic cancer, glioblastoma, cervical cancer, ovarian cancer, liver cancer, hepatoma, breast cancer (including metastatic breast cancer), bladder cancer, colon cancer, rectal cancer, colorectal cancer, endometrial or uterine carcinoma, salivary gland carcinoma, prostate cancer, vulval cancer, thyroid cancer, hepatic carcinoma, anal carcinoma, penile carcinoma, Merkel cell cancer, mycoses fungoids, testicular cancer, esophageal cancer, tumors of the biliary tract, head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome. In some embodiments, the cancer is kidney cancer. In particular embodiments, the kidney cancer is RCC (e.g., mRCC).

By "early stage cancer" or "early stage tumor" is meant a cancer that is not invasive or metastatic or is classified as a Stage 0, I, or II cancer.

An "advanced" cancer is one which has spread outside the site or organ of origin, either by local invasion or metastasis.

A "refractory" cancer is one which progresses even though an anti-tumor agent, such as a chemotherapeutic agent, is being administered to the cancer patient. An example of a refractory cancer is one which is platinum refractory.

A "recurrent" cancer is one which has regrown, either at the initial site or at a distant site, after a response to initial therapy.

The terms "cell proliferative disorder" and "proliferative disorder" refer to disorders that are associated with some degree of abnormal cell proliferation. In one embodiment, the cell proliferative disorder is cancer.

The term "tumor," as used herein, refers to all neoplastic cell growth and proliferation, whether malignant or benign, and all pre-cancerous and cancerous cells and tissues.

The terms "cancer," "cancerous," "cell proliferative disorder," "proliferative disorder," and "tumor" are not mutually exclusive as referred to herein.

A "disorder" is any condition that would benefit from treatment including, but not limited to, chronic and acute disorders or diseases including those pathological conditions which predispose the mammal to the disorder in question.

The term "detection" includes any means of detecting, including direct and indirect detection.

The term "sample," as used herein, refers to a composition that is obtained or derived from a patient and/or individual of interest that contains a cellular and/or other molecular entity that is to be characterized and/or identified, for example, based on physical, biochemical, chemical, and/or physiological characteristics. For example, the phrase "disease sample" and variations thereof refers to any sample obtained from a patient of interest that would be expected or is known to contain the cellular and/or molecular entity that is to be characterized. Samples include, but are not limited to, tissue samples, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, and combinations thereof.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, it will be clear from the context.

The terms "biomarker" and "marker" are used interchangeably herein to refer to a DNA, RNA, protein, carbohydrate, glycolipid, or cell-based molecular marker, the expression or presence of which in a patient's sample can be detected by standard methods (or methods disclosed herein). Such biomarkers include, but are not limited to, the genes and proteins set forth in Table 2. In some embodiments, a marker may be a cell (e.g., a CD8⁺ T cell or a CD68⁺/CD163⁺ macrophage). Expression of such a biomarker may be determined to be higher or lower in a sample obtained from a patient sensitive or responsive to a VEGF antagonist and/or a PD-L1 axis binding antagonist than a reference level (including, e.g., the median expression level of the biomarker in a sample from a group/population of patients, e.g., patients having cancer, and being tested for responsiveness to a VEGF antagonist and/or a PD-L1 axis binding antagonist; the median expression level of the biomarker in a sample from a group/population of patients, e.g., patients having cancer, and identified as not responding to a VEGF antagonist and/or a PD-L1 axis binding antagonist; the level in a sample previously obtained from the individual at a prior time; or the level in a sample from a patient who received prior treatment with a VEGF antagonist and/or a PD-L1 axis binding antagonist in a primary tumor setting, and who now may be experiencing metastasis).

The term "CD8A" as used herein, refers to any native CD8A from any vertebrate source, including mammals such as primates (e.g., humans) and rodents (e.g., mice and rats), unless otherwise indicated. The term encompasses "full-length," unprocessed CD8A as well as any form of CD8A that results from processing in the cell. The term also encompasses naturally occurring variants of CD8A e.g., splice variants or allelic variants. The nucleic acid sequence of an exemplary human CD8A is set forth in SEQ ID NO: 34. The amino acid sequence of an exemplary protein encoded by human CD8A is shown in SEQ ID NO: 35.

The term "CD8B" as used herein, refers to any native CD8B from any vertebrate source, including mammals such as primates (e.g., humans) and rodents (e.g., mice and rats), unless otherwise indicated. The term encompasses "full-length," unprocessed CD8B as well as any form of CD8B that results from processing in the cell. The term also encompasses naturally occurring variants of CD8B, e.g., splice variants or allelic variants. The nucleic acid sequence of an exemplary human CD8B is set forth in SEQ ID NO: 36. The amino acid sequence of an exemplary protein encoded by human CD8B is shown in SEQ ID NO: 37.

The term "EOMES" as used herein, refers to any native EOMES (Eomesodermin) from any vertebrate source, including mammals such as primates (e.g., humans) and rodents (e.g., mice and rats), unless otherwise indicated. The term encompasses "full-length," unprocessed EOMES as well as any form of EOMES that results from processing in the cell. The term also encompasses naturally occurring variants of EOMES e.g., splice variants or allelic variants. The nucleic acid sequence of an exemplary human EOMES is set forth in SEQ ID NO: 38. The amino acid sequence of an exemplary protein encoded by human EOMES is shown in SEQ ID NO: 39.

The term "GZMA" as used herein, refers to any native GZMA (Granzyme A) from any vertebrate source, including mammals such as primates (e.g., humans) and rodents (e.g., mice and rats), unless otherwise indicated. The term encompasses "full-length," unprocessed GZMA as well as any form of GZMA that results from processing in the cell. The term also encompasses naturally occurring variants of GZMA, e.g., splice variants or allelic variants. The nucleic acid sequence of an exemplary human GZMA is set forth in SEQ ID NO: 40. The amino acid sequence of an exemplary protein encoded by human GZMA is shown in SEQ ID NO: 41.

The term "GZMB" as used herein, refers to any native GZMB (Granzyme B) from any vertebrate source, including mammals such as primates (e.g., humans) and rodents (e.g., mice and rats), unless otherwise indicated. The term encompasses "full-length," unprocessed GZMB as well as any form of GZMB that results from processing in the cell. The term also encompasses naturally occurring variants of GZMB, e.g., splice variants or allelic variants. The nucleic acid sequence of an exemplary human GZMB is set forth in SEQ ID NO: 42. The amino acid sequence of an exemplary protein encoded by human GZMB is shown in SEQ ID NO: 43.

The term "IFNG" as used herein, refers to any native IFNG (Interferon, Gamma) from any vertebrate source, including mammals such as primates (e.g., humans) and rodents (e.g., mice and rats), unless otherwise indicated. The term encompasses "full-length," unprocessed IFNG as well as any form of IFNG that results from processing in the cell. The term also encompasses naturally occurring variants of IFNG, e.g., splice variants or allelic variants. The nucleic acid sequence of an exemplary human IFNG is set forth in SEQ ID NO: 44. The amino acid sequence of an exemplary protein encoded by human IFNG is shown in SEQ ID NO: 45.

The term "PRF1" as used herein, refers to any native PRF1 (Perforin 1; also known as Pore Forming Protein) from any vertebrate source, including mammals such as primates (e.g., humans) and rodents (e.g., mice and rats), unless otherwise indicated. The term encompasses "full-length," unprocessed PRF1 as well as any form of PRF1 that results from processing in the cell. The term also encompasses naturally occurring variants of PRF1, e.g., splice variants or allelic variants. The nucleic acid sequence of an exemplary human PRF1 is set forth in SEQ ID NO: 46. The amino acid sequence of an exemplary protein encoded by human PRF1 is shown in SEQ ID NO: 47.

The term "CXCL9" as used herein, refers to any native CXCL9 (Chemokine (C-X-C Motif) Ligand 9) from any vertebrate source, including mammals such as primates (e.g., humans) and rodents (e.g., mice and rats), unless otherwise indicated. The term encompasses "full-length," unprocessed CXCL9 as well as any form of CXCL9 that results from processing in the cell. The term also encompasses naturally occurring variants of CXCL9, e.g., splice variants or allelic variants. The nucleic acid sequence of an exemplary human CXCL9 is set forth in SEQ ID NO: 48. The amino acid sequence of an exemplary protein encoded by human CXCL9 is shown in SEQ ID NO: 49.

The term "CXCL10" as used herein, refers to any native CXCL10 (Chemokine (C-X-C Motif) Ligand 10) from any vertebrate source, including mammals such as primates (e.g., humans) and rodents (e.g., mice and rats), unless otherwise indicated. The term encompasses "full-length," unprocessed CXCL10 as well as any form of CXCL10 that results from processing in the cell. The term also encompasses naturally occurring variants of CXCL10, e.g., splice variants or allelic variants. The nucleic acid sequence of an exemplary human CXCL10 is set forth in SEQ ID NO: 50. The amino acid sequence of an exemplary protein encoded by human CXCL10 is shown in SEQ ID NO: 51.

The term "CXCL11" as used herein, refers to any native CXCL11 (Chemokine (C-X-C Motif) Ligand 11) from any vertebrate source, including mammals such as primates (e.g., humans) and rodents (e.g., mice and rats), unless otherwise indicated. The term encompasses "full-length," unprocessed CXCL11 as well as any form of CXCL11 that results from processing in the cell. The term also encompasses naturally occurring variants of CXCL11, e.g., splice variants or allelic variants. The nucleic acid sequence of an exemplary human CXCL11 is set forth in SEQ ID NO: 52. The amino acid sequence of an exemplary protein encoded by human CXCL11 is shown in SEQ ID NO: 53.

The term "CXCL13" as used herein, refers to any native CXCL13 (Chemokine (C-X-C Motif) Ligand 11) from any vertebrate source, including mammals such as primates (e.g., humans) and rodents (e.g., mice and rats), unless otherwise indicated. The term encompasses "full-length," unprocessed CXCL13 as well as any form of CXCL13 that results from processing in the cell. The term also encompasses naturally occurring variants of CXCL13, e.g., splice variants or allelic variants. The nucleic acid sequence of an exemplary human CXCL13 is set forth in SEQ ID NO: 54. The amino acid sequence of an exemplary protein encoded by human CXCL13 is shown in SEQ ID NO: 55.

The term "KLRK1" as used herein, refers to any native KLRK1 (Killer Cell Lectin-Like Receptor Subfamily K (Kappa), Member 1) from any vertebrate source, including mammals such as primates (e.g., humans) and rodents (e.g., mice and rats), unless otherwise indicated. The term encompasses "full-length," unprocessed KLRK1 as well as any form of KLRK1 that results from processing in the cell. The term also encompasses naturally occurring variants of KLRK1, e.g., splice variants or allelic variants. The nucleic acid sequence of an exemplary human KLRK1 is set forth in SEQ ID NO: 56. The amino acid sequence of an exemplary protein encoded by human KLRK1 is shown in SEQ ID NO: 57.

The term "SLAMF7" as used herein, refers to any native SLAMF7 (SLAM Family Member 7) from any vertebrate source, including mammals such as primates (e.g., humans) and rodents (e.g., mice and rats), unless otherwise indicated. The term encompasses "full-length," unprocessed SLAMF7 as well as any form of SLAMF7 that results from processing in the cell. The term also encompasses naturally occurring variants of SLAMF7, e.g., splice variants or allelic variants. The nucleic acid sequence of an exemplary human SLAMF7 is set forth in SEQ ID NO: 58. The amino acid sequence of an exemplary protein encoded by human SLAMF7 is shown in SEQ ID NO: 59.

The term "CX3CR1" as used herein, refers to any native CX3CR1 (CXC3 chemokine receptor 1, also known in the art as fractalkine receptor or G-protein coupled receptor 13 (GPR13)) from any vertebrate source, including mammals such as primates (e.g., humans) and rodents (e.g., mice and rats), unless otherwise indicated. The term encompasses "full-length," unprocessed CX3CR1 as well as any form of CX3CR1 that results from processing in the cell. The term also encompasses naturally occurring variants of CX3CR1, e.g., splice variants or allelic variants. The nucleic acid sequence of an exemplary human CX3CR1 is set forth in SEQ ID NO: 70. The amino acid sequence of an exemplary protein encoded by human CX3CR1 is shown in SEQ ID NO: 71.

The term "CCL2" as used herein, refers to any native CCL2 (Chemokine (C-C Motif) Ligand 2) from any vertebrate source, including mammals such as primates (e.g., humans) and rodents (e.g., mice and rats), unless otherwise indicated. The term encompasses "full-length," unprocessed CCL2 as well as any form of CCL2 that results from processing in the cell. The term also encompasses naturally occurring variants of CCL2, e.g., splice variants or allelic variants. The nucleic acid sequence of an exemplary human CCL2 is set forth in SEQ ID NO: 60. The amino acid sequence of an exemplary protein encoded by human CCL2 is shown in SEQ ID NO: 61.

The term "CCL5" as used herein, refers to any native CCL5 (Chemokine (C-C Motif) Ligand 5) from any vertebrate source, including mammals such as primates (e.g., humans) and rodents (e.g., mice and rats), unless otherwise indicated. The term encompasses "full-length," unprocessed CCL5 as well as any form of CCL5 that results from processing in the cell. The term also encompasses naturally occurring variants of CCL5, e.g., splice variants or allelic variants. The nucleic acid sequence of an exemplary human CCL5 is set forth in SEQ ID NO: 62. The amino acid sequence of an exemplary protein encoded by human CCL5 is shown in SEQ ID NO: 63.

The term "CCR5" as used herein, refers to any native CCR5 (Chemokine (C-C Motif) Receptor 5) from any vertebrate source, including mammals such as primates (e.g., humans) and rodents (e.g., mice and rats), unless otherwise indicated. The term encompasses "full-length," unprocessed CCR5 as well as any form of CCR5 that results from processing in the cell. The term also encompasses naturally occurring variants of CCR5, e.g., splice variants or allelic variants. The nucleic acid sequence of an exemplary human CCR5 is set forth in SEQ ID NO: 64. The amino acid sequence of an exemplary protein encoded by human CCR5 is shown in SEQ ID NO: 65.

The term "CX3CL1" as used herein, refers to any native CX3CL1 (Chemokine (C-X3-C Motif) Ligand 1; also known in the art as fractalkine) from any vertebrate source, including mammals such as primates (e.g., humans) and rodents (e.g., mice and rats), unless otherwise indicated. The term encompasses "full-length," unprocessed CX3CL1 as well as any form of CX3CL1 that results from processing in the cell. The term also encompasses naturally occurring variants of CX3CL1, e.g., splice variants or allelic variants. The nucleic acid sequence of an exemplary human CX3CL1 is set forth in SEQ ID NO: 66. The amino acid sequence of an exemplary protein encoded by human CX3CL1 is shown in SEQ ID NO: 67.

The term "CCR7" as used herein, refers to any native CCR7 (Chemokine (C-C Motif) Receptor 7) from any vertebrate source, including mammals such as primates (e.g., humans) and rodents (e.g., mice and rats), unless otherwise indicated. The term encompasses "full-length," unprocessed CCR7 as well as any form of CCR7 that results from processing in the cell. The term also encompasses naturally occurring variants of CCR7, e.g., splice variants or allelic variants. The nucleic acid sequence of an exemplary human CCR7 is set forth in SEQ ID NO: 68. The amino acid sequence of an exemplary protein encoded by human CCR7 is shown in SEQ ID NO: 69.

The term "MHC-I" as used herein, refers to any native MHC-I (Major Histocompatibility Complex-I) from any vertebrate source, including mammals such as primates (e.g., humans) and rodents (e.g., mice and rats), unless otherwise indicated. Human MHC-I is also referred to as human leukocyte antigen I (HLA-I). The expression level of MHC-I or HLA-I may be assessed by determining the expression level of any HLA-I gene or pseudogene (e.g., HLA-A, HLA-B, HLA-C, HLA-E, HLA-F, HLA-G, HLA-K, or HLA-L), or haplotype thereof. The expression level can be assessed by detection of all or a portion of the gene or pseudogene (e.g., MHC-I alpha chain or HLA-I histocompatibility antigen alpha chain).

The terms "level of expression" or "expression level" in general are used interchangeably and generally refer to the amount of a biomarker in a biological sample. "Expression" generally refers to the process by which information (e.g., gene-encoded and/or epigenetic information) is converted into the structures present and operating in the cell. Therefore, as used herein, "expression" may refer to transcription into a polynucleotide, translation into a polypeptide, or even polynucleotide and/or polypeptide modifications (e.g., posttranslational modification of a polypeptide). Fragments of the transcribed polynucleotide, the translated polypeptide, or polynucleotide and/or polypeptide modifications (e.g., posttranslational modification of a polypeptide) shall also be regarded as expressed whether they originate from a transcript generated by alternative splicing or a degraded transcript, or from a post-translational processing of the polypeptide, e.g., by proteolysis. "Expressed genes" include those that are transcribed into a polynucleotide as mRNA and then translated into a polypeptide, and also those that are transcribed into RNA but not translated into a polypeptide (for example, transfer and ribosomal RNAs).

"Increased expression," "increased expression level," "increased levels," "elevated expression," "elevated expression levels," or "elevated levels" refers to an increased expression or increased levels of a biomarker in an individual relative to a control, such as an individual or individuals who are not suffering from the disease or disorder (e.g., cancer), an internal control (e.g., a housekeeping biomarker), or the level of a biomarker in a sample obtained prior to administration of a therapy (e.g., an anti-cancer therapy that includes a VEGF antagonist and/or a PD-L1 antagonist).

"Decreased expression," "decreased expression level," "decreased levels," "reduced expression," "reduced expression levels," or "reduced levels" refers to a decrease expression or decreased levels of a biomarker in an individual relative to a control, such as an individual or individuals who are not suffering from the disease or disorder (e.g., cancer), an internal control (e.g., a housekeeping biomarker), or the level of a biomarker in a sample obtained prior to administration of a therapy (e.g., an anti-cancer therapy that includes a VEGF antagonist and/or a pD-L1 antagonist. In some embodiments, reduced expression is little or no expression).

A sample or cell that "expresses" a protein of interest is one in which mRNA encoding the protein, or the protein, including fragments thereof, is determined to be present in the sample or cell.

The "amount" or "level" of a biomarker associated with an increased clinical benefit to an individual is a detectable level in a biological sample. These can be measured by methods known to one skilled in the art and also disclosed herein. The expression level or amount of biomarker assessed can be used to determine the response to the treatment.

The phrase "based on" when used herein means that the information about one or more biomarkers is used to inform a treatment decision, information provided on a package insert, or marketing/promotional guidance, etc.

The term "housekeeping biomarker" refers to a biomarker or group of biomarkers (e.g., polynucleotides and/or polypeptides) which are typically similarly present in all cell types. In some embodiments, the housekeeping biomarker is a "housekeeping gene." A "housekeeping gene" refers herein to a gene or group of genes which encode proteins whose activities are essential for the maintenance of cell function and which are typically similarly present in all cell types.

"Amplification," as used herein generally refers to the process of producing multiple copies of a desired sequence. "Multiple copies" mean at least two copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

The term "multiplex-PCR" refers to a single PCR reaction carried out on nucleic acid obtained from a single source (e.g., an individual) using more than one primer set for the purpose of amplifying two or more DNA sequences in a single reaction.

The technique of "polymerase chain reaction" or "PCR" as used herein generally refers to a procedure wherein minute amounts of a specific piece of nucleic acid, RNA and/or DNA, are amplified as described, for example, in U.S. Pat. No. 4,683,195. Generally, sequence information from the ends of the region of interest or beyond needs to be available, such that oligonucleotide primers can be designed; these primers will be identical or similar in sequence to opposite strands of the template to be amplified. The 5' terminal nucleotides of the two primers may coincide with the ends of the amplified material. PCR can be used to amplify specific RNA sequences, specific DNA sequences from total genomic DNA, and cDNA transcribed from total cellular RNA, bacteriophage, or plasmid sequences, etc. See generally Mullis et al., Cold Spring Harbor Symp. Quant. Biol. 51:263 (1987) and Erlich, ed., PCR Technology, (Stockton Press, NY, 1989). As used herein, PCR is considered to be one, but not the only, example of a nucleic acid polymerase reaction method for amplifying a nucleic acid test sample, comprising the use of a known nucleic acid (DNA or RNA) as a primer and utilizes a nucleic acid polymerase to amplify or generate a specific piece of nucleic acid or to amplify or generate a specific piece of nucleic acid which is complementary to a particular nucleic acid.

"Quantitative real-time polymerase chain reaction" or "qRT-PCR" refers to a form of PCR wherein the amount of PCR product is measured at each step in a PCR reaction. This technique has been described in various publications including, for example, Cronin et al., Am. J. Pathol. 164(1):35-42 (2004) and Ma et al., Cancer Cell 5:607-616 (2004).

The term "microarray" refers to an ordered arrangement of hybridizable array elements, preferably polynucleotide probes, on a substrate.

The term "diagnosis" is used herein to refer to the identification or classification of a molecular or pathological state, disease or condition (e.g., cancer). For example, "diagnosis" may refer to identification of a particular type of cancer. "Diagnosis" may also refer to the classification of a particular subtype of cancer, for instance, by histopathological criteria, or by molecular features (e.g., a subtype characterized by expression of one or a combination of biomarkers (e.g., particular genes or proteins encoded by said genes)).

A "tumor-infiltrating immune cell," as used herein, refers to any immune cell present in a tumor or a sample thereof. Tumor-infiltrating immune cells include, but are not limited to, intratumoral immune cells, peritumoral immune cells, other tumor stroma cells (e.g., fibroblasts), or any combination thereof. Such tumor-infiltrating immune cells can be, for example, T lymphocytes (such as CD8⁺ T lymphocytes and/or CD4⁺ T lymphocytes), B lymphocytes, or other bone marrow-lineage cells, including granulocytes (e.g., neutrophils, eosinophils, and basophils), monocytes, macrophages (e.g., CD68⁺/CD163⁺ macrophages), dendritic cells (e.g., interdigitating dendritic cells), histiocytes, and natural killer (NK) cells.

A "tumor cell" as used herein, refers to any tumor cell present in a tumor or a sample thereof. Tumor cells may be distinguished from other cells that may be present in a tumor sample, for example, stromal cells and tumor-infiltrating immune cells, using methods known in the art and/or described herein.

A "reference sample," "reference cell," "reference tissue," "control sample," "control cell," or "control tissue," as used herein, refers to a sample, cell, tissue, standard, or level that is used for comparison purposes. In one embodiment, a reference sample, reference cell, reference tissue, control sample, control cell, or control tissue is obtained from a healthy and/or non-diseased part of the body (e.g., tissue or cells) of the same patient or individual. For example, the reference sample, reference cell, reference tissue, control sample, control cell, or control tissue may be healthy and/or non-diseased cells or tissue adjacent to the diseased cells or tissue (e.g., cells or tissue adjacent to a tumor). In another embodiment, a reference sample is obtained from an untreated tissue and/or cell of the body of the same patient or individual. In yet another embodiment, a reference sample, reference cell, reference tissue, control sample, control cell, or control tissue is obtained from a healthy and/or non-diseased part of the body (e.g., tissues or cells) of an individual who is not the patient or individual. In even another embodiment, a reference sample, reference cell, reference tissue, control sample, control cell, or control tissue is obtained from an untreated tissue and/or cell of the body of an individual who is not the patient or individual. In another embodiment, a reference sample, reference cell, reference tissue, control sample, control cell, or control tissue is obtained from a patient prior to administration of a therapy (e.g., an anti-cancer therapy that includes a VEGF antagonist and/or a PD-L1 axis binding antagonist).

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to the embodiment of polypeptide analysis or protocol, one may use the results of the polypeptide expression analysis or protocol to determine whether a specific therapeutic regimen should be performed. With respect to the embodiment of polynucleotide analysis or protocol, one may use the results of the polynucleotide expression analysis or protocol to determine whether a specific therapeutic regimen should be performed.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies (e.g., anti-VEGF antibodies, anti-PD-L1 antibodies, anti-PD-1 antibodies, or combinations thereof) are used to delay development of a disease or to slow the progression of a disease or disorder.

As used herein, "administering" is meant a method of giving a dosage of a compound (e.g., a VEGF antagonist (e.g., an anti-VEGF antibody) and/or a PD-L1 axis binding antagonist (e.g., an anti-PD-L1 antibody)) or a composition (e.g., a pharmaceutical composition, e.g., a pharmaceutical composition including a VEGF antagonist and/or a PD-L1 axis binding antagonist) to a patient. The compositions utilized in the methods described herein can be administered, for example, intramuscularly, intravenously, intradermally, percutaneously, intraarterially, intraperitoneally, intralesionally, intracranially, intraarticularly, intraprostatically, intrapleurally, intratracheally, intrathecally, intranasally, intravaginally, intrarectally, topically, intratumorally, peritoneally, subcutaneously, subconjunctivally, intravesicularly, mucosally, intrapericardially, intraumbilically, intraocularly, intraorbitally, intravitreally (e.g., by intravitreal injection), by eye drop, orally, topically, transdermally, by inhalation, by injection, by implantation, by infusion, by continuous infusion, by localized perfusion bathing target cells directly, by catheter, by lavage, in cremes, or in lipid compositions. The compositions utilized in the methods described herein can also be administered systemically or locally. The method of administration can vary depending on various factors (e.g., the compound or composition being administered and the severity of the condition, disease, or disorder being treated).

A "therapeutically effective amount" refers to an amount of a therapeutic agent to treat or prevent a disease or disorder in a mammal. In the case of cancers, the therapeutically effective amount of the therapeutic agent may reduce the number of cancer cells; reduce the primary tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the duration of survival, time to disease progression (TTP), response rates (e.g., complete response (CR) and partial response (PR)), duration of response, and/or quality of life.

The term "concurrently" is used herein to refer to administration of two or more therapeutic agents, where at least part of the administration overlaps in time. Accordingly, concurrent administration includes a dosing regimen when the administration of one or more agent(s) continues after discontinuing the administration of one or more other agent(s). For example, in some embodiments, a VEGF antagonist and a PD-L1 axis binding antagonist may be administered concurrently.

By "reduce or inhibit" is meant the ability to cause an overall decrease of 20%, 30%, 40%, 50%, 60%, 70%, 75%, 80%, 85%, 90%, 95%, or greater. Reduce or inhibit can refer, for example, to the symptoms of the disorder being treated, the presence or size of metastases, or the size of the primary tumor.

A "fixed" or "flat" dose of a therapeutic agent herein refers to a dose that is administered to a human patient without regard for the weight (WT) or body surface area (BSA) of the patient. The fixed or flat dose is therefore not provided as a mg/kg dose or a mg/m² dose, but rather as an absolute amount of the therapeutic agent.

A "loading" dose herein generally comprises an initial dose of a therapeutic agent administered to a patient, and is followed by one or more maintenance dose(s) thereof. Generally, a single loading dose is administered, but multiple loading doses are contemplated herein. Usually, the amount of loading dose(s) administered exceeds the amount of the maintenance dose(s) administered and/or the loading dose(s) are administered more frequently than the maintenance dose(s), so as to achieve the desired steady-state concentration of the therapeutic agent earlier than can be achieved with the maintenance dose(s).

A "maintenance" dose or "extended" dose herein refers to one or more doses of a therapeutic agent administered to the patient over a treatment period. Usually, the maintenance doses are administered at spaced treatment intervals, such as approximately every week, approximately every 2 weeks, approximately every 3 weeks, or approximately every 4 weeks.

The phrase "responsive to" in the context of the present invention indicates that a patient suffering, suspected to suffer or prone to suffer from cancer (e.g., a kidney cancer, e.g., RCC, e.g., metastatic RCC), shows a response to a therapy, e.g., an anti-cancer therapy comprising a VEGF antagonist (e.g., an anti-VEGF antibody, e.g., bevacizumab) and/or a PD-L1 axis binding antagonist (e.g., an anti-PD-L1 antibody, e.g., atezolizumab). A skilled person will readily be in a position to determine whether a person treated with a VEGF antagonist (e.g., an anti-VEGF antibody, e.g., bevacizumab) and/or a PD-L1 axis binding antagonist (e.g., an anti-PD-L1 antibody, e.g., atezolizumab) according to the methods of the invention shows a response. For example, a response may be reflected by decreased suffering from cancer, such as a diminished and/or halted tumor growth, reduction of the size of a tumor, and/or amelioration of one or more symptoms of cancer. Preferably, the response may be reflected by decreased or diminished indices of the metastatic conversion of the cancer or indices of the cancer, e.g., the prevention of the formation of metastases or a reduction of number or size of metastases.

The term "anti-cancer therapy" refers to a therapy useful in treating cancer. Examples of anti-cancer therapeutic agents include, but are limited to, cytotoxic agents, chemotherapeutic agents, growth inhibitory agents, agents used in radiation therapy, anti-angiogenesis agents, apoptotic agents, anti-tubulin agents, and other agents to treat cancer, for example, anti-CD20 antibodies, platelet derived growth factor inhibitors (e.g., GLEEVEC™ (imatinib mesylate)), a COX-2 inhibitor (e.g., celecoxib), interferons, cytokines, antagonists (e.g., neutralizing antibodies) that bind to one or more of the following targets: PDGFR-β, BIγS, APRIL, BCMA receptor(s), TRAIL/Apo2, other bioactive and organic chemical agents, and the like. Combinations thereof are also included in the invention.

An "anti-angiogenesis agent" or "angiogenesis inhibitor" refers to a small molecular weight substance, a polynucleotide, a polypeptide, an isolated protein, a recombinant protein, an antibody, or conjugates or fusion proteins thereof, that inhibits angiogenesis, vasculogenesis, or undesirable vascular permeability, either directly or indirectly. It should be understood that the anti-angiogenesis agent includes those agents that bind and block the angiogenic activity of the angiogenic factor or its receptor. For example, an anti-angiogenesis agent is an antibody or other antagonist to an angiogenic agent as defined above, e.g., antibodies to VEGF-A or the VEGF-A receptor (e.g., KDR receptor or Flt-1 receptor), anti-PDGFR inhibitors such as GLEEVEC™ (Imatinib Mesylate). Anti-angiogenesis agents also include native angiogenesis inhibitors, e.g., angiostatin, endostatin, etc. See, for example, Klagsbrun and D'Amore, Annu. Rev. Physiol., 53:217-39 (1991); Streit and Detmar, Oncogene, 22:3172-3179 (2003) (e.g., Table 3 listing anti-angiogenic therapy in malignant melanoma); Ferrara & Alitalo, Nature Medicine 5(12):1359-1364 (1999); Tonini et al., Oncogene, 22:6549-6556 (2003) and, Sato Int. J. Clin. Oncol., 8:200-206 (2003).

The term "cytotoxic agent" as used herein refers to a substance that inhibits or prevents the function of cells and/or causes destruction of cells. The term is intended to include radioactive isotopes (e.g., At²¹¹, I¹³¹, I¹²⁵, Y⁹⁰, Re¹⁸⁶, Re¹⁸⁸, Sm¹⁵³, Bi²¹², P³², and radioactive isotopes of Lu), chemotherapeutic agents, e.g., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. A tumoricidal agent causes destruction of tumor cells.

A "chemotherapeutic agent" includes chemical compounds useful in the treatment of cancer. Examples of chemotherapeutic agents include erlotinib (TARCEVA®, Genentech/OSI Pharm.), bortezomib (VELCADE®, Millennium Pharm.), disulfiram, epigallocatechin gallate, salinosporamide A, carfilzomib, 17-AAG (geldanamycin), radicicol, lactate dehydrogenase A (LDH-A), fulvestrant (FASLODEX®, AstraZeneca), sunitib (SUTENT®, Pfizer/Sugen), letrozole (FEMARA®, Novartis), imatinib mesylate (GLEEVEC®, Novartis), finasunate (VATALANIB®, Novartis), oxaliplatin (ELOXATIN®, Sanofi), 5-FU (5-fluorouracil), leucovorin, Rapamycin (Sirolimus, RAPAMUNE®, Pfizer), Lapatinib (TYKERB®, GSK572016, Glaxo Smith Kline), Lonafamib (SCH 66336), sorafenib (NEXAVAR®, Bayer Labs), gefitinib (IRESSA®, AstraZeneca), AG1478, alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including topotecan and irinotecan); bryostatin; callystatin; CC-1065 (including its adozelesin, carzelesin and bizelesin synthetic analogs); cryptophycins (particularly cryptophycin 1 and cryptophycin 8); adrenocorticosteroids (including prednisone and prednisolone); cyproterone acetate; 5α-reductases including finasteride and dutasteride; vorinostat, romidepsin, panobinostat, valproic acid, mocetinostat dolastatin; aldesleukin, talc duocarmycin (including the synthetic analogs, KW-2189 and CB1-TM1); eleutherobin; pancratistatin; a sarcodictyin; spongistatin; nitrogen mustards such as chlorambucil, chlomaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, especially calicheamicin γ1I and calicheamicin ω1I (Angew Chem. Intl. Ed. Engl. 33:183-186, 1994); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN® (doxorubicin), morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidamnol; nitraerine; pentostatin; phenamet; pirarubicin; losoxantrone; podophyllinic acid; 2-ethylhydrazide; procarbazine; PSK® polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL (paclitaxel; Bristol-Myers Squibb Oncology, Princeton, N.J.), ABRAXANE® (Cremophor-free), albumin-engineered nanoparticle formulations of paclitaxel (American Pharmaceutical Partners, Schaumberg, III.), and TAXOTERE® (docetaxel, doxetaxel; Sanofi-Aventis); chloranmbucil; GEMZAR® (gemcitabine); 6-thioguanine; mercaptopurine; methotrexate; platinum analogs such as cisplatin and carboplatin; vinblastine; etoposide (VP-16); ifosfamide; mitoxantrone; vincristine; NAVELBINE® (vinorelbine); novantrone; teniposide; edatrexate; daunomycin; aminopterin; capecitabine (XELODA®); ibandronate; CPT-11; topoisomerase inhibitor RFS 2000; difluoromethylornithine (DMFO); retinoids such as retinoic acid; and pharmaceutically acceptable salts, acids and derivatives of any of the above.

Chemotherapeutic agents also include anti-hormonal agents that act to regulate or inhibit hormone action on tumors such as anti-estrogens and selective estrogen receptor modulators (SERMs), including, for example, tamoxifen (including NOLVADEX®; tamoxifen citrate), raloxifene, droloxifene, iodoxyfene, 4-hydroxytamoxifen, trioxifene, keoxifene, LY117018, onapristone, and FARESTON® (toremifine citrate); aromatase inhibitors that inhibit the enzyme aromatase, which regulates estrogen production in the adrenal glands, such as, for example, 4(5)-imidazoles, aminoglutethimide, MEGASE® (megestrol acetate), AROMASIN® (exemestane; Pfizer), formestanie, fadrozole, RIVISOR® (vorozole), FEMARA® (letrozole; Novartis), and ARIMIDEX® (anastrozole; AstraZeneca); anti-androgens such as flutamide, nilutamide, bicalutamide, leuprolide and goserelin; buserelin, tripterelin, medroxyprogesterone acetate, diethylstilbestrol, premarin, fluoxymesterone, all transretionic acid, fenretinide, as well as troxacitabine (a 1,3-dioxolane nucleoside cytosine analog); protein kinase inhibitors; lipid kinase inhibitors; antisense oligonucleotides, particularly those which inhibit expression of genes in signaling pathways implicated in aberrant cell proliferation, such as, for example, PKC-alpha, Ralf and H-Ras; ribozymes such as VEGF expression inhibitors (e.g., ANGIOZYME®) and HER2 expression inhibitors; vaccines such as gene therapy vaccines, for example, ALLOVECTIN®, LEUVECTIN®, and VAXID®; PROLEUKIN®, rIL-2; a topoisomerase 1 inhibitor such as LURTOTECAN®; ABARELIX® rmRH; and pharmaceutically acceptable salts, acids and derivatives of any of the above.

Chemotherapeutic agents also include antibodies such as alemtuzumab (Campath), bevacizumab (AVASTIN®, Genentech); cetuximab (ERBITUX®, Imclone); panitumumab (VECTIBIX®, Amgen), rituximab (RITUXAN®, Genentech/Biogen Idec), pertuzumab (OMNITARG®, 2C4, Genentech), trastuzumab (HERCEPTIN®, Genentech), tositumomab (Bexxar, Corixia), and the antibody drug conjugate, gemtuzumab ozogamicin (MYLOTARG®, Wyeth). Additional humanized monoclonal antibodies with therapeutic potential as agents in combination with the compounds of the invention include: apolizumab, aselizumab, atlizumab, bapineuzumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, tucotuzumab celmoleukin, tucusituzumab, umavizumab, urtoxazumab, ustekinumab, visilizumab, and the anti-interleukin-12 (ABT-874/J695, Wyeth Research and Abbott Laboratories), which is a recombinant, exclusively human-sequence, full-length IgG1 λ antibody genetically modified to recognize interleukin-12 p40 protein.

Chemotherapeutic agents also include "EGFR inhibitors," which refers to compounds that bind to or otherwise interact directly with EGFR and prevent or reduce its signaling activity, and is alternatively referred to as an "EGFR antagonist." Examples of such agents include antibodies and small molecules that bind to EGFR. Examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533, Mendelsohn et al.) and variants thereof, such as chimerized 225 (C225 or Cetuximab; ERBUTIX®) and reshaped human 225 (H225) (see, WO 96/40210, Imclone Systems Inc.); IMC-11F8, a fully human, EGFR-targeted antibody (Imclone); antibodies that bind type II mutant EGFR (US Patent No. 5,212,290); humanized and chimeric antibodies that bind EGFR as described in US Patent No. 5,891,996; and human antibodies that bind EGFR, such as ABX-EGF or Panitumumab (see WO98/50433, Abgenix/Amgen); EMD 55900 (Stragliotto et al. Eur. J. Cancer 32A:636-640 (1996)); EMD7200 (matuzumab) a humanized EGFR antibody directed against EGFR that competes with both EGF and TGF-alpha for EGFR binding (EMD/Merck); human EGFR antibody, HuMax-EGFR (GenMab); fully human antibodies known as E1.1, E2.4, E2.5, E6.2, E6.4, E2.11, E6.3 and E7.6.3 and described in US 6,235,883; MDX-447 (Medarex Inc); and mAb 806 or humanized mAb 806 (Johns et al., J. Biol. Chem. 279(29):30375-30384 (2004)). The anti-EGFR antibody may be conjugated with a cytotoxic agent, thus generating an immunoconjugate (see, e.g., EP659,439A2, Merck Patent GmbH). EGFR antagonists include small molecules such as compounds described in US Patent Nos: 5,616,582, 5,457,105, 5,475,001, 5,654,307, 5,679,683, 6,084,095, 6,265,410, 6,455,534, 6,521,620, 6,596,726, 6,713,484, 5,770,599, 6,140,332, 5,866,572, 6,399,602, 6,344,459, 6,602,863, 6,391,874, 6,344,455, 5,760,041, 6,002,008, and 5,747,498, as well as the following PCT publications: WO98/14451, WO98/50038, WO99/09016, and WO99/24037. Particular small molecule EGFR antagonists include OSI-774 (CP-358774, erlotinib, TARCEVA® Genentech/OSI Pharmaceuticals); PD 183805 (CI 1033, 2-propenamide, N-[4-[(3-chloro-4-fluorophenyl)amino]-7-[3-(4-morpholinyl)propoxy]-6-quinazolinyl]-, dihydrochloride, Pfizer Inc.); ZD1839, gefitinib (IRESSA®) 4-(3'-Chloro-4'-fluoroanilino)-7-methoxy-6-(3-morpholinopropoxy)quinazoline, AstraZeneca); ZM 105180 ((6-amino-4-(3-methylphenyl-amino)-quinazoline, Zeneca); BIBX-1382 (N8-(3-chloro-4-fluoro-phenyl)-N2-(1-methyl-piperidin-4-yl)-pyrimido[5,4-d]pyrimidine-2,8-diamine, Boehringer Ingelheim); PKI-166 ((R)-4-[4-[(1-phenylethyl)amino]-1H-pyrrolo[2,3-d]pyrimidin-6-yl]-phenol); (R)-6-(4-hydroxyphenyl)-4-[(1-phenylethyl)amino]-7H-pyrrolo[2,3-d]pyrimidine); CL-387785 (N-[4-[(3-bromophenyl)amino]-6-quinazolinyl]-2-butynamide); EKB-569 (N-[4-[(3-chloro-4-fluorophenyl)amino]-3-cyano-7-ethoxy-6-quinolinyl]-4-(dimethylamino)-2-butenamide) (Wyeth); AG1478 (Pfizer); AG1571 (SU 5271; Pfizer); dual EGFR/HER2 tyrosine kinase inhibitors such as lapatinib (TYKERB®, GSK572016 or N-[3-chloro-4-[(3 fluorophenyl)methoxy]phenyl]-6[5[[[2methylsulfonyl)ethyl]amino]methyl]-2-furanyl]-4-quinazolinamine).

Chemotherapeutic agents also include "tyrosine kinase inhibitors" including the EGFR-targeted drugs noted in the preceding paragraph; small molecule HER2 tyrosine kinase inhibitor such as TAK165 available from Takeda; CP-724,714, an oral selective inhibitor of the ErbB2 receptor tyrosine kinase (Pfizer and OSI); dual-HER inhibitors such as EKB-569 (available from Wyeth) which preferentially binds EGFR but inhibits both HER2 and EGFR-overexpressing cells; lapatinib (GSK572016; available from Glaxo-SmithKline), an oral HER2 and EGFR tyrosine kinase inhibitor; PKI-166 (available from Novartis); pan-HER inhibitors such as canertinib (CI-1033; Pharmacia); Raf-1 inhibitors such as antisense agent ISIS-5132 available from ISIS Pharmaceuticals which inhibit Raf-1 signaling; non-HER targeted TK inhibitors such as imatinib mesylate (GLEEVEC®, available from Glaxo SmithKline); multi-targeted tyrosine kinase inhibitors such as sunitinib (SUTENT®, available from Pfizer); VEGF receptor tyrosine kinase inhibitors such as vatalanib (PTK787/ZK222584, available from Novartis/Schering AG); MAPK extracellular regulated kinase I inhibitor CI-1040 (available from Pharmacia); quinazolines, such as PD 153035,4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo[2,3-d] pyrimidines; curcumin (diferuloyl methane, 4,5-bis (4-fluoroanilino)phthalimide); tyrphostines containing nitrothiophene moieties; PD-0183805 (Warner-Lamber); antisense molecules (e.g. those that bind to HER-encoding nucleic acid); quinoxalines (US Patent No. 5,804,396); tryphostins (US Patent No. 5,804,396); ZD6474 (Astra Zeneca); PTK-787 (Novartis/Schering AG); pan-HER inhibitors such as CI-1033 (Pfizer); Affinitac (ISIS 3521; Isis/Lilly); imatinib mesylate (GLEEVEC®); PKI 166 (Novartis); GW2016 (Glaxo SmithKline); CI-1033 (Pfizer); EKB-569 (Wyeth); Semaxinib (Pfizer); ZD6474 (AstraZeneca); PTK-787 (Novartis/Schering AG); INC-1C11 (Imclone), rapamycin (sirolimus, RAPAMUNE®); or as described in any of the following patent publications: US Patent No. 5,804,396, WO 1999/09016, WO 1998/43960, WO 1997/38983, WO 1999/06378, WO 1999/06396, WO 1996/30347, WO 1996/33978, WO 1996/3397, and WO 1996/33980.

Chemotherapeutic agents also include dexamethasone, interferons, colchicine, metoprine, cyclosporine, amphotericin, metronidazole, alemtuzumab, alitretinoin, allopurinol, amifostine, arsenic trioxide, asparaginase, BCG live, bevacuzimab, bexarotene, cladribine, clofarabine, darbepoetin alfa, denileukin, dexrazoxane, epoetin alfa, elotinib, filgrastim, histrelin acetate, ibritumomab, interferon alfa-2a, interferon alfa-2b, lenalidomide, levamisole, mesna, methoxsalen, nandrolone, nelarabine, nofetumomab, oprelvekin, palifermin, pamidronate, pegademase, pegaspargase, pegfilgrastim, pemetrexed disodium, plicamycin, porfimer sodium, quinacrine, rasburicase, sargramostim, temozolomide, VM-26, 6-TG, toremifene, tretinoin, all-trans retinoic acid (ATRA), valrubicin, zoledronate, and zoledronic acid, and pharmaceutically acceptable salts thereof.

The term "prodrug" as used herein refers to a precursor or derivative form of a pharmaceutically active substance that is less cytotoxic to tumor cells compared to the parent drug and is capable of being enzymatically activated or converted into the more active parent form. See, for example, Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

A "growth inhibitory agent" when used herein refers to a compound or composition which inhibits growth and/or proliferation of a cell (e.g., a cell whose growth is dependent on PD-L1 expression) either *in vitro* or *in vivo.* Thus, the growth inhibitory agent may be one which significantly reduces the percentage of cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as the anthracycline antibiotic doxorubicin ((8S-cis)-10-[(3-amino-2,3,6-trideoxy-α-L-lyxo-hexapyranosyl)oxy]-7,8,9,10-tetrahydro-6,8,11-trihydroxy-8-(hydroxyacetyl)-1-methoxy-5,12-naphthacenedione), epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in *"*The Molecular Basis of Cancer," Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one-time administration and typical dosages range from 10 to 200 units (Grays) per day.

The term "pharmaceutical formulation" refers to a preparation which is in such form as to permit the biological activity of an active ingredient contained therein to be effective, and which contains no additional components which are unacceptably toxic to a patient to which the formulation would be administered.

A "pharmaceutically acceptable carrier" refers to an ingredient in a pharmaceutical formulation, other than an active ingredient, which is nontoxic to a patient. A pharmaceutically acceptable carrier includes, but is not limited to, a buffer, excipient, stabilizer, or preservative.

The term "package insert" is used to refer to instructions customarily included in commercial packages of therapeutic products, that contain information about the indications, usage, dosage, administration, combination therapy, contraindications, and/or warnings concerning the use of such therapeutic products.

A "sterile" formulation is aseptic or free from all living microorganisms and their spores.

An "article of manufacture" is any manufacture (e.g., a package or container) or kit comprising at least one reagent, e.g., a medicament for treatment of a disease or disorder (e.g., cancer), or a probe for specifically detecting a biomarker described herein. In certain embodiments, the manufacture or kit is promoted, distributed, or sold as a unit for performing the methods described herein.

The term "small molecule" refers to any molecule with a molecular weight of about 2000 daltons or less, preferably of about 500 daltons or less.

The word "label" when used herein refers to a compound or composition that is conjugated or fused directly or indirectly to a reagent such as a polynucleotide probe or an antibody and facilitates detection of the reagent to which it is conjugated or fused. The label may itself be detectable (e.g., radioisotope labels or fluorescent labels) or, in the case of an enzymatic label, may catalyze chemical alteration of a substrate compound or composition which is detectable. The term is intended to encompass direct labeling of a probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin.

The term "antibody" is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

An "isolated" antibody is one which has been identified and separated and/or recovered from a component of its natural environment. Contaminant components of its natural environment are materials which would interfere with research, diagnostic, and/or therapeutic uses for the antibody, and may include enzymes, hormones, and other proteinaceous or nonproteinaceous solutes. In some embodiments, an antibody is purified (1) to greater than 95% by weight of antibody as determined by, for example, the Lowry method, and in some embodiments, to greater than 99% by weight; (2) to a degree sufficient to obtain at least 15 residues of N-terminal or internal amino acid sequence by use of, for example, a spinning cup sequenator, or (3) to homogeneity by SDS-PAGE under reducing or nonreducing conditions using, for example, Coomassie blue or silver stain. An isolated antibody includes the antibody *in situ* within recombinant cells since at least one component of the antibody's natural environment will not be present. Ordinarily, however, an isolated antibody will be prepared by at least one purification step.

A "blocking" antibody or an antibody "antagonist" is one which inhibits or reduces biological activity of the antigen it binds. For example, a VEGF-specific antagonist antibody binds VEGF and inhibits the ability of VEGF to induce vascular endothelial cell proliferation. Preferred blocking antibodies or antagonist antibodies completely inhibit the biological activity of the antigen.

Unless indicated otherwise, the expression "multivalent antibody" is used throughout this specification to denote an antibody comprising three or more antigen binding sites. The multivalent antibody is preferably engineered to have the three or more antigen binding sites and is generally not a native sequence IgM or IgA antibody.

The "light chains" of antibodies (immunoglobulins) from any mammalian species can be assigned to one of two clearly distinct types, called kappa ("κ") and lambda ("λ"), based on the amino acid sequences of their constant domains.

The term "constant domain" refers to the portion of an immunoglobulin molecule having a more conserved amino acid sequence relative to the other portion of the immunoglobulin, the variable domain, which contains the antigen binding site. The constant domain contains the CH1, CH2, and CH3 domains (collectively, CH) of the heavy chain and the CHL (or CL) domain of the light chain.

The "variable region" or "variable domain" of an antibody refers to the amino-terminal domains of the heavy or light chain of the antibody. The variable domain of the heavy chain may be referred to as "VH." The variable domain of the light chain may be referred to as "VL." These domains are generally the most variable parts of an antibody and contain the antigen-binding sites.

The term "variable" refers to the fact that certain segments of the variable domains differ extensively in sequence among antibodies. The variable or "V" domain mediates antigen binding and defines specificity of a particular antibody for its particular antigen. However, the variability is not evenly distributed across the span of the variable domains. Instead, the V regions consist of relatively invariant stretches called framework regions (FRs) of 15-30 amino acids separated by shorter regions of extreme variability called "hypervariable regions" that are each 9-12 amino acids long. The term "hypervariable region" or "HVR" when used herein refers to the amino acid residues of an antibody which are responsible for antigen-binding. The hypervariable region generally comprises amino acid residues from, for example, around about residues 24-34 (L1), 50-56 (L2) and 89-97 (L3) in the VL, and around about residues 26-35 (H1), 49-65 (H2) and 95-102 (H3) in the VH (in one embodiment, H1 is around about residues 31-35); Kabat et al., Sequences of Proteins of Immunological Interest, 5th Ed. Public Health Service, National Institutes of Health, Bethesda, MD. (1991)) and/or those residues from a "hypervariable loop" (e.g., residues 26-32 (L1), 50-52 (L2), and 91-96 (L3) in the VL, and 26-32 (H1), 53-55 (H2), and 96-101 (H3) in the VH; Chothia and Lesk, J. Mol. Biol. 196:901-917 (1987). The variable domains of native heavy and light chains each comprise four FRs, largely adopting a beta-sheet configuration, connected by three hypervariable regions, which form loops connecting, and in some cases forming part of, the beta-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). Accordingly, the HVR and FR sequences generally appear in the following sequence in VH (or VL): FR1-H1(L1)-FR2-H2(L2)-FR3-H3(L3)-FR4. The constant domains are not involved directly in binding an antibody to an antigen, but exhibit various effector functions, such as participation of the antibody in antibody dependent cellular cytotoxicity (ADCC).

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

The term "hypervariable region," "HVR," or "HV," as used herein, refers to the regions of an antibody variable domain which are hypervariable in sequence and/or form structurally defined loops. Generally, antibodies comprise six HVRs; three in the VH (H1, H2, H3), and three in the VL (L1, L2, L3). In native antibodies, H3 and L3 display the most diversity of the six HVRs, and H3 in particular is believed to play a unique role in conferring fine specificity to antibodies. See, for example, Xu et al., Immunity 13:37-45 (2000); Johnson and Wu, in Methods in Molecular Biology 248:1-25 (Lo, ed., Human Press, Totowa, N.J., 2003). Indeed, naturally occurring camelid antibodies consisting of a heavy chain only are functional and stable in the absence of light chain. See, for example, Hamers-Casterman et al., Nature 363:446-448 (1993); Sheriff et al., Nature Struct. Biol. 3:733-736 (1996).

A number of HVR delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, Md. (1991)). Chothia refers instead to the location of the structural loops (Chothia and Lesk J. Mol. Biol. 196:901-917 (1987)). The AbM HVRs represent a compromise between the Kabat HVRs and Chothia structural loops, and are used by Oxford Molecular's AbM antibody modeling software. The "contact" HVRs are based on an analysis of the available complex crystal structures. The residues from each of these HVRs are noted below.

| *Loop* | *Kabat* | *AbM* | *Chothia* | *Contact* |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35b | H26-H35b | H26-H32 | H30-H35b (Kabat Numbering) |
| H1 | H31-H35 | H26-H35 | H26-H32 | H30-H35 (Chothia Numbering) |
| H2 | H50-H65 | H50-H58 | H53-H55 | H47-H58 |
| H3 | H95-H102 | H95-H102 | H96-H101 | H93-H101 |

HVRs may comprise "extended HVRs" as follows: 24-36 or 24-34 (L1), 46-56 or 50-56 (L2) and 89-97 or 89-96 (L3) in the VL and 26-35 (H1), 50-65 or 49-65 (H2) and 93-102, 94-102, or 95-102 (H3) in the VH. The variable domain residues are numbered according to Kabat et al., *supra,* for each of these definitions.

"Framework" or "FR" residues are those variable domain residues other than the HVR residues as herein defined.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., *supra.* In one embodiment, for the VH, the subgroup is subgroup III as in Kabat et al., *supra.*

The term "variable domain residue numbering as in Kabat" or "amino acid position numbering as in Kabat," and variations thereof, refers to the numbering system used for heavy chain variable domains or light chain variable domains of the compilation of antibodies in Kabat et al., *supra.* Using this numbering system, the actual linear amino acid sequence may contain fewer or additional amino acids corresponding to a shortening of, or insertion into, a FR or HVR of the variable domain. For example, a heavy chain variable domain may include a single amino acid insert (residue 52a according to Kabat) after residue 52 of H2 and inserted residues (e.g., residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

The Kabat numbering system is generally used when referring to a residue in the variable domain (approximately residues 1-107 of the light chain and residues 1-113 of the heavy chain) (e.g., Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (e.g., the EU index reported in Kabat et al., *supra*). The "EU index as in Kabat" refers to the residue numbering of the human IgG1 EU antibody. Unless stated otherwise herein, references to residue numbers in the variable domain of antibodies means residue numbering by the Kabat numbering system. Unless stated otherwise herein, references to residue numbers in the constant domain of antibodies means residue numbering by the EU numbering system (e.g., see United States Provisional Application No. 60/640,323, Figures for EU numbering).

Unless otherwise indicated, HVR residues and other residues in the variable domain (e.g., FR residues) are numbered herein according to Kabat et al., *supra.*

The terms "full-length antibody," "intact antibody," and "whole antibody" are used herein interchangeably to refer to an antibody in its substantially intact form, not antibody fragments as defined below. The terms particularly refer to an antibody with heavy chains that contain an Fc region.

"Antibody fragments" comprise a portion of an intact antibody, preferably comprising the antigen-binding region thereof. In some embodiments, the antibody fragment described herein is an antigen-binding fragment. Examples of antibody fragments include Fab, Fab', F(ab')₂, and Fv fragments; diabodies; linear antibodies; single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-combining sites and is still capable of cross-linking antigen.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD (1991).

"Effector functions" refer to those biological activities attributable to the Fc region of an antibody, which vary with the antibody isotype. Examples of antibody effector functions include: C1 q binding and complement dependent cytotoxicity (CDC); Fc receptor binding; antibody-dependent cell-mediated cytotoxicity (ADCC); phagocytosis; down-regulation of cell surface receptors (e.g. B cell receptor); and B cell activation.

"Fv" is the minimum antibody fragment which contains a complete antigen-binding site. In one embodiment, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three HVRs of each variable domain interact to define an antigen-binding site on the surface of the VH-VL dimer. Collectively, the six HVRs confer antigen-binding specificity to the antibody. However, even a single variable domain (or half of an Fv comprising only three HVRs specific for an antigen) has the ability to recognize and bind antigen, although at a lower affinity than the entire binding site.

The Fab fragment contains the heavy- and light-chain variable domains and also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv, see, e.g., Pluckthün, in The Pharmacology of Monoclonal Antibodies, vol. 113, Rosenburg and Moore eds., (Springer-Verlag, New York, 1994), pp. 269-315.

The term "multispecific antibody" is used in the broadest sense and specifically covers an antibody comprising a heavy chain variable domain (VH) and a light chain variable domain (VL), where the VH-VL unit has polyepitopic specificity (i.e., is capable of binding to two different epitopes on one biological molecule or each epitope on a different biological molecule). Such multispecific antibodies include, but are not limited to, full-length antibodies, antibodies having two or more VL and VH domains, antibody fragments such as Fab, Fv, dsFv, scFv, diabodies, bispecific diabodies and triabodies, antibody fragments that have been linked covalently or non-covalently. "Polyepitopic specificity" refers to the ability to specifically bind to two or more different epitopes on the same or different target(s). "Dual specificity" or "bispecificity" refers to the ability to specifically bind to two different epitopes on the same or different target(s). However, in contrast to bispecific antibodies, dual-specific antibodies have two antigen-binding arms that are identical in amino acid sequence and each Fab arm is capable of recognizing two antigens. Dual-specificity allows the antibodies to interact with high affinity with two different antigens as a single Fab or IgG molecule. According to one embodiment, the multispecific antibody in an IgG1 form binds to each epitope with an affinity of 5 µM to 0.001 pM, 3 µM to 0.001 pM, 1 µM to 0.001 pM, 0.5 µM to 0.001 pM or 0.1 µM to 0.001 pM. "Monospecific" refers to the ability to bind only one epitope.

The term "diabodies" refers to antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of antibodies are called α, δ, ε, γ, and µ, respectively.

The term "monoclonal antibody" as used herein refers to an antibody obtained from a population of substantially homogeneous antibodies, e.g., the individual antibodies comprising the population are identical except for possible mutations, e.g., naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity *in vivo,* to create a multispecific antibody, etc., and that an antibody comprising the altered target binding sequence is also a monoclonal antibody of this invention. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the invention may be made by a variety of techniques, including, for example, the hybridoma method (e.g., Kohler and Milstein, Nature 256:495-97 (1975); Hongo et al., Hybridoma 14 (3): 253-260 (1995), Harlow et al., Antibodies: A Laboratory Manual (Cold Spring Harbor Laboratory Press, 2nd ed. 1988); Hammerling et al., in: Monoclonal Antibodies and T cell Hybridomas 563-681 (Elsevier, N.Y., 1981)), recombinant DNA methods (see, e.g., U.S. Pat. No. 4,816,567), phage-display technologies (see, e.g., Clackson et al., Nature, 352: 624-628, 1991; Marks et al., J. Mol. Biol. 222: 581-597, 1992; Sidhu et al., J. Mol. Biol. 338(2): 299-310, 2004; Lee et al., J. Mol. Biol. 340(5): 1073-1093, 2004; Fellouse, Proc. Natl. Acad. Sci. USA 101(34): 12467-12472 ,2004; and Lee et al., J. Immunol. Methods 284(1-2): 119-132, 2004; and technologies for producing human or human-like antibodies in animals that have parts or all of the human immunoglobulin loci or genes encoding human immunoglobulin sequences (see, e.g., WO 1998/24893; WO 1996/34096; WO 1996/33735; WO 1991/10741; Jakobovits et al., Proc. Natl. Acad. Sci. USA 90: 2551, 1993; Jakobovits et al., Nature 362: 255-258, 1993; Bruggemann et al., Year in Immunol. 7:33 ,1993; U.S. Pat. Nos. 5,545,807; 5,545,806; 5,569,825; 5,625,126; 5,633,425; and 5,661,016; Marks et al., Bio/Technology 10: 779-783 (1992); Lonberg et al., Nature 368: 856-859, 1994; Morrison, Nature 368: 812-813, 1994; Fishwild et al., Nature Biotechnol. 14: 845-851, 1996; Neuberger, Nature Biotechnol. 14: 826, 1996; and Lonberg et al., Intern. Rev. Immunol. 13: 65-93, 1995.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see, e.g., U.S. Pat. No. 4,816,567; and Morrison et al., Proc. Natl. Acad. Sci. USA 81:6851-6855 (1984)). Chimeric antibodies include PRIMATIZED® antibodies wherein the antigen-binding region of the antibody is derived from an antibody produced by, e.g., immunizing macaque monkeys with the antigen of interest.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

"Humanized" forms of non-human (e.g., rodent) antibodies are chimeric antibodies that contain minimal sequence derived from the non-human antibody. For the most part, humanized antibodies are human immunoglobulins (recipient antibody) in which residues from a hypervariable region of the recipient are replaced by residues from a hypervariable region of a non-human species (donor antibody) such as mouse, rat, rabbit or non-human primate having the desired antibody specificity, affinity, and capability. In some instances, FR residues of the human immunoglobulin are replaced by corresponding non-human residues. Furthermore, humanized antibodies can comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525, 1986; Riechmann et al., Nature 332:323-329, 1988; and Presta, Curr. Op. Struct. Biol. 2:593-596, 1992.

A "variant" or "mutant" of a starting or reference polypeptide (e.g., a reference antibody or its variable domain(s)/HVR(s)), is a polypeptide that (1) has an amino acid sequence different from that of the starting or reference polypeptide and (2) was derived from the starting or reference polypeptide through either natural or artificial (man-made) mutagenesis. Such variants include, for example, deletions from, and/or insertions into and/or substitutions of, residues within the amino acid sequence of the polypeptide of interest, referred to herein as "amino acid residue alterations." Thus, a variant HVR refers to a HVR comprising a variant sequence with respect to a starting or reference polypeptide sequence (such as that of a source antibody or antigen binding fragment). An amino acid residue alteration, in this context, refers to an amino acid different from the amino acid at the corresponding position in a starting or reference polypeptide sequence (such as that of a reference antibody or fragment thereof). Any combination of deletion, insertion, and substitution may be made to arrive at the final variant or mutant construct, provided that the final construct possesses the desired functional characteristics. The amino acid changes also may alter post-translational processes of the polypeptide, such as changing the number or position of glycosylation sites.

A "wild-type (WT)" or "reference" sequence or the sequence of a "wild-type" or "reference" protein/polypeptide, such as an HVR or a variable domain of a reference antibody, may be the reference sequence from which variant polypeptides are derived through the introduction of mutations. In general, the "wild-type" sequence for a given protein is the sequence that is most common in nature. Similarly, a "wild-type" gene sequence is the sequence for that gene which is most commonly found in nature. Mutations may be introduced into a "wild-type" gene (and thus the protein it encodes) either through natural processes or through man-induced means. The products of such processes are "variant" or "mutant" forms of the original "wild-type" protein or gene.

A "reference antibody," as used herein, refers to an antibody or fragment thereof whose antigen-binding sequence serves as the template sequence upon which diversification according to the criteria described herein is performed. An antigen-binding sequence generally includes an antibody variable region, preferably at least one HVR, preferably including framework regions.

As used herein, "library" refers to a plurality of antibody or antibody fragment sequences (e.g., anti-VEGF antibodies or anti-PD-L1 antibodies of the invention), or the nucleic acids that encode these sequences, the sequences being different in the combination of variant amino acids that are introduced into these sequences according to the methods of the invention.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described herein.

With regard to the binding of an antibody to a target molecule, the term "specific binding" or "specifically binds to" or is "specific for" a particular polypeptide or an epitope on a particular polypeptide target means binding that is measurably different from a non-specific interaction. Specific binding can be measured, for example, by determining binding of a molecule compared to binding of a control molecule. For example, specific binding can be determined by competition with a control molecule that is similar to the target, for example, an excess of non-labeled target. In this case, specific binding is indicated if the binding of the labeled target to a probe is competitively inhibited by excess unlabeled target. The term "specific binding" or "specifically binds to" or is "specific for" a particular polypeptide or an epitope on a particular polypeptide target as used herein can be exhibited, for example, by a molecule having a Kd for the target of 10⁻⁴ M or lower, alternatively 10⁻⁵ M or lower, alternatively 10⁻⁶ M or lower, alternatively 10⁻⁷ M or lower, alternatively 10⁻⁸ M or lower, alternatively 10⁻⁹ M or lower, alternatively 10⁻¹⁰ M or lower, alternatively 10⁻¹¹ M or lower, alternatively 10⁻¹² M or lower or a Kd in the range of 10⁻⁴ M to 10⁻⁶ M or 10⁻⁶ M to 10⁻¹⁰ M or 10⁻⁷ M to 10⁻⁹ M. As will be appreciated by the skilled artisan, affinity and Kd values are inversely related. A high affinity for an antigen is measured by a low Kd value. In one embodiment, the term "specific binding" refers to binding where a molecule binds to a particular polypeptide or epitope on a particular polypeptide without substantially binding to any other polypeptide or polypeptide epitope.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more.

An "immunoconjugate" is an antibody conjugated to one or more heterologous molecule(s), including but not limited to a cytotoxic agent.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG1, IgG2 (including IgG2A and IgG2B), IgG3, or IgG4 subtypes, IgA (including IgA1 and IgA2), IgE, IgD or IgM. The Ig fusions preferably include the substitution of a domain of a polypeptide or antibody described herein in the place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130. For example, useful immunoadhesins as medicaments useful for therapy herein include polypeptides that comprise the extracellular domain (ECD) or PD-1 -binding portions of PD-L1 or PD-L2, or the extracellular or PD-L1 - or PD-L2-binding portions of PD-1, fused to a constant domain of an immunoglobulin sequence, such as a PD-L1 ECD-Fc, a PD-L2 ECD-Fc, and a PD-1 ECD-Fc, respectively. Immunoadhesin combinations of Ig Fc and ECD of cell surface receptors are sometimes termed soluble receptors.

A "fusion protein" and a "fusion polypeptide" refer to a polypeptide having two portions covalently linked together, where each of the portions is a polypeptide having a different property. The property may be a biological property, such as activity in vitro or in vivo. The property may also be simple chemical or physical property, such as binding to a target molecule, catalysis of a reaction, and the like. The two portions may be linked directly by a single peptide bond or through a peptide linker but are in reading frame with each other.

"Percent (%) amino acid sequence identity" with respect to the polypeptide sequences identified herein is defined as the percentage of amino acid residues in a candidate sequence that are identical with the amino acid residues in the polypeptide being compared, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full-length of the sequences being compared. For purposes herein, however, % amino acid sequence identity values are generated using the sequence comparison computer program ALIGN-2. The ALIGN-2 sequence comparison computer program was authored by Genentech, Inc. and the source code has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs.

A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps," substitution of one or more of the naturally-occurring nucleotides with an analog, internucleotide modifications such as, for example, those with uncharged linkages (e.g., methyl phosphonates, phosphotriesters, phosphoamidates, carbamates, and the like) and with charged linkages (e.g., phosphorothioates, phosphorodithioates, and the like), those containing pendant moieties, such as, for example, proteins (e.g., nucleases, toxins, antibodies, signal peptides, poly-L-lysine, and the like), those with intercalators (e.g., acridine, psoralen, and the like), those containing chelators (e.g., metals, radioactive metals, boron, oxidative metals, and the like), those containing alkylators, those with modified linkages (e.g., alpha anomeric nucleic acids), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro-, or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs, and abasic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), "(O)NR₂ ("amidate"), P(O)R, P(O)OR', CO or CH₂ ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, single stranded, polynucleotides that are, but not necessarily, less than about 250 nucleotides in length. Oligonucleotides may be synthetic. The terms "oligonucleotide" and "polynucleotide" are not mutually exclusive. The description above for polynucleotides is equally and fully applicable to oligonucleotides.

The term "primer" refers to a single-stranded polynucleotide that is capable of hybridizing to a nucleic acid and allowing polymerization of a complementary nucleic acid, generally by providing a free 3'-OH group.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

An "isolated" nucleic acid molecule is a nucleic acid molecule that is identified and separated from at least one contaminant nucleic acid molecule with which it is ordinarily associated in the natural source of the nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the antibody where, for example, the nucleic acid molecule is in a chromosomal location different from that of natural cells.

A "mutation" is a deletion, insertion, or substitution of a nucleotide(s) relative to a reference nucleotide sequence, such as a wild-type sequence.

As used herein, "codon set" refers to a set of different nucleotide triplet sequences used to encode desired variant amino acids. A set of oligonucleotides can be synthesized, for example, by solid phase synthesis, including sequences that represent all possible combinations of nucleotide triplets provided by the codon set and that will encode the desired group of amino acids. A standard form of codon designation is that of the IUB code, which is known in the art and described herein. A codon set typically is represented by 3 capital letters in italics, e.g., *NNK, NNS, XYZ, DVK* and the like. Synthesis of oligonucleotides with selected nucleotide "degeneracy" at certain positions is well known in that art, for example the TRIM approach (Knappek et al., J. Mol. Biol. 296:57-86, 1999); Garrard et al., Gene 128:103, 1993). Such sets of oligonucleotides having certain codon sets can be synthesized using commercial nucleic acid synthesizers (available from, for example, Applied Biosystems, Foster City, CA), or can be obtained commercially (for example, from Life Technologies, Rockville, MD). Therefore, a set of oligonucleotides synthesized having a particular codon set will typically include a plurality of oligonucleotides with different sequences, the differences established by the codon set within the overall sequence. Oligonucleotides, as used according to the invention, have sequences that allow for hybridization to a variable domain nucleic acid template and also can, but does not necessarily, include restriction enzyme sites useful for, for example, cloning purposes.

The expression "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "vascular endothelial growth factor" or "VEGF" refers to vascular endothelial growth factor protein A, as exemplified by Swiss Prot Accession Number P15692, Gene ID (NCBI): 7422. The term "VEGF" encompasses the protein having the amino acid sequence of Swiss Prot Accession Number P15692, Gene ID (NCBI): 7422 as well as homologues and isoforms thereof. The term "VEGF" also encompasses the known isoforms, e.g., splice isoforms, of VEGF, e.g., VEGF₁₁₁, VEGF₁₂₁, VEGF₁₄₅, VEGF₁₆₅, VEGF₁₈₉, and VEGF₂₀₆, together with the naturally-occurring allelic and processed forms thereof, including the 110 amino acid human vascular endothelial cell growth factor generated by plasmin cleavage of VEGF₁₆₅ as described in Ferrara Mol. Biol. Cell. 21:687, 2010; Leung et al., Science, 246:1306. 1989; and Houck et al., Mol. Endocrin., 5:1806, 1991. The term "VEGF" also refers to VEGFs from non-human species such as mouse, rat or primate. Sometimes the VEGF from a specific species are indicated by terms such as hVEGF for human VEGF, mVEGF for murine VEGF, and the like. The term "VEGF" is also used to refer to truncated forms of the polypeptide comprising amino acids 8 to 109 or 1 to 109 of the 165-amino acid human vascular endothelial cell growth factor. Reference to any such forms of VEGF may be identified in the present application, e.g., by "VEGF₁₀₉," "VEGF (8-109)," "VEGF (1-109)" or "VEGF₁₆₅." The amino acid positions for a "truncated" native VEGF are numbered as indicated in the native VEGF sequence. For example, amino acid position 17 (methionine) in truncated native VEGF is also position 17 (methionine) in native VEGF. The truncated native VEGF has binding affinity for the KDR and Flt-1 receptors comparable to native VEGF. The term "VEGF variant" as used herein refers to a VEGF polypeptide which includes one or more amino acid mutations in the native VEGF sequence. Optionally, the one or more amino acid mutations include amino acid substitution(s). For purposes of shorthand designation of VEGF variants described herein, it is noted that numbers refer to the amino acid residue position along the amino acid sequence of the putative native VEGF (provided in Leung et al., *supra* and Houck et al., *supra*). Unless specified otherwise, the term "VEGF" as used herein indicates VEGF-A.

A "VEGF antagonist" or "VEGF-specific antagonist" refers to a molecule capable of binding to VEGF, reducing VEGF expression levels, or neutralizing, blocking, inhibiting, abrogating, reducing, or interfering with VEGF biological activities, including, but not limited to, VEGF binding to one or more VEGF receptors, VEGF signaling, and VEGF mediated angiogenesis and endothelial cell survival or proliferation. For example, a molecule capable of neutralizing, blocking, inhibiting, abrogating, reducing, or interfering with VEGF biological activities can exert its effects by binding to one or more VEGF receptor (VEGFR) (e.g., VEGFR1, VEGFR2, VEGFR3, membrane-bound VEGF receptor (mbVEGFR), or soluble VEGF receptor (sVEGFR)). Included as VEGF-specific antagonists useful in the methods of the invention are polypeptides that specifically bind to VEGF, anti-VEGF antibodies and antigen-binding fragments thereof, receptor molecules and derivatives which bind specifically to VEGF thereby sequestering its binding to one or more receptors, fusions proteins (e.g., VEGF-Trap (Regeneron)), and VEGF₁₂₁-gelonin (Peregrine). VEGF-specific antagonists also include antagonist variants of VEGF polypeptides, antisense nucleobase oligomers complementary to at least a fragment of a nucleic acid molecule encoding a VEGF polypeptide; small RNAs complementary to at least a fragment of a nucleic acid molecule encoding a VEGF polypeptide; ribozymes that target VEGF; peptibodies to VEGF; and VEGF aptamers. VEGF antagonists also include polypeptides that bind to VEGFR, anti-VEGFR antibodies, and antigen-binding fragments thereof, and derivatives which bind to VEGFR thereby blocking, inhibiting, abrogating, reducing, or interfering with VEGF biological activities (e.g., VEGF signaling), or fusions proteins. VEGF-specific antagonists also include nonpeptide small molecules that bind to VEGF or VEGFR and are capable of blocking, inhibiting, abrogating, reducing, or interfering with VEGF biological activities. Thus, the term "VEGF activities" specifically includes VEGF mediated biological activities of VEGF. In certain embodiments, the VEGF antagonist reduces or inhibits, by at least 10%, 20%, 30%, 40%, 50%, 60%, 70%, 80%, 90% or more, the expression level or biological activity of VEGF. In some embodiments, the VEGF inhibited by the VEGF-specific antagonist is VEGF (8-109), VEGF (1-109), or VEGF₁₆₅.

As used herein VEGF antagonists can include, but are not limited to, anti-VEGFR2 antibodies and related molecules (e.g., ramucirumab, tanibirumab, aflibercept), anti-VEGFR1 antibodies and related molecules (e.g., icrucumab, aflibercept (VEGF Trap-Eye; EYLEA®), and ziv-aflibercept (VEGF Trap; ZALTRAP®)), bispecific VEGF antibodies (e.g., MP-0250, vanucizumab (VEGF-ANG2), and bispecific antibodies disclosed in US 2001/0236388), bispecific antibodies including combinations of two of anti-VEGF, anti-VEGFR1, and anti-VEGFR2 arms, anti-VEGFA antibodies (e.g., bevacizumab, sevacizumab), anti-VEGFB antibodies, anti-VEGFC antibodies (e.g., VGX-100), anti-VEGFD antibodies, and nonpeptide small molecule VEGF antagonists (e.g., pazopanib, axitinib, vandetanib, stivarga, cabozantinib, lenvatinib, nintedanib, orantinib, telatinib, dovitinig, cediranib, motesanib, sulfatinib, apatinib, foretinib, famitinib, and tivozanib).

An "anti-VEGF antibody" is an antibody that binds to VEGF with sufficient affinity and specificity. In certain embodiments, the antibody will have a sufficiently high binding affinity for VEGF, for example, the antibody may bind hVEGF with a Kd value of between 100 nM-1 pM. Antibody affinities may be determined, e.g., by a surface plasmon resonance based assay (such as the BIAcore® assay as described in PCT Application Publication No. WO2005/012359); enzyme-linked immunoabsorbent assay (ELISA); and competition assays (e.g. radioimmunoassays (RIAs)).

In certain embodiments, the anti-VEGF antibody can be used as a therapeutic agent in targeting and interfering with diseases or conditions wherein the VEGF activity is involved. Also, the antibody may be subjected to other biological activity assays, e.g., in order to evaluate its effectiveness as a therapeutic. Such assays are known in the art and depend on the target antigen and intended use for the antibody. Examples include the HUVEC inhibition assay; tumor cell growth inhibition assays (as described in WO 89/06692, for example); antibody-dependent cellular cytotoxicity (ADCC) and complement-mediated cytotoxicity (CDC) assays (U.S. Pat. No. 5,500,362); and agonistic activity or hematopoiesis assays (see WO 95/27062). An anti-VEGF antibody will usually not bind to other VEGF homologues such as VEGF-B or VEGF-C, nor other growth factors such as PIGF, PDGF, or bFGF. In one embodiment, anti-VEGF antibody is a monoclonal antibody that binds to the same epitope as the monoclonal anti-VEGF antibody A4.6.1 produced by hybridoma ATCC HB 10709. In another embodiment, the anti-VEGF antibody is a recombinant humanized anti-VEGF monoclonal antibody generated according to Presta et al. (Cancer Res. 57:4593-4599, 1997), including but not limited to the antibody known as bevacizumab (BV; AVASTIN®).

The anti-VEGF antibody "Bevacizumab (BV)," also known as "rhuMAb VEGF" or "AVASTIN®," is a recombinant humanized anti-VEGF monoclonal antibody generated according to Presta et al. (Cancer Res. 57:4593-4599, 1997). It comprises mutated human IgG1 framework regions and antigen-binding complementarity-determining regions from the murine anti-hVEGF monoclonal antibody A.4.6.1 that blocks binding of human VEGF to its receptors. Approximately 93% of the amino acid sequence of bevacizumab, including most of the framework regions, is derived from human IgG1, and about 7% of the sequence is derived from the murine antibody A4.6.1. Bevacizumab has a molecular mass of about 149,000 daltons and is glycosylated. Bevacizumab and other humanized anti-VEGF antibodies are further described in U.S. Pat. No. 6,884,879 issued Feb. 26, 2005. Additional preferred antibodies include the G6 or B20 series antibodies (e.g., G6-31, B20-4.1), as described in PCT Application Publication No. WO 2005/012359. For additional preferred antibodies see U.S. Pat. Nos. 7,060,269, 6,582,959, 6,703,020; 6,054,297; WO98/45332; WO 96/30046; WO94/10202; EP 0666868B1; U.S. Patent Application Publication Nos. 2006009360, 20050186208, 20030206899, 20030190317, 20030203409, and 20050112126; and Popkov et al., (Journal of Immunological Methods 288:149-164, 2004). Other preferred antibodies include those that bind to a functional epitope on human VEGF comprising of residues F17, M18, D19, Y21, Y25, Q89, 191, K101, E103, and C104 or, alternatively, comprising residues F17, Y21, Q22, Y25, D63, 183, and Q89.

Where a VEGF antagonist is administered as a "single anti-tumor agent" it is the only anti-tumor agent administered to treat the cancer, i.e., it is not administered in combination with another anti-tumor agent, such as chemotherapy.

A "nucleic acid encoding an anti-VEGF antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "dysfunction," in the context of immune dysfunction, refers to a state of reduced immune responsiveness to antigenic stimulation. The term includes the common elements of both "exhaustion" and/or "anergy" in which antigen recognition may occur, but the ensuing immune response is ineffective to control infection or tumor growth.

The term "dysfunctional," as used herein, also includes refractory or unresponsive to antigen recognition, specifically, impaired capacity to translate antigen recognition into down-stream T cell effector functions, such as proliferation, cytokine production (e.g., IL-2) and/or target cell killing.

The term "anergy" refers to the state of unresponsiveness to antigen stimulation resulting from incomplete or insufficient signals delivered through the T cell receptor (e.g., increase in intracellular Ca²⁺ in the absence of Ras activation). T cell anergy can also result upon stimulation with antigen in the absence of co-stimulation, resulting in the cell becoming refractory to subsequent activation by the antigen even in the context of co-stimulation. The unresponsive state can often be overridden by the presence of interleukin-2. Anergic T cells do not undergo clonal expansion and/or acquire effector functions.

The term "exhaustion" refers to T cell exhaustion as a state of T cell dysfunction that arises from sustained TCR signaling that occurs during many chronic infections and cancer. It is distinguished from anergy in that it arises not through incomplete or deficient signaling, but from sustained signaling. It is defined by poor effector function, sustained expression of inhibitory receptors and a transcriptional state distinct from that of functional effector or memory T cells. Exhaustion prevents optimal control of infection and tumors. Exhaustion can result from both extrinsic negative regulatory pathways (e.g., immunoregulatory cytokines) as well as cell-intrinsic negative regulatory (costimulatory) pathways (PD-1, B7-H3, B7-H4, etc.).

"Enhancing T cell function" means to induce, cause or stimulate a T cell to have a sustained or amplified biological function, or renew or reactivate exhausted or inactive T cells. Examples of enhancing T cell function include: increased secretion of γ-interferon from CD8⁺ T cells, increased proliferation, increased antigen responsiveness (e.g., viral, pathogen, or tumor clearance) relative to such levels before the intervention. In one embodiment, the level of enhancement is as least 50%, alternatively 60%, 70%, 80%, 90%, 100%, 120%, 150%, or 200% enhancement. The manner of measuring this enhancement is known to one of ordinary skill in the art.

"Tumor immunity" refers to the process in which tumors evade immune recognition and clearance. Thus, as a therapeutic concept, tumor immunity is "treated" when such evasion is attenuated, and the tumors are recognized and attacked by the immune system. Examples of tumor recognition include tumor binding, tumor shrinkage and tumor clearance.

"Immunogenicity" refers to the ability of a particular substance to provoke an immune response. Tumors are immunogenic and enhancing tumor immunogenicity aids in the clearance of the tumor cells by the immune response. Examples of enhancing tumor immunogenicity include treatment with a PD-L1 axis binding antagonist.

The terms "Programmed Death Ligand 1" and "PD-L1" refer herein to a native sequence PD-L1 polypeptide, polypeptide variants, and fragments of a native sequence polypeptide and polypeptide variants (which are further defined herein). The PD-L1 polypeptide described herein may be that which is isolated from a variety of sources, such as from human tissue types or from another source, or prepared by recombinant or synthetic methods.

A "native sequence PD-L1 polypeptide" comprises a polypeptide having the same amino acid sequence as the corresponding PD-L1 polypeptide derived from nature.

A "PD-L1 polypeptide variant," or variations thereof, means a PD-L1 polypeptide, generally an active PD-L1 polypeptide, as defined herein having at least about 80% amino acid sequence identity with any of the native sequence PD-L1 polypeptide sequences as disclosed herein. Such PD-L1 polypeptide variants include, for instance, PD-L1 polypeptides wherein one or more amino acid residues are added, or deleted, at the N- or C-terminus of a native amino acid sequence. Ordinarily, a PD-L1 polypeptide variant will have at least about 80% amino acid sequence identity, alternatively at least about 81%, 82%, 83%, 84%, 85%, 86%, 87%, 88%, 89%, 90%, 91%, 92%, 93%, 94%, 95%, 96%, 97%, 98%, or 99% amino acid sequence identity, to a native sequence PD-L1 polypeptide sequence as disclosed herein. Ordinarily, PD-L1 variant polypeptides are at least about 10 amino acids in length, alternatively at least about 20, 30, 40, 50, 60, 70, 80, 90, 100, 110, 120, 130, 140, 150, 160, 170, 180, 190, 200, 210, 220, 230, 240, 250, 260, 270, 280, 281, 282, 283, 284, 285, 286, 287, 288, or 289 amino acids in length, or more. Optionally, PD-L1 variant polypeptides will have no more than one conservative amino acid substitution as compared to a native PD-L1 polypeptide sequence, alternatively no more than 2, 3, 4, 5, 6, 7, 8, 9, or 10 conservative amino acid substitutions as compared to the native PD-L1 polypeptide sequence.

The term "PD-L1 axis binding antagonist" refers to a molecule that inhibits the interaction of a PD-L1 axis binding partner with one or more of its binding partners, so as to remove T cell dysfunction resulting from signaling on the PD-1 signaling axis, with a result being restored or enhanced T cell function. As used herein, a PD-L1 axis binding antagonist includes a PD-L1 binding antagonist and a PD-1 binding antagonist as well as molecules that interfere with the interaction between PD-L1 and PD-1 (e.g., a PD-L2-Fc fusion).

The terms "anti-PD-L1 antibody" and "an antibody that binds to PD-L1" refer to an antibody that is capable of binding PD-L1 with sufficient affinity such that the antibody is useful as a diagnostic and/or therapeutic agent in targeting PD-L1. In one embodiment, the extent of binding of an anti-PD-L1 antibody to an unrelated, non-PD-L1 protein is less than about 10% of the binding of the antibody to PD-L1 as measured, for example, by a RIA. In certain embodiments, an anti-PD-L1 antibody binds to an epitope of PD-L1 that is conserved among PD-L1 from different species.

The terms "anti-PD-1 antibody" and "an antibody that binds to PD-1" refer to an antibody that is capable of binding PD-1 with sufficient affinity such that the antibody is useful as a diagnostic and/or therapeutic agent in targeting PD-1. In one embodiment, the extent of binding of an anti-PD-1 antibody to an unrelated, non-PD-1 protein is less than about 10% of the binding of the antibody to PD-1 as measured, for example, by a RIA. In certain embodiments, an anti-PD-1 antibody binds to an epitope of PD-1 that is conserved among PD-1 from different species.

As used herein, a "PD-L1 binding antagonist" is a molecule that decreases, blocks, inhibits, abrogates or interferes with signal transduction resulting from the interaction of PD-L1 with either one or more of its binding partners, such as PD-1 and/or B7-1. In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some embodiments, PD-L1 binding antagonists include anti-PD-L1 antibodies and antigen-binding fragments thereof, immunoadhesins, fusion proteins, oligopeptides, small molecule antagonists, polynucleotide antagonists, and other molecules that decrease, block, inhibit, abrogate or interfere with signal transduction resulting from the interaction of PD-L1 with one or more of its binding partners, such as PD-1 and/or B7-1. In one embodiment, a PD-L1 binding antagonist reduces the negative signal mediated by or through cell surface proteins expressed on T lymphocytes, and other cells, mediated signaling through PD-L1 or PD-1 so as render a dysfunctional T cell less dysfunctional. In some embodiments, a PD-L1 binding antagonist is an anti-PD-L1 antibody. In a specific aspect, an anti-PD-L1 antibody is YW243.55.S70. In another specific aspect, an anti-PD-L1 antibody is MDX-1105. In still another specific aspect, an anti-PD-L1 antibody is MEDI4736 (druvalumab). In still another specific aspect, an anti-PD-L1 antibody is MSB0010718C (avelumab). In still another specific aspect, an anti-PD-L1 antibody is atezolizumab (MPDL3280A) described herein.

As used herein, a "PD-1 binding antagonist" is a molecule that decreases, blocks, inhibits, abrogates or interferes with signal transduction resulting from the interaction of PD-1 with one or more of its binding partners, such as PD-L1 and/or PD-L2. In some embodiments, the PD-1 binding antagonist is a molecule that inhibits the binding of PD-1 to its binding partners. In a specific aspect, the PD-1 binding antagonist inhibits the binding of PD-1 to PD-L1 and/or PD-L2. For example, PD-1 binding antagonists include anti-PD-1 antibodies and antigen-binding fragments thereof, immunoadhesins, fusion proteins, oligopeptides, small molecule antagonists, polynucleotide antagonists, and other molecules that decrease, block, inhibit, abrogate or interfere with signal transduction resulting from the interaction of PD-1 with PD-L1 and/or PD-L2. In one embodiment, a PD-1 binding antagonist reduces the negative signal mediated by or through cell surface proteins expressed on T lymphocytes, and other cells, mediated signaling through PD-1 or PD-L1 so as render a dysfunctional T cell less dysfunctional. In some embodiments, the PD-1 binding antagonist is an anti-PD-1 antibody. In a specific aspect, a PD-1 binding antagonist is MDX-1106 (nivolumab). In another specific aspect, a PD-1 binding antagonist is MK-3475 (pembrolizumab). In another specific aspect, a PD-1 binding antagonist is CT-011 (pidilizumab). In another specific aspect, a PD-1 binding antagonist is MEDI-0680 (AMP-514). In another specific aspect, a PD-1 binding antagonist is PDR001. In another specific aspect, a PD-1 binding antagonist is REGN2810. In another specific aspect, a PD-1 binding antagonist is BGB-108. In another specific aspect, a PD-1 binding antagonist is AMP-224.

Where a PD-L1 axis-binding antagonist is administered as a "single anti-tumor agent" it is the only anti-tumor agent administered to treat the cancer, i.e., it is not administered in combination with another anti-tumor agent, such as chemotherapy.

A "nucleic acid encoding a PD-L1 axis binding antagonist" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

"Individual response" or "response" can be assessed using any endpoint indicating a benefit to the individual, including, without limitation, inhibition, to some extent, of disease progression (e.g., cancer progression), including slowing down and complete arrest; a reduction in tumor size; inhibition (i.e., reduction, slowing down, or complete stopping) of cancer cell infiltration into adjacent peripheral organs and/or tissues; inhibition (i.e. reduction, slowing down, or complete stopping) of metastasis; relief, to some extent, of one or more symptoms associated with the disease or disorder (e.g., cancer); increase or extend in the length of survival, including overall survival and progression free survival; and/or decreased mortality at a given point of time following treatment.

An "effective response" of a patient or a patient's "responsiveness" to treatment with a medicament and similar wording refers to the clinical or therapeutic benefit imparted to a patient at risk for, or suffering from, a disease or disorder, such as cancer. In one embodiment, such benefit includes any one or more of: extending survival (including overall survival and progression-free survival); resulting in an objective response (including a complete response or a partial response); or improving signs or symptoms of cancer. In one embodiment, the biomarker (e.g., PD-L1 expression in tumor-infiltrating immune cells, for example, as determined using IHC) is used to identify the patient who is predicted to have an increased likelihood of being responsive to treatment with a medicament (e.g., treatment comprising a PD-L1 axis binding antagonist, e.g., an anti-PD-L1 antibody), relative to a patient who does not express the biomarker. In one embodiment, the biomarker (e.g., CD8A expression in tumor-infiltrating immune cells, for example, as determined using IHC) is used to identify the patient who is predicted to have an increase likelihood of being responsive to treatment with a medicament (e.g., an anti-VEGF antibody and/or an anti-PD-L1 antibody), relative to a patient who does not express the biomarker at the same level. In one embodiment, the presence of the biomarker is used to identify a patient who is more likely to respond to treatment with a medicament, relative to a patient that does not have the presence of the biomarker. In another embodiment, the presence of the biomarker is used to determine that a patient will have an increased likelihood of benefit from treatment with a medicament, relative to a patient that does not have the presence of the biomarker.

An "objective response" refers to a measurable response, including complete response (CR) or partial response (PR). In some embodiments, the "objective response rate (ORR)" refers to the sum of complete response (CR) rate and partial response (PR) rate.

By "complete response" or "CR" is intended the disappearance of all signs of cancer (e.g., disappearance of all target lesions) in response to treatment. This does not always mean the cancer has been cured.

As used herein, "partial response" or "PR" refers to a decrease in the size of one or more tumors or lesions, or in the extent of cancer in the body, in response to treatment. For example, in some embodiments, PR refers to at least a 30% decrease in the sum of the longest diameters (SLD) of target lesions, taking as reference the baseline SLD.

"Sustained response" refers to the sustained effect on reducing tumor growth after cessation of a treatment. For example, the tumor size may remain to be the same or smaller as compared to the size at the beginning of the administration phase. In some embodiments, the sustained response has a duration at least the same as the treatment duration, at least 1.5x, 2.0x, 2.5x, or 3.0x length of the treatment duration, or longer.

As used herein, "stable disease" or "SD" refers to neither sufficient shrinkage of target lesions to qualify for PR, nor sufficient increase to qualify for PD, taking as reference the smallest SLD since the treatment started.

As used herein, "progressive disease" or "PD" refers to at least a 20% increase in the SLD of target lesions, taking as reference the smallest SLD recorded since the treatment started or the presence of one or more new lesions.

The term "survival" refers to the patient remaining alive, and includes overall survival as well as progression-free survival
The phrase "progression-free survival" in the context of the present invention refers to the length of time during and after treatment during which, according to the assessment of the treating physician or investigator, a patient's disease does not become worse, i.e., does not progress. As the skilled person will appreciate, a patient's progression-free survival is improved or enhanced if the patient experiences a longer length of time during which the disease does not progress as compared to the average or mean progression free survival time of a control group of similarly situated patients.

As used herein, "overall survival" (OS) refers to the percentage of individuals in a group who are likely to be alive after a particular duration of time.

By "extending survival" is meant increasing overall or progression-free survival in a treated patient relative to an untreated patient (i.e. relative to a patient not treated with the medicament), or relative to a patient who does not express a biomarker at the designated level, and/or relative to a patient treated with an approved anti-tumor agent (e.g., an anti-VEGF antibody or a PD-L1 axis binding antagonist).

The term "benefit" is used in the broadest sense and refers to any desirable effect and specifically includes clinical benefit as defined herein. Clinical benefit can be measured by assessing various endpoints, e.g., inhibition, to some extent, of disease progression, including slowing down and complete arrest; reduction in the number of disease episodes and/or symptoms; reduction in lesion size; inhibition (i.e., reduction, slowing down, or complete stopping) of disease cell infiltration into adjacent peripheral organs and/or tissues; inhibition (i.e. reduction, slowing down, or complete stopping) of disease spread; decrease of auto-immune response, which may, but does not have to, result in the regression or ablation of the disease lesion; relief, to some extent, of one or more symptoms associated with the disorder; increase in the length of disease-free presentation following treatment, e.g., progression-free survival; increased overall survival; higher response rate; and/or decreased mortality at a given point of time following treatment.

### III. Methods

In one aspect, the invention is based, in part, on the discovery that changes in the expression level of one or more immunological biomarkers (e.g., CD8A, CD8B, EOMES, GZMA, GZMB, IFNG, PRF1, CXCL9, CXCL10, CXCL11, CXCL13, KLRK1, SLAMF7, MHC-I, CX3CR1, CCL2, CCL5, CCR5, CCR7, CX3CL1) and/or tumor infiltration of immune cells (e.g., CD8⁺ T_{eff} cells and/or CD68⁺/CD163⁺ macrophages) are associated with patient responses to anti-cancer therapy that includes a VEGF antagonist (e.g., an anti-VEGF antibody, e.g., bevacizumab) and/or a PD-L1 axis binding antagonist (e.g., a PD-L1 binding antagonist, e.g., atezolizumab). In certain embodiments, methods of monitoring a patient's response to treatment according to the expression of such biomarkers is provided. In other embodiments, methods of treating cancer (e.g., kidney cancer) according to the expression level or number of such biomarkers is provided. In some embodiments, methods of identifying a cancer patient who is likely to benefit from an anti-cancer therapy comprising a VEGF antagonist and a PD-L1 axis binding antagonist are provided. In some embodiments, methods of selecting an anti-cancer therapy comprising a VEGF antagonist and a PD-L1 axis binding antagonist for a cancer patient are provided. The present invention also provides methods for treating a patient having a cancer (e.g., a kidney cancer, such as RCC, e.g., mRCC). In some instances, the methods of the invention include administering to the patient an anti-cancer therapy that includes a VEGF antagonist and a PD-L1 axis binding antagonist based on the expression level of a biomarker of the invention.

### A. Methods of Monitoring Responses to Treatment, Diagnosis, Prognosis, and Patient Selection

The present invention relates to the identification, selection, and use of biomarkers (e.g., immunological biomarkers) of cancer, such as a kidney cancer (e.g., renal cell carcinoma (RCC), e.g., metastatic RCC (mRCC)), that correlate with response to VEGF antagonists (e.g., anti-VEGF antibodies, such as bevacizumab) and/or PD-L1 axis binding antagonists (e.g., PD-L1 binding antagonists, such as atezolizumab). In this respect, the invention relates to the use of expression profile(s) of one or more of biomarkers of the invention relative to reference level(s) for the one or more biomarkers to identify patients sensitive or responsive to the combination of VEGF antagonists (e.g., anti-VEGF antibodies, such as bevacizumab) and PD-L1 axis binding antagonists (e.g., PD-L1 binding antagonists, such as atezolizumab). In some instances, the administration of VEGF antagonists (e.g., anti-VEGF antibodies, such as bevacizumab) and PD-L1 axis binding antagonists (e.g., PD-L1 binding antagonists, such as atezolizumab) is based on a determination and/or comparison of tumor specific expression level(s) of one or more biomarkers relative to reference level(s).

The invention provides methods of monitoring the response of a patient having a cancer (e.g., kidney cancer) to treatment with an anti-cancer therapy comprising a VEGF antagonist (e.g., an anti-VEGF antibody, such as bevacizumab) and a PD-L1 axis binding antagonist (e.g., a PD-L1 binding antagonist, such as atezolizumab), involving determining the expression level of one or more immunological biomarkers in a sample obtained from the patient and comparing the expression level of the one or more biomarkers in the sample with a reference level, thereby monitoring the response of the patient to treatment with the anti-cancer therapy. In some embodiments, the expression level is increased relative to the reference level. A list of exemplary immunological biomarkers is set forth in Table 2 below. In some embodiments, the method involves selecting an anti-cancer therapy comprising a VEGF antagonist (e.g., an anti-VEGF antibody, such as bevacizumab) and a PD-L1 axis binding antagonist (e.g., a PD-L1 binding antagonist, such as atezolizumab) for the patient based on the expression level of a biomarker set forth in Table 2 below.

**Table 2. Immunological Biomarkers**

| **Biomarker** |
|---|
| CD8A |
| CD8B |
| EOMES |
| GZMA |
| GZMB |
| IFNG |
| PRF1 |
| CXCL9 |
| CXCL10 |
| CXCL11 |
| CXCL13 |
| KLRK1 |
| SLAMF7 |
| CX3CR1 |
| CCL2 |
| CCL5 |
| CCR5 |
| CX3CL1 |
| CCR7 |

In other embodiments, the invention provides a method of identifying a patient having a cancer (e.g., kidney cancer) who is likely to benefit from treatment with an anti-cancer therapy that includes a VEGF antagonist (e.g., an anti-VEGF antibody, such as bevacizumab) and a PD-L1 axis binding antagonist (e.g., a PD-L1 binding antagonist, such as atezolizumab), the method involving determining the expression level of one or more immunological biomarkers in a biological sample obtained from the patient and comparing the expression level of the one or more biomarkers in the sample with a reference level, thereby identifying the patient as likely to benefit from treatment with the anti-cancer therapy. In some embodiments, a change in the expression level (e.g., an increase or a decrease) of the one or more immunological biomarkers in the biological sample relative to the reference level identifies the patient as likely to benefit from treatment with the anti-cancer therapy. In some embodiments, the immunological biomarker is set forth in Table 2. In other embodiments, the immunological biomarker is MHC-I.

In another embodiment, the invention provides a method of diagnosing or prognosing a cancer (e.g., kidney cancer), the method involving determining the expression level of one or more immunological biomarkers in a biological sample obtained from the patient and comparing the expression level of the one or more biomarkers in the sample with a reference level, thereby diagnosing or prognosing the cancer. In some embodiments, a change in the expression level (e.g., an increase or a decrease) of the one or more immunological biomarkers in the biological sample relative to the reference level diagnoses or prognoses the patient. In some embodiments, the immunological biomarker is set forth in Table 2. In other embodiments, the immunological biomarker is MHC-I.

In yet another embodiment, the invention provides a method of determining whether a patient having a cancer (e.g., kidney cancer) is likely to respond to treatment with an anti-cancer therapy that includes a VEGF antagonist (e.g., an anti-VEGF antibody, such as bevacizumab) and a PD-L1 axis binding antagonist (e.g., a PD-L1 binding antagonist, such as atezolizumab), the method involving determining the expression level of one or more immunological biomarkers in a biological sample obtained from the patient and comparing the expression level of the one or more biomarkers in the sample with a reference level, thereby identifying the patient as one who is likely to respond to the anti-cancer therapy. In some embodiments, a change in the expression level (e.g., an increase or a decrease) of the one or more immunological biomarkers in the biological sample relative to the reference level identifies the patient as likely to respond to treatment with the anti-cancer therapy. In some embodiments, the immunological biomarker is set forth in Table 2. In other embodiments, the immunological biomarker is MHC-I.

In other embodiments, the invention provides a method of optimizing therapeutic efficacy of an anti-cancer therapy that includes a VEGF antagonist (e.g., an anti-VEGF antibody, such as bevacizumab) and a PD-L1 axis binding antagonist (e.g., a PD-L1 binding antagonist, such as atezolizumab), the method involving determining the expression level of one or more immunological biomarkers in a biological sample obtained from the patient and comparing the expression level of the one or more biomarkers in the sample with a reference level, wherein a change (e.g., an increase or decrease) in the expression level of the one or more immunological biomarkers in the biological sample relative to the reference level identifies a patient who is likely to respond to the anti-cancer therapy. In some embodiments, the immunological biomarker is set forth in Table 2. In other embodiments, the immunological biomarker is MHC-I.

In still further embodiments, the invention provides methods of selecting an anti-cancer therapy comprising a VEGF antagonist (e.g., an anti-VEGF antibody, such as bevacizumab) and a PD-L1 axis binding antagonist (e.g., a PD-L1 binding antagonist, such as atezolizumab) for a patient having a cancer (e.g., kidney cancer), the method involving determining the expression level of one or more immunological biomarkers in a biological sample obtained from the patient, comparing the expression level of the one or more biomarkers in the biological sample with a reference level, and selecting an anti-cancer therapy comprising a VEGF antagonist and a PD-L1 axis binding antagonist for the patient based on the expression level of the one or more immunological biomarkers. In some embodiments, a change in the expression level (e.g., an increase or a decrease) of the one or more immunological biomarkers in the biological sample relative to the reference level is used to select the anti-cancer therapy. In some embodiments, the immunological biomarker is set forth in Table 2. In other embodiments, the immunological biomarker is MHC-I.

In some embodiments, the invention provides a method of monitoring the response of a patient having a cancer (e.g., a kidney cancer) to treatment with an anti-cancer therapy comprising a VEGF antagonist (e.g., an anti-VEGF antibody, such as bevacizumab) and a PD-L1 axis binding antagonist (e.g., a PD-L1 binding antagonist, such as atezolizumab), involving determining, in a biological sample obtained from the patient at a time point following administration of the anti-cancer therapy, the expression level of one or more (e.g., 1, 2, 3, 4, 5, 6, 7, 8, 9, 10, 11, 12, or 13) of CD8A, CD8B, EOMES, GZMA, GZMB, IFNG, PRF1, CXCL9, CXCL10, CXCL11, CXCL13, KLRK1, and/or SLAMF7, and comparing the expression level of the one or more (e.g., 1, 2, 3, 4, 5, 6, 7, 8, 9, 10, 11, 12, or 13) of CD8A, CD8B, EOMES, GZMA, GZMB, IFNG, PRF1, CXCL9, CXCL10, CXCL11, CXCL13, KLRK1, and/or SLAMF7 with a reference level, thereby monitoring the response in the patient to treatment with the anti-cancer therapy.

In other embodiments, the invention provides a method of identifying a patient having a cancer (e.g., kidney cancer) who is likely to benefit from treatment with an anti-cancer therapy that includes a VEGF antagonist (e.g., an anti-VEGF antibody, such as bevacizumab) and a PD-L1 axis binding antagonist (e.g., a PD-L1 binding antagonist, such as atezolizumab), the method involving determining the expression level of one or more (e.g., 1, 2, 3, 4, 5, 6, 7, 8, 9, 10, 11, 12, or 13) of CD8A, CD8B, EOMES, GZMA, GZMB, IFNG, PRF1, CXCL9, CXCL10, CXCL11, CXCL13, KLRK1, and/or SLAMF7 in a biological sample obtained from the patient, and comparing the expression level of the one or more of CD8A, CD8B, EOMES, GZMA, GZMB, IFNG, PRF1, CXCL9, CXCL10, CXCL11, CXCL13, KLRK1, and/or SLAMF7 in the biological sample with a reference level, thereby identifying the patient as likely to benefit from treatment with the anti-cancer therapy. In some embodiments, a change in the expression level (e.g., an increase or a decrease) of one or more of CD8A, CD8B, EOMES, GZMA, GZMB, IFNG, PRF1, CXCL9, CXCL10, CXCL11, CXCL13, KLRK1, and/or SLAMF7 in the biological sample relative to the reference level identifies the patient as likely to benefit from treatment with the anti-cancer therapy. In some embodiments, the change is an increase. In other embodiments, the change is a decrease.

In another embodiment, the invention provides a method of diagnosing or prognosing a cancer (e.g., kidney cancer), the method involving determining the expression level of one or more (e.g., 1, 2, 3, 4, 5, 6, 7, 8, 9, 10, 11, 12, or 13) of CD8A, CD8B, EOMES, GZMA, GZMB, IFNG, PRF1, CXCL9, CXCL10, CXCL11, CXCL13, KLRK1, and/or SLAMF7 in a biological sample obtained from the patient, and comparing the expression level of the one or more of CD8A, CD8B, EOMES, GZMA, GZMB, IFNG, PRF1, CXCL9, CXCL10, CXCL11, CXCL13, KLRK1, and/or SLAMF7 in the biological sample with a reference level, thereby diagnosing or prognosing the cancer. In some embodiments, a change in the expression level (e.g., an increase or a decrease) of one or more of CD8A, CD8B, EOMES, GZMA, GZMB, IFNG, PRF1, CXCL9, CXCL10, CXCL11, CXCL13, KLRK1, and/or SLAMF7 in the biological sample relative to the reference level diagnoses or prognoses the patient. In some embodiments, the change is an increase. In other embodiments, the change is a decrease.

In yet another embodiment, the invention provides a method of determining whether a patient having a cancer (e.g., kidney cancer) is likely to respond to treatment with an anti-cancer therapy that includes a VEGF antagonist (e.g., an anti-VEGF antibody, such as bevacizumab) and a PD-L1 axis binding antagonist (e.g., a PD-L1 binding antagonist, such as atezolizumab), the method involving determining the expression level of one or more (e.g., 1, 2, 3, 4, 5, 6, 7, 8, 9, 10, 11, 12, or 13) of CD8A, CD8B, EOMES, GZMA, GZMB, IFNG, PRF1, CXCL9, CXCL10, CXCL11, CXCL13, KLRK1, and/or SLAMF7 in a biological sample obtained from the patient, and comparing the expression level of the one or more of CD8A, CD8B, EOMES, GZMA, GZMB, IFNG, PRF1, CXCL9, CXCL10, CXCL11, CXCL13, KLRK1, and/or SLAMF7 in the biological sample with a reference level, thereby identifying the patient as one who is likely to respond to the anti-cancer therapy. In some embodiments, a change in the expression level (e.g., an increase or a decrease) of one or more of CD8A, CD8B, EOMES, GZMA, GZMB, IFNG, PRF1, CXCL9, CXCL10, CXCL11, CXCL13, KLRK1, and/or SLAMF7 in the biological sample relative to the reference level identifies the patient as likely to respond to treatment with the anti-cancer therapy. In some embodiments, the change is an increase. In other embodiments, the change is a decrease.

In other embodiments, the invention provides a method of optimizing therapeutic efficacy of an anti-cancer therapy that includes a VEGF antagonist (e.g., an anti-VEGF antibody, such as bevacizumab) and a PD-L1 axis binding antagonist (e.g., a PD-L1 binding antagonist, such as atezolizumab), the method involving determining the expression level of one or more (e.g., 1, 2, 3, 4, 5, 6, 7, 8, 9, 10, 11, 12, or 13) of CD8A, CD8B, EOMES, GZMA, GZMB, IFNG, PRF1, CXCL9, CXCL10, CXCL11, CXCL13, KLRK1, and/or SLAMF7 in a biological sample obtained from the patient, and comparing the expression level of the one or more of CD8A, CD8B, EOMES, GZMA, GZMB, IFNG, PRF1, CXCL9, CXCL10, CXCL11, CXCL13, KLRK1, and/or SLAMF7 in the biological sample with a reference level, wherein a change (e.g., an increase or decrease) in the expression level of one or more of CD8A, CD8B, EOMES, GZMA, GZMB, IFNG, PRF1, CXCL9, CXCL10, CXCL11, CXCL13, KLRK1, and/or SLAMF7 in the biological sample relative to the reference level identifies a patient who is likely to respond to the anti-cancer therapy. In some embodiments, the change is an increase. In other embodiments, the change is a decrease.

In still further embodiments, the invention provides methods of selecting an anti-cancer therapy comprising a VEGF antagonist (e.g., an anti-VEGF antibody, such as bevacizumab) and a PD-L1 axis binding antagonist (e.g., a PD-L1 binding antagonist, such as atezolizumab) for a patient having a cancer (e.g., kidney cancer), the method involving determining the expression level of one or more (e.g., 1, 2, 3, 4, 5, 6, 7, 8, 9, 10, 11, 12, or 13) of CD8A, CD8B, EOMES, GZMA, GZMB, IFNG, PRF1, CXCL9, CXCL10, CXCL11, CXCL13, KLRK1, and/or SLAMF7 in a biological sample obtained from the patient, comparing the expression level of the one or more of CD8A, CD8B, EOMES, GZMA, GZMB, IFNG, PRF1, CXCL9, CXCL10, CXCL11, CXCL13, KLRK1, and/or SLAMF7 in the biological sample with a reference level, and selecting an anti-cancer therapy comprising a VEGF antagonist and a PD-L1 axis binding antagonist for the patient based on the expression level of one or more of CD8A, CD8B, EOMES, GZMA, GZMB, IFNG, PRF1, CXCL9, CXCL10, CXCL11, CXCL13, KLRK1, and/or SLAMF7 in the biological sample relative to the reference level. In some embodiments, a change in the expression level (e.g., an increase or a decrease) of one or more of CD8A, CD8B, EOMES, GZMA, GZMB, IFNG, PRF1, CXCL9, CXCL10, CXCL11, CXCL13, KLRK1, and/or SLAMF7 in the biological sample relative to the reference level is used to select the anti-cancer therapy. In some embodiments, the change is an increase. In other embodiments, the change is a decrease.

In some embodiments of any of the preceding methods, the expression level of one or more (e.g., 1, 2, 3, 4, 5, 6, or 7) of CD8A, CD8B, EOMES, GZMA, GZMB, IFNG, or PRF1 is correlated with the presence of CD8⁺ T effector (T_{eff}) cells in the tumor microenvironment. In some embodiments, the expression level of one or more (e.g., 1, 2, or 3) of GZMB, KLRK1, or SLAMF7 is correlated with the presence of natural killer (NK) cells in the tumor microenvironment. Methods of detecting the presence of CD8⁺ T_{eff} cells and/or NK cells are described herein and include, e.g., flow cytometry analysis on tumor sample cells (e.g., analysis of tumor-infiltrating immune cells in a tumor biopsy). In certain embodiments, the expression level of one or more (e.g., 1, 2, 3, or 4) of CXCL9, CXCL10, CXCL11, or CXCL13 is correlated with presence of Th1 chemokines in the tumor microenvironment. Methods for detecting the presence of Th1 chemokines in the tumor microenvironment are described herein and include, e.g., ELISA analysis on a tumor sample (e.g., a tumor biopsy lysate).

In certain embodiments of any of the preceding methods, the expression level of one or more (e.g., 1, 2, 3, 4, 5, 6, or 7) of CD8A, CD8B, EOMES, GZMA, GZMB, IFNG, or PRF1 is determined. In some embodiments, the expression level of at least 2, at least 3, at least 4, at least 5, or at least 6 of CD8A, CD8B, EOMES, GZMA, GZMB, IFNG, or PRF1 is determined. In some embodiments, the expression level of CD8A, CD8B, EOMES, GZMA, GZMB, IFNG, and PRF1 is determined. In some embodiments, the level of CD8A is between about 1-fold and about 60-fold (e.g., about 1-fold, about 2-fold, about 3-fold, about 4-fold, about 5-fold, about 10-fold, about 15-fold, about 17-fold, about 20-fold, about 25-fold, about 30-fold, about 35-fold, about 40-fold, about 45-fold, about 50-fold, about 55-fold, about 60-fold, or about 1-fold to about 50-fold) increased compared to a reference level. In some embodiments, the level of CD8A is about 50-fold or greater (e.g., about 50-fold, 100-fold, 200-fold, 250-fold, 500-fold, 1000-fold, or greater) increased compared to a reference level. In some embodiments, the level of CD8B is between about 1-fold and about 60-fold (e.g., about 1-fold, about 2-fold, about 3-fold, about 4-fold, about 5-fold, about 10-fold, about 15-fold, about 17-fold, about 20-fold, about 25-fold, about 30-fold, about 35-fold, about 40-fold, about 45-fold, about 50-fold, about 55-fold, about 60-fold, or about 1-fold to about 50-fold) increased compared to a reference level. In some embodiments, the level of CD8B is about 50-fold or greater (e.g., about 50-fold, 100-fold, 200-fold, 250-fold, 500-fold, 1000-fold, or greater) increased compared to a reference level. In some embodiments, the level of is between about 1-fold and about 60-fold (e.g., about 1-fold, about 2-fold, about 3-fold, about 4-fold, about 5-fold, about 10-fold, about 15-fold, about 17-fold, about 20-fold, about 25-fold, about 30-fold, about 35-fold, about 40-fold, about 45-fold, about 50-fold, about 55-fold, about 60-fold, or about 1-fold to about 50-fold) increased compared to a reference level. In some embodiments, the level of EOMES is about 50-fold or greater (e.g., about 50-fold, 100-fold, 200-fold, 250-fold, 500-fold, 1000-fold, or greater) increased compared to a reference level. In some embodiments, the level of GZMA is between about 1-fold and about 60-fold (e.g., about 1-fold, about 2-fold, about 3-fold, about 4-fold, about 5-fold, about 10-fold, about 15-fold, about 17-fold, about 20-fold, about 25-fold, about 30-fold, about 35-fold, about 40-fold, about 45-fold, about 50-fold, about 55-fold, about 60-fold, or about 1-fold to about 50-fold) increased compared to a reference level. In some embodiments, the level of GZMA is about 50-fold or greater (e.g., about 50-fold, 100-fold, 200-fold, 250-fold, 500-fold, 1000-fold, or greater) increased compared to a reference level. In some embodiments, the level of GZMB is between about 1-fold and about 60-fold (e.g., about 1-fold, about 2-fold, about 3-fold, about 4-fold, about 5-fold, about 10-fold, about 15-fold, about 17-fold, about 20-fold, about 25-fold, about 30-fold, about 35-fold, about 40-fold, about 45-fold, about 50-fold, about 55-fold, about 60-fold, or about 1-fold to about 50-fold) increased compared to a reference level. In some embodiments, the level of GZMB is about 50-fold or greater (e.g., about 50-fold, 100-fold, 200-fold, 250-fold, 500-fold, 1000-fold, or greater) increased compared to a reference level. In some embodiments, the level of IFNG is between about 1-fold and about 60-fold (e.g., about 1-fold, about 2-fold, about 3-fold, about 4-fold, about 5-fold, about 10-fold, about 15-fold, about 17-fold, about 20-fold, about 25-fold, about 30-fold, about 35-fold, about 40-fold, about 45-fold, about 50-fold, about 55-fold, about 60-fold, or about 1-fold to about 50-fold) increased compared to a reference level. In some embodiments, the level of IFNG is about 50-fold or greater (e.g., about 50-fold, 100-fold, 200-fold, 250-fold, 500-fold, 1000-fold, or greater) increased compared to a reference level. In some embodiments, the level of PRF1 is between about 1-fold and about 60-fold (e.g., about 1-fold, about 2-fold, about 3-fold, about 4-fold, about 5-fold, about 10-fold, about 15-fold, about 17-fold, about 20-fold, about 25-fold, about 30-fold, about 35-fold, about 40-fold, about 45-fold, about 50-fold, about 55-fold, about 60-fold, or about 1-fold to about 50-fold) increased compared to a reference level. In some embodiments, the level of PRF1 is about 50-fold or greater (e.g., about 50-fold, 100-fold, 200-fold, 250-fold, 500-fold, 1000-fold, or greater) increased compared to a reference level. In certain embodiments, the expression level of one or more of (e.g., 1, 2, 3, or 4) of CXCL9, CXCL10, CXCL11, or CXCL13 is determined. In some embodiments, the expression level of at least 2 or at least 3 of CXCL9, CXCL10, CXCL11, or CXCL13 is determined. In some embodiments, the expression level of CXCL9, CXCL10, CXCL11, and CXCL13 is determined. In some embodiments, the level of CXCL9 is between about 1-fold and about 300-fold (e.g., about 1-fold, about 2-fold, about 3-fold, about 4-fold, about 5-fold, about 10-fold, about 20-fold, about 30-fold, about 50-fold, about 60-fold, about 70-fold, about 80-fold, about 90-fold, about 100-fold, about 150-fold, about 200-fold, about 250-fold, about 3-fold to about 250-fold, or about 1-fold to about 80-fold) increased compared to a reference level. In some embodiments, the level of CXCL9 is about 50-fold or greater (e.g., about 50-fold, 80-fold, 100-fold, 150-fold, 200-fold, 250-fold, 1000-fold, or greater) increased compared to a reference level. In some embodiments, the level of CXCL10 is between about 1-fold and about 300-fold (e.g., about 1-fold, about 2-fold, about 3-fold, about 4-fold, about 5-fold, about 10-fold, about 20-fold, about 30-fold, about 50-fold, about 60-fold, about 70-fold, about 80-fold, about 90-fold, about 100-fold, about 150-fold, about 200-fold, about 250-fold, about 3-fold to about 250-fold, or about 1-fold to about 80-fold) increased compared to a reference level. In some embodiments, the level of CXCL10 is about 50-fold or greater (e.g., about 50-fold, 80-fold, 100-fold, 150-fold, 200-fold, 250-fold, 1000-fold, or greater) increased compared to a reference level. In some embodiments, the level of CXCL11 is between about 1-fold and about 300-fold (e.g., about 1-fold, about 2-fold, about 3-fold, about 4-fold, about 5-fold, about 10-fold, about 20-fold, about 30-fold, about 50-fold, about 60-fold, about 70-fold, about 80-fold, about 90-fold, about 100-fold, about 150-fold, about 200-fold, about 250-fold, about 3-fold to about 250-fold, or about 1-fold to about 80-fold) increased compared to a reference level. In some embodiments, the level of CXCL11 is about 50-fold or greater (e.g., about 50-fold, 80-fold, 100-fold, 150-fold, 200-fold, 250-fold, 1000-fold, or greater) increased compared to a reference level. In some embodiments, the level of CXCL13 is between about 1-fold and about 300-fold (e.g., about 1-fold, about 2-fold, about 3-fold, about 4-fold, about 5-fold, about 10-fold, about 20-fold, about 30-fold, about 50-fold, about 60-fold, about 70-fold, about 80-fold, about 90-fold, about 100-fold, about 150-fold, about 200-fold, about 250-fold, about 3-fold to about 250-fold, or about 1-fold to about 80-fold) increased compared to a reference level. In some embodiments, the level of CXCL13 is about 50-fold or greater (e.g., about 50-fold, 80-fold, 100-fold, 150-fold, 200-fold, 250-fold, 1000-fold, or greater) increased compared to a reference level.

In some embodiments of any of the preceding methods, the expression level of one or more (e.g., 1, 2, or 3) of GZMB, KLRK1, or SLAMF7 is determined. In some embodiments, the expression level of at least 2 of GZMB, KLRK1, or SLAMF7 is determined. In some embodiments, the expression level of GZMB, KLRK1, and SLAMF7 is determined. In some embodiments, the level of GZMB is between about 1-fold and about 20-fold (e.g., about 1-fold, about 2-fold, about 3-fold, about 4-fold, about 5-fold, about 6-fold, about 7-fold, about 8-fold, about 9-fold, about 10-fold, about 12-fold, about 13-fold, about 15-fold, about 20-fold, between about 1-fold and about 8-fold, or between about 1-fold and about 13-fold) increased compared to a reference level. In some embodiments, the level of GZMB is about 8-fold or greater (e.g., about 8-fold, 9-fold, 10-fold, 13-fold, 15-fold, 20-fold, 50-fold, 100-fold, 1000-fold, or greater) increased compared to a reference level. In some embodiments, the level of KLRK1 is between about 1-fold and about 20-fold (e.g., about 1-fold, about 2-fold, about 3-fold, about 4-fold, about 5-fold, about 6-fold, about 7-fold, about 8-fold, about 9-fold, about 10-fold, about 12-fold, about 13-fold, about 15-fold, about 20-fold, between about 1-fold and about 8-fold, or between about 1-fold and about 13-fold) increased compared to a reference level. In some embodiments, the level of KLRK1 is about 8-fold or greater (e.g., about 8-fold, 9-fold, 10-fold, 13-fold, 15-fold, 20-fold, 50-fold, 100-fold, 1000-fold, or greater) increased compared to a reference level. In some embodiments, the level of SLAMF7 is between about 1-fold and about 20-fold (e.g., about 1-fold, about 2-fold, about 3-fold, about 4-fold, about 5-fold, about 6-fold, about 7-fold, about 8-fold, about 9-fold, about 10-fold, about 12-fold, about 13-fold, about 15-fold, about 20-fold, between about 1-fold and about 8-fold, or between about 1-fold and about 13-fold) increased compared to a reference level. In some embodiments, the level of SLAMF7 is about 8-fold or greater (e.g., about 8-fold, 9-fold, 10-fold, 13-fold, 15-fold, 20-fold, 50-fold, 100-fold, 1000-fold, or greater) increased compared to a reference level.

In some embodiments of any of the preceding methods, the expression level of one or more (e.g., 1, 2, 3, 4, 5, 6, 7, 8, 9, 10, 11, 12, or 13) of CD8A, CD8B, EOMES, GZMA, GZMB, IFNG, PRF1, CXCL9, CXCL10, CXCL11, CXCL13, KLRK1, and/or SLAMF7 in the biological sample obtained from the patient is increased (e.g., by about 1.1-fold, about 1.2-fold, about 1.3-fold, about 1.4-fold, about 1.5-fold, about 1.6-fold, about 1.7-fold, about 1.8-fold, about 1.9-fold, about 2-fold, about 2.1-fold, about 2.2-fold, about 2.3-fold, about 2.4-fold, about 2.5-fold, about 3-fold, about 3.5-fold, about 4-fold, about 4.5-fold, about 5-fold, about 5.5-fold, about 6-fold, about 6.5-fold, about 7-fold, about 7.5-fold, about 8-fold, about 8.5-fold, about 9-fold, about 9.5-fold, about 10-fold, about 11-fold, about 12-fold, about 13-fold, about 14-fold, about 15-fold, about 16-fold, about 17-fold, about 18-fold, about 19-fold, about 20-fold, about 30-fold, about 40-fold, about 50-fold, about 100-fold, about 500-fold, about 1,000-fold or greater) relative to the reference level.

In some embodiments of any of the preceding methods, a reference level is the expression level of the one or more genes (e.g., CD8A, CD8B, EOMES, GZMA, GZMB, IFNG, PRF1, CXCL9, CXCL10, CXCL11, CXCL13, KLRK1, and/or SLAMF7) in a biological sample from the patient obtained prior to (e.g., minutes, hours, days, weeks (e.g., 1, 2, 3, 4, 5, 6, or 7 weeks), months, or years prior to) administration of the anti-cancer therapy. In certain embodiments, a reference level is the expression level of the one or more genes in a reference population. In certain embodiments, the reference level is a pre-assigned expression level for the one or more genes. In some embodiments, the reference level is the expression level of the one or more genes in a biological sample obtained from the patient at a previous time point, wherein the previous time point is following administration of the anti-cancer therapy. In other embodiments, the reference level is the expression level of the one or more genes in a biological sample obtained from the patient at a subsequent time point (e.g., minutes, hours, days, weeks, months, or years after administration of an anti-cancer therapy).

In another aspect, the invention provides a method of monitoring the response of a patient having a cancer (e.g., kidney cancer) to treatment with an anti-cancer therapy comprising a VEGF antagonist (e.g., an anti-VEGF antibody, such as bevacizumab) and a PD-L1 axis binding antagonist (e.g., a PD-L1 binding antagonist, such as atezolizumab), involving determining the expression level of MHC-I in a biological sample obtained from the patient at a time point following administration of the anti-cancer therapy and comparing the expression level of MHC-I in the biological sample with a reference level, thereby monitoring the response in the patient to treatment with the anti-cancer therapy. In some embodiments, the expression level in the biological sample obtained from the patient is increased relative to the reference level.

In other embodiments, the invention provides a method of identifying a patient having a cancer (e.g., kidney cancer) who is likely to benefit from treatment with an anti-cancer therapy that includes a VEGF antagonist (e.g., an anti-VEGF antibody, such as bevacizumab) and a PD-L1 axis binding antagonist (e.g., a PD-L1 binding antagonist, such as atezolizumab), the method involving determining the expression level of MHC-I in a biological sample obtained from the patient, and comparing the expression level of MHC-I in the biological sample with a reference level, thereby identifying the patient as likely to benefit from treatment with the anti-cancer therapy. In some embodiments, a change in the expression level (e.g., an increase or a decrease) of MHC-I in the biological sample relative to the reference level identifies the patient as likely to benefit from treatment with the anti-cancer therapy. In some embodiments, the change is an increase. In other embodiments, the change is a decrease.

In another embodiment, the invention provides a method of diagnosing or prognosing a cancer (e.g., kidney cancer), the method involving determining the expression level of MHC-I in a biological sample obtained from the patient, and comparing the expression level of MHC-I in the patient sample with a reference level, thereby diagnosing or prognosing the cancer. In some embodiments, a change in the expression level (e.g., an increase or a decrease) of MHC-I in the biological sample relative to the reference level diagnoses or prognoses the patient. In some embodiments, the change is an increase. In other embodiments, the change is a decrease.

In yet another embodiment, the invention provides a method of determining whether a patient having a cancer (e.g., kidney cancer) is likely to respond to treatment with an anti-cancer therapy that includes a VEGF antagonist (e.g., an anti-VEGF antibody, such as bevacizumab) and a PD-L1 axis binding antagonist (e.g., a PD-L1 binding antagonist, such as atezolizumab), the method involving determining the expression level of MHC-I in a biological sample obtained from the patient, and comparing the expression level of MHC-I in the patient sample with a reference level, thereby identifying the patient as one who is likely to respond to the anti-cancer therapy. In some embodiments, a change in the expression level (e.g., an increase or a decrease) of MHC-I in the biological sample relative to the reference level identifies the patient as likely to respond to treatment with the anti-cancer therapy. In some embodiments, the change is an increase. In other embodiments, the change is a decrease.

In other embodiments, the invention provides a method of optimizing therapeutic efficacy of an anti-cancer therapy that includes a VEGF antagonist (e.g., an anti-VEGF antibody, such as bevacizumab) and a PD-L1 axis binding antagonist (e.g., a PD-L1 binding antagonist, such as atezolizumab), the method involving determining the expression level of MHC-I in a biological sample obtained from the patient, and comparing the expression level of MHC-I in the patient sample with a reference level, wherein a change (e.g., an increase or decrease) in the expression level of MHC-I in the biological sample relative to the reference level identifies a patient who is likely to respond to the anti-cancer therapy. In some embodiments, the change is an increase. In other embodiments, the change is a decrease.

In still further embodiments, the invention provides methods of selecting an anti-cancer therapy comprising a VEGF antagonist (e.g., an anti-VEGF antibody, such as bevacizumab) for a patient having a cancer (e.g., kidney cancer) and a PD-L1 axis binding antagonist (e.g., a PD-L1 binding antagonist, such as atezolizumab), the method involving determining the expression level of MHC-I in a biological sample obtained from the patient, comparing the expression level of MHC-I in the biological sample with a reference level, and selecting an anti-cancer therapy comprising a VEGF antagonist and a PD-L1 antagonist for the patient based on the expression level of MHC-I in the patient sample relative to the reference level. In some embodiments, a change in the expression level (e.g., an increase or a decrease) of MHC-I in the biological sample relative to the reference level is used to select the anti-cancer therapy. In some embodiments, the change is an increase. In other embodiments, the change is a decrease.

In some embodiments of any of the preceding methods, the expression level of MHC-I is assessed by determining the expression level of any human leukocyte antigen class I (HLA-I) gene or pseudogene (e.g., HLA-A, HLA-B, HLA-C, HLA-E, HLA-F, HLA-G, HLA-K, or HLA-L), or haplotype thereof, by any of the detection method described herein. In some embodiments, the expression level of MHC-I is assessed by protein expression of MHC-I alpha chain, or HLA-I histocompatibility antigen alpha chain.

In some embodiments of any of the preceding methods, a reference level is the expression level of MHC-I in a biological sample from the patient obtained prior to (e.g., minutes, hours, days, weeks (e.g., 1, 2, 3, 4, 5, 6, or 7 weeks), months, or years prior to) administration of the anti-cancer therapy. In other embodiments, a reference level is the expression level of MHC-I in a reference population. In other embodiments, the reference level is a pre-assigned expression level for MHC-I. In other embodiments, the reference level is the expression level of MHC-I in a biological sample obtained from the patient at a previous time point, wherein the previous time point is following administration of the anti-cancer therapy. In other embodiments, the reference level is the expression level of MHC-I in a biological sample obtained from the patient at a subsequent time point (e.g., minutes, hours, days, weeks, months, or years after administration of an anti-cancer therapy).

In some embodiments of any of the preceding methods, the expression level of MHC-I in the biological sample obtained from the patient is increased (e.g., by about 1.1-fold, about 1.2-fold, about 1.3-fold, about 1.4-fold, about 1.5-fold, about 1.6-fold, about 1.7-fold, about 1.8-fold, about 1.9-fold, about 2-fold, about 2.1-fold, about 2.2-fold, about 2.3-fold, about 2.4-fold, about 2.5-fold, about 3-fold, about 3.5-fold, about 4-fold, about 4.5-fold, about 5-fold, about 5.5-fold, about 6-fold, about 6.5-fold, about 7-fold, about 7.5-fold, about 8-fold, about 8.5-fold, about 9-fold, about 9.5-fold, about 10-fold, about 11-fold, about 12-fold, about 13-fold, about 14-fold, about 15-fold, about 16-fold, about 17-fold, about 18-fold, about 19-fold, about 20-fold, about 30-fold, about 40-fold, about 50-fold, about 100-fold, about 500-fold, about 1,000-fold or greater) relative to the reference level. In some embodiments, the expression level of MHC-I in the biological sample obtained from the patient is increased by at least about 2-fold.

In a further aspect, the invention provides a method of monitoring the response of a patient having a cancer (e.g., kidney cancer) to treatment with an anti-cancer therapy comprising a VEGF antagonist (e.g., an anti-VEGF antibody, such as bevacizumab) and a PD-L1 axis binding antagonist (e.g., a PD-L1 binding antagonist, such as atezolizumab), involving determining the expression level of one or more (e.g., 1, 2, 3, 4, 5, 6, or 7) of the following genes: CX3CR1, CCL2, CCL5, CCR5, CX3CL1, CCR7, or CXCL10 in a biological sample obtained from the patient at a time point following administration of the anti-cancer therapy and comparing the expression level of the one or more of CX3CR1, CCL2, CCL5, CCR5, CX3CL1, CCR7, or CXCL10 in the biological sample with a reference level, thereby monitoring the response in the patient to treatment with the anti-cancer therapy.

In other embodiments, the invention provides a method of identifying a patient having a cancer (e.g., kidney cancer) who is likely to benefit from treatment with an anti-cancer therapy comprising a VEGF antagonist (e.g., an anti-VEGF antibody, such as bevacizumab) and a PD-L1 axis binding antagonist (e.g., a PD-L1 binding antagonist, such as atezolizumab), the method involving determining the expression level of one or more (e.g., 1, 2, 3, 4, 5, 6, or 7) of the following genes: CX3CR1, CCL2, CCL5, CCR5, CX3CL1, CCR7, or CXCL10 in a biological sample obtained from the patient, and comparing the expression level of the one or more of CX3CR1, CCL2, CCL5, CCR5, CX3CL1, CCR7, or CXCL10 in the biological sample with a reference level, thereby identifying the patient as likely to benefit from treatment with the anti-cancer therapy. In some embodiments, a change in the expression level (e.g., an increase or a decrease) of the one or more of CX3CR1, CCL2, CCL5, CCR5, CX3CL1, CCR7, or CXCL10 in the biological sample relative to the reference level identifies the patient as likely to benefit from treatment with the anti-cancer therapy. In some embodiments, the change is an increase. In other embodiments, the change is a decrease.

In another embodiment, the invention provides a method of diagnosing or prognosing a cancer (e.g., kidney cancer), the method involving determining the expression level of one or more (e.g., 1, 2, 3, 4, 5, 6, or 7) of the following genes: CX3CR1, CCL2, CCL5, CCR5, CX3CL1, CCR7, or CXCL10 in a biological sample obtained from the patient, and comparing the expression level of the one or more of CX3CR1, CCL2, CCL5, CCR5, CX3CL1, CCR7, or CXCL10 in the biological sample with a reference level, thereby diagnosing or prognosing the cancer. In some embodiments, a change in the expression level (e.g., an increase or a decrease) of the one or more of CX3CR1, CCL2, CCL5, CCR5, CX3CL1, CCR7, or CXCL10 in the biological sample relative to the reference level diagnoses or prognoses the patient. In some embodiments, the change is an increase. In other embodiments, the change is a decrease.

In yet another embodiment, the invention provides a method of determining whether a patient having a cancer (e.g., kidney cancer) is likely to respond to treatment with an anti-cancer therapy that includes a VEGF antagonist (e.g., an anti-VEGF antibody, such as bevacizumab) and a PD-L1 axis binding antagonist (e.g., a PD-L1 binding antagonist, such as atezolizumab), the method involving determining the expression level of one or more (e.g., 1, 2, 3, 4, 5, 6, or 7) of the following genes: CX3CR1, CCL2, CCL5, CCR5, CX3CL1, CCR7, or CXCL10 in a biological sample obtained from the patient, and comparing the expression level of the one or more of CX3CR1, CCL2, CCL5, CCR5, CX3CL1, CCR7, or CXCL10 in the biological sample with a reference level, thereby identifying the patient as one who is likely to respond to the anti-cancer therapy. In some embodiments, a change in the expression level (e.g., an increase or a decrease) of the one or more of CX3CR1, CCL2, CCL5, CCR5, CX3CL1, CCR7, or CXCL10 in the biological sample relative to the reference level identifies the patient as likely to respond to treatment with the anti-cancer therapy. In some embodiments, the change is an increase. In other embodiments, the change is a decrease.

In other embodiments, the invention provides a method of optimizing therapeutic efficacy of an anti-cancer therapy that includes a VEGF antagonist (e.g., an anti-VEGF antibody, such as bevacizumab) and a PD-L1 axis binding antagonist (e.g., a PD-L1 binding antagonist, such as atezolizumab), the method involving determining the expression level of one or more (e.g., 1, 2, 3, 4, 5, 6, or 7) of the following genes: CX3CR1, CCL2, CCL5, CCR5, CX3CL1, CCR7, or CXCL10 in a biological sample obtained from the patient, and comparing the expression level of the one or more of CX3CR1, CCL2, CCL5, CCR5, CX3CL1, CCR7, or CXCL10 in the biological sample with a reference level, wherein a change (e.g., an increase or decrease) in the expression level of the one or more of CX3CR1, CCL2, CCL5, CCR5, CX3CL1, CCR7, or CXCL10 in the biological sample relative to the reference level identifies a patient who is likely to respond to the anti-cancer therapy. In some embodiments, the change is an increase. In other embodiments, the change is a decrease.

In still further embodiments, the invention provides methods of selecting an anti-cancer therapy comprising a VEGF antagonist (e.g., an anti-VEGF antibody, such as bevacizumab) and a PD-L1 axis binding antagonist (e.g., a PD-L1 binding antagonist, such as atezolizumab) for a patient having a cancer (e.g., kidney cancer), the method involving determining the expression level of one or more (e.g., 1, 2, 3, 4, 5, 6, or 7) of the following genes: CX3CR1, CCL2, CCL5, CCR5, CX3CL1, CCR7, or CXCL10 in a biological sample obtained from the patient, comparing the expression level of the one or more of CX3CR1, CCL2, CCL5, CCR5, CX3CL1, CCR7, or CXCL10 in the biological sample with a reference level, and selecting an anti-cancer therapy comprising a VEGF antagonist and a PD-L1 axis binding antagonist for the patient based on the expression level of the one or more of CX3CR1, CCL2, CCL5, CCR5, CX3CL1, CCR7, or CXCL10 in the biological sample relative to the reference level. In some embodiments, a change in the expression level (e.g., an increase or a decrease) of the one or more of CX3CR1, CCL2, CCL5, CCR5, CX3CL1, CCR7, or CXCL10 in the biological sample relative to the reference level is used to select the anti-cancer therapy. In some embodiments, the change is an increase. In other embodiments, the change is a decrease.

In some embodiments of any of the preceding methods, the expression level in the biological sample obtained from the patient is increased relative to the reference level. In some embodiments, the expression level of at least two, at least three, at least four, at least five, or at least six of CX3CR1, CCL2, CCL5, CCR5, CX3CL1, CCR7, or CXCL10 is determined. In some embodiments, the expression level of CX3CR1, CCL2, CCL5, CCR5, CX3CL1, CCR7, and CXCL10 is determined.

In some embodiments of any of the preceding methods, the reference level is the expression level of the one or more genes (e.g., CX3CR1, CCL2, CCL5, CCR5, CX3CL1, CCR7, and/or CXCL10) in a biological sample from the patient obtained prior to (e.g., minutes, hours, days, weeks, months, or years prior to) administration of the anti-cancer therapy. In other embodiments, the reference level is the expression level of the one or more genes in a reference population. In other embodiments, the reference level is a pre-assigned expression level for the one or more genes. In other embodiments, the reference level is the expression level of the one or more genes in a biological sample obtained from the patient at a previous time point, wherein the previous time point is following administration of the anti-cancer therapy. In other embodiments, the reference level is the expression level of the one or more genes in a biological sample obtained from the patient at a subsequent time point.

In some embodiments of any of the preceding methods, the expression level of one or more (e.g., 1, 2, 3, 4, 5, 6, or 7) of CX3CR1, CCL2, CCL5, CCR5, CX3CL1, CCR7, and/or CXCL10 in the biological sample obtained from the patient is increased (e.g., by about 1.1-fold, about 1.2-fold, about 1.3-fold, about 1.4-fold, about 1.5-fold, about 1.6-fold, about 1.7-fold, about 1.8-fold, about 1.9-fold, about 2-fold, about 2.1-fold, about 2.2-fold, about 2.3-fold, about 2.4-fold, about 2.5-fold, about 3-fold, about 3.5-fold, about 4-fold, about 4.5-fold, about 5-fold, about 5.5-fold, about 6-fold, about 6.5-fold, about 7-fold, about 7.5-fold, about 8-fold, about 8.5-fold, about 9-fold, about 9.5-fold, about 10-fold, about 11-fold, about 12-fold, about 13-fold, about 14-fold, about 15-fold, about 16-fold, about 17-fold, about 18-fold, about 19-fold, about 20-fold, about 30-fold, about 40-fold, about 50-fold, about 100-fold, about 500-fold, about 1,000-fold or greater) relative to the reference level.

In some embodiments of any of the methods described above, the biological sample from the patient is obtained about 2 to about 10 weeks (e.g., 2, 3, 4, 5, 6, 7, 8, 9, or 10 weeks) following administration of the anti-cancer therapy. In some embodiments, the biological sample from the patient is obtained about 4 to about 6 weeks following administration of the anti-cancer therapy.

In certain embodiments of any of the preceding methods, the method of the invention further involves the step of administering one or more (e.g., 1, 2, 3, 4, 5, 6, 7, 8, 9, 10, or more) additional doses of the anti-cancer therapy to a patient whose expression level of MHC-I or the one or more genes (e.g., CD8A, CD8B, EOMES, GZMA, GZMB, IFNG, PRF1, CXCL9, CXCL10, CXCL11, CXCL13, KLRK1, SLAMF7, CX3CR1, CCL2, CCL5, CCR5, CX3CL1, and/or CCR7) is increased relative to the reference level.

The presence and/or expression level of any of the biomarkers described above may be assessed qualitatively and/or quantitatively based on any suitable criterion known in the art, including but not limited to DNA, mRNA, cDNA, proteins, protein fragments, and/or gene copy number. Methodologies for measuring such biomarkers are known in the art and understood by the skilled artisan, including, but not limited to, immunohistochemistry ("IHC"), Western blot analysis, immunoprecipitation, molecular binding assays, ELISA, ELIFA, fluorescence activated cell sorting ("FACS"), MassARRAY, proteomics, quantitative blood based assays (e.g., Serum ELISA), biochemical enzymatic activity assays, in situ hybridization, fluorescence in situ hybridization (FISH), Southern analysis, Northern analysis, whole genome sequencing, polymerase chain reaction (PCR) including quantitative real time PCR (qRT-PCR) and other amplification type detection methods, such as, for example, branched DNA, SISBA, TMA and the like, RNA-Seq, microarray analysis, gene expression profiling, whole-genome sequencing (WGS), and/or serial analysis of gene expression ("SAGE"), as well as any one of the wide variety of assays that can be performed by protein, gene, and/or tissue array analysis. Typical protocols for evaluating the status of genes and gene products are found, for example, in Ausubel et al. eds. (Current Protocols In Molecular Biology, 1995), Units 2 (Northern Blotting), 4 (Southern Blotting), 15 (Immunoblotting) and 18 (PCR Analysis). Multiplexed immunoassays such as those available from Rules Based Medicine or Meso Scale Discovery ("MSD") may also be used.

In any of the preceding methods, the expression level of a biomarker may be a protein expression level. In certain embodiments, the method comprises contacting the biological sample with antibodies that specifically bind to a biomarker described herein under conditions permissive for binding of the biomarker, and detecting whether a complex is formed between the antibodies and biomarker. Such method may be an *in vitro* or *in vivo* method. In some instances, an antibody is used to select patients eligible for therapy with a VEGF antagonist and/or a PD-L1 axis binding antagonist, e.g., a biomarker for selection of individuals. Any method of measuring protein expression levels known in the art or provided herein may be used. For example, in some embodiments, a protein expression level of a biomarker is determined using a method selected from the group consisting of flow cytometry (e.g., fluorescence-activated cell sorting (FACS™)), Western blot, enzyme-linked immunosorbent assay (ELISA), immunoprecipitation, immunohistochemistry (IHC), immunofluorescence, radioimmunoassay, dot blotting, immunodetection methods, HPLC, surface plasmon resonance, optical spectroscopy, mass spectrometry, and HPLC. In some embodiments, the protein expression level of the biomarker is determined in tumor-infiltrating immune cells. In some embodiments, the protein expression level of the biomarker is determined in tumor cells. In some embodiments, the protein expression level of the biomarker is determined in tumor-infiltrating immune cells and/or in tumor cells. In some embodiments, the protein expression level of the biomarker is determined in peripheral blood mononuclear cells (PBMCs).

In certain embodiments, the presence and/or expression level/amount of a biomarker protein in a sample is examined using IHC and staining protocols. IHC staining of tissue sections has been shown to be a reliable method of determining or detecting the presence of proteins in a sample. In some embodiments of any of the methods, assays and/or kits, the biomarker is one or more of the protein expression products of the following genes: CD8A, CD8B, EOMES, GZMA, GZMB, IFNG, PRF1, CXCL9, CXCL10, CXCL11, CXCL13, KLRK1, SLAMF7, CX3CR1, CCL2, CCL5, CCR5, CX3CL1, CCR7, and MHC-I. In one embodiment, an expression level of biomarker is determined using a method comprising: (a) performing IHC analysis of a sample (such as a tumor sample obtained from a patient) with an antibody; and (b) determining expression level of a biomarker in the sample. In some embodiments, IHC staining intensity is determined relative to a reference. In some embodiments, the reference is a reference value. In some embodiments, the reference is a reference sample (e.g., a control cell line staining sample, a tissue sample from non-cancerous patient, or a tumor sample that is determined to be negative for the biomarker of interest).

IHC may be performed in combination with additional techniques such as morphological staining and/or *in situ* hybridization (e.g., FISH). Two general methods of IHC are available; direct and indirect assays. According to the first assay, binding of antibody to the target antigen is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromogenic or fluorogenic substrate is added to provide visualization of the antigen. Signal amplification occurs because several secondary antibodies may react with different epitopes on the primary antibody.

The primary and/or secondary antibody used for IHC typically will be labeled with a detectable moiety. Numerous labels are available which can be generally grouped into the following categories: (a) radioisotopes, such as ³⁵S, ¹⁴C, ¹²⁵1, ³H, and ¹³¹I; (b) colloidal gold particles; (c) fluorescent labels including, but are not limited to, rare earth chelates (europium chelates), Texas Red, rhodamine, fluorescein, dansyl, lissamine, umbelliferone, phycocrytherin, phycocyanin, or commercially-available fluorophores such as SPECTRUM ORANGE7 and SPECTRUM GREEN7 and/or derivatives of any one or more of the above; (d) various enzyme-substrate labels are available and U.S. Patent No. 4,275,149 provides a review of some of these. Examples of enzymatic labels include luciferases (e.g., firefly luciferase and bacterial luciferase; see, e.g., U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like.

Examples of enzyme-substrate combinations include, for example, horseradish peroxidase (HRPO) with hydrogen peroxidase as a substrate; alkaline phosphatase (AP) with para-Nitrophenyl phosphate as chromogenic substrate; and β-D-galactosidase (β-D-Gal) with a chromogenic substrate (e.g., p-nitrophenyl-β-D-galactosidase) or fluorogenic substrate (e.g., 4-methylumbelliferyl-β-D-galactosidase). For a general review of these, see, for example, U.S. Patent Nos. 4,275,149 and 4,318,980.

Specimens may be prepared, for example, manually, or using an automated staining instrument (e.g., a Ventana BenchMark XT or Benchmark ULTRA instrument). Specimens thus prepared may be mounted and coverslipped. Slide evaluation is then determined, for example, using a microscope, and staining intensity criteria, routinely used in the art, may be employed. In one embodiment, it is to be understood that when cells and/or tissue from a tumor is examined using IHC, staining is generally determined or assessed in tumor cell(s) and/or tissue (as opposed to stromal or surrounding tissue that may be present in the sample). In some embodiments, it is understood that when cells and/or tissue from a tumor is examined using IHC, staining includes determining or assessing in tumor-infiltrating immune cells, including intratumoral or peritumoral immune cells. In some embodiments, the presence of a biomarker is detected by IHC in >0% of the sample, in at least 1% of the sample, in at least 5% of the sample, in at least 10% of the sample, in at least 15% of the sample, in at least 15% of the sample, in at least 20% of the sample, in at least 25% of the sample, in at least 30% of the sample, in at least 35% of the sample, in at least 40% of the sample, in at least 45% of the sample, in at least 50% of the sample, in at least 55% of the sample, in at least 60% of the sample, in at least 65% of the sample, in at least 70% of the sample, in at least 75% of the sample, in at least 80% of the sample, in at least 85% of the sample, in at least 90% of the sample, in at least 95% of the sample, or more. Samples may be scored using any method known in the art, for example, by a pathologist or automated image analysis.

In some embodiments of any of the methods, the biomarker is detected by immunohistochemistry using a diagnostic antibody (i.e., primary antibody). In some embodiments, the diagnostic antibody specifically binds human antigen. In some embodiments, the diagnostic antibody is a non-human antibody. In some embodiments, the diagnostic antibody is a rat, mouse, or rabbit antibody. In some embodiments, the diagnostic antibody is a rabbit antibody. In some embodiments, the diagnostic antibody is a monoclonal antibody. In some embodiments, the diagnostic antibody is directly labeled. In other embodiments, the diagnostic antibody is indirectly labeled.

In other embodiments of any of the preceding methods, the expression level of a biomarker may be a nucleic acid expression level (e.g., a DNA expression level or an RNA expression level (e.g., an mRNA expression level). Any suitable method of determining a nucleic acid expression level may be used. In some embodiments, the nucleic acid expression level is determined using qPCR, rtPCR, RNA-seq, multiplex qPCR or RT-qPCR, microarray analysis, serial analysis of gene expression (SAGE), MassARRAY technique, *in situ* hybridization (e.g., FISH), or combinations thereof.

Methods for the evaluation of mRNAs in cells are well known and include, for example, serial analysis of gene expression (SAGE), whole genome sequencing (WGS), hybridization assays using complementary DNA probes (such as in situ hybridization using labeled riboprobes specific for the one or more genes, Northern blot and related techniques) and various nucleic acid amplification assays (such as RT-PCR (e.g., qRT-PCR) using complementary primers specific for one or more of the genes, and other amplification type detection methods, such as, for example, branched DNA, SISBA, TMA and the like). In addition, such methods can include one or more steps that allow one to determine the levels of target mRNA in a biological sample (e.g., by simultaneously examining the levels a comparative control mRNA sequence of a "housekeeping" gene such as an actin family member). Optionally, the sequence of the amplified target cDNA can be determined. Optional methods include protocols which examine or detect mRNAs, such as target mRNAs, in a tissue or cell sample by microarray technologies. Using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. For example, a selection of genes whose expression correlates with increased or reduced clinical benefit of treatment comprising a PD-L1 axis binding antagonist may be arrayed on a solid support. Hybridization of a labeled probe with a particular array member indicates that the sample from which the probe was derived expresses that gene.

In some embodiments, the invention provides methods of monitoring the response of a patient having a cancer (e.g., kidney cancer) treated with an anti-cancer therapy including a VEGF antagonist (e.g., an anti-VEGF antibody, such as bevacizumab) and a PD-L1 axis binding antagonists (e.g., a PD-L1 binding antagonists, such as atezolizumab) by determining the number of CD8⁺ T cells and/or CD68+/CD163+ macrophages in a tumor sample obtained from the patient at a time point following administration of the anti-cancer therapy and comparing the number of CD8⁺ T cells and/or CD68+/CD163+ macrophages in the tumor sample with a reference sample, thereby monitoring the response in the patient undergoing treatment with the anti-cancer therapy.

In other embodiments, the invention provides a method of identifying a patient having a cancer (e.g., kidney cancer) who is likely to benefit from treatment with an anti-cancer therapy comprising a VEGF antagonist (e.g., an anti-VEGF antibody, such as bevacizumab) and a PD-L1 axis binding antagonist (e.g., a PD-L1 binding antagonist, such as atezolizumab), the method involving determining the number of CD8⁺ T cells and/or CD68⁺/CD163⁺ macrophages in a tumor sample obtained from the patient, and comparing the number of CD8⁺ T cells and/or CD68⁺/CD163⁺ macrophages in the tumor sample with a reference sample, thereby identifying the patient as likely to benefit from treatment with the anti-cancer therapy. In some embodiments, a change in the number of CD8⁺ T cells and/or CD68⁺/CD163⁺ macrophages (e.g., an increase or a decrease) in the tumor sample relative to the reference sample identifies the patient as likely to benefit from treatment with the anti-cancer therapy. In some embodiments, the change is an increase. In other embodiments, the change is a decrease.

In another embodiment, the invention provides a method of diagnosing or prognosing a cancer (e.g., kidney cancer), the method involving determining the number of CD8⁺ T cells and/or CD68⁺/CD163⁺ macrophages in a tumor sample obtained from the patient, and comparing the number of CD8⁺ T cells and/or CD68⁺/CD163⁺ macrophages in the tumor sample with a reference sample, thereby diagnosing or prognosing the cancer. In some embodiments, a change in the number of CD8⁺ T cells and/or CD68⁺/CD163⁺ macrophages (e.g., an increase or a decrease) in the tumor sample relative to the reference sample diagnoses or prognoses the patient. In some embodiments, the change is an increase. In other embodiments, the change is a decrease.

In yet another embodiment, the invention provides a method of determining whether a patient having a cancer (e.g., kidney cancer) is likely to respond to treatment with an anti-cancer therapy that includes a VEGF antagonist (e.g., an anti-VEGF antibody, such as bevacizumab) and a PD-L1 axis binding antagonist (e.g., a PD-L1 binding antagonist, such as atezolizumab), the method involving determining the number of CD8⁺ T cells and/or CD68⁺/CD163⁺ macrophages in a tumor sample obtained from the patient, and comparing the number of CD8⁺ T cells and/or CD68⁺/CD163⁺ macrophages in the tumor sample with a reference sample, thereby identifying the patient as one who is likely to respond to the anti-cancer therapy. In some embodiments, a change in the number of CD8⁺ T cells and/or CD68⁺/CD163⁺ macrophages (e.g., an increase or a decrease) in the tumor sample relative to the reference sample identifies the patient as likely to respond to treatment with the anti-cancer therapy. In some embodiments, the change is an increase. In other embodiments, the change is a decrease.

In other embodiments, the invention provides a method of optimizing therapeutic efficacy of an anti-cancer therapy that includes a VEGF antagonist (e.g., an anti-VEGF antibody, such as bevacizumab) and a PD-L1 axis binding antagonist (e.g., a PD-L1 binding antagonist, such as atezolizumab), the method involving determining the number of CD8⁺ T cells and/or CD68⁺/CD163⁺ macrophages in a tumor sample obtained from the patient, and comparing the number of CD8⁺ T cells and/or CD68⁺/CD163⁺ macrophages in the tumor sample with a reference sample, wherein determining the number of CD8⁺ T cells and/or CD68⁺/CD163⁺ macrophages in a tumor sample obtained from the patient, and comparing the number of CD8⁺ T cells and/or CD68⁺/CD163⁺ macrophages in the tumor sample with a reference sample identifies a patient who is likely to respond to the anti-cancer therapy. In some embodiments, the change is an increase. In other embodiments, the change is a decrease.

In still further embodiments, the invention provides methods of selecting an anti-cancer therapy comprising a VEGF antagonist (e.g., an anti-VEGF antibody, such as bevacizumab) and a PD-L1 axis binding antagonist (e.g., a PD-L1 binding antagonist, such as atezolizumab) for a patient having a cancer (e.g., kidney cancer), the method involving determining the number of CD8⁺ T cells and/or CD68⁺/CD163⁺ macrophages in a tumor sample obtained from the patient, comparing the number of CD8⁺ T cells and/or CD68⁺/CD163⁺ macrophages in the tumor sample with a reference sample, and selecting an anti-cancer therapy comprising a VEGF antagonist and a PD-L1 axis binding antagonist for the patient based on the number of CD8⁺ T cells and/or CD68⁺/CD163⁺ macrophages in the tumor sample with a reference sample. In some embodiments, a change in the number of CD8⁺ T cells and/or CD68⁺/CD163⁺ macrophages in the tumor sample with a reference sample is used to select the anti-cancer therapy. In some embodiments, the change is an increase. In other embodiments, the change is a decrease.

In some embodiments of any of the preceding methods, the tumor sample obtained from the patient has an increased number (e.g., by at least about 1.1-fold, about 1.2-fold, about 1.3-fold, about 1.4-fold, about 1.5-fold, about 1.6-fold, about 1.7-fold, about 1.8-fold, about 1.9-fold, about 2-fold, about 2.1-fold, about 2.2-fold, about 2.3-fold, about 2.4-fold, about 2.5-fold, about 2.6-fold, about 2.7-fold, about 2.8-fold, about 2.9-fold, about 3-fold, about 3.1-fold, about 3.2-fold, about 3.3-fold, about 3.4-fold, about 3.5-fold, about 3.6-fold, about 3.7-fold, about 3.8-fold, about 3.9-fold, about 4-fold, about 4.1-fold, about 4.2-fold, about 4.3-fold, about 4.4-fold, about 4.5-fold, about 4.6-fold, about 4.7-fold, about 4.8-fold, about 4.9-fold, about 5-fold, about 5.5-fold, about 6-fold, about 6.5-fold, about 7-fold, about 7.5-fold, about an 8-fold, about an 8.5-fold, about 9-fold, about 9.5-fold, about 10-fold, about an 11-fold, about 12-fold, about 13-fold, about 14-fold, about 15-fold, about 16-fold, about 17-fold, about 18-fold, about 19-fold, about 20-fold, about 30-fold, about 40-fold, about 50-fold, about 100-fold, about 500-fold, about 1,000-fold or greater) of CD8⁺ T cells and/or macrophages (e.g., CD68⁺/CD163⁺ macrophages) relative to the reference sample.

In some embodiments of any of the preceding methods, the biological sample from the patient is obtained about 2 to about 10 weeks (e.g., 2, 3, 4, 5, 6, 7, 8, 9, or 10 weeks) following administration of the anti-cancer therapy. In some embodiments, the biological sample from the patient is obtained about 4 to about 6 weeks following administration of the anti-cancer therapy.

In some embodiments of any of the preceding methods, the expression level or number of a biomarker is detected in a tissue sample, a primary or cultured cells or cell line, a cell supernatant, a cell lysate, platelets, serum, plasma, vitreous fluid, lymph fluid, synovial fluid, follicular fluid, seminal fluid, amniotic fluid, milk, whole blood, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, or any combination thereof.

For example, in some embodiments of any of the preceding methods, the expression level of a biomarker is detected in tumor-infiltrating immune cells, tumor cells, PBMCs, or combinations thereof using known techniques (e.g., flow cytometry or IHC). Tumor-infiltrating immune cells include, but are not limited to, intratumoral immune cells, peritumoral immune cells or any combinations thereof, and other tumor stroma cells (e.g., fibroblasts). Such tumor infiltrating immune cells may be T lymphocytes (such as CD8⁺ T lymphocytes (e.g., CD8⁺ T effector (T_{eff}) cells) and/or CD4⁺ T lymphocytes (e.g., CD4⁺ T_{eff} cells), B lymphocytes, or other bone marrow-lineage cells including granulocytes (neutrophils, eosinophils, basophils), monocytes, macrophages, dendritic cells (e.g., interdigitating dendritic cells), histiocytes, and natural killer (NK) cells. In some embodiments, the staining for a biomarker is detected as membrane staining, cytoplasmic staining, or combinations thereof. In other embodiments, the absence of a biomarker is detected as absent or no staining in the sample, relative to a reference sample.

In some embodiments of any of the preceding methods, the sample obtained from the patient may be selected from the group consisting of tissue, whole blood, plasma, serum, and combinations thereof. In some embodiments, the sample is a tissue sample. In some embodiments, the tissue sample is a tumor sample. In some embodiments, the tumor sample comprises tumor-infiltrating immune cells, tumor cells, stromal cells, or any combinations thereof. In any of the preceding embodiments, the tumor sample may be a formalin-fixed and paraffin-embedded (FFPE) tumor sample, an archival tumor sample, a fresh tumor sample, or a frozen tumor sample.

In particular embodiments of any of the preceding methods, the expression level of a biomarker is assessed in a biological sample that contains or is suspected to contain cancer cells. The sample may be, for example, a tissue biopsy or a metastatic lesion obtained from a patient suffering from, suspected to suffer from, or diagnosed with cancer (e.g., a kidney cancer, in particular renal cell carcinoma). In some embodiments, the sample is a sample of kidney tissue, a biopsy of an kidney tumor, a known or suspected metastatic kidney cancer lesion or section, or a blood sample, e.g., a peripheral blood sample, known or suspected to comprise circulating cancer cells, e.g., kidney cancer cells. The sample may comprise both cancer cells, i.e., tumor cells, and non-cancerous cells (e.g., lymphocytes, such as T cells or NK cells), and, in certain embodiments, comprises both cancerous and non-cancerous cells. Methods of obtaining biological samples including tissue resections, biopsies, and body fluids, e.g., blood samples comprising cancer/tumor cells, are well known in the art.

In some embodiments of any of the preceding methods, the sample obtained from the patient is collected after the beginning of an anti-cancer therapy, e.g., therapy for the treatment of cancer or the management or amelioration of a symptom thereof. Therefore, in some embodiments, the sample is collected after the administration of chemotherapeutics or the start of a chemotherapy regimen.

In some embodiments of any of the preceding methods, the patient has carcinoma, lymphoma, blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, melanoma, and leukemia or lymphoid malignancies. In some embodiments, the cancer is kidney cancer (e.g., renal cell carcinoma (RCC), e.g., metastatic RCC), squamous cell cancer (e.g., epithelial squamous cell cancer), lung cancer (including small-cell lung cancer (SCLC), non-small cell lung cancer (NSCLC), adenocarcinoma of the lung, and squamous carcinoma of the lung), cancer of the peritoneum, hepatocellular cancer, gastric or stomach cancer including gastrointestinal cancer, pancreatic cancer, glioblastoma, cervical cancer, ovarian cancer, liver cancer, hepatoma, breast cancer (including metastatic breast cancer), bladder cancer, colon cancer, rectal cancer, colorectal cancer, endometrial or uterine carcinoma, salivary gland carcinoma, prostate cancer, vulval cancer, thyroid cancer, hepatic carcinoma, anal carcinoma, penile carcinoma, Merkel cell cancer, mycoses fungoids, testicular cancer, esophageal cancer, tumors of the biliary tract, head and neck cancer, B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), abnormal vascular proliferation associated with phakomatoses, edema (such as that associated with brain tumors), or Meigs' syndrome. In preferred embodiments, the patient has a kidney cancer (e.g., RCC, e.g., mRCC). The patient may optionally have an advanced, refractory, recurrent, chemotherapy-resistant, and/or platinum-resistant form of the cancer.

In certain embodiments, the presence and/or expression levels/amount of a biomarker in a first sample is increased or elevated as compared to presence/absence and/or expression levels/amount in a second sample. In certain embodiments, the presence/absence and/or expression levels/amount of a biomarker in a first sample is decreased or reduced as compared to presence and/or expression levels/amount in a second sample. In certain embodiments, the second sample is a reference sample, reference cell, reference tissue, control sample, control cell, or control tissue.

In certain embodiments, a reference sample, reference cell, reference tissue, control sample, control cell, or control tissue is a single sample or combined multiple samples from the same patient or individual that are obtained at one or more different time points than when the test sample is obtained. For example, a reference sample, reference cell, reference tissue, control sample, control cell, or control tissue is obtained at an earlier time point from the same patient or individual than when the test sample is obtained. Such reference sample, reference cell, reference tissue, control sample, control cell, or control tissue may be useful if the reference sample is obtained during initial diagnosis of cancer and the test sample is later obtained when the cancer becomes metastatic.

In certain embodiments, a reference sample, reference cell, reference tissue, control sample, control cell, or control tissue is a combined multiple samples from one or more healthy individuals who are not the patient. In certain embodiments, a reference sample, reference cell, reference tissue, control sample, control cell, or control tissue is a combined multiple samples from one or more individuals with a disease or disorder (e.g., cancer) who are not the patient or individual. In certain embodiments, a reference sample, reference cell, reference tissue, control sample, control cell, or control tissue is pooled RNA samples from normal tissues or pooled plasma or serum samples from one or more individuals who are not the patient. In certain embodiments, a reference sample, reference cell, reference tissue, control sample, control cell, or control tissue is pooled RNA samples from tumor tissues or pooled plasma or serum samples from one or more individuals with a disease or disorder (e.g., cancer) who are not the patient.

In some embodiments of any of the preceding methods, elevated or increased expression or number refers to an overall increase of about any of 10%, 20%, 30%, 40%, 50%, 60%, 70%, 80%, 90%, 95%, 96%, 97%, 98%, 99% or greater, in the level or number of a biomarker (e.g., protein, nucleic acid (e.g., gene or mRNA), or cell), detected by methods such as those described herein and/or known in the art, as compared to a reference sample, reference cell, reference tissue, control sample, control cell, or control tissue. In certain embodiments, the elevated expression or number refers to the increase in expression level/amount of a biomarker in the sample wherein the increase is at least about any of 1.1x, 1.2x, 1.3x, 1.4x, 1.5x, 1.6x, 1.7x, 1.8x, 1.9x, 2x, 2.1x, 2.2x, 2.3x, 2.4x, 2.5x, 2.6x, 2.7x, 2.8x, 2.9x, 3x, 3.5x, 4x, 4.5x, 5x, 6x, 7x, 8x, 9x, 10x, 15x, 20x, 30x, 40x, 50x, 100x, 500x, or 1000x the expression level/amount of the respective biomarker in a reference sample, reference cell, reference tissue, control sample, control cell, or control tissue. In some embodiments, elevated expression or number refers to an overall increase of greater than about 1.1-fold, about 1.2-fold, about 1.3-fold, about 1.4-fold, about 1.5-fold, about 1.6-fold, about 1.7-fold, about 1.8-fold, about 1.9-fold, about 2-fold, about 2.1-fold, about 2.2-fold, about 2.3-fold, about 2.4-fold, about 2.5-fold, about 2.6-fold, about 2.7-fold, about 2.8-fold, about 2.9-fold, about 3-fold, about 3.5-fold, about 4-fold, about 4.5-fold, about 5-fold, about 6-fold, about 7-fold, about 8-fold, about 9-fold, about 10-fold, about 15-fold, about 20-fold, about 30-fold, about 40-fold, about 50-fold, about 100-fold, about 500-fold, about 1,000-fold or greater as compared to a reference sample, reference cell, reference tissue, control sample, control cell, control tissue, or internal control (e.g., housekeeping gene).

In some embodiments of any of the preceding methods, reduced expression or number refers to an overall reduction of about any of 10%, 20%, 30%, 40%, 50%, 60%, 70%, 80%, 90%, 95%, 96%, 97%, 98%, 99% or greater, in the level of biomarker (e.g., protein, nucleic acid (e.g., gene or mRNA), or cell), detected by standard art known methods such as those described herein, as compared to a reference sample, reference cell, reference tissue, control sample, control cell, or control tissue. In certain embodiments, reduced expression or number refers to the decrease in expression level/amount of a biomarker in the sample wherein the decrease is at least about any of 0.9x, 0.8x, 0.7x, 0.6x, 0.5x, 0.4x, 0.3x, 0.2x, 0.1x, 0.05x, or 0.01x the expression level/amount of the respective biomarker in a reference sample, reference cell, reference tissue, control sample, control cell, or control tissue. In some embodiments, a decreased expression level of a biomarker listed above following administration of an anti-cancer therapy including a VEGF antagonist and a PD-L1 axis binding antagonist indicates that the patient is not responding appropriately to the anti-cancer therapy.

### B. Methods of Treatment

The present invention provides methods for treating a patient having a cancer (e.g., a kidney cancer, such as RCC, e.g., mRCC). In some instances, the methods of the invention include administering to the patient an anti-cancer therapy that includes a VEGF antagonist and a PD-L1 axis binding antagonist based on the expression level of a biomarker of the invention. Any of the VEGF antagonists, PD-L1 axis binding antagonists, or other anti-cancer agents described herein (e.g., as described in the "Compositions" or "Examples" sections) or known in the art may be used in the methods.

In some instances, the methods involve determining the presence and/or expression level of a biomarker (e.g., an immunological biomarker, such as a biomarker listed in Table 2) in a biological sample obtained from the patient at a time point following administration of the anti-cancer therapy, comparing the expression level of the one or more of the genes in the biological sample with a reference level, and continuing to administer the anti-cancer therapy to the patient if the expression level is increased relative to the expression level. Gene expression levels can be determined or compared using any of the methods described herein or known in the art. The invention further relates to methods for improving progression-free survival (PFS) and/or overall survival (OS) of a patient suffering from kidney cancer (e.g., RCC, e.g., mRCC) by administration of an anti-cancer therapy that includes a VEGF antagonist (e.g., an anti-VEGF antibody, such as bevacizumab) and a PD-L1 axis binding antagonist (e.g., a PD-L1 binding antagonist, such as atezolizumab). The expression level or number of any of the biomarkers described herein may be determined using any method known in the art and/or described herein, for example, in Section A above and/or in the working Examples.

In some embodiments, the invention provides a method of treating a patient having a cancer (e.g., a kidney cancer) with an anti-cancer therapy comprising a VEGF antagonist (e.g., an anti-VEGF antibody, such as bevacizumab) and a PD-L1 axis binding antagonist (e.g., a PD-L1 binding antagonist, such as atezolizumab), by determining, in a biological sample obtained from the patient, the expression level of one or more (e.g., 1, 2, 3, 4, 5, 6, 7, 8, 9, 10, 11, 12, or 13) of CD8A, CD8B, EOMES, GZMA, GZMB, IFNG, PRF1, CXCL9, CXCL10, CXCL11, CXCL13, KLRK1, SLAMF7, comparing the expression level of the one or more of CD8A, CD8B, EOMES, GZMA, GZMB, IFNG, PRF1, CXCL9, CXCL10, CXCL11, CXCL13, KLRK1, SLAMF7 with a reference level, and administering the anti-cancer therapy to the patient if the expression level of their one or more genes is changed (e.g., increased or decreased) in the patient's sample relative to the reference level. In some embodiments, the anti-cancer therapy is administered to the patient if the expression level of their one or more genes is increased relative to the reference level.

In other embodiments, the invention provides a method of treating a patient having a cancer (e.g., a kidney cancer) with an anti-cancer therapy comprising a VEGF antagonist (e.g., an anti-VEGF antibody, such as bevacizumab) and a PD-L1 axis binding antagonist (e.g., a PD-L1 binding antagonist, such as atezolizumab), by determining, in a biological sample obtained from the patient at a time point following administration of the anti-cancer therapy, the expression level of one or more (e.g., 1, 2, 3, 4, 5, 6, 7, 8, 9, 10, 11, 12, or 13) of CD8A, CD8B, EOMES, GZMA, GZMB, IFNG, PRF1, CXCL9, CXCL10, CXCL11, CXCL13, KLRK1, SLAMF7, and comparing the expression level of the one or more (e.g., 1, 2, 3, 4, 5, 6, 7, 8, 9, 10, 11, 12, or 13) of CD8A, CD8B, EOMES, GZMA, GZMB, IFNG, PRF1, CXCL9, CXCL10, CXCL11, CXCL13, KLRK1, SLAMF7 with a reference level, and continuing to administer the anti-cancer therapy to the patient if the expression level of their one or more genes is increased relative to the reference level.

In certain embodiments, the expression level of one or more (e.g., 1, 2, 3, 4, 5, 6, or 7) of CD8A, CD8B, EOMES, GZMA, GZMB, IFNG, or PRF1 is determined. In some embodiments, the expression level of one or more (e.g., 1, 2, 3, 4, 5, 6, or 7) of CD8A, CD8B, EOMES, GZMA, GZMB, IFNG, or PRF1 is correlated with the presence of CD8⁺ T_{eff} cells in the tumor microenvironment. In certain embodiments, the expression level of one or more (1, 2, 3, or 4) of CXCL9, CXCL10, CXCL11, or CXCL13 is determined. In some embodiments, the expression level of one or more (1, 2, 3, or 4) of CXCL9, CXCL10, CXCL11, or CXCL13 is correlated with the presence of Th1 chemokines in the tumor microenvironment. In some embodiments, the presence of one or more (e.g., 1, 2, or 3) of GZMB, KLRK1, or SLAMF7 is determined. In some embodiments, the presence of one or more (e.g., 1, 2, or 3) of GZMB, KLRK1, or SLAMF7 is correlated with the presence of natural killer (NK) cells in the tumor microenvironment.

In certain embodiments, the expression level of one or more (e.g., 1, 2, 3, 4, 5, 6, or 7) of CD8A, CD8B, EOMES, GZMA, GZMB, IFNG, or PRF1 is determined. In some embodiments, the expression level of at least 2, at least 3, at least 4, at least 5, or at least 6 of CD8A, CD8B, EOMES, GZMA, GZMB, IFNG, or PRF1 is determined. In some embodiments, the expression level of CD8A, CD8B, EOMES, GZMA, GZMB, IFNG, and PRF1 is determined. In some embodiments, the level of CD8A is between about 1-fold and about 60-fold (e.g., about 1-fold, about 2-fold, about 3-fold, about 4-fold, about 5-fold, about 10-fold, about 15-fold, about 17-fold, about 20-fold, about 25-fold, about 30-fold, about 35-fold, about 40-fold, about 45-fold, about 50-fold, about 55-fold, about 60-fold, or about 1-fold to about 50-fold) increased compared to a reference level. In some embodiments, the level of CD8A is about 50-fold or greater (e.g., about 50-fold, 100-fold, 200-fold, 250-fold, 500-fold, 1000-fold, or greater) increased compared to a reference level. In some embodiments, the level of CD8B is between about 1-fold and about 60-fold (e.g., about 1-fold, about 2-fold, about 3-fold, about 4-fold, about 5-fold, about 10-fold, about 15-fold, about 17-fold, about 20-fold, about 25-fold, about 30-fold, about 35-fold, about 40-fold, about 45-fold, about 50-fold, about 55-fold, about 60-fold, or about 1-fold to about 50-fold) increased compared to a reference level. In some embodiments, the level of CD8B is about 50-fold or greater (e.g., about 50-fold, 100-fold, 200-fold, 250-fold, 500-fold, 1000-fold, or greater) increased compared to a reference level. In some embodiments, the level of is between about 1-fold and about 60-fold (e.g., about 1-fold, about 2-fold, about 3-fold, about 4-fold, about 5-fold, about 10-fold, about 15-fold, about 17-fold, about 20-fold, about 25-fold, about 30-fold, about 35-fold, about 40-fold, about 45-fold, about 50-fold, about 55-fold, about 60-fold, or about 1-fold to about 50-fold) increased compared to a reference level. In some embodiments, the level of EOMES is about 50-fold or greater (e.g., about 50-fold, 100-fold, 200-fold, 250-fold, 500-fold, 1000-fold, or greater) increased compared to a reference level. In some embodiments, the level of GZMA is between about 1-fold and about 60-fold (e.g., about 1-fold, about 2-fold, about 3-fold, about 4-fold, about 5-fold, about 10-fold, about 15-fold, about 17-fold, about 20-fold, about 25-fold, about 30-fold, about 35-fold, about 40-fold, about 45-fold, about 50-fold, about 55-fold, about 60-fold, or about 1-fold to about 50-fold) increased compared to a reference level. In some embodiments, the level of GZMA is about 50-fold or greater (e.g., about 50-fold, 100-fold, 200-fold, 250-fold, 500-fold, 1000-fold, or greater) increased compared to a reference level. In some embodiments, the level of GZMB is between about 1-fold and about 60-fold (e.g., about 1-fold, about 2-fold, about 3-fold, about 4-fold, about 5-fold, about 10-fold, about 15-fold, about 17-fold, about 20-fold, about 25-fold, about 30-fold, about 35-fold, about 40-fold, about 45-fold, about 50-fold, about 55-fold, about 60-fold, or about 1-fold to about 50-fold) increased compared to a reference level. In some embodiments, the level of GZMB is about 50-fold or greater (e.g., about 50-fold, 100-fold, 200-fold, 250-fold, 500-fold, 1000-fold, or greater) increased compared to a reference level. In some embodiments, the level of IFNG is between about 1-fold and about 60-fold (e.g., about 1-fold, about 2-fold, about 3-fold, about 4-fold, about 5-fold, about 10-fold, about 15-fold, about 17-fold, about 20-fold, about 25-fold, about 30-fold, about 35-fold, about 40-fold, about 45-fold, about 50-fold, about 55-fold, about 60-fold, or about 1-fold to about 50-fold) increased compared to a reference level. In some embodiments, the level of IFNG is about 50-fold or greater (e.g., about 50-fold, 100-fold, 200-fold, 250-fold, 500-fold, 1000-fold, or greater) increased compared to a reference level. In some embodiments, the level of PRF1 is between about 1-fold and about 60-fold (e.g., about 1-fold, about 2-fold, about 3-fold, about 4-fold, about 5-fold, about 10-fold, about 15-fold, about 17-fold, about 20-fold, about 25-fold, about 30-fold, about 35-fold, about 40-fold, about 45-fold, about 50-fold, about 55-fold, about 60-fold, or about 1-fold to about 50-fold) increased compared to a reference level. In some embodiments, the level of PRF1 is about 50-fold or greater (e.g., about 50-fold, 100-fold, 200-fold, 250-fold, 500-fold, 1000-fold, or greater) increased compared to a reference level.

In certain embodiments, the expression level of one or more of (e.g., 1, 2, 3, or 4) of CXCL9, CXCL10, CXCL11, or CXCL13 is determined. In some embodiments, the expression level of at least 2 or at least 3 of CXCL9, CXCL10, CXCL11, or CXCL13 is determined. In some embodiments, the expression level of CXCL9, CXCL10, CXCL11, and CXCL13 is determined. In some embodiments, the level of CXCL9 is between about 1-fold and about 300-fold (e.g., about 1-fold, about 2-fold, about 3-fold, about 4-fold, about 5-fold, about 10-fold, about 20-fold, about 30-fold, about 50-fold, about 60-fold, about 70-fold, about 80-fold, about 90-fold, about 100-fold, about 150-fold, about 200-fold, about 250-fold, about 3-fold to about 250-fold, or about 1-fold to about 80-fold) increased compared to a reference level. In some embodiments, the level of CXCL9 is about 50-fold or greater (e.g., about 50-fold, 80-fold, 100-fold, 150-fold, 200-fold, 250-fold, 1000-fold, or greater) increased compared to a reference level. In some embodiments, the level of CXCL10 is between about 1-fold and about 300-fold (e.g., about 1-fold, about 2-fold, about 3-fold, about 4-fold, about 5-fold, about 10-fold, about 20-fold, about 30-fold, about 50-fold, about 60-fold, about 70-fold, about 80-fold, about 90-fold, about 100-fold, about 150-fold, about 200-fold, about 250-fold, about 3-fold to about 250-fold, or about 1-fold to about 80-fold) increased compared to a reference level. In some embodiments, the level of CXCL10 is about 50-fold or greater (e.g., about 50-fold, 80-fold, 100-fold, 150-fold, 200-fold, 250-fold, 1000-fold, or greater) increased compared to a reference level. In some embodiments, the level of CXCL11 is between about 1-fold and about 300-fold (e.g., about 1-fold, about 2-fold, about 3-fold, about 4-fold, about 5-fold, about 10-fold, about 20-fold, about 30-fold, about 50-fold, about 60-fold, about 70-fold, about 80-fold, about 90-fold, about 100-fold, about 150-fold, about 200-fold, about 250-fold, about 3-fold to about 250-fold, or about 1-fold to about 80-fold) increased compared to a reference level. In some embodiments, the level of CXCL11 is about 50-fold or greater (e.g., about 50-fold, 80-fold, 100-fold, 150-fold, 200-fold, 250-fold, 1000-fold, or greater) increased compared to a reference level. In some embodiments, the level of CXCL13 is between about 1-fold and about 300-fold (e.g., about 1-fold, about 2-fold, about 3-fold, about 4-fold, about 5-fold, about 10-fold, about 20-fold, about 30-fold, about 50-fold, about 60-fold, about 70-fold, about 80-fold, about 90-fold, about 100-fold, about 150-fold, about 200-fold, about 250-fold, about 3-fold to about 250-fold, or about 1-fold to about 80-fold) increased compared to a reference level. In some embodiments, the level of CXCL13 is about 50-fold or greater (e.g., about 50-fold, 80-fold, 100-fold, 150-fold, 200-fold, 250-fold, 1000-fold, or greater) increased compared to a reference level.

In some embodiments, the expression level of one or more (e.g., 1, 2, or 3) of GZMB, KLRK1, or SLAMF7 is determined. In some embodiments, the expression level of at least 2 of GZMB, KLRK1, or SLAMF7 is determined. In some embodiments, the expression level of GZMB, KLRK1, and SLAMF7 is determined. In some embodiments, the level of GZMB is between about 1-fold and about 20-fold (e.g., about 1-fold, about 2-fold, about 3-fold, about 4-fold, about 5-fold, about 6-fold, about 7-fold, about 8-fold, about 9-fold, about 10-fold, about 12-fold, about 13-fold, about 15-fold, about 20-fold, between about 1-fold and about 8-fold, or between about 1-fold and about 13-fold) increased compared to a reference level. In some embodiments, the level of GZMB is about 8-fold or greater (e.g., about 8-fold, 9-fold, 10-fold, 13-fold, 15-fold, 20-fold, 50-fold, 100-fold, 1000-fold, or greater) increased compared to a reference level. In some embodiments, the level of KLRK1 is between about 1-fold and about 20-fold (e.g., about 1-fold, about 2-fold, about 3-fold, about 4-fold, about 5-fold, about 6-fold, about 7-fold, about 8-fold, about 9-fold, about 10-fold, about 12-fold, about 13-fold, about 15-fold, about 20-fold, between about 1-fold and about 8-fold, or between about 1-fold and about 13-fold) increased compared to a reference level. In some embodiments, the level of KLRK1 is about 8-fold or greater (e.g., about 8-fold, 9-fold, 10-fold, 13-fold, 15-fold, 20-fold, 50-fold, 100-fold, 1000-fold, or greater) increased compared to a reference level. In some embodiments, the level of SLAMF7 is between about 1-fold and about 20-fold (e.g., about 1-fold, about 2-fold, about 3-fold, about 4-fold, about 5-fold, about 6-fold, about 7-fold, about 8-fold, about 9-fold, about 10-fold, about 12-fold, about 13-fold, about 15-fold, about 20-fold, between about 1-fold and about 8-fold, or between about 1-fold and about 13-fold) increased compared to a reference level. In some embodiments, the level of SLAMF7 is about 8-fold or greater (e.g., about 8-fold, 9-fold, 10-fold, 13-fold, 15-fold, 20-fold, 50-fold, 100-fold, 1000-fold, or greater) increased compared to a reference level.

In some embodiments, the expression level of one or more (e.g., 1, 2, 3, 4, 5, 6, 7, 8, 9, 10, 11, 12, or 13) of CD8A, CD8B, EOMES, GZMA, GZMB, IFNG, PRF1, CXCL9, CXCL10, CXCL11, CXCL13, KLRK1, and/or SLAMF7 in the biological sample obtained from the patient is increased (e.g., by about 1.1-fold, about 1.2-fold, about 1.3-fold, about 1.4-fold, about 1.5-fold, about 1.6-fold, about 1.7-fold, about 1.8-fold, about 1.9-fold, about 2-fold, about 2.1-fold, about 2.2-fold, about 2.3-fold, about 2.4-fold, about 2.5-fold, about 3-fold, about 3.5-fold, about 4-fold, about 4.5-fold, about 5-fold, about 5.5-fold, about 6-fold, about 6.5-fold, about 7-fold, about 7.5-fold, about 8-fold, about 8.5-fold, about 9-fold, about 9.5-fold, about 10-fold, about 11-fold, about 12-fold, about 13-fold, about 14-fold, about 15-fold, about 16-fold, about 17-fold, about 18-fold, about 19-fold, about 20-fold, about 30-fold, about 40-fold, about 50-fold, about 100-fold, about 500-fold, about 1,000-fold or greater) relative to the reference level.

In some embodiments, a reference level is the expression level of the one or more genes (e.g., CD8A, CD8B, EOMES, GZMA, GZMB, IFNG, PRF1, CXCL9, CXCL10, CXCL11, CXCL13, KLRK1, and/or SLAMF7) in a biological sample from the patient obtained prior to (e.g., minutes, hours, days, weeks (e.g., 1, 2, 3, 4, 5, 6, or 7 weeks), months, or years prior to) administration of the anti-cancer therapy. In certain embodiments, a reference level is the expression level of the one or more genes in a reference population. In certain embodiments, the reference level is a pre-assigned expression level for the one or more genes. In some embodiments, the reference level is the expression level of the one or more genes in a biological sample obtained from the patient at a previous time point, wherein the previous time point is following administration of the anti-cancer therapy. In other embodiments, the reference level is the expression level of the one or more genes in a biological sample obtained from the patient at a subsequent time point (e.g., minutes, hours, days, weeks, months, or years after administration of an anti-cancer therapy).

In some embodiments, the invention provides a method of treating a patient having a cancer (e.g., a kidney cancer), the method involving determining the expression level of MHC-I in a biological sample (e.g., a tumor sample) obtained from the patient at a time point, comparing the expression level of MHC-I with a reference level, and administering the anti-cancer therapy to the patient if the expression level of MHC-I is changed (e.g., increased or decreased) in the patient's sample relative to the reference level. In some embodiments, the anti-cancer therapy is administered to the patient if the expression level of MHC-I is increased in the patient's sample relative to the reference level.

In some embodiments the invention provides a method of treating a patient having a cancer (e.g., a kidney cancer) the method involving determining the expression level of MHC-I in a biological sample (e.g., a tumor sample) obtained from the patient at a time point following administration of the anti-cancer therapy, comparing the expression level of MHC-I with a reference level, and continuing to administer the anti-cancer therapy to the patient if the expression level of MHC-I is increased. In some embodiments, a reference level is the expression level of MHC-I in a biological sample from the patient obtained prior to (e.g., minutes, hours, days, weeks (e.g., 1, 2, 3, 4, 5, 6, or 7 weeks), months, or years prior to) administration of the anti-cancer therapy. In other embodiments, a reference level is the expression level of MHC-I in a reference population. In other embodiments, the reference level is a pre-assigned expression level for MHC-I. In other embodiments, the reference level is the expression level of MHC-I in a biological sample obtained from the patient at a previous time point, wherein the previous time point is following administration of the anti-cancer therapy. In other embodiments, the reference level is the expression level of MHC-I in a biological sample obtained from the patient at a subsequent time point (e.g., minutes, hours, days, weeks, months, or years after administration of an anti-cancer therapy).

In some embodiments, the expression level of MHC-I is increased (e.g., about 1.1-fold, about 1.2-fold, about 1.3-fold, about 1.4-fold, about 1.5-fold, about 1.6-fold, about 1.7-fold, about 1.8-fold, about 1.9-fold, about 2-fold, about 2.1-fold, about 2.2-fold, about 2.3-fold, about 2.4-fold, about 2.5-fold, about 2.6-fold, about 2.7-fold, about 2.8-fold, about 2.9-fold, about 3-fold, about 3.1-fold, about 3.2-fold, about 3.3-fold, about 3.4-fold, about 3.5-fold, about 3.6-fold, about 3.7-fold, about 3.8-fold, about 3.9-fold, about 4-fold, about 4.1-fold, about 4.2-fold, about 4.3-fold, about 4.4-fold, about 4.5-fold, about 4.6-fold, about 4.7-fold, about 4.8-fold, about 4.9-fold, about 5-fold, about 5.5-fold, about 6-fold, about 6.5-fold, about 7-fold, about 7.5-fold, about an 8-fold, about an 8.5-fold, about 9-fold, about 9.5-fold, about 10-fold, about an 11-fold, about 12-fold, about 13-fold, about 14-fold, about 15-fold, about 16-fold, about 17-fold, about 18-fold, about 19-fold, about 20-fold, about 30-fold, about 40-fold, about 50-fold, about 100-fold, about 500-fold, about 1,000-fold or greater) relative to the reference level. In some embodiments, the expression level of MHC-I is increased at least 2-fold relative to the reference level.

In some embodiments, the invention provides a method of treating a cancer patient (e.g., kidney cancer), the method involving determining the expression level of one or more (e.g., 1, 2, 3, 4, 5, 6, or 7) of CX3CR1, CCL2, CCL5, CCR7, CX3CL1, CCR7, or CXCL10 in a biological sample obtained from the patient, comparing the expression level with a reference level, and administering the anti-cancer therapy to the patient if the expression level is changed (e.g., increased or decreased) relative to the reference level. In some embodiments, the anti-cancer therapy is administered to the patient if the expression level of the one or more genes is increased in the patient's sample relative to the reference level. In some embodiments, the expression level of at least 2, at least 3, at least 4, at least 5, or at least 6 of CX3CR1, CCL2, CCL5, CCR5, CX3CL1, CCR7, or CXCL10 is determined. In some embodiments, the expression level of CX3CR1, CCL2, CCL5, CCR5, CX3CL1, CCR7, and CXCL10 is determined.

In other embodiments, the invention provides a method of treating a cancer patient (e.g., kidney cancer), the method involving determining the expression level of one or more (e.g., 1, 2, 3, 4, 5, 6, or 7) of CX3CR1, CCL2, CCL5, CCR7, CX3CL1, CCR7, or CXCL10 in a biological sample obtained from the patient at a time point following administration of the anti-cancer therapy, comparing the expression level with a reference level, and continuing to administer the anti-cancer therapy to the patient if the expression level is increased relative to the reference level. In some embodiments, the expression level of at least 2, at least 3, at least 4, at least 5, or at least 6 of CX3CR1, CCL2, CCL5, CCR5, CX3CL1, CCR7, or CXCL10 is determined. In some embodiments, the expression level of CX3CR1, CCL2, CCL5, CCR5, CX3CL1, CCR7, and CXCL10 is determined.

In some embodiments, the reference level is the expression level of the one or more genes (e.g., CX3CR1, CCL2, CCL5, CCR5, CX3CL1, CCR7, and/or CXCL10) in a biological sample from the patient obtained prior to (e.g., minutes, hours, days, weeks, months, or years prior to) administration of the anti-cancer therapy. In other embodiments, the reference level is the expression level of the one or more genes in a reference population. In other embodiments, the reference level is a pre-assigned expression level for the one or more genes. In other embodiments, the reference level is the expression level of the one or more genes in a biological sample obtained from the patient at a previous time point, wherein the previous time point is following administration of the anti-cancer therapy. In other embodiments, the reference level is the expression level of the one or more genes in a biological sample obtained from the patient at a subsequent time point.

In some embodiments, the expression level of one or more (e.g., 1, 2, 3, 4, 5, 6, or 7) of CX3CR1, CCL2, CCL5, CCR5, CX3CL1, CCR7, and/or CXCL10 in the biological sample obtained from the patient is increased (e.g., by about 1.1-fold, about 1.2-fold, about 1.3-fold, about 1.4-fold, about 1.5-fold, about 1.6-fold, about 1.7-fold, about 1.8-fold, about 1.9-fold, about 2-fold, about 2.1-fold, about 2.2-fold, about 2.3-fold, about 2.4-fold, about 2.5-fold, about 3-fold, about 3.5-fold, about 4-fold, about 4.5-fold, about 5-fold, about 5.5-fold, about 6-fold, about 6.5-fold, about 7-fold, about 7.5-fold, about 8-fold, about 8.5-fold, about 9-fold, about 9.5-fold, about 10-fold, about 11-fold, about 12-fold, about 13-fold, about 14-fold, about 15-fold, about 16-fold, about 17-fold, about 18-fold, about 19-fold, about 20-fold, about 30-fold, about 40-fold, about 50-fold, about 100-fold, about 500-fold, about 1,000-fold or greater) relative to the reference level.

In another aspect, the invention provides a method of treating a patient having a cancer (e.g., kidney cancer), the method involving determining the number of T cells (e.g., CD8⁺ T cells, e.g., CD8⁺ T_{eff} cells) and/or macrophages (e.g., CD68⁺, CD168⁺, or CD68⁺CD168⁺ macrophages) in a tumor sample obtained from the patient, comparing the number of T cells (e.g., CD8⁺ T cells, e.g., CD8⁺ T_{eff} cells) and/or macrophages (e.g., CD68⁺, CD168⁺, or CD68⁺CD168⁺ macrophages) in a tumor sample with the number of T cells (e.g., CD8⁺ T cells, e.g., CD8⁺ T_{eff} cells) and/or macrophages (e.g., CD68⁺, CD168⁺, or CD68+CD168+ macrophages) in a reference sample, and administering an anti-cancer therapy comprising a VEGF antagonist and a PD-L1 axis binding antagonist to the patient if the number of T cells (e.g., CD8⁺ T cells, e.g., CD8⁺ T_{eff} cells) and/or macrophages (e.g., CD68⁺, CD168⁺, or CD68⁺CD168⁺ macrophages) in the patient's sample is changed (e.g., increased or decreased) relative to the reference sample. In some embodiments, the anti-cancer therapy is administered to the patient if the number of T cells (e.g., CD8⁺ T cells, e.g., CD8⁺ T_{eff} cells) and/or macrophages (e.g., CD68⁺, CD168⁺, or CD68⁺CD168⁺ macrophages) in the biological sample obtained from the patient is increased relative to the reference level.

In yet another aspect, the invention provides a method of treating a patient having a cancer (e.g., kidney cancer) by determining the number of T cells (e.g., CD8⁺ T cells, e.g., CD8⁺ T_{eff} cells) and/or macrophages (e.g., CD68⁺, CD168⁺, or CD68⁺CD168⁺ macrophages) in a tumor sample obtained from the patient at a time point following administration of an anti-cancer agent, comparing the number of T cells (e.g., CD8⁺ T cells, e.g., CD8⁺ T_{eff} cells) and/or macrophages (e.g., CD68⁺, CD168⁺, or CD68⁺CD168⁺ macrophages) in a tumor sample with the number of T cells (e.g., CD8⁺ T cells, e.g., CD8⁺ T_{eff} cells) and/or macrophages (e.g., CD68⁺, CD168⁺, or CD68⁺CD168⁺ macrophages) in a reference sample, and continuing to administer the anti-cancer therapy to the patient if the number of T cells (e.g., CD8+ T cells, e.g., CD8⁺ T_{eff} cells) and/or macrophages (e.g., CD68⁺, CD168⁺, or CD68⁺CD168⁺ macrophages) in the patient's sample is increased relative to the reference sample. In some embodiments, the anti-cancer agent comprises a VEGF antagonist and a PD-L1 axis binding antagonist.

In some embodiments of any of the preceding methods, the tumor sample obtained from the patient has an increased number (e.g., by at least about 1.1-fold, about 1.2-fold, about 1.3-fold, about 1.4-fold, about 1.5-fold, about 1.6-fold, about 1.7-fold, about 1.8-fold, about 1.9-fold, about 2-fold, about 2.1-fold, about 2.2-fold, about 2.3-fold, about 2.4-fold, about 2.5-fold, about 2.6-fold, about 2.7-fold, about 2.8-fold, about 2.9-fold, about 3-fold, about 3.1-fold, about 3.2-fold, about 3.3-fold, about 3.4-fold, about 3.5-fold, about 3.6-fold, about 3.7-fold, about 3.8-fold, about 3.9-fold, about 4-fold, about 4.1-fold, about 4.2-fold, about 4.3-fold, about 4.4-fold, about 4.5-fold, about 4.6-fold, about 4.7-fold, about 4.8-fold, about 4.9-fold, about 5-fold, about 5.5-fold, about 6-fold, about 6.5-fold, about 7-fold, about 7.5-fold, about an 8-fold, about an 8.5-fold, about 9-fold, about 9.5-fold, about 10-fold, about an 11-fold, about 12-fold, about 13-fold, about 14-fold, about 15-fold, about 16-fold, about 17-fold, about 18-fold, about 19-fold, about 20-fold, about 30-fold, about 40-fold, about 50-fold, about 100-fold, about 500-fold, about 1,000-fold or greater) of CD8⁺ T cells and/or macrophages relative to the reference sample.

In some embodiments of any of the preceding methods, therapy with a VEGF antagonist (e.g., an anti-VEGF antibody, such as bevacizumab) in combination with a PD-L1 axis binding antagonist (e.g., a PD-L1 binding antagonist, such as atezolizumab) preferably extends and/or improves survival, including progression free survival (PFS) and/or overall survival (OS). In one embodiment, therapy with the VEGF antagonist (e.g., an anti-VEGF antibody, such as bevacizumab) in combination with a PD-L1 axis binding antagonist (e.g., a PD-L1 binding antagonist, such as atezolizumab) extends survival by at least about 10%, 20%, 30%, 40%, 50%, 60%, 70%, 80%, 90%, 100%, 200%, or more, relative to the survival achieved by administering an approved anti-tumor agent, or standard of care, for the cancer being treated.

In some embodiments of any of the preceding methods, the expression level or number of a biomarker is detected in a tissue sample, a primary or cultured cells or cell line, a cell supernatant, a cell lysate, platelets, serum, plasma, vitreous fluid, lymph fluid, synovial fluid, follicular fluid, seminal fluid, amniotic fluid, milk, whole blood, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, or any combination thereof.

For example, in some embodiments of any of the preceding methods, the expression level of a biomarker is detected in tumor-infiltrating immune cells, tumor cells, PBMCs, or combinations thereof using known techniques (e.g., flow cytometry or IHC). Tumor-infiltrating immune cells include, but are not limited to, intratumoral immune cells, peritumoral immune cells or any combinations thereof, and other tumor stroma cells (e.g., fibroblasts). Such tumor infiltrating immune cells may be T lymphocytes (such as CD8⁺ T lymphocytes (e.g., CD8⁺ T effector (T_{eff}) cells) and/or CD4⁺ T lymphocytes (e.g., CD4⁺ T_{eff} cells), B lymphocytes, or other bone marrow-lineage cells including granulocytes (neutrophils, eosinophils, basophils), monocytes, macrophages, dendritic cells (e.g., interdigitating dendritic cells), histiocytes, and natural killer (NK) cells. In some embodiments, the staining for a biomarker is detected as membrane staining, cytoplasmic staining, or combinations thereof. In other embodiments, the absence of a biomarker is detected as absent or no staining in the sample, relative to a reference sample.

In some embodiments of any of the preceding methods, the sample obtained from the patient may be selected from the group consisting of tissue, whole blood, plasma, serum, and combinations thereof. In some embodiments, the sample is a tissue sample. In some embodiments, the tissue sample is a tumor sample. In some embodiments, the tumor sample comprises tumor-infiltrating immune cells, tumor cells, stromal cells, or any combinations thereof. In any of the preceding embodiments, the tumor sample may be a formalin-fixed and paraffin-embedded (FFPE) tumor sample, an archival tumor sample, a fresh tumor sample, or a frozen tumor sample.

In particular embodiments of any of the preceding methods, the expression level or number of a biomarker is assessed in a biological sample that contains or is suspected to contain cancer cells. The sample may be, for example, a tissue biopsy or a metastatic lesion obtained from a patient suffering from, suspected to suffer from, or diagnosed with cancer (e.g., a kidney cancer, in particular renal cell carcinoma). In some embodiments, the sample is a sample of kidney tissue, a biopsy of an kidney tumor, a known or suspected metastatic kidney cancer lesion or section, or a blood sample, e.g., a peripheral blood sample, known or suspected to comprise circulating cancer cells, e.g., kidney cancer cells. The sample may comprise both cancer cells, i.e., tumor cells, and non-cancerous cells (e.g., lymphocytes, such as T cells or NK cells), and, in certain embodiments, comprises both cancerous and non-cancerous cells. Methods of obtaining biological samples including tissue resections, biopsies, and body fluids, e.g., blood samples comprising cancer/tumor cells, are well known in the art.

In some embodiments, the sample obtained from the patient is collected after the beginning of an anti-cancer therapy, e.g., therapy for the treatment of cancer or the management or amelioration of a symptom thereof. Therefore, in some embodiments, the sample is collected after the administration of chemotherapeutics or the start of a chemotherapy regimen.

In some embodiments of any of the preceding methods, the patient has carcinoma, lymphoma, blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, melanoma, and leukemia or lymphoid malignancies. In some embodiments, the cancer is kidney cancer (e.g., renal cell carcinoma (RCC), e.g., metastatic RCC), squamous cell cancer (e.g., epithelial squamous cell cancer), lung cancer (including small-cell lung cancer (SCLC), non-small cell lung cancer (NSCLC), adenocarcinoma of the lung, and squamous carcinoma of the lung), cancer of the peritoneum, hepatocellular cancer, gastric or stomach cancer including gastrointestinal cancer, pancreatic cancer, glioblastoma, cervical cancer, ovarian cancer, liver cancer, hepatoma, breast cancer (including metastatic breast cancer), bladder cancer, colon cancer, rectal cancer, colorectal cancer, endometrial or uterine carcinoma, salivary gland carcinoma, prostate cancer, vulval cancer, thyroid cancer, hepatic carcinoma, anal carcinoma, penile carcinoma, Merkel cell cancer, mycoses fungoids, testicular cancer, esophageal cancer, tumors of the biliary tract, head and neck cancer, B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), abnormal vascular proliferation associated with phakomatoses, edema (such as that associated with brain tumors), or Meigs' syndrome. In preferred embodiments, the patient has a kidney cancer (e.g., RCC, e.g., mRCC). The patient may optionally have an advanced, refractory, recurrent, chemotherapy-resistant, and/or platinum-resistant form of the cancer.

For the prevention or treatment of cancer, the dose of a VEGF antagonist (e.g., an anti-VEGF antibody, such as bevacizumab) and/or a PD-L1 axis binding antagonist (e.g., a PD-L1 binding antagonist, such as atezolizumab) will depend on the type of cancer to be treated, as defined above, the severity and course of the cancer, whether the antibody is administered for preventive or therapeutic purposes, previous therapy, the patient's clinical history and response to the drug, and the discretion of the attending physician.

In some embodiments, the VEGF antagonist (e.g., an anti-VEGF antibody, such as bevacizumab) and/or PD-L1 axis binding antagonist (e.g., a PD-L1 binding antagonist, such as atezolizumab) may be suitably administered to the patient at one time or over a series of treatments. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. Such doses may be administered intermittently, e.g., every week or every three weeks (e.g., such that the patient receives, for example, from about two to about twenty, or e.g., about six doses of the VEGF antagonist and/or the PD-L1 axis binding antagonist). An initial higher loading dose, followed by one or more lower doses may be administered. However, other dosage regimens may be useful. The progress of this therapy is easily monitored by conventional techniques and assays.

For example, as a general proposition, the therapeutically effective amount of a VEGF antagonist (e.g., an anti-VEGF antibody, such as bevacizumab) and/or PD-L1 axis binding antagonist (e.g., a PD-L1 binding antagonist, such as atezolizumab) administered to human will be in the range of about 0.01 to about 50 mg/kg of patient body weight, whether by one or more administrations. In some embodiments, the antibody used is about 0.01 mg/kg to about 45 mg/kg, about 0.01 mg/kg to about 40 mg/kg, about 0.01 mg/kg to about 35 mg/kg, about 0.01 mg/kg to about 30 mg/kg, about 0.01 mg/kg to about 25 mg/kg, about 0.01 mg/kg to about 20 mg/kg, about 0.01 mg/kg to about 15 mg/kg, about 0.01 mg/kg to about 10 mg/kg, about 0.01 mg/kg to about 5 mg/kg, or about 0.01 mg/kg to about 1 mg/kg administered daily, weekly, every two weeks, every three weeks, or monthly, for example. In some embodiments, the antibody is administered at 15 mg/kg. However, other dosage regimens may be useful. In one embodiment, an VEGF antagonist (e.g., an anti-VEGF antibody, such as bevacizumab) and/or PD-L1 axis binding antagonist (e.g., a PD-L1 binding antagonist, such as atezolizumab) is administered to a human at a dose of about 100 mg, about 200 mg, about 300 mg, about 400 mg, about 420 mg, about 500 mg, about 525 mg, about 600 mg, about 700 mg, about 800 mg, about 840mg, about 900 mg, about 1000 mg, about 1050 mg, about 1100 mg, about 1200 mg, about 1300 mg, about 1400 mg, about 1500 mg, about 1600 mg, about 1700 mg, or about 1800 mg on day 1 of 21-day cycles (every three weeks, q3w).

In some embodiments, atezolizumab is administered at 1200 mg intravenously every three weeks (q3w). In some embodiments, bevacizumab is administered at a fixed dose at one time or over a series of treatments. Where a fixed dose is administered, preferably it is in the range from about 5 mg to about 2000 mg. For example, the fixed dose may be approximately 420 mg, approximately 525 mg, approximately 840 mg, or approximately 1050 mg. In some embodiments, bevacizumab is administered at 10 mg/kg intravenously every two weeks. In some embodiments, bevacizumab is administered at 15 mg/kg intravenously every three weeks. The dose of VEGF antagonist and/or PD-L1 axis binding antagonist may be administered as a single dose or as multiple doses (e.g., 2, 3, 4, 5, 6, 7, 8, 9, or 10 or more doses). Where a series of doses are administered, these may, for example, be administered approximately every week, approximately every 2 weeks, approximately every 3 weeks, or approximately every 4 weeks. The dose of the antibody administered in a combination treatment may be reduced as compared to a single treatment. The progress of this therapy is easily monitored by conventional techniques.

VEGF antagonists (e.g., anti-VEGF antibodies, e.g., bevacizumab) and PD-L1 axis binding antagonists (e.g., an antibody, binding polypeptide, and/or small molecule) described herein (any additional therapeutic agent) may be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The PD-L1 axis binding antagonist and/or VEGF antagonist need not be, but is optionally formulated with and/or administered concurrently with one or more agents currently used to prevent or treat the disorder in question. The effective amount of such other agents depends on the amount of the PD-L1 axis binding antagonist and/or VEGF antagonist present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

In some embodiments, a VEGF antagonist (e.g., an anti-VEGF antibody, e.g., bevacizumab) is administered concurrently with a PD-L1 axis binding antagonist (e.g., an anti-PD-L1 antibody, e.g., atezolizumab). In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) are administered as part of the same formulation. In other embodiments, a VEGF antagonist (e.g., an anti-VEGF antibody, e.g., bevacizumab) is administered separately from a PD-L1 axis binding antagonist (e.g., an anti-PD-L1 antibody, e.g., atezolizumab).

In some embodiments, any of the preceding methods may further include administering an additional therapeutic agent. In some embodiments, the additional therapeutic agent is selected from the group consisting of an immunotherapy agent, a cytotoxic agent, a growth inhibitory agent, a radiation therapy agent, an anti-angiogenic agent, and combinations thereof.

In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) is administered concurrently with an agonist directed against an activating co-stimulatory molecule. In some embodiments, an activating co-stimulatory molecule may include CD40, CD226, CD28, OX40, GITR, CD137, CD27, HVEM, or CD127. In some embodiments, the agonist directed against an activating co-stimulatory molecule is an agonist antibody that binds to CD40, CD226, CD28, OX40, GITR, CD137, CD27, HVEM, or CD127. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an antagonist directed against an inhibitory co-stimulatory molecule. In some embodiments, an inhibitory co-stimulatory molecule may include CTLA-4 (also known as CD152), TIM-3, BTLA, VISTA, LAG-3, B7-H3, B7-H4, IDO, TIGIT, MICA/B, or arginase. In some embodiments, the antagonist directed against an inhibitory co-stimulatory molecule is an antagonist antibody that binds to CTLA-4, TIM-3, BTLA, VISTA, LAG-3, B7-H3, B7-H4, IDO, TIGIT, MICA/B, or arginase.

In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an antagonist directed against CTLA-4 (also known as CD152), e.g., a blocking antibody. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with ipilimumab (also known as MDX-010, MDX-101, or YERVOY®). In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with tremelimumab (also known as ticilimumab or CP-675,206). In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an antagonist directed against B7-H3 (also known as CD276), e.g., a blocking antibody. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with MGA271. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an antagonist directed against a TGF-beta, e.g., metelimumab (also known as CAT-192), fresolimumab (also known as GC1008), or LY2157299.

In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an agonist directed against CD137 (also known as TNFRSF9, 4-1BB, or ILA), e.g., an activating antibody. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with urelumab (also known as BMS-663513). In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an agonist directed against CD40, e.g., an activating antibody. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with CP-870893. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an agonist directed against OX40 (also known as CD134), e.g., an activating antibody. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an anti-OX40 antibody (e.g., AgonOX). In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an agonist directed against CD27, e.g., an activating antibody. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with CDX-1127. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an antagonist directed against TIGIT, for example, an anti-TIGIT antibody. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an antagonist directed against indoleamine-2,3-dioxygenase (IDO). In some embodiments, the IDO antagonist is 1-methyl-D-tryptophan (also known as 1-D-MT).

In some embodiments, VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with a cancer vaccine. In some embodiments, the cancer vaccine is a peptide cancer vaccine, which in some embodiments is a personalized peptide vaccine. In some embodiments the peptide cancer vaccine is a multivalent long peptide, a multi-peptide, a peptide cocktail, a hybrid peptide, or a peptide-pulsed dendritic cell vaccine (see, e.g., Yamada et al., Cancer Sci. 104:14-21, 2013). In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an adjuvant. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with a treatment comprising a TLR agonist, e.g., Poly-ICLC (also known as HILTONOL®), LPS, MPL, or CpG ODN. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with tumor necrosis factor (TNF) alpha. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with IL-1. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with HMGB1. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an IL-10 antagonist. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an IL-4 antagonist. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an IL-13 antagonist. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an HVEM antagonist. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an ICOS agonist, e.g., by administration of ICOS-L, or an agonistic antibody directed against ICOS. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with a treatment targeting CX3CL1. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with a treatment targeting CXCL9. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with a treatment targeting CXCL10. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with a treatment targeting CCL5. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with an LFA-1 or ICAM1 agonist. In some embodiments, a VEGF antagonist (e.g., an anti-VEGF antibody, e.g., bevacizumab) and a PD-L1 axis binding antagonist (e.g., an anti-PD-L1 antibody, e.g., atezolizumab) may be administered in conjunction with a Selectin agonist.

A chemotherapeutic agent, if administered, is usually administered at dosages known therefore, or optionally lowered due to combined action of the drugs or negative side effects attributable to administration of the chemotherapeutic agent. Preparation and dosing schedules for such chemotherapeutic agents may be used according to manufacturers' instructions or as determined empirically by the skilled practitioner. Where the chemotherapeutic agent is paclitaxel, preferably, it is administered at a dose between about 130 mg/m² to 200 mg/m² (e.g., approximately 175 mg/m²), for instance, over 3 hours, once every 3 weeks. Where the chemotherapeutic agent is carboplatin, preferably it is administered by calculating the dose of carboplatin using the Calvert formula which is based on a patient's preexisting renal function or renal function and desired platelet nadir. Renal excretion is the major route of elimination for carboplatin. The use of this dosing formula, as compared to empirical dose calculation based on body surface area, allows compensation for patient variations in pretreatment renal function that might otherwise result in either underdosing (in patients with above average renal function) or overdosing (in patients with impaired renal function). The target AUC of 4-6 mg/mL/min using single agent carboplatin appears to provide the most appropriate dose range in previously treated patients.

In addition to the above therapeutic regimes, the patient may be subjected to surgical removal of tumors and/or cancer cells.

Such combination therapies noted above encompass combined administration (where two or more therapeutic agents are included in the same or separate formulations), and separate administration, in which case, administration of a VEGF antagonist and/or a PD-L1 axis binding antagonist can occur prior to, simultaneously, and/or following, administration of the additional therapeutic agent or agents. In one embodiment, administration of VEGF antagonist and/or a PD-L1 axis binding antagonist and administration of an additional therapeutic agent occur within about one month, or within about one, two or three weeks, or within about one, two, three, four, five, or six days, of each other.

In embodiments where either the VEGF antagonist or the PD-L1 axis binding antagonist is an antibody (e.g., bevacizumab or atezolizumab), the administered antibody may be a naked antibody. The VEGF antagonist (e.g., an anti-VEGF antibody, such as bevacizumab) and/or the PD-L1 axis binding antagonist (e.g., a PD-L1 binding antagonist, such as atezolizumab) administered may be conjugated with a cytotoxic agent. Preferably, the conjugated and/or antigen to which it is bound is/are internalized by the cell, resulting in increased therapeutic efficacy of the conjugate in killing the cancer cell to which it binds. In a preferred embodiment, the cytotoxic agent targets or interferes with nucleic acid in the cancer cell. Examples of such cytotoxic agents include maytansinoids, calicheamicins, ribonucleases, and DNA endonucleases.

The compositions utilized in the methods described herein can be administered by any suitable method, including, for example, intravenously, intramuscularly, subcutaneously, intradermally, percutaneously, intraarterially, intraperitoneally, intralesionally, intracranially, intraarticularly, intraprostatically, intrapleurally, intratracheally, intrathecally, intranasally, intravaginally, intrarectally, topically, intratumorally, peritoneally, subconjunctivally, intravesicularly, mucosally, intrapericardially, intraumbilically, intraocularly, intraorbitally, orally, topically, transdermally, intravitreally (e.g., by intravitreal injection), by eye drop, by inhalation, by injection, by implantation, by infusion, by continuous infusion, by localized perfusion bathing target cells directly, by catheter, by lavage, in cremes, or in lipid compositions. The compositions utilized in the methods described herein can also be administered systemically or locally. The method of administration can vary depending on various factors (e.g., the compound or composition being administered and the severity of the condition, disease, or disorder being treated). In some embodiments, the PD-L1 axis binding antagonist is administered intravenously, intramuscularly, subcutaneously, topically, orally, transdermally, intraperitoneally, intraorbitally, by implantation, by inhalation, intrathecally, intraventricularly, or intranasally. Dosing can be by any suitable route, e.g., by injections, such as intravenous or subcutaneous injections, depending in part on whether the administration is brief or chronic. Various dosing schedules including but not limited to single or multiple administrations over various time-points, bolus administration, and pulse infusion are contemplated herein.

### IV. Compositions

In one aspect, the invention is based, in part, on the discovery that combinations of VEGF antagonists (e.g., anti-VEGF antibodies, such as bevacizumab) and PD-L1 axis binding antagonists (e.g., anti-PD-L1 antibodies, such as atezolizumab), have anti-tumor effects in cancers such as kidney cancer. In certain embodiments, VEGF antagonists and PD-L1 axis binding antagonists are provided. These agents, and combinations thereof, are useful for the treatment of cancer, e.g., as part of any of the methods described herein.

### A. Exemplary VEGF Antagonists

VEGF antagonists of the invention include any molecule capable of binding VEGF, reducing VEGF expression levels, or neutralizing, blocking, inhibiting, abrogating, reducing, or interfering with VEGF biological activities. An exemplary human VEGF is shown under UniProtKB/Swiss-Prot Accession No. P15692, Gene ID (NCBI): 7422.

In some instances, the VEGF antagonist is an anti-VEGF antibody. In some embodiments, the anti-VEGF antibody is bevacizumab, also known as "rhuMab VEGF" or "AVASTIN®." Bevacizumab is a recombinant humanized anti-VEGF monoclonal antibody generated according to Presta et al. (Cancer Res. 57:4593-4599, 1997). It comprises mutated human IgG1 framework regions and antigen-binding complementarity-determining regions from the murine anti-hVEGF monoclonal antibody A.4.6.1 that blocks binding of human VEGF to its receptors. Approximately 93% of the amino acid sequence of bevacizumab, including most of the framework regions, is derived from human IgG1, and about 7% of the sequence is derived from the murine antibody A4.6.1. Bevacizumab has a molecular mass of about 149,000 daltons and is glycosylated. Bevacizumab and other humanized anti-VEGF antibodies are further described in U.S. Pat. No. 6,884,879 issued Feb. 26, 2005. Additional preferred antibodies include the G6 or B20 series antibodies (e.g., G6-31, B20-4.1), as described in PCT Application Publication No. WO 2005/012359. For additional preferred antibodies see U.S. Pat. Nos. 7,060,269, 6,582,959, 6,703,020; 6,054,297; WO98/45332; WO 96/30046; WO94/10202; EP 0666868B1; U.S. Patent Application Publication Nos. 2006009360, 20050186208, 20030206899, 20030190317, 20030203409, and 20050112126; and Popkov et al. (Journal of Immunological Methods 288:149-164, 2004). Other preferred antibodies include those that bind to a functional epitope on human VEGF comprising of residues F17, M18, D19, Y21, Y25, Q89, 191, K101, E103, and C104 or, alternatively, comprising residues F17, Y21, Q22, Y25, D63, 183, and Q89.

In other instances, the VEGF antagonist is an anti-VEGFR2 antibody or related molecule (e.g., ramucirumab, tanibirumab, aflibercept); an anti-VEGFR1 antibody or related molecules (e.g., icrucumab, aflibercept (VEGF Trap-Eye; EYLEA®), or ziv-aflibercept (VEGF Trap; ZALTRAP®)); a bispecific VEGF antibody (e.g., MP-0250, vanucizumab (VEGF-ANG2), or bispecific antibodies disclosed in US 2001/0236388); a bispecific antibody including a combination of two of anti-VEGF, anti-VEGFR1, and anti-VEGFR2 arms; an anti-VEGFA antibody (e.g., bevacizumab, sevacizumab); an anti-VEGFB antibody; an anti-VEGFC antibody (e.g., VGX-100), an anti-VEGFD antibody; or a nonpeptide small molecule VEGF antagonist (e.g., pazopanib, axitinib, vandetanib, stivarga, cabozantinib, lenvatinib, nintedanib, orantinib, telatinib, dovitinig, cediranib, motesanib, sulfatinib, apatinib, foretinib, famitinib, or tivozanib).

It is expressly contemplated that such VEGF antagonist antibodies or other antibodies described herein (e.g., anti-VEGF antibodies for detection of VEGF expression levels) for use in any of the embodiments enumerated above may have any of the features, singly or in combination, described in Sections i-vii of Section C below.

### B. Exemplary PD-L1 Axis Binding Antagonists

PD-L1 axis binding antagonists of the invention include PD-1 binding antagonists, PD-L1 binding antagonists, and PD-L2 binding antagonists. PD-1 (programmed death 1) is also referred to in the art as "programmed cell death 1," "PDCD1," "CD279," and "SLEB2." An exemplary human PD-1 is shown in UniProtKB/Swiss-Prot Accession No. Q15116. PD-L1 (programmed death ligand 1) is also referred to in the art as "programmed cell death 1 ligand 1," "PDCD1LG1," "CD274," "B7-H," and "PDL1." An exemplary human PD-L1 is shown in UniProtKB/Swiss-Prot Accession No.Q9NZQ7.1. PD-L2 (programmed death ligand 2) is also referred to in the art as "programmed cell death 1 ligand 2," "PDCD1LG2," "CD273," "B7-DC," "Btdc," and "PDL2." An exemplary human PD-L2 is shown in UniProtKB/Swiss-Prot Accession No. Q9BQ51. In some embodiments, PD-1, PD-L1, and PD-L2 are human PD-1, PD-L1 and PD-L2.

In some instances, the PD-L1 axis binding antagonist is a PD-L1 binding antagonist. In some instances, the PD-L1 binding antagonist inhibits the binding of PD-L1 to one or more of its ligand binding partners. In other instances, the PD-L1 binding antagonist inhibits the binding of PD-L1 to PD-1. In yet other instances, the PD-L1 binding antagonist inhibits the binding of PD-L1 to B7-1. In some instances, the PD-L1 binding antagonist inhibits the binding of PD-L1 to both PD-1 and B7-1. In some instances, the PD-L1 binding antagonist is an antibody. In some embodiments, the antibody is selected from the group consisting of: YW243.55.S70, MPDL3280A (atezolizumab), MDX-1105, MEDI4736 (durvalumab), and MSB0010718C (avelumab). In some embodiments, the antibody comprises a heavy chain comprising HVR-H1 sequence of SEQ ID NO: 19, HVR-H2 sequence of SEQ ID NO: 20, and HVR-H3 sequence of SEQ ID NO: 21; and a light chain comprising HVR-L1 sequence of SEQ ID NO: 22, HVR-L2 sequence of SEQ ID NO: 23, and HVR-L3 sequence of SEQ ID NO: 24. In some embodiments, the antibody comprises a heavy chain variable region comprising the amino acid sequence of SEQ ID NO: 26 and a light chain variable region comprising the amino acid sequence of SEQ ID NO: 4.

In some instances, the PD-L1 axis binding antagonist is a PD-1 binding antagonist. For example, in some instances, the PD-1 binding antagonist inhibits the binding of PD-1 to one or more of its ligand binding partners. In some instances, the PD-1 binding antagonist inhibits the binding of PD-1 to PD-L1. In other instances, the PD-1 binding antagonist inhibits the binding of PD-1 to PD-L2. In yet other instances, the PD-1 binding antagonist inhibits the binding of PD-1 to both PD-L1 and PD-L2. In some instances, the PD-1 binding antagonist is an antibody. In some instances, the antibody is selected from the group consisting of: MDX 1106 (nivolumab), MK-3475 (pembrolizumab), CT-011 (pidilizumab), MEDI-0680 (AMP-514), PDR001, REGN2810, and BGB-108. In some instances, the PD-1 binding antagonist is an Fc-fusion protein. For example, in some instances, the Fc-fusion protein is AMP-224. In a further aspect, the invention provides for the use of a PD-L1 axis binding antagonist in the manufacture or preparation of a medicament. In one embodiment, the medicament is for treatment of a cancer. In a further embodiment, the medicament is for use in a method of treating a cancer comprising administering to a patient suffering from kidney cancer (e.g., a renal cell carcinoma (RCC), e.g., metastatic RCC (mRCC)) an effective amount of the medicament. In one such embodiment, the method further comprises administering to the individual an effective amount of at least one additional therapeutic agent, e.g., as described below.

In some embodiments, the PD-1 binding antagonist is a molecule that inhibits the binding of PD-1 to its ligand binding partners. In a specific aspect the PD-1 ligand binding partners are PD-L1 and/or PD-L2. In another embodiment, a PD-L1 binding antagonist is a molecule that inhibits the binding of PD-L1 to its binding ligands. In a specific aspect, PD-L1 binding partners are PD-1 and/or B7-1. In another embodiment, the PD-L2 binding antagonist is a molecule that inhibits the binding of PD-L2 to its ligand binding partners. In a specific aspect, the PD-L2 binding ligand partner is PD-1. The antagonist may be an antibody, an antigen binding fragment thereof, an immunoadhesin, a fusion protein, or oligopeptide.

In some embodiments, the PD-1 binding antagonist is an anti-PD-1 antibody (e.g., a human antibody, a humanized antibody, or a chimeric antibody), for example, as described below. In some embodiments, the anti-PD-1 antibody is selected from the group consisting of MDX-1106 (nivolumab), MK-3475 (pembrolizumab), CT-011 (pidilizumab), MEDI-0680 (AMP-514), PDR001, REGN2810, and BGB-108. MDX-1106, also known as MDX-1106-04, ONO-4538, BMS-936558, or nivolumab, is an anti-PD-1 antibody described in WO2006/121168. MK-3475, also known as pembrolizumab or lambrolizumab, is an anti-PD-1 antibody described in WO 2009/114335. CT-011, also known as hBAT, hBAT-1 or pidilizumab, is an anti-PD-1 antibody described in WO 2009/101611. In some embodiments, the PD-1 binding antagonist is an immunoadhesin (e.g., an immunoadhesin comprising an extracellular or PD-1 binding portion of PD-L1 or PD-L2 fused to a constant region (e.g., an Fc region of an immunoglobulin sequence). In some embodiments, the PD-1 binding antagonist is AMP-224. AMP-224, also known as B7-DCIg, is a PD-L2-Fc fusion soluble receptor described in WO 2010/027827 and WO 2011/066342.

In some embodiments, the anti-PD-1 antibody is MDX-1106. Alternative names for "MDX-1106" include MDX-1106-04, ONO-4538, BMS-936558, and nivolumab. In some embodiments, the anti-PD-1 antibody is nivolumab (CAS Registry Number: 946414-94-4). In a still further embodiment, provided is an isolated anti-PD-1 antibody comprising a heavy chain variable region comprising the heavy chain variable region amino acid sequence from SEQ ID NO: 1 and/or a light chain variable region comprising the light chain variable region amino acid sequence from SEQ ID NO: 2. In a still further embodiment, provided is an isolated anti-PD-1 antibody comprising a heavy chain and/or a light chain sequence, wherein:
(a) the heavy chain sequence has at least 85%, at least 90%, at least 91%, at least 92%, at least 93%, at least 94%, at least 95%, at least 96%, at least 97%, at least 98%, at least 99% or 100% sequence identity to the heavy chain sequence:
(b) the light chain sequences has at least 85%, at least 90%, at least 91%, at least 92%, at least 93%, at least 94%, at least 95%, at least 96%, at least 97%, at least 98%, at least 99% or 100% sequence identity to the light chain sequence:

In some embodiments, the PD-L1 axis binding antagonist is a PD-L2 binding antagonist. In some embodiments, the PD-L2 binding antagonist is an anti-PD-L2 antibody (e.g., a human antibody, a humanized antibody, or a chimeric antibody). In some embodiments, the PD-L2 binding antagonist is an immunoadhesin.

In some embodiments, the PD-L1 binding antagonist is an anti-PD-L1 antibody, for example, as described below. In some embodiments, the anti-PD-L1 antibody is capable of inhibiting binding between PD-L1 and PD-1 and/or between PD-L1 and B7-1. In some embodiments, the anti-PD-L1 antibody is a monoclonal antibody. In some embodiments, the anti-PD-L1 antibody is an antibody fragment selected from the group consisting of Fab, Fab'-SH, Fv, scFv, and (Fab')₂ fragments. In some embodiments, the anti-PD-L1 antibody is a humanized antibody. In some embodiments, the anti-PD-L1 antibody is a human antibody. In some embodiments, the anti-PD-L1 antibody is selected from the group consisting of YW243.55.S70, MPDL3280A (atezolizumab), MDX-1105, and MEDI4736 (durvalumab), and MSB0010718C (avelumab). Antibody YW243.55.S70 is an anti-PD-L1 described in WO 2010/077634. MDX-1105, also known as BMS-936559, is an anti-PD-L1 antibody described in WO2007/005874. MEDI4736 (durvalumab) is an anti-PD-L1 monoclonal antibody described in WO2011/066389 and US2013/034559. Examples of anti-PD-L1 antibodies useful for the methods of this invention, and methods for making thereof are described in PCT patent application WO 2010/077634, WO 2007/005874, WO 2011/066389, U.S. Pat. No. 8,217,149, and US 2013/034559.

Anti-PD-L1 antibodies described in WO 2010/077634 A1 and US 8,217,149 may be used in the methods described herein. In some embodiments, the anti-PD-L1 antibody comprises a heavy chain variable region sequence of SEQ ID NO: 3 and/or a light chain variable region sequence of SEQ ID NO: 4. In a still further embodiment, provided is an isolated anti-PD-L1 antibody comprising a heavy chain variable region and/or a light chain variable region sequence, wherein:
(a) the heavy chain sequence has at least 85%, at least 90%, at least 91%, at least 92%, at least 93%, at least 94%, at least 95%, at least 96%, at least 97%, at least 98%, at least 99% or 100% sequence identity to the heavy chain sequence:
(b) the light chain sequence has at least 85%, at least 90%, at least 91%, at least 92%, at least 93%, at least 94%, at least 95%, at least 96%, at least 97%, at least 98%, at least 99% or 100% sequence identity to the light chain sequence:

In one embodiment, the anti-PD-L1 antibody comprises a heavy chain variable region comprising an HVR-H1, HVR-H2 and HVR-H3 sequence, wherein:
(a) the HVR-H1 sequence is GFTFSX₁SWIH (SEQ ID NO: 5);
(b) the HVR-H2 sequence is AWIX₂PYGGSX₃YYADSVKG (SEQ ID NO: 6);
(c) the HVR-H3 sequence is RHWPGGFDY (SEQ ID NO: 7);
further wherein: X₁ is D or G; X₂ is S or L; X₃ is T or S. In one specific aspect, X₁ is D; X₂ is S and X₃ is T. In another aspect, the polypeptide further comprises variable region heavy chain framework sequences juxtaposed between the HVRs according to the formula: (FR-H1)-(HVR-H1)-(FR-H2)-(HVR-H2)-(FR-H3)-(HVR-H3)-(FR-H4). In yet another aspect, the framework sequences are derived from human consensus framework sequences. In a further aspect, the framework sequences are VH subgroup III consensus framework. In a still further aspect, at least one of the framework sequences is the following:
FR-H1 is EVQLVESGGGLVQPGGSLRLSCAAS (SEQ ID NO: 8)
FR-H2 is WVRQAPGKGLEWV (SEQ ID NO: 9)
FR-H3 is RFTISADTSKNTAYLQMNSLRAEDTAVYYCAR (SEQ ID NO: 10)
FR-H4 is WGQGTLVTVSA (SEQ ID NO: 11).

In a still further aspect, the heavy chain polypeptide is further combined with a variable region light chain comprising an HVR-L1, HVR-L2 and HVR-L3, wherein:
(a) the HVR-L1 sequence is RASQX₄X₅X₆TX₇X₈A (SEQ ID NO: 12);
(b) the HVR-L2 sequence is SASX₉LX₁₀S, (SEQ ID NO: 13);
(c) the HVR-L3 sequence is QQX₁₁X₁₂X₁₃X₁₄PX₁₅T (SEQ ID NO: 14);
wherein: X₄ is D or V; X₅ is V or I; X₆ is S or N; X₇ is A or F; X₈ is V or L; X₉ is F or T; X₁₀ is Y or A; X₁₁ is Y, G, F, or S; X₁₂ is L, Y, For W; X₁₃ is Y, N, A, T, G, F or I; X₁₄ is H, V, P, T or I; X₁₅ is A, W, R, P or T. In a still further aspect, X₄ is D; X₅ is V; X₆ is S; X₇ is A; X₈ is V; X₉ is F; X₁₀ is Y; X₁₁ is Y; X₁₂ is L; X₁₃ is Y; X₁₄ is H; X₁₅ is A.

In a still further aspect, the light chain further comprises variable region light chain framework sequences juxtaposed between the HVRs according to the formula: (FR-L1)-(HVR-L1)-(FR-L2)-(HVR-L2)-(FR-L3)-(HVR-L3)-(FR-L4). In a still further aspect, the framework sequences are derived from human consensus framework sequences. In a still further aspect, the framework sequences are VL kappa I consensus framework. In a still further aspect, at least one of the framework sequence is the following:
FR-L1 is DIQMTQSPSSLSASVGDRVTITC (SEQ ID NO: 15)
FR-L2 is WYQQKPGKAPKLLIY (SEQ ID NO: 16)
FR-L3 is GVPSRFSGSGSGTDFTLTISSLQPEDFATYYC (SEQ ID NO: 17)
FR-L4 is FGQGTKVEIKR (SEQ ID NO: 18).

In another embodiment, provided is an isolated anti-PD-L1 antibody or antigen binding fragment comprising a heavy chain and a light chain variable region sequence, wherein:
(a) the heavy chain comprises an HVR-H1, HVR-H2 and HVR-H3, wherein further:
   (i) the HVR-H1 sequence is GFTFSX₁SWIH; (SEQ ID NO: 5)
   (ii) the HVR-H2 sequence is AWIX₂PYGGSX₃YYADSVKG (SEQ ID NO: 6)
   (iii) the HVR-H3 sequence is RHWPGGFDY, and (SEQ ID NO: 7)
(b) the light chain comprises an HVR-L1, HVR-L2 and HVR-L3, wherein further:
   (i) the HVR-L1 sequence is RASQX₄X₅X₆TX₇X₈A (SEQ ID NO: 12)
   (ii) the HVR-L2 sequence is SASX₉LX₁₀S; and (SEQ ID NO: 13)
   (iii) the HVR-L3 sequence is QQX₁₁X₁₂X₁₃X₁₄PX₁₅T; (SEQ ID NO: 14)
   wherein: X₁ is D or G; X₂ is S or L; X₃ is T or S; X₄ is D or V; X₅ is V or I; X₆ is S or N; X₇ is A or F; X₈ is V or L; X₉ is F or T; X₁₀ is Y or A; X₁₁ is Y, G, F, or S; X₁₂ is L, Y, F or W; X₁₃ is Y, N, A, T, G, F or I; X₁₄ is H, V, P, T or I; X₁₅ is A, W, R, P or T. In a specific aspect, X₁ is D; X₂ is S and X₃ is T. In another aspect, X₄ is D; X₅ is V; X₆ is S; X₇ is A; X₈ is V; X₉ is F; X₁₀ is Y; X₁₁ is Y; X₁₂ is L; X₁₃ is Y; X₁₄ is H; X₁₅ is A. In yet another aspect, X₁ is D; X₂ is S and X₃ is T, X₄ is D; X₅ is V; X₆ is S; X₇ is A; X₈ is V; X₉ is F; X₁₀ is Y; X₁₁ is Y; X₁₂ is L; X₁₃ is Y; X₁₄ is H and X₁₅ is A.

In a further aspect, the heavy chain variable region comprises one or more framework sequences juxtaposed between the HVRs as: (FR-H1)-(HVR-H1)-(FR-H2)-(HVR-H2)-(FR-H3)-(HVR-H3)-(FR-H4), and the light chain variable regions comprises one or more framework sequences juxtaposed between the HVRs as: (FR-L1)-(HVR-L1)-(FR-L2)-(HVR-L2)-(FR-L3)-(HVR-L3)-(FR-L4). In a still further aspect, the framework sequences are derived from human consensus framework sequences. In a still further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences are set forth as SEQ ID NOs: 8, 9, 10 and 11. In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences are set forth as SEQ ID NOs: 15, 16, 17 and 18.

In a still further specific aspect, the antibody further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region is IgG2A. In a still further specific aspect, the antibody has reduced or minimal effector function. In a still further specific aspect the minimal effector function results from an "effector-less Fc mutation" or aglycosylation. In still a further embodiment, the effector-less Fc mutation is an N297A or D265A/N297A substitution in the constant region.

In yet another embodiment, provided is an anti-PD-L1 antibody comprising a heavy chain and a light chain variable region sequence, wherein:
(a) the heavy chain further comprises an HVR-H1, HVR-H2 and an HVR-H3 sequence having at least 85% sequence identity to GFTFSDSWIH (SEQ ID NO: 19), AWISPYGGSTYYADSVKG (SEQ ID NO: 20) and RHWPGGFDY (SEQ ID NO: 21), respectively, or
(b) the light chain further comprises an HVR-L1, HVR-L2 and an HVR-L3 sequence having at least 85% sequence identity to RASQDVSTAVA (SEQ ID NO: 22), SASFLYS (SEQ ID NO: 23) and QQYLYHPAT (SEQ ID NO: 24), respectively.

In a specific aspect, the sequence identity is 86%, 87%, 88%, 89%, 90%, 91%, 92%, 93%, 94%, 95%, 96%, 97%, 98%, 99% or 100%.

In another aspect, the heavy chain variable region comprises one or more framework sequences juxtaposed between the HVRs as: (FR-H1)-(HVR-H1)-(FR-H2)-(HVR-H2)-(FR-H3)-(HVR-H3)-(FR-H4), and the light chain variable regions comprises one or more framework sequences juxtaposed between the HVRs as: (FR-L1)-(HVR-L1)-(FR-L2)-(HVR-L2)-(FR-L3)-(HVR-L3)-(FR-L4). In yet another aspect, the framework sequences are derived from human consensus framework sequences. In a still further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences are set forth as SEQ ID NOs: 8, 9, 10 and 11. In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences are set forth as SEQ ID NOs: 15, 16, 17 and 18.

In a still further specific aspect, the antibody further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region is IgG2A. In a still further specific aspect, the antibody has reduced or minimal effector function. In a still further specific aspect the minimal effector function results from an "effector-less Fc mutation" or aglycosylation. In still a further embodiment, the effector-less Fc mutation is an N297A or D265A/N297A substitution in the constant region.

In another further embodiment, provided is an isolated anti-PD-L1 antibody comprising a heavy chain and a light chain variable region sequence, wherein:
(a) the heavy chain sequence has at least 85% sequence identity to the heavy chain sequence:
(b) the light chain sequences has at least 85% sequence identity to the light chain sequence:

In a specific aspect, the sequence identity is 86%, 87%, 88%, 89%, 90%, 91%, 92%, 93%, 94%, 95%, 96%, 97%, 98%, 99% or 100%. In another aspect, the heavy chain variable region comprises one or more framework sequences juxtaposed between the HVRs as: (FR-H1)-(HVR-H1)-(FR-H2)-(HVR-H2)-(FR-H3)-(HVR-H3)-(FR-H4), and the light chain variable regions comprises one or more framework sequences juxtaposed between the HVRs as: (FR-L1)-(HVR-L1)-(FR-L2)-(HVR-L2)-(FR-L3)-(HVR-L3)-(FR-L4). In yet another aspect, the framework sequences are derived from human consensus framework sequences. In a further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences are set forth as SEQ ID NOs: 8, 9, 10 and WGQGTLVTVSS (SEQ ID NO: 27).

In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences are set forth as SEQ ID NOs: 15, 16, 17 and 18.

In a still further specific aspect, the antibody further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region is IgG2A. In a still further specific aspect, the antibody has reduced or minimal effector function. In a still further specific aspect, the minimal effector function results from production in prokaryotic cells. In a still further specific aspect the minimal effector function results from an "effector-less Fc mutation" or aglycosylation. In still a further embodiment, the effector-less Fc mutation is an N297A or D265A/N297A substitution in the constant region.

In a further aspect, the heavy chain variable region comprises one or more framework sequences juxtaposed between the HVRs as: (FR-H1)-(HVR-H1)-(FR-H2)-(HVR-H2)-(FR-H3)-(HVR-H3)-(FR-H4), and the light chain variable regions comprises one or more framework sequences juxtaposed between the HVRs as: (FR-L1)-(HVR-L1)-(FR-L2)-(HVR-L2)-(FR-L3)-(HVR-L3)-(FR-L4). In a still further aspect, the framework sequences are derived from human consensus framework sequences. In a still further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences is the following:

| | | |
|---|---|---|
| FR-H1 | EVQLVESGGGLVQPGGSLRLSCAASGFTFS | (SEQ ID NO: 29) |
| FR-H2 | WVRQAPGKGLEWVA | (SEQ ID NO: 30) |
| FR-H3 | RFTISADTSKNTAYLQMNSLRAEDTAVYYCAR | (SEQ ID NO: 10) |
| FR-H4 | WGQGTLVTVSS | (SEQ ID NO: 27) |

In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences is the following:

| | | |
|---|---|---|
| FR-L1 | DIQMTQSPSSLSASVGDRVTITC | (SEQ ID NO: 15) |
| FR-L2 | WYQQKPGKAPKLLIY | (SEQ ID NO: 16) |
| FR-L3 | GVPSRFSGSGSGTDFTLTISSLQPEDFATYYC | (SEQ ID NO: 17) |
| FR-L4 | FGQGTKVEIK | (SEQ ID NO: 28) |

In a still further specific aspect, the antibody further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region is IgG2A. In a still further specific aspect, the antibody has reduced or minimal effector function. In a still further specific aspect the minimal effector function results from an "effector-less Fc mutation" or aglycosylation. In still a further embodiment, the effector-less Fc mutation is an N297A or D265A/N297A substitution in the constant region.

In yet another embodiment, provided is an anti-PD-L1 antibody comprising a heavy chain and a light chain variable region sequence, wherein:
(c) the heavy chain further comprises an HVR-H1, HVR-H2 and an HVR-H3 sequence having at least 85% sequence identity to GFTFSDSWIH (SEQ ID NO: 19), AWISPYGGSTYYADSVKG (SEQ ID NO: 20) and RHWPGGFDY (SEQ ID NO: 21), respectively, and/or
(d) the light chain further comprises an HVR-L1, HVR-L2 and an HVR-L3 sequence having at least 85% sequence identity to RASQDVSTAVA (SEQ ID NO: 22), SASFLYS (SEQ ID NO: 23) and QQYLYHPAT (SEQ ID NO: 24), respectively.

In a specific aspect, the sequence identity is 86%, 87%, 88%, 89%, 90%, 91%, 92%, 93%, 94%, 95%, 96%, 97%, 98%, 99% or 100%.

In another aspect, the heavy chain variable region comprises one or more framework sequences juxtaposed between the HVRs as: (FR-H1)-(HVR-H1)-(FR-H2)-(HVR-H2)-(FR-H3)-(HVR-H3)-(FR-H4), and the light chain variable regions comprises one or more framework sequences juxtaposed between the HVRs as: (FR-L1)-(HVR-L1)-(FR-L2)-(HVR-L2)-(FR-L3)-(HVR-L3)-(FR-L4). In yet another aspect, the framework sequences are derived from human consensus framework sequences. In a still further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences are set forth as SEQ ID NOs: 8, 9, 10 and WGQGTLVTVSSASTK (SEQ ID NO: 31).

In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences are set forth as SEQ ID NOs: 15, 16, 17 and 18. In a still further specific aspect, the antibody further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region is IgG2A. In a still further specific aspect, the antibody has reduced or minimal effector function. In a still further specific aspect the minimal effector function results from an "effector-less Fc mutation" or aglycosylation. In still a further embodiment, the effector-less Fc mutation is an N297A or D265A/N297A substitution in the constant region.

In a still further embodiment, provided is an isolated anti-PD-L1 antibody comprising a heavy chain and a light chain variable region sequence, wherein:
(a) the heavy chain sequence has at least 85% sequence identity to the heavy chain sequence:
(b) the light chain sequences has at least 85% sequence identity to the light chain sequence:

In some embodiments, provided is an isolated anti-PD-L1 antibody comprising a heavy chain and a light chain variable region sequence, wherein the light chain variable region sequence has at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to the amino acid sequence of SEQ ID NO: 4. In some embodiments, provided is an isolated anti-PD-L1 antibody comprising a heavy chain and a light chain variable region sequence, wherein the heavy chain variable region sequence has at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to the amino acid sequence of SEQ ID NO: 26. In some embodiments, provided is an isolated anti-PD-L1 antibody comprising a heavy chain and a light chain variable region sequence, wherein the light chain variable region sequence has at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the amino acid sequence of SEQ ID NO: 4 and the heavy chain variable region sequence has at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the amino acid sequence of SEQ ID NO: 26. In some embodiments, one, two, three, four or five amino acid residues at the N-terminal of the heavy and/or light chain may be deleted, substituted or modified.

In a still further embodiment, provided is an isolated anti-PD-L1 antibody comprising a heavy chain and a light chain sequence, wherein:
(a) the heavy chain sequence has at least 85% sequence identity to the heavy chain sequence:
(b) the light chain sequences has at least 85% sequence identity to the light chain sequence:

In some embodiments, provided is an isolated anti-PD-L1 antibody comprising a heavy chain and a light chain sequence, wherein the light chain sequence has at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence of SEQ ID NO: 33. In some embodiments, provided is an isolated anti-PD-L1 antibody comprising a heavy chain and a light chain sequence, wherein the heavy chain sequence has at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence of SEQ ID NO: 32. In some embodiments, provided is an isolated anti-PD-L1 antibody comprising a heavy chain and a light chain sequence, wherein the light chain sequence has at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence of SEQ ID NO: 33 and the heavy chain sequence has at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence of SEQ ID NO: 32.

In some embodiments, the isolated anti-PD-L1 antibody is aglycosylated. Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. Removal of glycosylation sites form an antibody is conveniently accomplished by altering the amino acid sequence such that one of the above-described tripeptide sequences (for N-linked glycosylation sites) is removed. The alteration may be made by substitution of an asparagine, serine or threonine residue within the glycosylation site another amino acid residue (e.g., glycine, alanine or a conservative substitution).

In any of the embodiments herein, the isolated anti-PD-L1 antibody can bind to a human PD-L1, for example a human PD-L1 as shown in UniProtKB/Swiss-Prot Accession No.Q9NZQ7.1, or a variant thereof.

In a still further embodiment, provided is an isolated nucleic acid encoding any of the antibodies described herein. In some embodiments, the nucleic acid further comprises a vector suitable for expression of the nucleic acid encoding any of the previously described anti-PD-L1 antibodies. In a still further specific aspect, the vector is in a host cell suitable for expression of the nucleic acid. In a still further specific aspect, the host cell is a eukaryotic cell or a prokaryotic cell. In a still further specific aspect, the eukaryotic cell is a mammalian cell, such as Chinese hamster ovary (CHO) cell.

The antibody or antigen binding fragment thereof, may be made using methods known in the art, for example, by a process comprising culturing a host cell containing nucleic acid encoding any of the previously described anti-PD-L1 antibodies or antigen-binding fragment in a form suitable for expression, under conditions suitable to produce such antibody or fragment, and recovering the antibody or fragment.

It is expressly contemplated that such PD-L1 axis binding antagonist antibodies (e.g., anti-PD-L1 antibodies, anti-PD-1 antibodies, and anti-PD-L2 antibodies), or other antibodies described herein (e.g., anti-PD-L1 antibodies for detection of PD-L1 expression levels) for use in any of the embodiments enumerated above may have any of the features, singly or in combination, described in Sections i-vii of Section C below.

### C. Antibodies

### i. Antibody Affinity

In certain embodiments, an antibody provided herein (e.g., an anti-VEGF antibody, an anti-PD-L1 antibody or an anti-PD-1 antibody) has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (e.g., 10⁻⁸ M or less, e.g., from 10⁻⁸ M to 10⁻¹³ M, e.g., from 10⁻⁹ M to 10⁻¹³ M).

In one embodiment, Kd is measured by a radiolabeled antigen binding assay (RIA). In one embodiment, an RIA is performed with the Fab version of an antibody of interest and its antigen. For example, solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, e.g., Chen et al., J. Mol. Biol. 293:865-881, 1999). To establish conditions for the assay, MICROTITER® multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [¹²⁵I]-antigen are mixed with serial dilutions of a Fab of interest (e.g., consistent with assessment of the anti-VEGF antibody, Fab-12, in Presta et al., Cancer Res. 57:4593-4599, 1997). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20®) in PBS. When the plates have dried, 150 µl/well of scintillant (MICROSCINT-20™; Packard) is added, and the plates are counted on a TOPCOUNT™ gamma counter (Packard) for ten minutes. Concentrations of each Fab that give less than or equal to 20% of maximal binding are chosen for use in competitive binding assays.

According to another embodiment, Kd is measured using a BIACORE® surface plasmon resonance assay. For example, an assay using a BIACORE®-2000 or a BIACORE®-3000 (BIAcore, Inc., Piscataway, NJ) is performed at 25°C with immobilized antigen CM5 chips at -10 response units (RU). In one embodiment, carboxymethylated dextran biosensor chips (CM5, BIACORE, Inc.) are activated with *N*-ethyl-*N'*-(3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 µg/ml (∼0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 µl/min. Association rates (kₒₙ) and dissociation rates (k_{off}) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio k_{off}/kₒₙ. See, for example, Chen et al., (J. Mol. Biol. 293:865-881, 1999). If the on-rate exceeds 10⁶ M⁻¹s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO™ spectrophotometer (ThermoSpectronic) with a stirred cuvette.

### ii. Antibody Fragments

In certain embodiments, an antibody (e.g., an anti-PD-L1 antibody or an anti-PD-1 antibody) provided herein is an antibody fragment. Antibody fragments include, but are not limited to, Fab, Fab', Fab'-SH, F(ab')₂, Fv, and scFv fragments, and other fragments described below. For a review of certain antibody fragments, see Hudson et al. (Nat. Med. 9:129-134, 2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994). See also WO 93/16185; and U.S. Patent Nos. 5,571,894 and 5,587,458. For discussion of Fab and F(ab')₂ fragments comprising salvage receptor binding epitope residues and having increased *in vivo* half-life, see U.S. Patent No. 5,869,046.

Diabodies are antibody fragments with two antigen-binding sites that may be bivalent or bispecific. See, for example, EP 404,097, WO 1993/01161, Hudson et al. Nat. Med. 9:129-134, 2003, and Hollinger et al. Proc. Natl. Acad. Sci. USA 90: 6444-6448, 1993. Triabodies and tetrabodies are also described in Hudson et al. (Nat. Med. 9:129-134, 2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see, e.g., U.S. Patent No. 6,248,516 B1).

Antibody fragments can be made by various techniques, including but not limited to proteolytic digestion of an intact antibody as well as production by recombinant host cells (e.g., *E. coli* or phage), according to known methods.

### iii. Chimeric and Humanized Antibodies

In certain embodiments, an antibody (e.g., an anti-VEGF antibody, an anti-PD-L1 antibody or an anti-PD-1 antibody) provided herein is a chimeric antibody. Certain chimeric antibodies are described, e.g., in U.S. Patent No. 4,816,567; and Morrison et al. (Proc. Natl. Acad. Sci. USA, 81:6851-6855, 1984). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, (Front. Biosci. 13:1619-1633, 2008), and are further described, e.g., in Riechmann et al. (Nature 332:323-329, 1988); Queen et al. (Proc. Natl. Acad. Sci. USA 86:10029-10033, 1989); US Patent Nos. 5, 821,337, 7,527,791, 6,982,321, and 7,087,409; Kashmiri et al. (Methods 36:25-34, 2005) (describing specificity determining region (SDR) grafting); Padlan, (Mol. Immunol. 28:489-498, 1991) (describing "resurfacing"); Dall'Acqua et al. (Methods 36:43-60, 2005) (describing "FR shuffling"); Osbourn et al. (Methods 36:61-68, 2005), and Klimka et al. (Br. J. Cancer, 83:252-260, 2000) (describing the "guided selection" approach to FR shuffling).

Human framework regions that may be used for humanization include but are not limited to: framework regions selected using the "best-fit" method (see, e.g., Sims et al. J. Immunol. 151:2296, 1993); framework regions derived from the consensus sequence of human antibodies of a particular subgroup of light or heavy chain variable regions (see, e.g., Carter et al. Proc. Natl. Acad. Sci. USA, 89:4285, 1992; and Presta et al. J. Immunol., 151:2623, 1993); human mature (somatically mutated) framework regions or human germline framework regions (see, e.g., Almagro and Fransson, Front. Biosci. 13:1619-1633, 2008); and framework regions derived from screening FR libraries (see, e.g., Baca et al., J. Biol. Chem. 272:10678-10684, 1997; and Rosok et al. J. Biol. Chem. 271:22611-22618, 1996).

### iv. Human Antibodies

In certain embodiments, an antibody (e.g., an anti-VEGF antibody, an anti-PD-L1 antibody or an anti-PD-1 antibody) provided herein is a human antibody. Human antibodies can be produced using various techniques known in the art. Human antibodies are described generally in van Dijk and van de Winkel, (Curr. Opin. Pharmacol. 5: 368-74, 2001) and Lonberg (Curr. Opin. Immunol. 20:450-459, 2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, (Nat. Biotech. 23:1117-1125, 2005). See also, e.g., U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. See, e.g., Kozbor, (J. Immunol. 133: 3001, 1984); Brodeur et al. (Monoclonal Antibody Production Techniques and Applications, pp. 51-63, Marcel Dekker, Inc., New York, 1987); and Boerner et al. (J. Immunol., 147: 86, 1991). Human antibodies generated via human B-cell hybridoma technology are also described in Li et al., Proc. Natl. Acad. Sci. USA, 103:3557-3562, 2006. Additional methods include those described, for example, in U.S. Patent No. 7,189,826 (describing production of monoclonal human IgM antibodies from hybridoma cell lines) and Ni, (Xiandai Mianyixue, 26(4):265-268, 2006) (describing human-human hybridomas). Human hybridoma technology (Trioma technology) is also described in Vollmers and Brandlein, (Histology and Histopathology, 20(3):927-937, 2005) and Vollmers and Brandlein, (Methods and Findings in Experimental and Clinical Pharmacology, 27(3):185-91, 2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### v. Library-Derived Antibodies

Antibodies of the invention (e.g., anti-VEGF antibodies, anti-PD-L1 antibodies, or anti-PD-1 antibodies) may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. (Methods in Molecular Biology 178:1-37, O'Brien et al., ed., Human Press, Totowa, NJ, 2001) and further described, e.g., in McCafferty et al. (Nature 348:552-554, 1990); Clackson et al. (Nature 352: 624-628, 1991); Marks et al. (J. Mol. Biol. 222: 581-597, 1992); Marks and Bradbury, (Methods in Molecular Biology 248:161-175, Lo, ed., Human Press, Totowa, NJ, 2003); Sidhu et al. (J. Mol. Biol. 338(2): 299-310, 2004); Lee et al. (J. Mol. Biol. 340(5): 1073-1093, 2004); Fellouse, (Proc. Natl. Acad. Sci. USA 101(34): 12467-12472, 2004); and Lee et al. (J. Immunol. Methods 284(1-2): 119-132, 2004).

In certain phage display methods, repertoires of VH and VL genes are separately cloned by polymerase chain reaction (PCR) and recombined randomly in phage libraries, which can then be screened for antigen-binding phage as described in Winter et al. (Ann. Rev. Immunol., 12: 433-455, 1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and self antigens without any immunization as described by Griffiths et al. (EMBO J, 12: 725-734, 1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, (J. Mol. Biol., 227: 381-388, 1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### vi. Multispecific Antibodies

In any one of the above aspects, an antibody (e.g., an anti-VEGF antibody, an anti-PD-L1 antibody, or an anti-PD-1 antibody) provided herein may be a multispecific antibody, for example, a bispecific antibody. Multispecific antibodies are monoclonal antibodies that have binding specificities for at least two different sites. In certain embodiments, an antibody provided herein is a multispecific antibody, e.g., a bispecific antibody. In certain embodiments, one of the binding specificities is for PD-L1 and the other is for any other antigen. In certain embodiments, one of the binding specificities is for VEGF and the other is for any other antigen. In certain embodiments, bispecific antibodies may bind to two different epitopes of PD-L1. In certain embodiments, bispecific antibodies may bind to two different epitopes of VEGF. Bispecific antibodies may also be used to localize cytotoxic agents to cells which express PD-L1 or VEGF. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537, 1983), WO 93/08829 and Traunecker et al. EMBO J. 10: 3655, 1991) and "knob-in-hole" engineering (see, e.g., U.S. Patent No. 5,731,168). Multi-specific antibodies may also be made by engineering electrostatic steering effects for making antibody Fc-heterodimeric molecules (see, e.g., WO 2009/089004A1); cross-linking two or more antibodies or fragments (see, e.g., US Patent No. 4,676,980, and Brennan et al. Science 229: 81, 1985); using leucine zippers to produce bi-specific antibodies (see, e.g., Kostelny et al. J. Immunol. 148(5): 1547-1553, 1992); using "diabody" technology for making bispecific antibody fragments (see, e.g., Hollinger et al. Proc. Natl. Acad. Sci. USA 90:6444-6448, 1993); and using single-chain Fv (sFv) dimers (see, e.g., Gruber et al. J. Immunol. 152:5368, 1994); and preparing trispecific antibodies as described, e.g., in Tutt et al. J. Immunol. 147: 60, 1991).

Engineered antibodies with three or more functional antigen binding sites, including "Octopus antibodies," are also included herein (see, e.g., US 2006/0025576A1).

The antibody or fragment herein includes a "Dual Acting FAb" or "DAF" comprising an antigen binding site that binds to PD-L1 and another, different antigen. The antibody or fragment herein also includes a DAF comprising an antigen binding site that binds to VEGF and another, different antigen.

### vii. Antibody Variants

In certain embodiments, amino acid sequence variants of the antibodies of the invention (e.g., anti-VEGF antibodies, anti-PD-L1 antibodies, and anti-PD-1 antibodies) are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, for example, antigen-binding.

### a. Substitution, insertion, and deletion variants

In certain embodiments, antibody variants having one or more amino acid substitutions are provided. Sites of interest for substitutional mutagenesis include the HVRs and FRs. Conservative substitutions are shown in Table 1 under the heading of "preferred substitutions." More substantial changes are provided in Table 1 under the heading of "exemplary substitutions," and as further described below in reference to amino acid side chain classes. Amino acid substitutions may be introduced into an antibody of interest and the products screened for a desired activity, for example, retained/improved antigen binding, decreased immunogenicity, or improved ADCC or CDC.

**Table 1. Exemplary and Preferred Amino Acid Substitutions**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (e.g., a humanized or human antibody). Generally, the resulting variant(s) selected for further study will have modifications (e.g., improvements) in certain biological properties (e.g., increased affinity and/or reduced immunogenicity) relative to the parent antibody and/or will have substantially retained certain biological properties of the parent antibody. An exemplary substitutional variant is an affinity matured antibody, which may be conveniently generated, for example, using phage display-based affinity maturation techniques such as those described herein. Briefly, one or more HVR residues are mutated and the variant antibodies displayed on phage and screened for a particular biological activity (e.g., binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196, 2008), and/or residues that contact antigen, with the resulting variant VH or VL being tested for binding affinity. Affinity maturation by constructing and reselecting from secondary libraries has been described, e.g., in Hoogenboom et al. (Methods in Molecular Biology 178:1-37, O'Brien et al., ed., Human Press, Totowa, NJ, 2001). In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen-contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (Science, 244:1081-1085, 1989). In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g., for ADEPT) or a polypeptide which increases the serum half-life of the antibody.

### b. Glycosylation variants

In certain embodiments, antibodies of the invention can be altered to increase or decrease the extent to which the antibody is glycosylated. Addition or deletion of glycosylation sites to an antibody of the invention may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32, 1997. The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, for example, U.S. Patent Publication Nos. US 2003/0157108; US 2004/0093621. Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. (J. Mol. Biol. 336:1239-1249, 2004); and Yamane-Ohnuki et al. (Biotech. Bioeng. 87: 614, 2004). Examples of cell lines capable of producing defucosylated antibodies include Lec13 CHO cells deficient in protein fucosylation (Ripka et al. Arch. Biochem. Biophys. 249:533-545, 1986); U.S. Pat. Appl. No. US 2003/0157108 A1; and WO 2004/056312 A1, especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614, 2004; Kanda, Y. et al. Biotechnol. Bioeng. 94(4):680-688, 2006; and WO 2003/085107).

Antibody variants are further provided with bisected oligosaccharides, for example, in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878; US Patent No. 6,602,684; and US 2005/0123546. Antibody variants with at least one galactose residue in the oligosaccharide attached to the Fc region are also provided. Such antibody variants may have improved CDC function. Such antibody variants are described, e.g., in WO 1997/30087; WO 1998/58964; and WO 1999/22764.

### c. Fc region variants

In certain embodiments, one or more amino acid modifications may be introduced into the Fc region of an antibody of the invention, thereby generating an Fc region variant. The Fc region variant may comprise a human Fc region sequence (e.g., a human IgG1, IgG2, IgG3 or IgG4 Fc region) comprising an amino acid modification (e.g., a substitution) at one or more amino acid positions.

In certain embodiments, the invention contemplates an antibody variant that possesses some but not all effector functions, which make it a desirable candidate for applications in which the half-life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, (Annu. Rev. Immunol. 9:457-492, 1991). Nonlimiting examples of *in vitro* assays to assess ADCC activity of a molecule of interest is described in U.S. Patent No. 5,500,362 (see, e.g., Hellstrom, I. et al. Proc. Natl. Acad. Sci. USA 83:7059-7063, 1986) and Hellstrom, I et al. Proc. Natl. Acad. Sci. USA 82:1499-1502, 1985; U.S. Patent No. 5,821,337; Bruggemann et al. J. Exp. Med. 166:1351-1361, 1987). Alternatively, non-radioactive assays methods may be employed (see, for example, ACTI™ non-radioactive cytotoxicity assay for flow cytometry (CellTechnology, Inc. Mountain View, CA; and CYTOTOX 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo,* e.g., in an animal model such as that disclosed in Clynes et al. (Proc. Natl. Acad. Sci. USA 95:652-656, 1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, e.g., Gazzano-Santoro et al. J. Immunol. Methods 202:163, 1996; Cragg et al. Blood. 101:1045-1052, 2003; and Cragg et al. Blood. 103:2738-2743, 2004). FcRn binding and *in vivo* clearance/half-life determinations can also be performed using methods known in the art (see, e.g., Petkova et al. Int'l. Immunol. 18(12):1759-1769, 2006).

Antibodies with reduced effector function include those with substitution of one or more of Fc region residues 238, 265, 269, 270, 297, 327 and 329 (U.S. Patent Nos. 6,737,056 and 8,219,149). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581 and 8,219,149).

Certain antibody variants with improved or diminished binding to FcRs are described (see, e.g., U.S. Patent No. 6,737,056; WO 2004/056312, and Shields et al., J. Biol. Chem. 9(2): 6591-6604, 2001).

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (i.e., either improved or diminished) C1q binding and/or Complement Dependent Cytotoxicity (CDC), e.g., as described in US Patent No. 6,194,551, WO 99/51642, and Idusogie et al. (J. Immunol. 164: 4178-4184, 2000).

Antibodies with increased half-lives and improved binding to the neonatal Fc receptor (FcRn), which is responsible for the transfer of maternal IgGs to the fetus (Guyer et al., J. Immunol. 117:587, 1976; and Kim et al. J. Immunol. 24:249, 1994), are described in U.S. Pub. No. 2005/0014934A1. Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (U.S. Patent No. 7,371,826).

See also Duncan and Winter, (Nature 322:738-40, 1988); U.S. Patent No. 5,648,260; U.S. Patent No. 5,624,821; and WO 94/29351, concerning other examples of Fc region variants.

### d. Cysteine engineered antibody variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

### e. Antibody derivatives

In certain embodiments, an antibody provided herein may be further modified to contain additional nonproteinaceous moieties that are known in the art and readily available. The moieties suitable for derivatization of the antibody include but are not limited to water soluble polymers. Nonlimiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone) polyethylene glycol, propropylene glycol homopolymers, prolypropylene oxide/ethylene oxide copolymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al. Proc. Natl. Acad. Sci. USA 102: 11600-11605, 2005). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### f. Immunoconjugates

The invention also provides immunoconjugates comprising an antibody herein (e.g., an anti-VEGF antibody, an anti-PD-L1 antibody, or an anti-PD-1 antibody) conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (see U.S. Patent Nos. 5,208,020 and 5,416,064 and European Patent EP 0 425 235 B1); an auristatin such as monomethylauristatin drug moieties DE and DF (MMAE and MMAF) (see U.S. Patent Nos. 5,635,483, 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877,296; Hinman et al. Cancer Res. 53:3336-3342, 1993; and Lode et al. Cancer Res. 58:2925-2928, 1998); an anthracycline such as daunomycin or doxorubicin (see Kratz et al. Current Med. Chem. 13:477-523, 2006; Jeffrey et al. Bioorganic & Med. Chem. Letters 16:358-362, 2006; Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532, 2002; King et al., J. Med. Chem. 45:4336-4343, 2002; and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another embodiment, an immunoconjugate comprises an antibody as described herein conjugated to a radioactive atom to form a radioconjugate. A variety of radioactive isotopes are available for the production of radioconjugates. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the radioconjugate is used for detection, it may comprise a radioactive atom for scintigraphic studies, for example tc99m or 1123, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron. Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCI), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al. (Science 238:1098, 1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al. Cancer Res. 52:127-131, 1992; and U.S. Patent No. 5,208,020) may be used.

The immunoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

### D. Pharmaceutical Formulations

Therapeutic formulations of the VEGF antagonists and the PD-L1 axis binding antagonists used in accordance with the present invention (e.g., an anti-VEGF antibody, such as bevacizumab, and an anti-PD-L1 antibody, such as atezolizumab) are prepared for storage by mixing the antagonist having the desired degree of purity with optional pharmaceutically acceptable carriers, excipients, or stabilizers in the form of lyophilized formulations or aqueous solutions. For general information concerning formulations, see, e.g., Gilman et al. (eds.) The Pharmacological Bases of Therapeutics, 8th Ed., Pergamon Press, 1990; A. Gennaro (ed.), Remington's Pharmaceutical Sciences, 18th Edition, Mack Publishing Co., Pennsylvania, 1990; Avis et al. (eds.) Pharmaceutical Dosage Forms: Parenteral Medications Dekker, New York, 1993; Lieberman et al. (eds.) Pharmaceutical Dosage Forms: Tablets Dekker, New York, 1990; Lieberman et al. (eds.), Pharmaceutical Dosage Forms: Disperse Systems Dekker, New York, 1990; and Walters (ed.) Dermatological and Transdermal Formulations (Drugs and the Pharmaceutical Sciences), Vol 119, Marcel Dekker, 2002.

Acceptable carriers, excipients, or stabilizers are non-toxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™, or polyethylene glycol (PEG).

The formulation herein may also contain more than one active compound, preferably those with complementary activities that do not adversely affect each other. The type and effective amounts of such medicaments depend, for example, on the amount and type of antagonist present in the formulation, and clinical parameters of the patients.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed., 1980.

Sustained-release preparations may be prepared. Suitable examples of sustained-release preparations include semi-permeable matrices of solid hydrophobic polymers containing the antagonist, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Patent No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for *in vivo* administration must be sterile. This is readily accomplished by filtration through sterile filtration membranes.

It is to be understood that any of the above articles of manufacture may include an immunoconjugate described herein in place of or in addition to a PD-L1 axis binding antagonist.

### EXAMPLES

### Example 1: Materials and Experimental Methods

### A. Study Design

The goal of the phase Ib study described in Examples 1-4 was to evaluate the safety and tolerability of the anti-PD-L1 antibody atezolizumab, in combination with bevacizumab, a human, monoclonal, engineered anti-VEGF antibody concurrently administered by intravenous infusion every 3 weeks (q3w) to patients with previously untreated advanced metastatic renal cell carcinoma (mRCC). Treatment was continued as long as patients were experiencing clinical benefit in the opinion of the investigator (i.e. in the absence of unacceptable toxicity or symptomatic deterioration attributed to disease progression). Patients were allowed to continue to receive study treatment at the discretion of the investigator if pseudoprogression was suspected or if there was evidence of a mixed response. Study objectives included an evaluation of tumor and circulating pharmacodynamic markers associated with the administration of bevacizumab and atezolizumab and preliminary assessment of the antitumor activity of the treatment combination.

Safety evaluations (clinical and laboratory) were performed at screening and throughout the trial. A final evaluation occurred by 30 days after the last dose. The incidence, nature and severity of adverse events (AEs) were graded according to National Cancer Institute Common Terminology Criteria for Adverse Events (CTCAE), version 4.0.

Any evaluable or measurable disease was documented at screening and reassessed at each tumor evaluation. Tumor evaluations were performed at the ends of cycles 2, 4, 6, 8, 12 and 16 or as clinically indicated. Assessments were performed during the last week of the drug administration cycle and before the start of treatment in the next cycle. Patients who discontinued study treatment for reasons other than disease progression continued to have tumor assessments every 12 weeks until the patient experienced disease progression, initiated further systemic cancer therapy, or died.

Protocol-defined dose-limiting toxicity (DLT) criteria included standard Grade ≥ 3 or 4 hematologic and non-hematologic toxicities. Dosing commenced with the recommended Phase 2 dose of atezolizumab administered in combination with the labeled q3w dose of bevacizumab, and no DLTs were reported.

### B. Patients

Patients were eligible to participate in this cohort of the phase Ib study if they had advanced or metastatic RCC for which they had not received prior systemic therapy. Patients were required to be at least 18 years old; have adequate hematological and end-organ function; and have an Eastern Cooperative Oncology Group performance status of 0 or 1. Disease had to be measurable per Response Evaluation Criteria in Solid Tumors (RECIST). Patients with known primary central nervous system (CNS) malignancy or symptomatic CNS metastases, history or risk of autoimmune disease, or history of human immunodeficiency virus, hepatitis B, or hepatitis C infection were excluded. Also excluded were patients who received prior treatment with anti-CTLA-4, anti-PD-1, or anti-PD-L1 therapeutic antibodies or pathway-targeting agents as well as patients who were treated with systemic immunostimulatory agents or systemic immunosuppressive medications within a specified period before study start.

Of the ten patients on study, six yielded biopsies with sufficient viable tumor cells at both post treatment time points. Of the six pairs (i.e., biopsies from the same patient at both on-treatment time points), seven were derived from kidney lesions, four from the abdominal/chest wall, one from a lung lesion, one from lymph node, and five were from undisclosed lesions.

### C. Immunohistochemical Analysis for PD-L1, CD8, and MHC-I

Formalin-fixed, paraffin-embedded (FFPE) tissue sections of 4 µm thickness were stained for PD-L1 with an anti-human PD-L1 rabbit monoclonal antibody (clone SP142; Ventana, Tucson, AZ) on an automated staining platform (BenchMark; Ventana) using a concentration of 4.3 mg/ml, with signal visualization by diaminobenzidine; sections were counter-stained with haematoxylin. PD-L1 expression was evaluated on tumor cells and tumor-infiltrating immune cells. For tumor cells, the proportion of PD-L1-positive tumor cells was estimated as a percentage of the total number of tumor cells; tumor cells typically showed membranous staining with a variably strong component of cytoplasmic staining. The distribution of PD-L1-positive tumor cells within a given tumor sample was typically very focal; in tumors growing as solid aggregates, PD-L1-positive tumor cells were more commonly observed at the interface between malignant cells and stroma containing tumor-infiltrating immune cells. For tumor-infiltrating immune cells, the percentage of PD-L1-positive tumor-infiltrating immune cells occupying the tumor was determined. Tumor-infiltrating immune cells with clearly discernible cytoplasm, such as macrophages and dendritic cells, showed a membranous staining pattern for PD-L1. This was more difficult to determine for cells of small lymphoid morphology with scant amounts of cytoplasm. PD-L1-positive tumor-infiltrating immune cells were typically seen as variably-sized aggregates towards the periphery of the tumor mass, in stromal bands dissecting the tumor mass, as single cells scattered in stroma, or within tumor-infiltrating immune cell aggregates. Specimens were scored as IHC 0, 1, 2, or 3 if < 1%, ≥ 1% but < 5%, ≥ 5% but < 10%, or ≥ 10% of cells per area were PD-L1 positive, respectively. PD-L1 IHC scores in patients with multiple specimens were based on the highest score. CD8 (clone SP16 (Epitomics)) IHC was performed on a Discovery XT autostainer (Ventana) using CC1 antigen retrieval and OMNIMAP™ (Ventana) detection technology.

All MHC-I IHC steps were carried out on the Ventana Discovery XT automated platform (Ventana Medical Systems; Tucson, AZ). Sections were treated with Cell Conditioner 1, standard time, and then incubated in primary antibody, MHC Class I (EP1395Y, Novus, cat. # NB110-57201) at a 1:5000 dilution for 60 min at 37°C. Bound primary antibody was detected by the OMNIMAP™ anti-rabbit HRP detection kit, followed by DAB (Ventana Medical Systems; Tucson, AZ). Sections were counterstained with Hematoxylin II (Ventana Medical Systems; Tucson, AZ) for 4 min, bluing solution for 4 min, then dehydrated and cover-slipped. Human cell pellets endogenously expressing low, medium, and high MHC-I were used in parallel as positive controls. Negative controls were performed using rabbit monoclonal (Clone DA1E, Cell Signaling Technology, Cat#3900S) isotype antibody. MHC-I staining in tumor cells was scored using an H-score system. Briefly, staining intensity of tumor cell membranes was assigned a numerical value of 0, 1, 2, or 3 corresponding to no, low, medium, or high 3,3'-diaminobenzidine (DAB) signal intensity, respectively. Relative to the overall tumor area, the percentage of cells at different staining intensities was determined by visual assessment. A final score was calculated by multiplying the membrane intensity score by the area percentage for each population present in a given tumor sample as follows: 1 × (% of 1 + cells) + 2 × (% of 2 + cells) + 3 × (% of 3 + cells) = H score. Cases were scored by two independent pathologists. Scoring brackets were defined as scores of ≤100, 101-200, and 201-300, and concordance was defined as independent scores falling within the same bracket. Any discordance was resolved upon mutual review of the cases.

### D. Dual- and Triple-Color Immunohistochemistry and a Whole Slide Digital Analysis

Consecutive 4 µm thickness sections of FFPE tumor tissues were stained with the following in-house developed IHC assays using Ventana Benchmark XT or Benchmark Ultra automated platforms (Ventana Medical Systems; Tucson, AZ): Ki67/CD8, PDPN/CD34/ASMA, and CD163/CD68.

For the Ki67/CD8 assay, sections were treated with Cell Conditioner 1 for 64 min. Sections were then incubated in primary antibody, Ki67 (30-9, RTU, Ventana) for 4 min at 37°C. Bound primary antibody was detected by the OptiView DAB IHC detection kit (Ventana Medical Systems; Tucson, AZ). Subsequently, slides were incubated in primary antibody anti-CD8 (SP239, Spring Biosciences) at a 1:100 dilution for 60 min at 37°C. Bound primary antibody was detected by the UltraView Universal AP Red detection kit (Ventana Medical Systems; Tucson, AZ). Sections were counterstained with Hematoxylin II (Ventana Medical Systems; Tucson, AZ) for 4 min, bluing solution for 4 min, then dehydrated and cover-slipped.

For the PDPN/CD34/ASMA assay, sections were treated with Cell Conditioner 1 for 32 min. Sections were then incubated in the primary antibody anti-Podoplanin (D2-40, RTU, Ventana) for 16 min at 37°C. Bound primary antibody was detected by the OptiView DAB IHC detection kit (Ventana Medical Systems; Tucson, AZ). Subsequently, slides were incubated in primary antibody anti-CD34 (QBEnd/10; RTU, Ventana) for 16 min at 37°C. Bound primary antibody was detected by the iView Blue Plus detection kit (Ventana Medical Systems; Tucson, AZ). Finally, slides were incubated in primary antibody anti-smooth muscle actin ("SMActin") (1A4; RTU, Ventana) for 16 min at 37°C. Bound primary antibody was detected by the UltraView Universal AP Red detection kit (Ventana Medical Systems; Tucson, AZ). Sections were counterstained with Hematoxylin II (Ventana Medical Systems; Tucson, AZ) for 4 min, bluing solution for 4 min, then dehydrated and cover-slipped.

For the CD163/CD68 assay, sections were treated with Cell Conditioner 1 for 32 min and incubated in primary antibody anti-CD163 (MRQ-26, RTU, Ventana), for 8 min at 37°C. Bound primary antibody was detected by the OptiView DAB IHC detection kit (Ventana Medical Systems; Tucson, AZ). Subsequently, slides were incubated in primary antibody anti-CD68 (KP-1, RTU, Ventana) for 8 min at 37°C. Bound primary antibody was detected by the UltraView Universal AP Red detection kit (Ventana Medical Systems; Tucson, AZ). Sections were counterstained with Hematoxylin II (Ventana Medical Systems; Tucson, AZ) for 4 min, bluing solution for 4 min, then dehydrated and cover-slipped. Appropriate negative and positive controls were performed according to known methods.

Algorithms for the detection and classification of IHC-stained objects on a whole slide basis were written in Matlab. Following brightfield stain unmixing, IHC-stained objects were detected as cell candidates. For all cell candidates, quantitative features were extracted. Candidates were then classified into the various cell classes (e.g. CD8⁺/Ki67⁻ cells) using supervised machine learning. The classification method was trained using a ground truth gallery of true and false stained objects (provided by a pathologist). Finally, classified cells and tumor areas (provided by a pathologist through digital slide annotation) were reported and quality control (QC) images were generated for pathology review. The results of automated digital slide analysis were reported for tumor areas as follows: Ki67⁻/CD8⁺ and Ki67⁺/CD8⁺ cell densities (number of cell counts per mm²), CD68⁺/CD163⁺ and CD68⁺/CD163⁻ percent of area coverage (area coverage in relation to the whole tumor area), CD34⁺/αSMA⁻ and CD34⁺/αSMA⁺ vessel densities (vessel count per mm²).

### E. RNA Isolation from FFPE Tumor Tissue

RNA isolation was performed as described by Schleifman et al. (PLoS One 8:e74231, 2014). Briefly, tumor FFPE sections were macro dissected to enrich for neoplastic tissue, and tissue was lysed using tumor lysis buffer and Proteinase K to allow for complete digestion and release of nucleic acids. RNA was isolated using the High Pure FFPE RNA Micro Kit (Roche Applied Sciences, Indianapolis, IN) according to the manufacturer's protocol. DNA was isolated using the QIAAMP® DNA FFPE Tissue Kit (Qiagen, Hilden, Germany) according to the manufacturer's protocol. RNA and DNA were stored at 280 uC until the analyses were performed.

### F. Fluidigm and Nanostring Expression Analysis

Gene-expression analysis was performed using the BioMark HD™ real-time PCR Platform (Fluidigm) as described by Schleifman et al. (PLoS One 8:e74231, 2014). All TAQMAN® assays in the expression panel used FAM™ dye-labeled TAQMAN® minor groove binder (MGB) probes and ordered through Life Technologies either made-to-order or custom-designed, including four reference genes: SP2, GUSB, TMEM55B and VPS33B. A geometric median of the Ct values for the four reference genes (SP2, GUSB, TMEM55B and VPS33B) was calculated for each sample, and expression levels were determined using the delta Ct (ΔCt) method as follows: Ct (target Gene)2GeoMedian Ct (reference genes). Median mRNA expression levels (as measured by immunochip (iChip)) across patients on study were used as cutoffs to derive high- versus low-expression categorization. P values were determined by t test.

NanoString gene expression data were processed using the R/Bioconductor package "NanoStringQCPro." Raw counts were adjusted by positive control counts before probe- and lane-specific background was calculated based on both negative controls and blank measurements. After background correction, counts were log₂ transformed and normalized by housekeeping gene expression (TMEM55B, VPS33B, TBP, and TUBB).

### G. TCR Sequencing

The amplification and sequencing of TCRβ repertoire were performed at Adaptive Biotechnologies as described by Klinger et al. (PLoS One 8:e74231, 2013).

### H. Flow Cytometry

Whole blood flow cytometry for CD3, CD8, HLA-DR, and Ki-67 expression was performed at LabCorp central laboratory according to established protocols. Peripheral blood mononuclear cells (PBMCs) were isolated at Precision Bioservices and cryopreserved samples were shipped to Genentech for analysis of fractalkine receptor expression and detection of tumor-specific T cells. PBMCs were thawed and rested overnight, and a small aliquot of cells were stained with anti-HLA-A2-FITC (BB7.2, BD) and anti-CD45-APC-H7 (2D1, BD) to determine HLA-A2 status. The remaining cells were stained with a mixture of HLA-A*0201/peptide dextramers and pentamers (Immudex and Proimmune, see Table 1) for 10 min at room temperature followed by staining with anti-CD3-BV510 (UCHTI, Biolegend), anti-CD8-A700 (RPA-T8, BD), anti-CD4-PE-Cy7 (RPA-T4, eBioscience), anti-CD45RA-eVolve605 (HI100, eBioscience), anti-CCR7-BV421 (G043H7, Biolegend), anti-CX3CR1-PerCP-eFluor710 (2A9-1, eBioscience), and Fixable Viability Dye eFluor780 (eBioscience) for 30 minutes on ice. Samples were washed twice prior to data acquisition and sorting on a BD FACSARIA™ running FACSDIVA™ v8 software. A minimum of 10 dextramer-positive events out of 50,000 CD8⁺ T cells is considered a tumor-specific response. Table 3 shows a list of dextramers used for flow cytometry.

**Table 3. Dextramers for flow cytometry**

| **RCC-specific/associated antigens** | | |
|---|---|---|
| **Dex-FITC** | **Dex-PE** | **Dex-APC** |
| APOL1 | MAGE-A1 | G250 217-225 |
| APOL1 | PRAME-1 | NY-ESO-1 |
| MUC-1 12-20 | PRAME-2 | PRAME-4 |
| MUC-1 13-21 | PRAME-3 | PRAME-5 |
| SSX-2 | Survivin | PRAME-6 |
| SSX-2 | CCND1 | Survivin |
| DLK1 | Hsp70-2 | MET |
| EphA2 | IDO | PLIN |
| NRP1 | FLT1 | PRUNE2 |
| TEM1 | KDR | |

### I. Statistical Analysis

Data from all ten patients with RCC who received more than one dose of atezolizumab and bevacizumab intravenously every 21 days were used to determine baseline characteristics and rates of adverse events. Efficacy was assessed according to RECIST v1.1. The best confirmed overall objective response rate was derived from investigator-reported assessments. Objective response rate (ORR) was defined as the number of patients with a best overall objective response of complete or partial response divided by the total number of patients with a baseline tumor assessment.

Patients who were alive and did not experience disease progression at the cutoff date were censored at the time of last tumor assessment. Duration of response was obtained by Kaplan Meier method. Summaries of all AEs, AEs related to treatment, and grade 3-4 AEs are provided from all ten patients.

### Example 2: Gene Expression Analysis Identifies Biomarkers Associated with Bevacizumab Monotherapy and Bevacizumab and Atezolizumab Combination Therapy

A phase 1b clinical study was performed in which 10 patients with previously untreated mRCC received a single dose of bevacizumab on C1D1, followed by combined administration of atezolizumab and bevacizumab every three weeks beginning on C2D1. Baseline demographics of the patient cohort are shown in Table 4. Partial responses (PR) were observed in four out of ten patients using RECIST v1.1, while an additional five patients had prolonged stable disease (SD) (Figs. 1 and 2). The clinical activity observed in this small cohort was higher than previously obtained with either monotherapy. The duration of response has not been reached and the median time to response was 4.2 months.

**Table 4. Baseline Demographics**

| **Characteristics** | **N = 10** |
|---|---|
| Median age (range), y | 62 (42-74) |
| Male, n (%) | 7 (70%) |
| Patients with metastatic disease, n (%) | 8 (80%) |
| Liver or lung | 5 (50%) |
| Other than liver | 6 (60%) |

In addition to safety, tolerability, and clinical activity, one key objective of the phase Ib study described above was to evaluate the mechanism of combination activity. The trial design included a run-in period with bevacizumab to specifically interrogate the effects of bevacizumab on the local tumor immune microenvironment, followed by combination therapy with immune checkpoint blockade using atezolizumab. Tumor biopsies and blood were collected prior to treatment, 15-18 days following bevacizumab, and 4-6 weeks after the atezolizumab and bevacizumab combination treatment had been initiated.

To identify tumor markers associated with bevacizumab monotherapy or combination therapy, gene expression analysis was performed using both a 90 gene PCR-based Fluidigm panel and an 800 gene custom NanoString panel. Genes associated with the neo-vasculature, which reflect VEGF downstream signaling activity, were significantly decreased at both on-treatment time points in all patients (Fig. 3), confirming anti-angiogenic activity of bevacizumab. Surprisingly, comparison of the pre-treatment time point and the bevacizumab treatment alone time point revealed that there was increased gene expression of Th1 chemokines (CXCL9, CXLC10, CXCL11, and CXCL13) (ranging from about 0.7-fold to 6.9-fold relative to pre-treatment levels), CD8 T-effector markers (CD8A, CD8B, EOMES, GZMA, GZMB, IFNG, and PRF1) (ranging from about 0.4-fold to 6.2-fold relative to pre-treatment levels), as well as NK cell markers (GZMB, KLRK1, and SLAMF7) (ranging from about 0.7-fold to 8.2-fold relative to pre-treatment levels) (Fig. 3). Bevacizumab treatment resulted in four of the six patients showing a significant increase in gene signatures related to Th1 signaling. Importantly, at the individual patient level, these signatures were delinked from the degree of reduction of the VEGF dependent signature. FasL expression by IHC has been described as a potential barrier to immune cells in several cancers including RCC (Motz et al. Nat. Med. 20:607-615, 2014). In this study, consistent changes in FasL gene expression with bevacizumab or combination treatment were not observed. Overall, these differences indicate that bevacizumab treatment alone results in modulation of tumor immune microenvironment with Th1-related signatures reflecting the most significant treatment-induced alterations in the tumor microenvironment.

The increased expression of Th1 chemokines (CXCL9, CXLC10, CXCL11, and CXCL13), CD8 T-effector markers (CD8A, CD8B, EOMES, GZMA, GZMB, IFNG, and PRF1), and NK cell markers (GZMB, KLRK1, and SLAMF7) were enhanced upon administration of atezolizumab in combination with bevacizumab (Fig. 4). The expression level of the Th1 chemokine signature was increased 2.9- to 250.4-fold at the bevacizumab+atezolizumab time point relative to pre-treatment, and increased 0.9- to 81.8-fold compared to the bevacizumab alone time point. The expression level of the CD8 T_{eff} signature was increased 0.8- to 51.8-fold at the bevacizumab+atezolizumab time point relative to pre-treatment, and increased 0.3- to 17.6-fold compared to the bevacizumab alone time point. The expression level of the NK cell signature was increased 0.7- to 7.8-fold at the bevacizumab+atezolizumab time point relative to pre-treatment, and increased 0.4- to 13.1-fold compared to the bevacizumab alone time point.

### Example 3: Characterization of Biomarkers of Vascular and Immune Responses Following Bevacizumab Monotherapy or Combination Therapy in Both On-Treatment Time Points

To confirm the immune and vascular gene expression changes observed in the tumor, immune and vascular protein expression changes in pre-treatment and on-treatment tissue were evaluated by immunohistochemistry. A decrease in CD31, a marker of vessel-lining endothelial cells, was observed (Figs. 5 and 6). Dual staining of CD34, another marker of endothelial cells, with alpha-smooth muscle actin (αSMA) showed that immature or unstable vessels (CD34⁺/αSMA⁻) were primarily affected with bevacizumab treatment (Figs. 7 and 8), consistent with other published reports (see, e.g., Gasparini et al. Nat. Clin. Pract. Oncol. 2:562-577, 2005). Morphological changes in endothelial cells were also evident for the combination treatment, consistent with findings in metastatic melanoma following ipilumimab and bevacizumab treatment (see, e.g., Hodi et al. Cancer Immunol. Res. 2:632-642, 2014). In addition, contextual localization of CD68+/CD163+ but not CD68+/CD163- macrophages was observed in four patients on-treatment adjacent to the immature vessels but not the mature vessels, which were largely unaffected by bevacizumab therapy (Figs. 7, 9, and 10). Without wishing to be bound by theory, one potential explanation is that the macrophages localized to unstable vessels could be responding to the inflammation and vascular-induced changes caused by bevacizumab. Macrophages have been shown to promote vascularization by secreting VEGF (Lamagna et al. J. Leukoc. Biol. 80:705-713, 2006), and VEGF transcript expression was upregulated in the tumors on-treatment (Fig. 11).

Intratumoral CD8⁺ T cell increases were pronounced following combination treatment in all but one of the patients (Figs. 5, 6, and 12). However, upregulation of PD-L1, which is an IFN-γ response gene, was only detected by immunohistochemistry in one patient, who demonstrated a PR (Fig. 5). Conversely and unexpectedly, a concomitant increase in MHC-I staining was observed following both bevacizumab and combination treatment (Figs. 5 and 6). The modulation of MHC-I by anti-VEGF antibody therapy has not been previously described and was not consistently associated with an increase in CD8⁺ T cells. Previous studies have found that hypoxia is linked to increased MHC-I expression through HIF-1α (Ghosh et al. Mol. Cell. Biol. 33: 2718-2731, 2013).

To address if the increase in CD8⁺ T cell densities upon combination therapy were attributed to enhanced intratumoral proliferation or increased trafficking, dual immunohistochemistry staining of CD8 with the proliferation marker Ki67 was employed. The ratio of Ki67⁺/CD8⁺ cells to Ki67⁻/CD8⁺ cells remained unchanged on-treatment (Figs. 7, 13, 14A-14C, 15A-15C, and 16A-16C), suggesting that the CD8⁺ T cell increase was not due to enhanced intratumoral proliferation but rather due to increased trafficking and infiltration of proliferating CD8⁺ T cells.

### Example 4: Characterization of Antigen-Specific T cell Response Following Combination Therapy

To confirm whether the elevation in intratumoral CD8⁺ T cells was due to increased trafficking, cell populations in the periphery were phenotyped by flow cytometry. HLA-A2 dextramers containing previously described RCC tumor antigen peptides (Table 1) were used to determine if antigen-specific T cells were present in patient blood and if these cell populations changed with treatment. Of the 10 patients, in only two HLA-A2 positive patients were positive for cells in the Dex-APC channel at the pre-treatment timepoint (Fig. 19). Of these two patients, only patient 6 demonstrated an increase in intratumoral CD8⁺ T cells. Interestingly, Dex-APC-positive staining decreased at least 3-fold by the post combination treatment timepoint for patient 6 but not for patient 2, who did not show an increase in intratumoral CD8⁺ T cells. These changes may suggest that RCC antigen-specific T cells traffic from the periphery into tumors.

Gene expression data also indicated that several other chemokines and chemokine receptors increased in patient tumors on-treatment (Fig. 20). The most significant change occurred with fractalkine (CX3CL1), which is known to be expressed on the membrane of activated endothelial cells in inflammatory or hypoxic environments (Szukiewicz et al. Mediators Inflamm. 2013:437576, 2013; Umehara et al. Arterioscler. Thromb. Vasc. Biol. 24:34-40, 2004).

The receptor for fractalkine, CX3CR1, has been shown to be expressed on armed CD8⁺ T cells (perforin+/granzyme B+) (Nishimura et al. J. Immunol. 168:6173-6180, 2002). In the present study, CX3CR1 was upregulated on peripheral CD8+ T cells following combination treatment (Fig. 13). Furthermore, the majority of dextramer-positive cells (Fig. 19) also expressed CX3CR1 (84% and 100% for patients 2 and 6, respectively; Figs. 16A and 16C). The concordant upregulation of fractalkine and other chemokines in the tumor and CX3CR1 on CD8⁺ T cells on-treatment suggest a mechanism for the increased tumor infiltration of CD8⁺ T cells.

T cell receptor (TCR) sequencing was performed on tumors and CD8⁺ T cells were sorted from matched PBMCs to investigate treatment-induced changes in T cell repertoire and trafficking of T cells into the tumor. Comparison of the top clones from pre-treatment and on-treatment TILs for patients 3 and 6 showed that some clones were retained on-treatment (Figs. 21A-21C and 22). There were clones that appeared following bevacizumab alone while other clones emerged only after the combination therapy. There were also clones detected in pre-treatment but not on-treatment tumors. Altogether, these dynamic changes in intratumoral T cell composition suggest that the anti-tumor T cell response is evolving on-treatment.

Evaluation of TCR sequences from sorted peripheral CD8⁺ T cells showed that many of the top clones were maintained between pre-treatment and on-treatment samples, but there was also some overlap between clones found in PBMCs versus on-treatment TILs (Figs. 23A and 23B). In particular, there were no shared clones between PBMCs and TILs of patient 2, the patient in which intratumoral CD8⁺ T cells did not increase on-treatment. For patients 3 and 6, there were some clones present at similar frequencies between PBMCs and TILs. A blast of the Adaptive Public Clone Database revealed that some of the top PBMC clones likely recognize viral antigens, but only some of these clones were detected in TILs, further suggesting that tumor antigen-specific T cells may be migrating into the tumor (Fig. 24). The majority of top clones in on-treatment TILs were present at much lower levels in the blood while the most dominant clones in the blood are not detected in the tumor. Because the relative proportions of top clones are not maintained in PBMCs compared to TILs, this may suggest that the increase in CD8⁺ T cells in the tumor induced by combination treatment occurs through a selective trafficking mechanism. It is also possible that the infiltration is non-biased and there is retention of antigen-specific T cells in the tumor.

## Claims

1. A method of monitoring the response of a patient having a kidney cancer to treatment with an anti-cancer therapy comprising a VEGF antagonist and a PD-L1 axis binding antagonist, the method comprising:
(a) determining, in a biological sample obtained from the patient at a time point following administration of the anti-cancer therapy, the mRNA expression level of CD8A, CD8B, EOMES, GZMA, GZMB, IFNG, and PRF1; and
(b) comparing the mRNA expression level of CD8A, CD8B, EOMES, GZMA, GZMB, IFNG, and PRF1 in the biological sample with a reference level, thereby monitoring the response in the patient to treatment with the anti-cancer therapy.

2. The method of claim 1, wherein:
(i) the expression level of CD8A, CD8B, EOMES, GZMA, GZMB, IFNG, and PRF1 is correlated with the presence of CD8⁺ T effector (T_{eff}) cells in the tumor microenvironment;
(ii) the method further comprises determining the expression level of one or more of CXCL9, CXCL10, CXCL11, or CXCL13, optionally wherein the expression level of one or more of CXCL9, CXCL10, CXCL1 1, or CXCL13 is correlated with the presence of Th1 chemokines in the tumor microenvironment, further optionally wherein the expression level of at least two or at least three of CXCL9, CXCL10, CXCL11, or CXCL13 is determined, further optionally wherein the expression level of CXCL9, CXCL10, CXCL11, and CXCL13 is determined; and/or
(iii) the method further comprises determining the expression level of one or more of GZMB, KLRK1, or SLAMF7, optionally wherein the presence of GZMB, KLRK1, or SLAMF7 is correlated with the presence of natural killer (NK) cells in the tumor microenvironment, further optionally wherein the expression level of at least two of GZMB, KLRK1, or SLAMF7 is determined, further optionally wherein the expression level of GZMB, KLRK1, and SLAMF7 is determined.

3. The method of claim 1 or 2, wherein the reference level is selected from the group consisting of (i) the expression level of CD8A, CD8B, EOMES, GZMA, GZMB, IFNG, and PRF1 in a biological sample from the patient obtained prior to administration of the anti-cancer therapy; (ii) the expression level of CD8A, CD8B, EOMES, GZMA, GZMB, IFNG, and PRF1 in a reference population; (iii) a pre-assigned expression level for CD8A, CD8B, EOMES, GZMA, GZMB, IFNG, and PRF1; (iv) the expression level of CD8A, CD8B, EOMES, GZMA, GZMB, IFNG, and PRF1 in a biological sample obtained from the patient at a previous time point, wherein the previous time point is following administration of the anti-cancer therapy; or (v) the expression level of CD8A, CD8B, EOMES, GZMA, GZMB, IFNG, and PRF1 in a biological sample obtained from the patient at a subsequent time point.

4. The method of any one of claims 1-3, wherein:
(i) the expression level of CD8A, CD8B, EOMES, GZMA, GZMB, IFNG, and PRF1 is increased in the biological sample obtained from the patient relative to the reference level, optionally wherein:
(a) the expression level of CD8A, CD8B, EOMES, GZMA, GZMB, IFNG, and PRF1 is increased at least about 2-fold, about 15-fold, or about 50-fold relative to the reference level;
(b) the expression level of one or more of CXCL9, CXCL10, CXCL11, or CXCL13 is increased at least about 3-fold, about 80-fold, or about 250-fold relative to a reference level for the one or more genes; and/or
(c) the expression level of one or more of GZMB, KLRK1, or SLAMF7 is increased at least about 2-fold, about 8-fold, or about 13-fold relative to a reference level for the one or more genes;
(ii) the increased expression level of CD8A, CD8B, EOMES, GZMA, GZMB, IFNG, and PRF1 indicates that the patient is responding to the anti-cancer therapy; and/or
(iii) the biological sample from the patient is obtained about 4 to about 6 weeks following administration of the anti-cancer therapy.

5. An anti-cancer therapy comprising a VEGF antagonist and a PD-L1 axis binding antagonist for use in a method of treating a patient having a kidney cancer, the method comprising:
(a) determining, in a biological sample obtained from the patient at a time point following administration of the anti-cancer therapy, the mRNA expression level of CD8A, CD8B, EOMES, GZMA, GZMB, IFNG, and PRF1;
(b) comparing the mRNA expression level of CD8A, CD8B, EOMES, GZMA, GZMB, IFNG, and PRF1 in the biological sample with a reference level; and
(c) continuing to administer the anti-cancer therapy to the patient if the expression level of CD8A, CD8B, EOMES, GZMA, GZMB, IFNG, and PRF1 is increased relative to the reference level.

6. The anti-cancer therapy for use of claim 5, wherein:
(i) the expression level of CD8A, CD8B, EOMES, GZMA, GZMB, IFNG, and PRF1 is correlated with the presence of CD8⁺ T effector (T_{eff}) cells in the tumor microenvironment;
(ii) the method further comprises determining the expression level of one or more of CXCL9, CXCL10, CXCL1 1, or CXCL13, optionally wherein the expression level of one or more of CXCL9, CXCL10, CXCL1 1, or CXCL13 is correlated with the presence of Th1 chemokines in the tumor microenvironment, further optionally wherein the expression level of at least two or at least three of CXCL9, CXCL10, CXCL11, or CXCL13 is determined, further optionally wherein the expression level of CXCL9, CXCL10, CXCL11, and CXCL13 is determined; and/or
(iii) the method further comprises determining the expression level of one or more of GZMB, KLRK1, or SLAMF7, optionally wherein the presence of GZMB, KLRK1, or SLAMF7 is correlated with the presence of natural killer (NK) cells in the tumor microenvironment, further optionally wherein the expression level of at least two of GZMB, KLRK1, or SLAMF7 is determined, further optionally wherein the expression level of GZMB, KLRK1, and SLAMF7 is determined.

7. The anti-cancer therapy for use of claim 5 or 6, wherein the reference level is selected from the group consisting of (i) the expression level of CD8A, CD8B, EOMES, GZMA, GZMB, IFNG, and PRF1 in a biological sample from the patient obtained prior to administration of the anti-cancer therapy; (ii) the expression level of CD8A, CD8B, EOMES, GZMA, GZMB, IFNG, and PRF1 in a reference population; (iii) a pre-assigned expression level for CD8A, CD8B, EOMES, GZMA, GZMB, IFNG, and PRF1; (iv) the expression level of CD8A, CD8B, EOMES, GZMA, GZMB, IFNG, and PRF1 in a biological sample obtained from the patient at a previous time point, wherein the previous time point is following administration of the anti-cancer therapy; or (v) the expression level of CD8A, CD8B, EOMES, GZMA, GZMB, IFNG, and PRF1 in a biological sample obtained from the patient at a subsequent time point.

8. The anti-cancer therapy for use of any one of claims 5-7, wherein:
(i) the expression level of CD8A, CD8B, EOMES, GZMA, GZMB, IFNG, and PRF1 is increased at least about 2-fold, about 15-fold, or about 50-fold relative to the reference level;
(ii) the expression level of one or more of CXCL9, CXCL10, CXCL11, or CXCL13 is increased at least about 3-fold, about 80-fold, or about 250-fold relative to a reference level for the one or more genes;
(iii) the expression level of one or more of GZMB, KLRK1, or SLAMF7 is increased at least about 2-fold, about 8-fold, or about 13-fold relative to a reference level for the one or more genes; and/or
(iv) the biological sample from the patient is obtained about 4 to about 6 weeks following administration of the anti-cancer therapy.

9. The method of any one of claims 1-4, or the anti-cancer therapy for use of any one of claims 5-8, wherein:
(i) the VEGF antagonist is an anti-VEGF antibody; and/or
(ii) the PD-L1 axis binding antagonist is selected from the group consisting of a PD-L1 binding antagonist, a PD-1 binding antagonist, and a PD-L2 binding antagonist.

10. The method or anti-cancer therapy for use of claim 9, wherein the anti-VEGF antibody is bevacizumab.

11. The method of any one of claims 1-4, 9, or 10, or the anti-cancer therapy for use of any one of claims 5-10, wherein:
(i) the PD-L1 axis binding antagonist is a PD-L1 binding antagonist, optionally wherein the PD-L1 binding antagonist inhibits the binding of PD-L1 to one or more of its ligand binding partners, further optionally wherein the PD-L1 binding antagonist inhibits the binding of PD-L1 to PD-1, B7-1, or both PD-1 and B7-1, further optionally wherein the PD-L1 binding antagonist is an antibody; or
(ii) the PD-L1 axis binding antagonist is a PD-1 binding antagonist, optionally wherein the PD-1 binding antagonist inhibits the binding of PD-1 to one or more of its ligand binding partners, further optionally wherein the PD-1 binding antagonist inhibits the binding of PD-1 to PD-L1, PD-L2, or both PD-L1 and PD-L2, further optionally wherein the PD-1 binding antagonist is an antibody or an Fc-fusion protein, optionally wherein the antibody is selected from the group consisting of: MDX-1106 (nivolumab), MK-3475 (pembrolizumab), MEDI-0680 (AMP-514), PDR001, REGN2810, and BGB-108, or the Fc-fusion protein is AMP-224.

12. The method of any one of claims 1-4 or 9-11, or the anti-cancer therapy for use of any one of claims 5-11, wherein:
(i) the antibody is selected from the group consisting of: atezolizumab, YW243.55.S70, MDX-1105, MEDI4736 (durvalumab), and MSB0010718C (avelumab), preferably wherein the antibody is atezolizumab;
(ii) the antibody comprises a heavy chain comprising HVR-H1 sequence of SEQ ID NO:19, HVR-H2 sequence of SEQ ID NO:20, and HVR-H3 sequence of SEQ ID NO:21; and a light chain comprising HVR-L1 sequence of SEQ ID NO:22, HVR-L2 sequence of SEQ ID NO:23, and HVR-L3 sequence of SEQ ID NO:24; or
(iii) the antibody comprises a heavy chain variable region comprising the amino acid sequence of SEQ ID NO:25 and a light chain variable region comprising the amino acid sequence of SEQ ID NO:4.

13. The method of any one of claims 1-4 or 9-12, or the anti-cancer therapy for use of any one of claims 5-12, wherein:
(i) the anti-cancer therapy is administered in combination with an additional therapeutic agent, optionally wherein the additional therapeutic agent is selected from the group consisting of an immunotherapy agent, a cytotoxic agent, a growth inhibitory agent, a radiation therapy agent, an anti-angiogenic agent, and combinations thereof; and/or
(ii) the kidney cancer is a renal cell carcinoma, optionally wherein the renal cell carcinoma is a metastatic renal cell carcinoma.

14. The method of any one of claims 1-4 or 9-13, or the VEGF antagonist for use of any one of claims 5-13, wherein the mRNA expression level is determined using a method selected from the group consisting of quantitative polymerase chain reaction (qPCR), reverse transcription qPCR (RT-qPCR), RNA sequencing, microarray analysis, in situ hybridization, and serial analysis of gene expression (SAGE).

15. The method of any one of claims 1-4 or 9-14, or the VEGF antagonist for use of any one of claims 5-14, wherein:
(i) the biological sample obtained from the patient is a tumor sample or a cell sample, optionally wherein the tumor sample is formalin-fixed and paraffin-embedded, fresh, archival, or frozen or wherein the cell sample comprises peripheral CD8⁺ T cells; and/or
(ii) the patient is a human patient.

## Patentansprüche

1. Verfahren zum Überwachen des Ansprechens eines Patienten mit einem Nierenkrebs auf Behandlung mit einer Antikrebstherapie, umfassend einen VEGF-Antagonisten und einen PD-L1-achsenbindenden Antagonisten, wobei das Verfahren Folgendes umfasst:
(a) Bestimmen des mRNA-Expressionsniveaus von CD8A, CD8B, EOMES, GZMA, GZMB, IFNG und PRF1 in einer von dem Patienten zu einem Zeitpunkt nach Verabreichung der Antikrebstherapie erhaltenen biologischen Probe und
(b) Vergleichen des mRNA-Expressionsniveaus von CD8A, CD8B, EOMES, GZMA, GZMB, IFNG und PRF1 in der biologischen Probe mit einem Referenzniveau, wodurch das Ansprechen in dem Patienten auf die Behandlung mit der Antikrebstherapie überwacht wird.

2. Verfahren nach Anspruch 1, wobei:
(i) das Expressionsniveau von CD8A, CD8B, EOMES, GZMA, GZMB, IFNG und PRF1 mit der Gegenwart von CD8⁺-T-Effektor-(T_{eff})-Zellen in der Tumormikroumgebung korreliert wird;
(ii) das Verfahren ferner das Bestimmen des Expressionsniveaus von einem oder mehreren von CXCL9, CXCL10, CXCL11 oder CXCL13 umfasst, gegebenenfalls wobei das Expressionsniveau von einem oder mehreren von CXCL9, CXCL10, CXCL11 oder CXCL13 mit der Gegenwart von Th1-Chemokinen in der Tumormikroumgebung korreliert wird, ferner gegebenenfalls wobei das Expressionsniveau von mindestens zwei oder mindestens drei von CXCL9, CXCL10, CXCL11 oder CXCL13 bestimmt wird, ferner gegebenenfalls wobei das Expressionsniveau von CXCL9, CXCL10, CXCL11 und CXCL13 bestimmt wird und/oder
(iii) das Verfahren ferner das Bestimmen des Expressionsniveaus von einem oder mehreren von GZMB, KLRK1 oder SLAMF7 umfasst, gegebenenfalls wobei die Gegenwart von GZMB, KLRK1 oder SLAMF7 mit der Gegenwart natürlicher Killer-(NK)-Zellen in der Tumormikroumgebung korreliert wird, ferner gegebenenfalls wobei das Expressionsniveau von mindestens zwei von GZMB, KLRK1 oder SLAMF7 bestimmt wird, ferner gegebenenfalls wobei das Expressionsniveau von GZMB, KLRK1 und SLAMF7 bestimmt wird.

3. Verfahren nach Anspruch 1 oder 2, wobei das Referenzniveau ausgewählt ist aus der Gruppe, bestehend aus (i) dem Expressionsniveau von CD8A, CD8B, EOMES, GZMA, GZMB, IFNG und PRF1 in einer von dem Patienten vor Verabreichung der Antikrebstherapie erhaltenen biologischen Probe; (ii) dem Expressionsniveau von CD8A, CD8B, EOMES, GZMA, GZMB, IFNG und PRF1 in einer Referenzpopulation; (iii) einem vorher zugeordneten Expressionsniveau für CD8A, CD8B, EOMES, GZMA, GZMB, IFNG und PRF1; (iv) dem Expressionsniveau von CD8A, CD8B, EOMES, GZMA, GZMB, IFNG und PRF1 in einer zu einem früheren Zeitpunkt von dem Patienten erhaltenen biologischen Probe, wobei der frühere Zeitpunkt nach Verabreichung der Antikrebstherapie liegt; oder (v) dem Expressionsniveau von CD8A, CD8B, EOMES, GZMA, GZMB, IFNG und PRF1 in einer zu einem späteren Zeitpunkt von dem Patienten erhaltenen biologischen Probe.

4. Verfahren nach einem der Ansprüche 1-3, wobei:
(i) das Expressionsniveau von CD8A, CD8B, EOMES, GZMA, GZMB, IFNG und PRF1 in der von dem Patienten erhaltenen biologischen Probe in Bezug auf das Referenzniveau erhöht ist, gegebenenfalls wobei:
(a) das Expressionsniveau von CD8A, CD8B, EOMES, GZMA, GZMB, IFNG und PRF1 um mindestens etwa das 2-Fache, etwa das 15-Fache oder etwa das 50-Fache in Bezug auf das Referenzniveau erhöht ist;
(b) das Expressionsniveau von einem oder mehreren von CXCL9, CXCL10, CXCL11 oder CXCL13 um mindestens etwa das 3-Fache, etwa das 80-Fache oder etwa das 250-Fache in Bezug auf ein Referenzniveau für das eine oder die mehreren Gene erhöht ist und/oder
(c) das Expressionsniveau von einem oder mehreren von GZMB, KLRK1 oder SLAMF7 um mindestens etwa das 2-Fache, etwa das 8-Fache oder etwa das 13-Fache in Bezug auf ein Referenzniveau für das eine oder die mehreren Gene erhöht ist;
(ii) das erhöhte Expressionsniveau von CD8A, CD8B, EOMES, GZMA, GZMB, IFNG und PRF1 angibt, dass der Patient auf die Antikrebstherapie anspricht, und/oder
(iii) die biologische Probe von dem Patienten etwa 4 bis etwa 6 Wochen nach Verabreichung der Antikrebstherapie erhalten wird.

5. Antikrebstherapie, umfassend einen VEGF-Antagonisten und einen PD-L1-achsenbindenden Antagonisten zur Verwendung in einem Verfahren zum Behandeln eines Patienten mit einem Nierenkrebs, wobei das Verfahren Folgendes umfasst:
(a) Bestimmen des mRNA-Expressionsniveaus von CD8A, CD8B, EOMES, GZMA, GZMB, IFNG und PRF1 in einer von dem Patienten zu einem Zeitpunkt nach Verabreichung der Antikrebstherapie erhaltenen biologischen Probe;
(b) Vergleichen des mRNA-Expressionsniveaus von CD8A, CD8B, EOMES, GZMA, GZMB, IFNG und PRF1 in der biologischen Probe mit einem Referenzniveau und
(c) Fortsetzen des Verabreichens der Antikrebstherapie an den Patienten, wenn das Expressionsniveau von CD8A, CD8B, EOMES, GZMA, GZMB, IFNG und PRF1 in Bezug auf das Referenzniveau erhöht ist.

6. Antikrebstherapie zur Verwendung nach Anspruch 5, wobei:
(i) das Expressionsniveau von CD8A, CD8B, EOMES, GZMA, GZMB, IFNG und PRF1 mit der Gegenwart von CD8⁺-T-Effektor-(T_{eff})-Zellen in der Tumormikroumgebung korreliert wird;
(ii) das Verfahren ferner das Bestimmen des Expressionsniveaus von einem oder mehreren von CXCL9, CXCL10, CXCL11 oder CXCL13 umfasst, gegebenenfalls wobei das Expressionsniveau von einem oder mehreren von CXCL9, CXCL10, CXCL11 oder CXCL13 mit der Gegenwart von Th1-Chemokinen in der Tumormikroumgebung korreliert wird, ferner gegebenenfalls wobei das Expressionsniveau von mindestens zwei oder mindestens drei von CXCL9, CXCL10, CXCL11 oder CXCL13 bestimmt wird, ferner gegebenenfalls wobei das Expressionsniveau von CXCL9, CXCL10, CXCL11 und CXCL13 bestimmt wird und/oder
(iii) das Verfahren ferner das Bestimmen des Expressionsniveaus von einem oder mehreren von GZMB, KLRK1 oder SLAMF7 umfasst, gegebenenfalls wobei die Gegenwart von GZMB, KLRK1 oder SLAMF7 mit der Gegenwart natürlicher Killer-(NK)-Zellen in der Tumormikroumgebung korreliert wird, ferner gegebenenfalls wobei das Expressionsniveau von mindestens zwei von GZMB, KLRK1 oder SLAMF7 bestimmt wird, ferner gegebenenfalls wobei das Expressionsniveau von GZMB, KLRK1 und SLAMF7 bestimmt wird.

7. Antikrebstherapie zur Verwendung nach Anspruch 5 oder 6, wobei das Referenzniveau ausgewählt ist aus der Gruppe, bestehend aus (i) dem Expressionsniveau von CD8A, CD8B, EOMES, GZMA, GZMB, IFNG und PRF1 in einer von dem Patienten vor Verabreichung der Antikrebstherapie erhaltenen biologischen Probe; (ii) dem Expressionsniveau von CD8A, CD8B, EOMES, GZMA, GZMB, IFNG und PRF1 in einer Referenzpopulation; (iii) einem vorher zugeordneten Expressionsniveau für CD8A, CD8B, EOMES, GZMA, GZMB, IFNG und PRF1; (iv) dem Expressionsniveau von CD8A, CD8B, EOMES, GZMA, GZMB, IFNG und PRF1 in einer zu einem früheren Zeitpunkt von dem Patienten erhaltenen biologischen Probe , wobei der frühere Zeitpunkt nach Verabreichung der Antikrebstherapie liegt; oder (v) dem Expressionsniveau von CD8A, CD8B, EOMES, GZMA, GZMB, IFNG und PRF1 in einer zu einem späteren Zeitpunkt von dem Patienten erhaltenen biologischen Probe.

8. Antikrebstherapie zur Verwendung nach einem der Ansprüche 5-7, wobei:
(i) das Expressionsniveau von CD8A, CD8B, EOMES, GZMA, GZMB, IFNG und PRF1 um mindestens etwa das 2-Fache, etwa das 15-Fache oder etwa das 50-Fache in Bezug auf das Referenzniveau erhöht ist;
(ii) das Expressionsniveau von einem oder mehreren von CXCL9, CXCL10, CXCL11 oder CXCL13 um mindestens etwa das 3-Fache, etwa das 80-Fache oder etwa das 250-Fache in Bezug auf ein Referenzniveau für das eine oder die mehreren Gene erhöht ist;
(iii) das Expressionsniveau von einem oder mehreren von GZMB, KLRK1 oder SLAMF7 um mindestens etwa das 2-Fache, etwa das 8-Fache oder etwa das 13-Fache in Bezug auf ein Referenzniveau für das eine oder die mehreren Gene erhöht ist und/oder
(iv) die biologische Probe von dem Patienten etwa 4 bis etwa 6 Wochen nach Verabreichung der Antikrebstherapie erhalten wurde.

9. Verfahren nach einem der Ansprüche 1-4 oder Antikrebstherapie zur Verwendung nach einem der Ansprüche 5-8, wobei:
(i) der VEGF-Antagonist ein Anti-VEGF-Antikörper ist und/oder
(ii) der PD-L1-achsenbindende Antagonist ausgewählt ist aus der Gruppe, bestehend aus einem PD-L1-bindenden Antagonisten, einem PD-1-bindenden Antagonisten und einem PD-L2-bindenden Antagonisten.

10. Verfahren oder Antikrebstherapie zur Verwendung nach Anspruch 9, wobei der Anti-VEGF-Antikörper Bevacizumab ist.

11. Verfahren nach einem der Ansprüche 1-4, 9 oder 10 oder Antikrebstherapie zur Verwendung nach einem der Ansprüche 5-10, wobei:
(i) der PD-L1-achsenbindende Antagonist ein PD-L1-bindender Antagonist ist, gegebenenfalls wobei der PD-L1-bindende Antagonist das Binden von PD-L1 an einen oder mehrere seiner ligandenbindenden Partner inhibiert, ferner gegebenenfalls wobei der PD-L1-bindende Antagonist das Binden von PD-L1 an PD-1, B7-1 oder sowohl an PD-1 als auch an B7-1 inhibiert, ferner gegebenenfalls wobei der PD-L1-bindende Antagonist ein Antikörper ist; oder
(ii) der PD-L1-achsenbindende Antagonist ein PD-1-bindender Antagonist ist, gegebenenfalls wobei der PD-1-bindende Antagonist das Binden von PD-1 an einen oder mehrere seiner ligandenbindenden Partner inhibiert, ferner gegebenenfalls wobei der PD-1-bindende Antagonist das Binden von PD-1 an PD-L1, PD-L2 oder sowohl an PD-L1 als auch an PD-L2 inhibiert, ferner gegebenenfalls wobei der PD-1-bindende Antagonist ein Antikörper oder ein Fc-Fusionsprotein ist, gegebenenfalls wobei der Antikörper ausgewählt ist aus der Gruppe, bestehend aus: MDX-1106 (Nivolumab), MK-3475 (Pembrolizumab), MEDI-0680 (AMP-514), PDR001, REGN2810 und BGB-108, oder das Fc-Fusionsprotein AMP-224 ist.

12. Verfahren nach einem der Ansprüche 1-4 oder 9-11 oder Antikrebstherapie zur Verwendung nach einem der Ansprüche 5-11, wobei:
(i) der Antikörper ausgewählt ist aus der Gruppe, bestehend aus: Atezolizumab, YW243.55.S70, MDX-1105, MEDI4736 (Durvalumab) und MSB0010718C (Avelumab), vorzugsweise wobei der Antikörper Atezolizumab ist;
(ii) der Antikörper eine schwere Kette, umfassend die HVR-H1-Sequenz von SEQ ID NO:19, die HVR-H2-Sequenz von SEQ ID NO:20 und die HVR-H3-Sequenz von SEQ ID NO:21, und eine leichte Kette, umfassend die HVR-L1-Sequenz von SEQ ID NO:22, die HVR-L2-Sequenz von SEQ ID NO:23 und die HVR-L3-Sequenz on SEQ ID NO:24, umfasst oder
(iii) der Antikörper eine variable Region der schweren Kette, umfassend die Aminosäuresequenz von SEQ ID NO:25, und eine variable Region der leichten Kette, umfassend die Aminosäuresequenz von SEQ ID NO:4, umfasst.

13. Verfahren nach einem der Ansprüche 1-4 oder 9-12 oder Antikrebstherapie zur Verwendung nach einem der Ansprüche 5-12, wobei:
(i) die Antikrebstherapie in Kombination mit einem zusätzlichen Therapeutikum verabreicht wird, gegebenenfalls wobei das zusätzliche Therapeutikum ausgewählt ist aus der Gruppe, bestehend aus einem Immuntherapeutikum, einem zytotoxischen Mittel, einem wachstumshemmenden Mittel, einem Strahlentherapeutikum, einem anti-angiogenen Mittel und Kombinationen davon, und/oder
(ii) der Nierenkrebs ein Nierenzellkarzinom ist, gegebenenfalls wobei das Nierenzellkarzinom ein metastatisches Nierenzellkarzinom ist.

14. Verfahren nach einem der Ansprüche 1-4 oder 9-13 oder VEGF-Antagonist zur Verwendung nach einem der Ansprüche 5-13, wobei das mRNA-Expressionsniveau unter Anwendung eines Verfahrens bestimmt wird, das ausgewählt ist aus der Gruppe, bestehend aus quantitativer Polymerase-Kettenreaktion (qPCR), Umkehrtranskription-qPCR (RT-qPCR), RNA-Sequenzierung, Mikroarray-Analyse, In-situ-Hybridisierung und serieller Analyse der Genexpression (SAGE).

15. Verfahren nach einem der Ansprüche 1-4 oder 9-14 oder VEGF-Antagonist zur Verwendung nach einem der Ansprüche 5-14, wobei:
(i) die von dem Patienten erhaltene biologische Probe eine Tumorprobe oder eine Zellprobe ist, gegebenenfalls wobei die Tumorprobe formalinfixiert und paraffineingebettet, frisch, archivarisch oder gefroren ist, oder wobei die Zellprobe periphere CD8⁺-T-Zellen umfasst, und/oder
(ii) der Patient ein menschlicher Patient ist.

## Revendications

1. Méthode de suivi de la réponse d'un patient ayant un cancer du rein à un traitement avec une thérapie anticancéreuse comprenant un antagoniste du VEGF et un antagoniste de liaison à l'axe PD-L1, la méthode comprenant :
(a) la détermination, dans un échantillon biologique obtenu auprès du patient à un point temporel après l'administration de la thérapie anticancéreuse, du niveau d'expression d'ARNm de CD8A, CD8B, EOMES, GZMA, GZMB, IFNG et PRF1 ; et
(b) la comparaison du niveau d'expression d'ARNm de CD8A, CD8B, EOMES, GZMA, GZMB, IFNG et PRF1 dans l'échantillon biologique à un niveau de référence, suivant ainsi la réponse du patient au traitement avec la thérapie anticancéreuse.

2. Méthode selon la revendication 1, dans laquelle :
(i) le niveau d'expression de CD8A, CD8B, EOMES, GZMA, GZMB, IFNG et PRF1 est corrélé à la présence de cellules effectrices T (T_{eff}) CD8⁺ dans le micro-environnement tumoral ;
(ii) la méthode comprend en outre la détermination du niveau d'expression d'un ou de plusieurs de CXCL9, CXCL10, CXCL11 ou CXCL13, éventuellement dans laquelle le niveau d'expression d'un ou de plusieurs de CXCL9, CXCL10, CXCL11 ou CXCL13 est corrélé à la présence de chimiokines Th1 dans le micro-environnement tumoral, en outre éventuellement dans laquelle le niveau d'expression d'au moins deux ou d'au moins trois de CXCL9, CXCL10, CXCL11 ou CXCL13 est déterminé, en outre éventuellement dans laquelle le niveau d'expression de CXCL9, CXCL10, CXCL11 et CXCL13 est déterminé ; et/ou
(iii) la méthode comprend en outre la détermination du niveau d'expression d'un ou de plusieurs de GZMB, KLRK1 ou SLAMF7, éventuellement dans laquelle la présence de GZMB, KLRK1 ou SLAMF7 est corrélée à la présence de cellules tueuses naturelles (NK) dans le micro-environnement tumoral, en outre éventuellement dans laquelle le niveau d'expression d'au moins deux de GZMB, KLRK1 ou SLAMF7 est déterminé, en outre éventuellement dans laquelle le niveau d'expression de GZMB, KLRK1 et SLAMF7 est déterminé.

3. Méthode selon la revendication 1 ou 2, dans laquelle le niveau de référence est choisi dans le groupe constitué par (i) le niveau d'expression de CD8A, CD8B, EOMES, GZMA, GZMB, IFNG et PRF1 dans un échantillon biologique du patient obtenu avant l'administration de la thérapie anticancéreuse ; (ii) le niveau d'expression de CD8A, CD8B, EOMES, GZMA, GZMB, IFNG et PRF1 dans une population de référence ; (iii) un niveau d'expression pré-attribué pour CD8A, CD8B, EOMES, GZMA, GZMB, IFNG et PRF1 ; (iv) le niveau d'expression de CD8A, CD8B, EOMES, GZMA, GZMB, IFNG et PRF1 dans un échantillon biologique obtenu auprès du patient à un point temporel antérieur, dans laquelle le point temporel antérieur suit l'administration de la thérapie anticancéreuse ; ou (v) le niveau d'expression de CD8A, CD8B, EOMES, GZMA, GZMB, IFNG et PRF1 dans un échantillon biologique obtenu auprès du patient à un point temporel ultérieur.

4. Méthode selon l'une quelconque des revendications 1 à 3, dans laquelle :
(i) le niveau d'expression de CD8A, CD8B, EOMES, GZMA, GZMB, IFNG et PRF1 est augmenté dans l'échantillon biologique obtenu auprès du patient par rapport au niveau de référence, éventuellement dans laquelle :
(a) le niveau d'expression de CD8A, CD8B, EOMES, GZMA, GZMB, IFNG et PRF1 est augmenté d'au moins environ un facteur 2, environ un facteur 15 ou environ un facteur 50 par rapport au niveau de référence ;
(b) le niveau d'expression d'un ou de plusieurs de CXCL9, CXCL10, CXCL11 ou CXCL13 est augmenté d'au moins environ un facteur 3, environ un facteur 80 ou environ un facteur 250 par rapport à un niveau de référence pour le un ou plusieurs gènes ; et/ou
(c) le niveau d'expression d'un ou plusieurs de GZMB, KLRK1 or SLAMF7 est augmenté d'au moins environ un facteur 2, environ un facteur 8 ou environ un facteur 13 par rapport à un niveau de référence pour le un ou plusieurs gènes ;
(ii) le niveau d'expression augmenté de CD8A, CD8B, EOMES, GZMA, GZMB, IFNG et PRF1 indique que le patient répond à la thérapie anticancéreuse ; et/ou
(iii) l'échantillon biologique du patient est obtenu environ 4 à environ 6 semaines après l'administration de la thérapie anticancéreuse.

5. Thérapie anticancéreuse comprenant un antagoniste du VEGF et un antagoniste de liaison à l'axe PD-L1 pour une utilisation dans une méthode de traitement d'un patient ayant un cancer du rein, la méthode comprenant :
(a) la détermination, dans un échantillon biologique obtenu auprès du patient à un point temporel après l'administration de la thérapie anticancéreuse, du niveau d'expression d'ARNm de CD8A, CD8B, EOMES, GZMA, GZMB, IFNG et PRF1 ;
(b) la comparaison du niveau d'expression d'ARNm de CD8A, CD8B, EOMES, GZMA, GZMB, IFNG et PRF1 dans l'échantillon biologique à un niveau de référence ; et
(c) la poursuite de l'administration de la thérapie anticancéreuse au patient si le niveau d'expression de CD8A, CD8B, EOMES, GZMA, GZMB, IFNG et PRF1 est augmenté par rapport au niveau de référence.

6. Thérapie anticancéreuse pour une utilisation selon la revendication 5, dans laquelle :
(i) le niveau d'expression de CD8A, CD8B, EOMES, GZMA, GZMB, IFNG et PRF1 est corrélé à la présence de cellules effectrices T (Teff) CD8+ dans le micro-environnement tumoral ;
(ii) la méthode comprend en outre la détermination du niveau d'expression d'un ou de plusieurs de CXCL9, CXCL10, CXCL11 ou CXCL13, éventuellement dans laquelle le niveau d'expression d'un ou de plusieurs de CXCL9, CXCL10, CXCL11 ou CXCL13 est corrélé à la présence de chimiokines Th1 dans le micro-environnement tumoral, en outre éventuellement dans laquelle le niveau d'expression d'au moins deux ou d'au moins trois de CXCL9, CXCL10, CXCL11 ou CXCL13 est déterminé, en outre éventuellement dans laquelle le niveau d'expression de CXCL9, CXCL10, CXCL11 et CXCL13 est déterminé ; et/ou
(iii) la méthode comprend en outre la détermination du niveau d'expression d'un ou de plusieurs de GZMB, KLRK1 ou SLAMF7, éventuellement dans laquelle la présence de GZMB, KLRK1 ou SLAMF7 est corrélée à la présence de cellules tueuses naturelles (NK) dans le micro-environnement tumoral, en outre éventuellement dans laquelle le niveau d'expression d'au moins deux de GZMB, KLRK1 ou SLAMF7 est déterminé, en outre éventuellement dans laquelle le niveau d'expression de GZMB, KLRK1 et SLAMF7 est déterminé.

7. Thérapie anticancéreuse pour une utilisation selon la revendication 5 ou 6, dans laquelle le niveau de référence est choisi dans le groupe constitué par (i) le niveau d'expression de CD8A, CD8B, EOMES, GZMA, GZMB, IFNG et PRF1 dans un échantillon biologique du patient obtenu avant l'administration de la thérapie anticancéreuse ; (ii) le niveau d'expression de CD8A, CD8B, EOMES, GZMA, GZMB, IFNG et PRF1 dans une population de référence ; (iii) un niveau d'expression pré-attribué pour CD8A, CD8B, EOMES, GZMA, GZMB, IFNG et PRF1 ; (iv) le niveau d'expression de CD8A, CD8B, EOMES, GZMA, GZMB, IFNG et PRF1 dans un échantillon biologique obtenu auprès du patient à un point temporel antérieur, dans laquelle le point temporel antérieur suit l'administration de la thérapie anticancéreuse ; ou (v) le niveau d'expression de CD8A, CD8B, EOMES, GZMA, GZMB, IFNG et PRF1 dans un échantillon biologique obtenu auprès du patient à un point temporel ultérieur.

8. Thérapie anticancéreuse pour une utilisation selon l'une quelconque des revendications 5 à 7, dans laquelle :
(i) le niveau d'expression de CD8A, CD8B, EOMES, GZMA, GZMB, IFNG et PRF1 est augmenté d'au moins environ un facteur 2, environ un facteur 15 ou environ un facteur 50 par rapport au niveau de référence ;
(ii) le niveau d'expression d'un ou de plusieurs de CXCL9, CXCL10, CXCL11 ou CXCL13 est augmenté d'au moins environ 3 fois, environ 80 fois ou environ 250 fois par rapport à un niveau de référence pour le un ou plusieurs gènes ;
(iii) le niveau d'expression d'un ou plusieurs de GZMB, KLRK1 or SLAMF7 est augmenté d'au moins environ un facteur 2, environ un facteur 8 ou environ un facteur 13 par rapport à un niveau de référence pour le un ou plusieurs gènes ; et/ou
(iv) l'échantillon biologique du patient est obtenu environ 4 à environ 6 semaines après l'administration de la thérapie anticancéreuse.

9. Méthode selon l'une quelconque des revendications 1 à 4, ou thérapie anticancéreuse pour une utilisation selon l'une quelconque des revendications 5 à 8, dans laquelle :
(i) l'antagoniste du VEGF est un anticorps anti-VEGF ; et/ou
(ii) l'antagoniste de liaison à l'axe PD-L1 est choisi dans le groupe constitué par un antagoniste de liaison à PD-L1, un antagoniste de liaison à PD-1 et un antagoniste de liaison à PD-L2.

10. Méthode ou thérapie anticancéreuse pour une utilisation selon la revendication 9, dans laquelle l'anticorps anti-VEGF est le bévacizumab.

11. Méthode selon l'une quelconque des revendications 1 à 4, 9 ou 10, ou thérapie anticancéreuse pour une utilisation selon l'une quelconque des revendications 5 à 10, dans laquelle :
(i) l'antagoniste de liaison à l'axe PD-L1 est un antagoniste de liaison à PD-L1, éventuellement dans laquelle l'antagoniste de liaison à PD-L1 inhibe la liaison de PD-L1 à un ou plusieurs de ses partenaires de liaison de type ligand, en outre éventuellement dans laquelle l'antagoniste de liaison à PD-L1 inhibe la liaison de PD-L1 à PD-1, B7-1, ou à la fois à PD-1 et B7-1, en outre éventuellement dans laquelle l'antagoniste de liaison à PD-L1 est un anticorps ; ou
(ii) l'antagoniste de liaison à l'axe PD-L1 est un antagoniste de liaison à PD-1, éventuellement dans laquelle l'antagoniste de liaison à PD-1 inhibe la liaison de PD-1 à un ou plusieurs de ses partenaires de liaison de type ligand, en outre éventuellement dans laquelle l'antagoniste de liaison à PD-1 inhibe la liaison de PD-1 à PD-L1, PD-L2, ou à la fois à PD-L1 et PD-L2, en outre éventuellement dans laquelle l'antagoniste de liaison à PD-1 est un anticorps ou une protéine de fusion Fc, éventuellement dans laquelle l'anticorps est choisi dans le groupe constitué par : MDX-1106 (nivolumab), MK-3475 (pembrolizumab), MEDI-0680 (AMP-514), PDR001, REGN2810 et BGB-108, ou la protéine de fusion Fc est l'AMP-224.

12. Méthode selon l'une quelconque des revendications 1 à 4 ou 9 à 11, ou thérapie anticancéreuse pour une utilisation selon l'une quelconque des revendications 5 à 11, dans laquelle :
(i) l'anticorps est choisi dans le groupe constitué par : l'atézolizumab, YW243.55.S70, MDX-1105, MEDI4736 (durvalumab) et MSB0010718C (avélumab), préférentiellement dans laquelle l'anticorps est l'atézolizumab ;
(ii) l'anticorps comprend une chaîne lourde comprenant une séquence HVR-H1 de SEQ ID NO : 19, une séquence HVR-H2 de SEQ ID NO : 20 et une séquence HVR-H3 de SEQ ID NO : 21 ; et une chaîne légère comprenant une séquence HVR-L1 de SEQ ID NO : 22, une séquence HVR-L2 de SEQ ID NO : 23 et une séquence HVR-L3 de SEQ ID NO : 24 ; ou
(iii) l'anticorps comprend une région variable de chaîne lourde comprenant la séquence d'acides aminés de SEQ ID NO : 25 et une région variable de chaîne légère comprenant la séquence d'acides aminés de SEQ ID NO : 4.

13. Méthode selon l'une quelconque des revendications 1 à 4 ou 9 à 12, ou thérapie anticancéreuse pour une utilisation selon l'une quelconque des revendications 5 à 12, dans laquelle :
(i) la thérapie anticancéreuse est administrée en association avec un agent thérapeutique supplémentaire, éventuellement dans laquelle l'agent thérapeutique supplémentaire est choisi dans le groupe constitué par un agent immunothérapeutique, un agent cytotoxique, un agent inhibiteur de croissance, un agent de radiothérapie, un agent anti-angiogénique, et des combinaisons de ceux-ci ; et/ou
(ii) le cancer du rein est un carcinome à cellules rénales, éventuellement dans lequel le carcinome à cellules rénales est un carcinome à cellules rénales métastatique.

14. Méthode selon l'une quelconque des revendications 1 à 4 ou 9 à 13, ou antagoniste du VEGF pour une utilisation selon l'une quelconque des revendications 5 à 13, dans lesquels le niveau d'expression d'ARNm est déterminé en utilisant une méthode choisie dans le groupe constitué par la réaction en chaîne par polymérase quantitative (qPCR), la transcription inverse couplée à une qPCR (RT-qPCR), le séquençage de l'ARN, l'analyse par microréseau, l'hybridation in situ et l'analyse en série de l'expression génétique (SAGE).

15. Méthode selon l'une quelconque des revendications 1 à 4 ou 9 à 14, ou antagoniste du VEGF pour une utilisation selon l'une quelconque des revendications 5 à 14, dans lesquels :
(i) l'échantillon biologique obtenu auprès du patient est un échantillon tumoral ou un échantillon cellulaire, éventuellement dans lesquels l'échantillon tumoral est fixé au formol et inclus en paraffine, frais, archivé ou congelé ou dans lesquels l'échantillon cellulaire comprend des cellules T CD8+ périphériques ; et/ou
(ii) le patient est un patient humain.
